(19) Europäisches Patentamt
European Patent Office
Office européen des brevets

(11) EP 3 628 672 A1

(12) EUROPEAN PATENT APPLICATION

(43) Date of publication:
01.04.2020 Bulletin 2020/14

(21) Application number: 18290105.8

(22) Date of filing: 25.09.2018

(51) Int Cl.:
C07D 417/12 (2006.01)
C07D 417/14 (2006.01)
C07D 277/28 (2006.01)
A61P 25/00 (2006.01)
A61P 31/04 (2006.01)
A61K 31/427 (2006.01)
A61K 31/4523 (2006.01)
A61K 31/505 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: **Antabio SAS**
**31670 Labège (FR)**

(72) Inventor: **The designation of the inventor has not yet been filed**

(74) Representative: **J A Kemp LLP**
**14 South Square**
**Gray's Inn**
**London WC1R 5JJ (GB)**

Remarks:
The references to parts of the description (originally filed page 183 and parts of originally filed pages 22 and 212) are deemed to be deleted (Rule 56(4) EPC).

# (54) INDANE DERIVATIVES FOR USE IN THE TREATMENT OF BACTERIAL INFECTION

(57) The invention relates to a compound which is an indane according to Formula (I), or a pharmaceutically acceptable salt thereof,

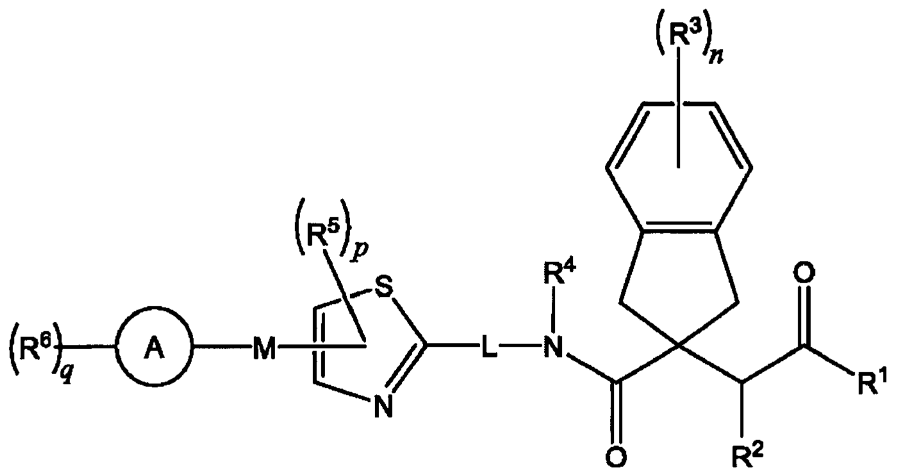

[FORMULA (I)]

wherein $R^1$, $R^2$, $R^3$, $R^4$, $R^5$, $R^6$, L, M, Ⓐ, *n*, *p*, and *q* are as defined herein. The compounds are useful in the treatment of antibacterial infection either as stand alone antibiotics, or in combination with further antibiotics.

EP 3 628 672 A1

## Description

### Field of the Invention

The present invention relates to compounds which find use in the prevention or treatment of bacterial infection. The invention also provides such compounds *per se* and pharmaceutical compositions comprising such compounds.

### Background

Cystic fibrosis (CF) is a life-threatening disease affecting approximately 70,000 sufferers worldwide. CF is the most common lethal, hereditary disease in Caucasian populations, resulting from mutations in the cystic fibrosis transmembrane conductance regulator (CFTR) gene. The prevalence of CF in Europe is 1 in every 2,000-3,000 live births, and in North America is about 1 in every 3,500 births. In the UK there are approximately 9,800 people with CF.

The organs of individuals with CF typically have significantly thickened secretions. This in turn can lead to a range of pathological problems. For instance, individuals with CF typically have impaired ciliary clearance, and the lungs of such individuals are typically colonized and infected by bacteria from an early age. Such bacteria include *Staphylococcus aureus, Haemophilus influenza, Pseudomonas aeruginosa* and *Burkholderia cepacia. Pseudomonas aeruginosa* (PA) is the most common cause of chronic lung infection in individuals with CF, and chronic infection with PA is found in 9% of pre-school children, 32% of 10-15 year olds and the majority (between 59% and 80%) of adults with CF, leading to progressive lung damage and early death.

As the lung of the individual with CF is colonised by PA, the growth pattern of the bacteria changes and its capacity for survival improves. In chronic infection, PA bacteria on mucosal and epithelial surfaces, or in sputum, form biofilms as well as producing large quantities of alginate (the so-called mucoid phenotype) which reduce the effectiveness of phagocytosis and antibiotic therapy. This leads to chronic colonisation of the lung by PA that is not cleared by conventional antibiotic therapy.

Antibiotics are a broad range of substances exhibiting anti-bacterial activity. A large number of antibiotic compounds are known and have been shown to exhibit antibacterial activity against a wide range of bacteria. However, currently available antibiotics are incapable of controlling some bacterial infections. This is because the target bacteria have acquired antibiotic resistance, for example via horizontal gene transfer or because the target bacteria are found in a state in which the efficacy of antibiotics which would otherwise be highly active is reduced. One such state is a bacterial biofilm.

Bacteria in biofilms are enclosed in a self-produced extracellular biopolymer matrix, which may include polysaccharides, proteins and DNA. Bacteria in biofilms typically exhibit different properties from free-living bacteria of the same species. Such properties typically include increased resistance to antibiotics and detergents and increased lateral gene transfer. For example, bacteria in biofilms typically display up to 1,000-fold higher tolerance to antibiotic challenge than their single cell, planktonic (free-living) counterparts.

This limitation in the efficacy of antibacterial compounds is especially important for individuals who through immunodeficiency or other diseases or conditions cannot adequately combat bacterial infection. Such individuals include those suffering from cystic fibrosis.

CF patients who are colonised with PA show also a more rapid decline in lung function, faster decline in chest radiograph score, poor weight gain, increased hospitalisation rates and an increased need for antibiotic therapy. Median survival is reduced and mortality increased (2.6x risk of death). Most disease-related morbidity and mortality in CF is caused by progressive lung disease as a result of bacterial infection and airway inflammation, primarily associated with the effects of chronic PA lung infection and the persistence of PA biofilms. Despite intensive antibiotic treatment, adaptive mechanisms such as biofilm formation allow PA to resist both immune and antibiotic pressures, leading to recurrent exacerbations and respiratory failure.

Pathogenic bacteria such as PA are not only of importance in the context of CF. For example, the opportunistic pathogen PA can also cause septic shock, particularly in neutropenic patients, and can be responsible for infections of the urinary tract, the gastrointestinal network and skin and soft tissues. PA is also a frequent coloniser of medical devices such as catheters, nebulizers, and the like.

Accordingly, there is a clear need for new antibiotic compounds and compositions and adjuvant therapies for treating bacterial infection.

### Summary of the Invention

The inventors have surprisingly found that compounds of Formula (I) are potent inhibitors of the *Pseudomonas aeruginosa*-derived elastase enzyme LasB, which is important in *Pseudomonas aeruginosa* pathogenesis and persistence through biofilm formation.

LasB is implicated in bacterial disease pathology, since secreted LasB degrades many host immune proteins and causes tissue damage. LasB, also known as pseudolysin, is massively secreted into the environment of the producer organism where it is able to proteolytically attack numerous host immune proteins (e.g. immunoglobulins, cytokines, SP-A, antimicrobial peptides (e.g. Trappin 2)) and tissue proteins (e.g. elastin). There are no mammalian homologues of LasB. The ability of LasB to attack host proteins contributes to immune evasion (e.g. avoidance of SP-A mediated phagocytosis, and degradation of immunoglobulin, degradation of antimicrobial peptides (e.g. Trappin 2)) whilst promoting tissue invasion and long term colonization. Inhibition of LasB therefore better equips the host to deal with immune attack.

LasB also has an important internal role within the bacterial cell cleaving nucleoside diphosphate kinase (NDK) to a smaller active form. Active form of NDK leads to increased GTP levels within the cell, increasing production of alginate. Alginate is a polysaccharide which is a major component of the extracellular biofilm matrix and which is required for swarming motility. Those two virulence phenotypes are associated with bacterial persistence in response to immune and antibiotic pressures. LasB activity has also been shown to upregulate rhamnolipid production, which is necessary for biofilm formation/ maturation. Accordingly, inhibition of LasB assists impairment of biofilm formation and disruption of the established biofilm. This in turn is believed to better enable antibiotics currently in use to deal effectively with infection.

Accordingly, the invention provides the following aspects:

1. A compound which is an indane according to Formula (I), or a pharmaceutically acceptable salt thereof,

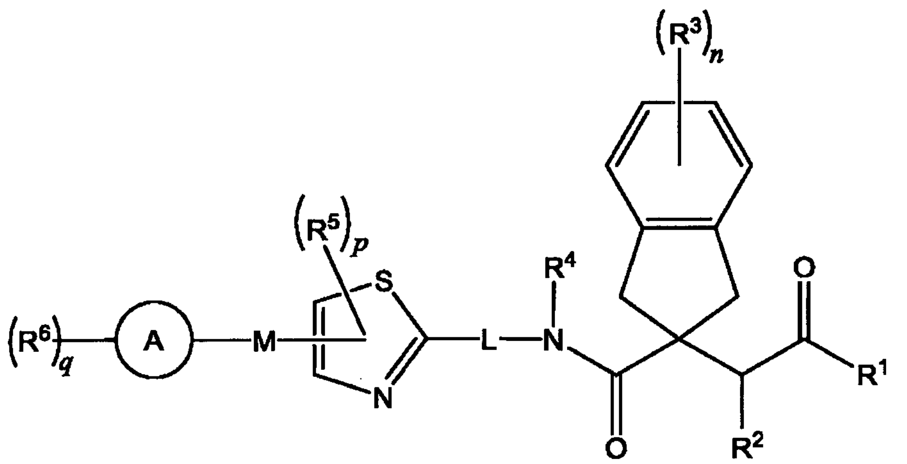

[FORMULA (I)]

wherein

- $R^1$ is selected from:
  - NHOH, OH, $OR^{1a}$ and $-OCH_2OC(O)R^{1a}$, wherein $R^{1a}$ is selected from an unsubstituted $C_1$ to $C_4$ alkyl group and phenyl; and
  - where the compound of Formula (I) contains a positively charged nitrogen atom, $R^1$ may be $O^-$, such that the compound forms a zwitterion;
- $R^2$ is selected from H and unsubstituted $C_1$ to $C_2$ alkyl;
- each $R^3$ group is independently selected from halogen, -OH, $-NH_2$, methyl and $-CF_3$;
- *n* is an integer from 0 to 4;
- $R^4$ is selected from H and unsubstituted $C_1$ to $C_2$ alkyl;
- L is selected from a bond and a $C_1$ to $C_3$ alkylene group which is unsubstituted or is substituted by one group selected from halogen, -OH, -OMe, $-NR^{20}R^{21}$; $-N^+R^{20}R^{22}$, and $-CF_3$;
- M is selected from a bond and a $C_1$ to $C_3$ alkylene group which is unsubstituted or is substituted by one group selected from halogen, -OH, -OMe, $-NR^{20}R^{21}$; $-N^+R^{20}R^{21}R^{22}$, and $-CF_3$,
- Ⓐ is a cyclic group selected from phenyl, 5- to 10- membered heteroaryl, and 4- to 10-membered carbocyclic and heterocyclic groups; wherein when Ⓐ is a heterocyclic or heteroaryl group comprising at least one nitrogen atom, said nitrogen atom(s) are independently selected from secondary, tertiary and quaternary nitrogen atom(s);

- *p* is 0 or 1;
- $R^5$ is selected from halogen, -OH, -CN, $-NH_2$, methyl, $-CF_3$, and -OMe;
- *q* is 0, 1 or 2;
- each $R^6$ is independently selected from:

    ◦ halogen, -CN, -OH;
    ◦ oxo, providing that Ⓐ is a carbocyclic or heterocyclic group;
    ◦ $-R^{6a}$, $-OR^{6a}$, $NR^{20}R^{6a}$, $-C(O)NR^{20}R^{6a}$, $-NR^{20}C(O)R^{6a}$, $-SO_2R^{6a}$, $-SO_2NR^{20}R^{6a}$, $-NR^{20}SO_2R^{6a}$, $C(O)R^{6a}$, $-OC(O)R^{6a}$, and $-C(O)OR^{6a}$; wherein
    each $R^{6a}$ is independently selected from $C_1$ to $C_4$ alkyl, $C_2$ to $C_4$ alkenyl and $C_2$ to $C_4$ alkynyl, and wherein each $R^{6a}$ is independently unsubstituted or is substituted with one, two or three groups selected from -OH, halogen; $-NR^{20}R^{21}$; $-N^+R^{20}R^{21}R^{22}$; $-NR^{20}C(NR^{21})NR^{22}R^{23}$; $-NR^{20}C(N^+R^{21}R^{22})NR^{23}R^{24}$; $-NR^{20}C(NR^{21})R^{22}$; $-NR^{20}C(N^+R^{21}R^{22})R^{23}$; $-C(NR^{20})NR^{21}R^{22}$; $-C(N^+R^{20}R^{21})NR^{22}R^{23}$; $-C(NR^{20})R^{21}$; $-C(N^+R^{20}R^{21})R^{22}$; $-C(O)NR^{20}R^{21}$; $-C(O)N^+R^{20}R^{21}R^{22}$; $-C(O)-R^{20}$, and methoxy which is substituted by one, two or three halogen substituents;
    ◦ $-R^7-(Het^1)_v-R^9-R^{R1}$ and $-R^7-R^{R1}-Het^2-R^{R2}$; wherein

    - $Het^1$ is selected from -O-, $-NR^{20}-$, -C(O)-, $-SO_2-$, $-C(O)NR^{20}-$, $-NR^{20}C(O)-$, $-O-R^8-C(O)-$, $-C(O)-R^8-O-$, $-SO_2NR^{20}-$, $-NR^{20}SO_2-$, -OC(O)-, and -C(O)O-;
    - $Het^2$ is selected from -C(O)-, $-R^8-C(O)-$ and $-C(O)-R^8-$;
    - *v* is 0 or 1;
    - $R^{R1}$ and $R^{R2}$ are each independently a 4- to 10- membered heteroaryl or heterocyclic group comprising at least one nitrogen atom, and said nitrogen atom(s) are independently selected from secondary, tertiary and quaternary nitrogen atom(s); and wherein each $R^{R1}$ and $R^{R2}$ is independently unsubstituted or is substituted with one, two or three groups independently selected from

        ◦ halogen, -CN;
        ◦ oxo, providing that said $R^{R1}$ or $R^{R2}$ group is a heterocyclic group;
        ◦ $-R^{20}$, $-R^7-OR^{20}$; $-R^7-NR^{20}R^{21}$; $-R^7-N^+R^{20}R^{21}R^{22}$; $-R^7-NR^{20}C(NR^{21})NR^{22}R^{23}$; $-R^7-NR^{20}C(N^+R^{21}R^{22})NR^{23}R^{24}$; $-R^7-NR^{20}C(NR^{21})R^{22}$; $-R^7-NR^{20}C(N^+R^{21}R^{22})R^{23}$; $-R^7-C(NR^{20})NR^{21}R^{22}$; $-R^7-C(N+R^{20}R^{21})NR^{22}R^{23}$; $-R^7-C(NR^{20})R^{21}$; $-R^7-(N^+R^{20}R^{21})R^{22}$; $-R^7-C(O)-R^9-NR^{20}R^{21}$; $R^7-C(O)-R^9-N^+R^{20}R^{21}R^{22}$; $R^7-C(O)-R^{20}$, and methoxy which is substituted by one, two or three halogen substituents;

- $R^7$ is selected from a bond and unsubstituted $C_1$ to $C_3$ alkylene;
- $R^8$ is unsubstituted $C_1$ to $C_3$ alkylene;
- $R^9$ is selected from a bond and unsubstituted $C_1$ to $C_3$ alkylene;
- $R^{20}$, $R^{21}$, $R^{22}$, $R^{23}$ and $R^{24}$ are each independently selected from H and unsubstituted $C_1$ to $C_2$ alkyl.

2. A compound according to aspect 1 wherein $R^1$ is selected from OH and NHOH; or where the compound of Formula (I) contains a positively charged nitrogen atom, $R^1$ may be $O^-$, such that the compound forms a zwitterion.

3. A compound according to aspects 1 or 2, wherein $R^2$ is selected from H and unsubstituted methyl.

4. A compound according to any one of aspects 1 to 3 wherein $R^4$ is H.

5. A compound according to any one of the preceding aspects, wherein *n* is an integer from 0 to 2

6. A compound according to any one of the preceding aspects, wherein each $R^3$ group is independently selected from halogen and -OH.

7. A compound according to any one of the preceding aspects, wherein L is an unsubstituted $C_1$ alkylene group

8. A compound according to any one of the preceding aspects, wherein M is selected from a bond and an unsubstituted $C_1$ alkylene group.

9. A compound according to any one of the preceding aspects, wherein $R^5$ is selected from halogen, -CN, and methyl.

10. A compound according to any one of aspects 1 to 9, wherein *p* is 0.

11. A compound according to any one of the preceding aspects wherein Ⓐ is selected from phenyl and 5- to 6-membered heteroaryl and heterocyclic groups, wherein when Ⓐ is a heterocyclic or heteroaryl group it comprises at least one nitrogen atom, said nitrogen atom(s) being independently selected from secondary, tertiary and quaternary nitrogen atom(s).

12. A compound according to any one of the preceding aspects wherein Ⓐ is selected from benzene, pyridine, pyrimidine, pyrazine, pyrazole, triazole, dihydropyridine, piperazine and piperidine.

13. A compound according to any one of the preceding aspects wherein Ⓐ is aromatic

14. A compound according to any one of the preceding aspects wherein Ⓐ is phenyl.

15. A compound according to any one of the preceding aspects wherein each $R^6$ is independently selected from:

◦ halogen, -OH;
◦ oxo, providing that Ⓐ is a carbocyclic or heterocyclic group;
◦ $-R^{6a}$, $-OR^{6a}$, $NR^{20}R^{6a}$, $-C(O)NR^{20}R^{6a}$, $-NR^{20}C(O)R^{6a}$, $-SO_2R^{6a}$, $-SO_2NR^{20}R^{6a}$, and $-NR^{20}SO_2R^{6a}$; wherein
each $R^{6a}$ is independently selected from $C_1$ to $C_3$ alkyl and $C_2$ to $C_3$ alkynyl, and wherein each $R^{6a}$ is independently unsubstituted or is substituted with one or two groups selected from -OH; $-NR^{20}R^{21}$; $-N^+R^{20}R^{21}R^2$; $-NR^{20}C(NR^{21})NR^{22}R^{23}$; $-C(NR^{20})NR^{21}R^{22}$; and $-C(N^+R^{20}R^{21})NR^{22}R^{23}$, or with one, two or three halogen groups
◦ $-R^7-(Het^1)_v-R^9-R^{R1}$ and $-R^7-R^{R1}-Het^2-R^{R2}$; wherein
$R^{R1}$ and $R^{R2}$ are each independently a 5- to 6- membered heteroaryl or 4- to 8-membered heterocyclic group comprising at least one nitrogen atom, and said nitrogen atom(s) are independently selected from secondary, tertiary and quaternary nitrogen atom(s); and wherein each $R^{R1}$ and $R^{R2}$ is independently unsubstituted or is substituted with one or two groups independently selected from

- oxo, providing that said $R^{R1}$ or $R^{R2}$ group is a heterocyclic group;
- $-R^{20}$, $-R^7-OR^{20}$; $-R^7-NR^{20}R^{21}$; $-R^7-N^+R^{20}R^{21}R^{22}$; $-R^7-NR^{20}C(NR^{21})NR^{22}R^{23}$; $-R^7-NR^{20}C(N^+R^{21}R^{22})NR^{23}R^{24}$; $-R^7-NR^{20}C(NR^{21})R^{22}$; $-R^7-NR^{20}C(N^+R^{21}R^{22})R^{23}$; $-R^7-C(NR^{20})NR^{21}R^{22}$; $-R^7-C(N^+R^{20}R^{21})NR^{22}R^{23}$; $-R^7-C(NR^{20})R^{21}$; $-R^7-(N^+R^{20}R^{21})R^{22}$; $-R^7-C(O)-R^9-NR^{20}R^{21}$; $R^7-C(O)-R^9-N^+R^{20}R^{21}R^{22}$; and $R^7-C(O)-R^{20}$;
  or
- with one, two or three halogen groups;

16. A compound according to any one of the preceding aspects wherein each $R^6$ is independently selected from: halogen; -OH; $-OCF_3$; $-(CH_2)_z-H$; $-(CH_2)_z-OH$; $-(CH_2)_z-N(R^X)_2$; $-(CH_2)_z-N^+(R^X)_3$, $-O-(CH_2)_z-H$; $-O-(CH_2)_z-OH$; $-O-(CH_2)_z-N(R^X)_2$; $-O-(CH_2)_z-N^+(R^X)_3$; $-O-(CH_2)_z-N(R^X)-C(NR^X)-CH_3$; $-O-(CH_2)_z-N(R^X)-C(NR^X)-N(R^X)_2$; $-C(O)NR^X-(CH_2)_z-N(R^X)_2$; $-C(O)NR^X-(CH_2)_z-N^+(R^X)_3$; $-NR^XC(O)-(CH_2)_z-N(R^X)_2$; $-NR^XC(O)-(CH_2)_z-N^+(R^X)_3$; $-SO_2-(CH_2)_z-H$; $-SO_2-NR^X-(CH_2)_z-N(R^X)_2$; $-SO_2-NR^X-(CH_2)_z-N^+(R^X)3$; $-C{\equiv}C-CH_2-N(R^X)_2$ and $-C{\equiv}C-CH_2-N^+(R^X)_3$; wherein $R^X$ is H or Me and z is 1, 2 or 3

17. A compound according to any one of aspects 1 to 15 wherein each $R^6$ is independently selected from $-R^{R1}$; $-(CH_2)_z-R^{R1}$; $-O-(CH_2)_z-R^{R1}$; $-C(O)-R^{R1}$; $-(CH_2)_z-C(O)-R^{R1}$; $-N(R^X)-C(O)-R^{R1}$; $-N(R^X)-C(O)-(CH_2)_z-R^{R1}$; $-O-(CH_2)_z-C(O)-R^{R1}$; $-SO_2-R^{R1}$; $R^{R1}-C(O)-R^{R2}$; $-(CH_2)_z-R^{R1}-C(O)-R^{R2}$; $-R^{R1}-(CH_2)_z-C(O)-R^{R2}$; and each $R^{R1}$ and $R^{R2}$ is independently unsubstituted or is substituted with one or two groups selected from $-CH_3$; $-N(R^X)_2$;$-N^+(R^X)_3$; $-(CH_2)_z-N(R^X)_2$; $-(CH_2)_z-N^+(R^X)_3$; $-OCH_3$; oxo; $-C(O)-CH_3$; $-(CO)-N(R^X)_2$; $-(CO)-(CH_2)_z-N(R^X)_2$; $-C(NR^X)-N(R^X)_2$ and $-C(NR^X)-CH_3$;
wherein $R^X$ is H or Me and z is 1, 2 or 3.

18. A compound according to any one of the preceding aspects wherein each $R^{R1}$ and $R^{R2}$ if present are independently selected from azetidine, imidazole, morpholine, 1,4-oxazepane, octahydropyrrolo[3,4-c]pyrrole, piperazine, piperidine, pyridine, and pyrrolidine.

19. A compound according to aspect 1, which compound is selected from

1. 2-[2-[(4-phenylthiazol-2-yl)methylcarbamoyl]indan-2-yl]acetic acid
2. 2-[2-[(5-phenylthiazol-2-yl)methylcarbamoyl]indan-2-yl]acetic acid
3. 2-[5,6-difluoro-2-[(4-phenylthiazol-2-yl)methylcarbamoyl]indan-2-yl]acetic acid
4. 2-(2-((benzo[d]thiazol-2-ylmethyl)carbamoyl)-5,6-dichloro-2,3-dihydro-1H-inden-2-yl)acetic acid
5. 2-[2-[[4-(3-methoxyphenyl)thiazol-2-yl]methylcarbamoyl]indan-2-yl]acetic acid
6. 2-[2-[[4-(4-methoxyphenyl)thiazol-2-yl]methylcarbamoyl]indan-2-yl]acetic acid
7. 2-[2-[[4-(4-chlorophenyl)thiazol-2-yl]methylcarbamoyl]indan-2-yl]acetic acid
8. 2-[2-[[4-(3-pyridyl)thiazol-2-yl]methylcarbamoyl]indan-2-yl]acetic acid
9. 2-[2-[[4-(2-pyridyl)thiazol-2-yl]methylcarbamoyl]indan-2-yl]acetic acid
10. 2-[2-[[4-(2-hydroxyphenyl)thiazol-2-yl]methytcarbamoyl]indan-2-yl]acetic acid
11. 2-[2-[[4-(4-hydroxyphenyl)thiazol-2-yl]methylcarbamoyl]indan-2-yl]acetic acid
12. 2-[2-[[4-(3-hydroxyphenyl)thiazol-2-yl]methylcarbamoyl]indan-2-yl]acetic acid
13. 2-[2-[[4-[2-(2-hydroxyethoxy)phenyl]thiazol-2-yl]methylcarbamoyl]indan-2-yl]acetic acid
14. 2-[2-[[4-[2-(2-aminoethoxy)phenyl]thiazol-2-yl]methylcarbamoyl]indan-2-yl]acetic acid
15. 2-[2-[[4-[4-[2-(dimethylamino)ethylcarbamoyl]phenyl]thiazol-2-yl]methylcarbamoyl]indan-2-yl]acetic acid
16. 2-[2-[[4-[4-(4-methylpiperazine-1-carbonyl)phenyl]thiazol-2-yl]methylcarbamoyl]indan-2-yl]acetic acid
17. 2-[2-[[4-[4-[4-(dimethylamino)piperidine-1-carbonyl]phenyl]thiazol-2-yl]methylcarbamoyl]indan-2-yl]acetic acid
18. 2-[2-[[4-[4-[(3S)-3-(dimethylamino)pyrrolidine-1-carbonyl]phenyl]thiazol-2-yl]methylcarbamoyl]indan-2-yl]acetic acid
19. 2-[2-[[4-[4-[(3R)-3-(dimethylamino)pyrrolidine-1-carbonyl]phenyl]thiazol-2-yl]methylcarbamoyl]indan-2-yl]acetic acid
20. 2-[2-[[4-[4-[3-(dimethylamino)azetidine-1-carbonyl]phenyl]thiazol-2-yl]methylcarbamoyl]indan-2-yl]acetic acid
21. 2-[2-[[4-[2-[3-(dimethylamino)propylcarbamoyl]phenyl]thiazol-2-yl]methylcarbamoyl]indan-2-yl]acetic acid
22. 2-[2-[[4-[4-(4,4-dimethylpiperazin-4-ium-1-carbonyl)phenyl]thiazol-2-yl]methylcarbamoyl]indan-2-yl]acetate
23. 2-[2-[[4-[3-(4-methylpiperazine-1-carbonyl)phenyl]thiazol-2-yl]methylcarbamoyl]indan-2-yl]acetic acid
24. 2-[2-[[4-[5-[2-(dimethylamino)ethylcarbamoyl]-2-methoxy-phenyl]thiazol-2-yl]methylcarbamoyl]indan-2-yl]acetic acid
25. 2-[2-[[4-[2-methoxy-5-(4-methylpiperazine-1-carbonyl)phenyl]thiazol-2-yl]methylcarbamoyl]indan-2-yl]acetic acid
26. 2-[2-[[4-[4-(4-methylpiperazin-1-yl)phenyl]thiazol-2-yl]methylcarbamoyl]indan-2-yl]acetic acid
27. 2-[2-[[4-[4-(4-methylpiperazine-1-carbonyl)phenyl]thiazol-2-yl]methylcarbamoyl]indan-2-yl]acetic acid
28. 2-[2-[[4-[3-(4-methylpiperazin-1-yl)phenyl]thiazol-2-yl]methylcarbamoyl]indan-2-yl]acetic acid
29. 2-[2-[[4-[3-[3-(dimethylamino)azetidin-1-yl]phenyl]thiazol-2-yl]methylcarbamoyl]indan-2-yl]acetic acid
30. 2-[2-[[4-[4-[(3aS,6aR)-2-methyl-1,3,3a,4,6,6a-hexahydropyrrolo[3,4-c]pyrrol-5-yl]phenyl]thiazol-2-yl]methylcarbamoyl]indan-2-yl]acetic acid
31. 2-[2-[[4-[2-methoxy-4-(4-methylpiperazin-1-yl)phenyl]thiazol-2-yl]methylcarbamoyl]indan-2-yl]acetic acid
32. 2-[2-[[4-[4-[3-(dimethylamino)azetidin-1-yl]-2-methoxy-phenyl]thiazol-2-yl]methylcarbamoyl]indan-2-yl]acetic acid
33. 2-[5,6-difluoro-2-[[4-[2-methoxy-4-[4-(trimethylammonio)-1-piperidyl]phenyl]thiazol-2-yl]methylcarbamoyl]indan-2-yl]acetate
34. 2-[2-[[4-[6-(4-methylpiperazin-1-yl)-3-pyridyl]thiazol-2-yl]methylcarbamoyl]indan-2-yl]acetic acid
35. 2-[2-[[4-[6-[3-(dimethylamino)azetidin-1-yl]-3-pyridyl]thiazol-2-yl]methylcarbamoyl]indan-2-yl]acetic acid
36. 2-[2-[[4-[4-[4-[2-(4-methylpiperazin-1-yl)-2-oxo-ethyl]piperazin-1-yl]phenyl]thiazol-2-yl]methylcarbamoyl]indan-2-yl]acetic acid
37. 2-[2-[[4-[4-[3-(dimethylamino)prop-1-ynyl]phenyl]thiazol-2-yl]methylcarbamoyl]indan-2-yl]acetic acid
38. 2-[2-[[4-[6-[2-(4-methylpiperazin-1-yl)-2-oxo-ethoxy]-3-pyridyl]thiazol-2-yl]methylcarbamoyl]indan-2-yl]acetic acid
39. 2-[2-[[4-[4-[[2-(dimethylamino)acetyl]amino]phenyl]thiazol-2-yl]methylcarbamoyl]indan-2-yl]acetic acid
40. 2-[2-[[4-[4-[[2-(trimethylammonio)acetyl]ainino]phenyl]thiazol-2-yl]methylcarbamoyl]indan-2-yl]acetate
41. 2-[2-[[4-[3-[[2-(dimethylamino)acetyl]amino]phenyl]thiazol-2-yl]methylcarbamoyl]indan-2-yl]acetic acid
42. 2-[2-[[4-[3-[[2-(trimethylammonio)acetyl]amino]phenyl]thiazol-2-yl]methylcarbamoyl]indan-2-yl]acetate
43. 2-[2-[[4-[4-[(1-methylpiperidine-4-carbonyl)amino]phenyl]thiazol-2-yl]methylcarbamoyl]indan-2-yl]acetic acid
44. 2-[2-[[4-[4-[(4-methylpiperazine-1-carbonyl)amino]phenyl]thiazol-2-yl]methylcarbamoyl]indan-2-yl]acetic acid

45. 2-[2-[[4-[4-[[2-(4-methylpiperazin-1-yl)acetyl]amino]phenyl]thiazol-2-yl]methylcarbamoyl]indan-2-yl]acetic acid
46. 2-[2-[[4-[4-[2-(4-methylpiperazin-1-yl)-2-oxo-ethoxy]phenyl]thiazol-2-yl]methylcarbamoyl]indan-2-yl]acetic acid
47. 2-[2-[[4-[4-[2-(4,4-dimethylpiperazin-4-ium-1-yl)-2-oxo-ethoxy]phenyl]thiazol-2-yl]methylcarbamoyl]indan-2-yl]acetate
48. 2-[2-[[4-[4-[2-(dimethylamino)ethoxy]phenyl]thiazol-2-yl]methylcarbamoyl]indan-2-yl]acetic acid
49. 2-[2-[[4-[4-(2-morpholinoethoxy)phenyl]thiazol-2-yl]methylcarbamoyl]indan-2-yl]acetic acid
50. 2-[2-[[4-[4-[2-(4-methylmorpholin-4-ium-4-yl)ethoxy]phenyl]thiazol-2-yl]methylcarbamoyl]indan-2-yl]acetate
51. 2-[2-[[4-[4-[(1-methylpyridin-1-ium-4-yl)methoxy]phenyl]thiazol-2-yl]methylcarbamoyl]indan-2-yl]acetate
52. 2-[2-[[4-[4-[2-(4-amino-1-piperidyl)-2-oxo-ethoxy]phenyl]thiazol-2-yl]methylcarbamoyl]indan-2-yl]acetic acid
53. 2-[2-[[4-[3-[2-(dimethylamino)ethoxy]phenyl]thiazol-2-yl]methylcarbamoyl]indan-2-yl]acetic acid
54. 2-[2-[[4-[3-[2-(4-methylpiperazin-1-yl)-2-oxo-ethoxy]phenyl]thiazol-2-yl]methylcarbamoyl]indan-2-yl]acetic acid
55. 2-[2-[[4-[4-(3-morpholinopropoxy)phenyl]thiazol-2-yl]methylcarbamoyl]indan-2-yl]acetic acid
56. 2-[2-[[4-[4-[3-(4-methylmorpholin-4-ium-4-yl)propoxy]phenyl]thiazol-2-yl]methylcarbamoyl]indan-2-yl]acetate
57. 2-[2-[[4-[4-[3-(4-methylpiperazin-1-yl)propoxy]phenyl]thiazol-2-yl]methylcarbamoyl]indan-2-yl]acetic acid
58. 2-[2-[[4-[4-(3-pyrrolidin-1-ylpropoxy)phenyl]thiazol-2-yl]methylcarbamoyl]indan-2-yl]acetic acid
59. 2-[2-[[4-[4-(3-pyridin-1-ium-1-ylpropoxy)phenyl]thiazol-2-yl]methylcarbamoyl]indan-2-yl]acetate
60. 2-[2-[[4-[3-methylsulfonyl-4-(3-morpholinopropoxy)phenyl]thiazol-2-yl]methylcarbamoyl]indan-2-yl]acetic acid
61. 2-[2-[[4-[4-[3-(4-methylmorpholin-4-ium-4-yl)propoxy]-3-methylsulfonyl-phenyl]thiazol-2-yl]methylcarbamoyl]indan-2-yl]acetate
62. 2-[5,6-difluoro-2-[[4-[4-[3-(4-methylmorpholin-4-ium-4-yl)propoxy]-3-methylsulfonyl-phenyl]thiazol-2-yl]methylcarbamoyl]indan-2-yl]acetate
63. 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid
64. 2-[5,6-difluoro-2-[[4-[2-methoxy-4-[2-oxo-2-[3-(trimethylammonio)azetidin-1-yl]ethoxy]phenyl]thiazol-2-yl]methylcarbamoyl]indan-2-yl]acetate
65. 2-[2-[[4-[4-[2-[4-[3-(dimethylamino)propyl]piperazin-1-yl]-2-oxo-ethoxy]-2-methoxy-phenyl]thiazol-2-yl]methylcarbamoyl]indan-2-yl]acetic acid
66. 2-[2-[[4-[2-methoxy-4-[2-(2-methyl-1,3,3a,4,6,6a-hexahydropyrrolo[3,4-c]pyrrol-5-yl)-2-oxo-ethoxy]phenyl]thiazol-2-yl]methylcarbamoyl]indan-2-yl]acetic acid
67. 2-[2-[[4-[2-methoxy-4-[2-(4-methylmorpholin-4-ium-4-yl)ethoxy]phenyl]thiazol-2-yl]methylcarbamoyl]indan-2-yl]acetate
68. 2-[5,6-difluoro-2-[[4-[2-methoxy-4-[2-(4-methylmorpholin-4-ium-4-yl)ethoxy]phenyl]thiazol-2-yl]methylcarbamoyl]indan-2-yl]acetate
69. 2-[2-[[4-[2-methoxy-4-[3-(4-methylmorpholin-4-ium-4-yl)propoxy]phenyl]thiazol-2-yl]methylcarbamoyl]indan-2-yl]acetate
70. 2-[5,6-difluoro-2-[[4-[2-methoxy-4-[3-(4-methylmorpholin-4-ium-4-yl)propoxy]phenyl]thiazol-2-yl]methylcarbamoyl]indan-2-yl]acetate
71. 2-[2-[[4-[2-methoxy-4-(3-morpholinopropoxy)phenyl]thiazol-2-yl]methylcarbamoyl]indan-2-yl]acetic acid
72. 2-[5,6-difluoro-2-[[4-[2-methoxy-4-(3-pyrrolidin-1-ylpropoxy)phenyl]thiazol-2-yl]methylcarbamoyl]indan-2-yl]acetic acid
73. 2-[5,6-difluoro-2-[[4-[2-methoxy-4-[3-(1-methylpyrrolidin-1-ium-1-yl)propoxy]phenyl]thiazol-2-yl]methylcarbamoyl]indan-2-yl]acetate
74. 2-[2-[[4-[2-methoxy-4-(4-pyridylmethoxy)phenyl]thiazol-2-yl]methylcarbamoyl]indan-2-yl]acetic acid
75. 2-[2-[[4-[2-methoxy-4-[(1-methylpyridin-1-ium-4-yl)methoxy]phenyl]thiazol-2-yl]methylcarbamoyl]indan-2-yl]acetate
76. 2-[2-[[4-[2-methoxy-4-(2-morpholinoethoxy)phenyl]thiazol-2-yl]methylcarbamoyl]indan-2-yl]acetic acid
77. 2-[2-[[4-[2-methoxy-4-[(1-methylpyridin-1-ium-3-yl)methoxy]phenyl]thiazol-2-yl]methylcarbamoyl]indan-2-yl]acetate
78. 2-[2-[[4-[2-methoxy-4-(3-pyridylmethoxy)phenyl]thiazol-2-yl]methylcarbamoyl]indan-2-yl]acetic acid
79. 2-[2-[[4-[4-[2-(4,4-dimethylpiperazin-4-ium-1-yl)-2-oxo-ethoxy]-2-methoxy-phenyl]thiazol-2-yl]methylcarbamoyl]indan-2-yl]acetate

80. 2-[2-[[4-[4-[3-(dimethylamino)propoxy]-2-methoxy-phenyl]thiazol-2-yl]methylcarbamoyl]-5,6-difluoro-indan-2-yl]acetic acid
81. 2-[5,6-difluoro-2-[[4-[2-methoxy-4-[3-(trimethylammonio)propoxy]phenyl]thiazol-2-yl]methylcarbamoyl]indan-2-yl]acetate
82. 2-[2-[[4-[4-[2-(4,4-dimethylpiperazin-4-ium-1-yl)-2-oxo-ethoxy]-2-methoxy-phenyl]thiazol-2-yl]methylcarbamoyl]-5,6-difluoro-indan-2-yl]acetate
83. 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid
84. 2-[2-[[4-[4-[2-(1,4-dimethyl-3-oxo-piperazin-1-ium-1-yl)ethoxy]-2-methoxy-phenyl]thiazol-2-yl]methylcarbamoyl]indan-2-yl]acetate
85. 2-[2-[[4-[2-methoxy-4-[2-(1-methylimidazol-2-yl)ethoxy]phenyl]thiazol-2-yl]methylcarbamoyl]indan-2-yl]acetic acid
86. 2-[2-[[4-[4-[2-(1,3-dimethylimidazol-1-ium-2-yl)ethoxy]-2-methoxy-phenyl]thiazol-2-yl]methylcarbamoyl]-5,6-difluoro-indan-2-yl]acetate
87. 2-[2-[[4-[4-[2-(1,3-dimethylimidazol-1-ium-2-yl)ethoxy]phenyl]thiazol-2-yl]methylcarbamoyl]indan-2-yl]acetate
88. 2-[2-[[4-[4-[2-(4-acetylpiperazin-1-yl)ethoxy]-2-methoxy-phenyl]thiazol-2-yl]methylcarbamoyl]indan-2-yl]acetic acid
89. 2-[2-[[4-(1-methylpyrazol-4-yl)thiazol-2-yl]methylcarbamoyl]indan-2-yl]acetic acid
90. 2-[2-[[4-(1-methylpyrazol-3-yl)thiazol-2-yl]methylcarbamoyl]indan-2-yl]acetic acid
91. 2-[2-[[4-(1,5-dimethylpyrazol-4-yl)thiazol-2-yl]methylcarbamoyl]indan-2-yl]acetic acid
92. 2-[2-[[4-(2-methylpyrazol-3-yl)thiazol-2-yl]methylcarbamoyl]indan-2-yl]acetic acid
93. 2-[2-[[4-(1,3-dimethylpyrazol-4-yl)thiazol-2-yl]methylcarbamoyl]indan-2-yl]acetic acid
94. 2-[2-[(4-pyrimidin-5-ylthiazol-2-yl)methylcarbamoyl]indan-2-yl]acetic acid
95. 2-[2-[[4-(4-pyridyl)thiazol-2-yl]methylcarbamoyl]indan-2-yl]acetic acid
96. 2-[2-[[4-[1-(2-morpholinoethyl)pyrazol-4-yl]thiazol-2-yl]methylcarbamoyl]indan-2-yl]acetic acid
97. 2-[2-[[4-[1-[2-(dimethylamino)ethyl]pyrazol-4-yl]thiazol-2-yl]methylcarbamoyl]indan-2-yl]acetic acid
98. 2-[2-[[4-(5-methyl-1H-pyrazol-3-yl)thiazol-2-yl]methylcarbamoyl]indan-2-yl]acetic acid
99. 2-[2-[[4-[1-(3-morpholinopropyl)pyrazol-4-yl]thiazol-2-yl]methylcarbamoyl]indan-2-yl]acetic acid
100. 2-[2-[[4-[1-[3-(4-methylmorpholin-4-ium-4-yl)propyl]pyrazol-4-yl]thiazol-2-yl]methylcarbamoyl]indan-2-yl]acetate
101. 2-[2-[[4-[1-(3-morpholinopropyl)pyrazol-4-yl]thiazol-2-yl]methylcarbamoyl]indan-2-yl]acetic acid
102. 2-[2-[[4-[1-(1-methyl-4-piperidyl)pyrazol-4-yl]thiazol-2-yl]methylcarbamoyl]indan-2-yl]acetic acid
103. 2-[2-[[4-[1-[1-[2-(dimethylamino)acetyl]-4-piperidyl]pyrazol-4-yl]thiazol-2-yl]methylcarbamoyl]indan-2-yl]acetic acid
104. 2-[2-[[4-[1-[1-(4-methylpiperazine-1-carbonyl)-4-piperidyl]pyrazol-4-yl]thiazol-2-yl]methylcarbamoyl]indan-2-yl]acetic acid
105. 2-[2-[[4-[1-[1-[2-(4-methylpiperazin-1-yl)-2-oxo-ethyl]-4-piperidyl]pyrazol-4-yl]thiazol-2-yl]methylcarbamoyl]indan-2-yl]acetic acid
106. 2-[2-[[4-[1-[3-(4-acetylpiperazin-1-yl)propyl]pyrazol-4-yl]thiazol-2-yl]methylcarbamoyl]indan-2-yl]acetic acid
107. 2-[2-[[4-[1-[3-(4-acetyl-1-methyl-piperazin-1-ium-1-yl)propyl]pyrazol-4-yl]thiazol-2-yl]methylcarbamoyl]indan-2-yl]acetate
108. 2-[2-[[4-[1-[3-(4-methylpiperazin-1-yl)propyl]pyrazol-4-yl]thiazol-2-yl]methylcarbamoyl]indan-2-yl]acetic acid
109. 2-[2-[[4-[1-[3-(1,4-oxazepan-4-yl)propyl]pyrazol-4-yl]thiazol-2-yl]methylcarbamoyl]indan-2-yl]acetic acid
110. 2-[2-[[4-[1-[3-(4-methoxy-1-piperidyl)propyl]pyrazol-4-yl]thiazol-2-yl]methylcarbamoyl]indan-2-yl]acetic acid
111. 2-[2-[[4-[1-[3-(1-piperidyl)propyl]pyrazol-4-yl]thiazol-2-yl]methylcarbamoyl]indan-2-yl]acetic acid
112. 2-[2-[[4-[1-[3-[4-(4-methylpiperazine-1-carbonyl)piperazin-1-yl]propyl]pyrazol-4-yl]thiazol-2-yl]methylcarbamoyl]indan-2-yl]acetic acid
113. 2-[2-[[4-[1-[3-[4-(dimethylcarbamoyl)-1-piperidyl]propyl]pyrazol-4-yl]thiazol-2-yl]methylcarbamoyl]indan-2-yl]acetic acid
114. 2-[2-[[4-[1-[3-[4-(1-methylpiperidine-4-carbonyl)piperazin-1-yl]propyl]pyrazol-4-yl]thiazol-2-yl]methylcarbamoyl]indan-2-yl]acetic acid
115. 2-[2-[[4-[1-[3-[4-(1,1-dimethylpiperidin-1-ium-4-carbonyl)-1-methyl-piperazin-1-ium-1-yl]propyl]pyrazol-4-yl]thiazol-2-yl]methylcarbamoyl]indan-2-yl]acetate
116. 2-[2-[[4-[1-[3-(4-methyl-3-oxo-piperazin-1-yl)propyl]pyrazol-4-yl]thiazol-2-yl]methylcarbamoyl]indan-2-yl]acetic acid
117. 2-[2-[[4-[1-[2-(1-methylpyridin-1-ium-4-yl)ethyl]pyrazol-4-yl]thiazol-2-yl]methylcarbamoyl]indan-2-yl]acetate
118. 2-[2-[[4-[1-[3-(1-methylpyridin-1-ium-4-yl)propyl]pyrazol-4-yl]thiazol-2-yl]methylcarbamoyl]indan-2-yl]acetate
119. 2-[2-[[4-[1-[2-(4-acetylpiperazin-1-yl)ethyl]pyrazol-4-yl]thiazol-2-yl]methylcarbamoyl]indan-2-yl]acetic acid
120. 2-[2-[[4-[1-[(1-methylpyridin-1-ium-4-yl)methyl]pyrazol-4-yl]thiazol-2-yl]methylcarbamoyl]indan-2-yl]acetate
121. 2-[2-[[4-[3-[[4-(dimethylamino)-1-piperidyl]sulfonyl]-4-methoxy-phenyl]thiazol-2-yl]methylcarbamoyl]indan-2-yl]acetic acid
122. 2-[2-[[4-[3-[[4-(dimethylamino)-1-piperidyl]sulfonyl]-4-methoxy-phenyl]thiazol-2-yl]methylcarbamoyl]indan-2-yl]acetic acid
123. 2-[2-[[4-[3-[[4-(dimethylamino)-1-piperidyl]sulfonyl]-4-methoxy-phenyl]thiazol-2-yl]methylcarbamoyl]-5,6-difluoro-indan-2-yl]acetic acid
124. 2-[5,6-difluoro-2-[[4-[4-methoxy-3-[[4-(trimethylammonio)-1-piperidyl]sulfonyl]phenyl]thiazol-2-yl]methylcarbamoyl]indan-2-yl]acetate
125. 2-[2-[[4-[5-[[4-(dimethylamino)-1-piperidyl]sulfonyl]-2-methoxy-phenyl]thiazol-2-yl]methylcarbamoyl]-5,6-difluoro-indan-2-yl]acetic acid
126. 2-[5,6-difluoro-2-[[4-[2-methoxy-5-[[4-(trimethylammonio)-1-piperidyl]sulfonyl]phenyl]thiazol-2-yl]methylcarbamoyl]indan-2-yl]acetate
127. 2-[2-[[4-[3-(4-methylpiperazin-1-yl)sulfonylphenyl]thiazol-2-yl]methylcarbamoyl]indan-2-yl]acetic acid
128. 2-[2-[[4-[3-(4,4-dimethylpiperazin-4-ium-1-yl)sulfonylphenyl]thiazol-2-yl]methylcarbamoyl]indan-2-yl]acetate
129. 2-[2-[[4-[3-[2-(dimethylamino)ethylsulfamoyl]phenyl]thiazol-2-yl]methylcarbamoyl]indan-2-yl]acetic acid
130. 2-[2-[[4-[4-(4-methylpiperazin-1-yl)sulfonylphenyl]thiazol-2-yl]methylcarbamoyl]indan-2-yl]acetic acid
131. 2-[2-[[4-[4-[2-(dimethylamino)ethylsulfamoyl]phenyl]thiazol-2-yl]methylcarbamoyl]indan-2-yl]acetic acid
132. 2-[2-[[4-[3-(4-methylpiperazin-1-yl)sulfonyl-4-(2-morpholinoethoxy)phenyl]thiazol-2-yl]methylcarbamoyl]indan-2-yl]acetic acid
133. 2-[2-[[4-[3-(4-methylpiperazin-1-yl)sulfonyl-4-(3-morpholinopropoxy)phenyl]thiazol-2-yl]methylcarbamoyl]indan-2-yl]acetic acid
134. 2-[2-[[4-[3-[[4-(dimethylamino)-1-piperidyl]sulfonyl]-4-(2-morpholinoethoxy)phenyl]thiazol-2-yl]methylcarbamoyl]-5,6-difluoro-indan-2-yl]acetic acid
135. 2-[2-[[4-[3-methylsulfonyl-4-(2-morpholinoethoxy)phenyl]thiazol-2-yl]methylcarbamoyl]indan-2-yl]acetic acid
136. 2-[2-[[4-[4-[2-(4-methylmorpholin-4-ium-4-yl)ethoxy]-3-methylsulfonyl-phenyl]thiazol-2-yl]methylcarbamoyl]indan-2-yl]acetate
137. 2-[2-[[4-[3-[4-(dimethylamino)piperidine-1-carbonyl]-4-methoxy-phenyl]thiazol-2-yl]methylcarbamoyl]indan-2-yl]acetic acid
138. 2-[2-[[4-[4-methoxy-3-[4-(trimethylammonio)piperidine-1-carbonyl]phenyl]thiazol-2-yl]methylcarbamoyl]indan-2-yl]acetate
139. 2-[2-[[4-[3-[[4-(dimethylamino)-1-piperidyl]sulfonyl]-4-(3-pyrrolidin-1-ylpropoxy)phenyl]thiazol-2-yl]methylcarbamoyl]-5,6-difluoro-indan-2-yl]acetic acid
140. 2-[2-[[4-[6-(2-morpholinoethyl)-3-pyridyl]thiazol-2-yl]methylcarbamoyl]indan-2-yl]acetic acid
141. 2-[2-[[4-[6-[2-(4-methylmorpholin-4-ium-4-yl)ethyl]-3-pyridyl]thiazol-2-yl]methylcarbamoyl]indan-2-yl]acetate
142. 2-[2-[[4-[6-(4-pyridylmethoxy)-3-pyridyl]thiazol-2-yl]methylcarbamoyl]indan-2-yl]acetic acid
143. 2-[2-[[4-[6-[(1-methylpyridin-1-ium-4-yl)methoxy]-3-pyridyl]thiazol-2-yl]methylcarbamoyl]indan-2-yl]acetate
144. 2-[2-[[4-[5-(4-methylpiperazine-1-carbonyl)-3-pyridyl]thiazol-2-yl]methylcarbamoyl]indan-2-yl]acetic acid
145. 2-[2-[[4-[2-(trifluoromethoxy)phenyl]thiazol-2-yl]methylcarbamoyl]indan-2-yl]acetic acid
146. 2-[2-[[4-(2-methoxyphenyl)thiazol-2-yl]methylcarbamoyl]indan-2-yl]acetic acid
147. 2-[2-[[4-(2-ethylphenyl)thiazol-2-yl]methylcarbamoyl]indan-2-yl]acetic acid
148. 2-[2-[[4-[2-(hydroxymethyl)phenyl]thiazol-2-yl]methylcarbamoyl]indan-2-yl]acetic acid
149. 2-[2-[[4-[3-(hydroxymethyl)phenyl]thiazol-2-yl]methylcarbamoyl]indan-2-yl]acetic acid
150. 2-[2-[[4-(2-methoxy-3-pyridyl)thiazol-2-yl]methylcarbamoyl]indan-2-yl]acetic acid
151. 2-[2-[[4-(4-methoxy-3-pyridyl)thiazol-2-yl]methylcarbamoyl]indan-2-yl]acetic acid
152. 2-[2-[[4-(2-oxo-1H-pyridin-3-yl)thiazol-2-yl]methylcarbamoyl]indan-2-yl]acetic acid
153. 2-[2-[[4-[1-(4-methylpiperazine-1-carbonyl)-4-piperidyl]thiazol-2-yl]methylcarbamoyl]indan-2-yl]acetic acid
154. 2-[2-[[4-[1-(4-methylpiperazin-1-yl)sulfonyl-3-piperidyl]thiazol-2-yl]methylcarbamoyl]indan-2-yl]acetic acid
155. 2-[2-[[4-[4-(4-carbamimidoylpiperazin-1-yl)phenyl]thiazol-2-yl]methylcarbamoyl]indan-2-yl]acetic acid
156. 2-[2-[[4-[4-(4-ethanimidoylpiperazin-1-yl)phenyl]thiazol-2-yl]methylcarbamoyl]indan-2-yl]acetic acid
157. 2-[2-(hydroxyamino)-2-oxo-ethyl]-N-[(4-phenylthiazol-2-yl)methyl]indane-2-carboxamide
158. 2-[2-(hydroxyamino)-2-oxo-ethyl]-N-[[4-[1-(2-morpholinoethyl)pyrazol-4-yl]thiazol-2-yl]methyl]indane-2-carboxamide
159. 2-[2-(hydroxyamino)-2-oxo-ethyl]-N-[[4-[1-[2-(4-methylpiperazin-1-yl)-2-oxoethyl]pyrazol-4-yl]thiazol-2-yl]methyl]indane-2-carboxamide
160. 2-[2-[[4-[3-[(4-methylpiperazin-1-yl)methyl]phenyl]thiazol-2-yl]methylcarbamoyl]indan-2-yl]acetic acid
161. 2-[2-[[4-[4-[(4-methylpiperazin-1-yl)methyl]phenyl]thiazol-2-yl]methylcarbamoyl]indan-2-yl]acetic acid
162. 2-[2-[[4-[4-[(dimethylamino)methyl]phenyl]thiazol-2-yl]methylcarbamoyl]indan-2-yl]acetic acid
163. 2-[2-[[4-[4-(2-aminoethoxy)phenyl]thiazol-2-yl]methylcarbamoyl]indan-2-yl]acetic acid
164. 2-[2-[[4-[4-[2-(ethanimidoylamino)ethoxy]phenyl]thiazol-2-yl]methylcarbamoyl]indan-2-yl]acetic acid
165. 2-[2-[[4-[4-(2-guanidinoethoxy)phenyl]thiazol-2-yl]methylcarbamoyl]indan-2-yl]acetic acid
166. 2-[2-[(4-benzylthiazol-2-yl)methylcarbamoyl]indan-2-yl]acetic acid
167. 2-[2-[(5-methyl-4-phenyl-thiazol-2-yl)methylcarbamoyl]indan-2-yl]acetic acid
168. 2-[2-[[4-(5-methyl-1H-1,2,4-triazol-3-yl)thiazol-2-yl]methylcarbamoyl]indan-2-yl]acetic acid
A. 2-[2-[(4-pyrazin-2-ylthiazol-2-yl)methylcarbamoyl]indan-2-yl]acetic acid
B. 2-[2-[(5-chloro-4-phenyl-thiazol-2-yl)methylcarbamoyl]indan-2-yl] acetic acid
C. 2-[2-[(5-cyano-4-phenyl-thiazol-2-yl)methylcarbamoyl]indan-2-yl]acetic acid
D. 2-[2-[[4-[4-(4-methyl-2-oxo-piperazin-1-yl)phenyl]thiazol-2-yl]methylcarbamoyl]indan-2-yl]acetic acid

or a pharmaceutically acceptable salt thereof.

20. A pharmaceutical composition comprising (i) a compound according to any one of the preceding aspects and (ii) at least one pharmaceutically acceptable carrier or diluent; and optionally further comprising (iii) an antibiotic agent.

21. A pharmaceutical composition according to aspect 20 wherein the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

22. A combination of (i) a compound according to any one of aspects 1 to 19 and (ii) an antibiotic agent.

23. A combination according to aspect 22 wherein the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

24. A compound according to any one of aspects 1 to 19; a composition according to aspect 20 or 21 or a combination according to aspect 22 or 23 for use in medicine.

25. A compound according to any one of aspects 1 to 19; a composition according to aspect 20 or 21 or a combination according to aspect 22 or 23 for use in treating or preventing bacterial infection in a subject.

26. A compound for use, composition for use or combination for use according to aspect 25 wherein the bacterial infection is caused by *Bacillus, Pseudomonas, Staphylococcus, Streptococcus, Listeria, Burkholderia or Escherichia.*

27. A compound for use, composition for use or combination for use according to aspect 26 wherein the bacterial infection is caused by *Pseudomonas aeruginosa.*

28. A compound for use, composition for use or combination for use according to any one of aspects 25 to 27 wherein the compound for use, composition for use or combination for use is for use in the treatment or prevention of pneumonia

29. A compound for use, composition for use or combination for use according to any one of aspects 25 to 28 wherein the subject suffers from cystic fibrosis.

## BRIEF DESCRIPTION OF THE FIGURES

**Figure 1** shows lung burden in a rat model of chronic lung infection following infection with WT PA (which expresses LasB) and a mutant form of PA (*ΔlasB* PA) in which LasB is not expressed. Results are described in Example 169.

## DETAILED DESCRIPTION OF THE INVENTION

*Definitions*

As used herein, a $C_1$ to $C_4$ alkyl group is a linear or branched alkyl group containing from 1 to 4 carbon atoms. A $C_1$ to $C_4$ alkyl group is often a $C_1$ to $C_3$ alkyl group. Examples of $C_1$ to $C_4$ alkyl groups include methyl, ethyl, n-propyl, iso-propyl, n-butyl, sec-butyl, and tert-butyl. A $C_1$ to $C_3$ alkyl group is typically a $C_1$ to $C_2$ alkyl group. A $C_1$ to $C_2$ alkyl group is methyl or ethyl, typically methyl. For the avoidance of doubt, where two alkyl groups are present, the alkyl groups may be the same or different.

As used herein, an alkoxy group is typically a said alkyl group attached to an oxygen atom. Thus, a $C_2$ to $C_4$ alkoxy group is a $C_2$ to $C_4$ alkyl group attached to an oxygen atom. A $C_1$ to $C_3$ alkoxy group is a $C_1$ to $C_3$ alkyl group attached to an oxygen atom. Examples of $C_2$ to $C_4$ alkoxy groups include ethoxy, n-propyoxy, iso-propoxy, n-butoxy, sec-butoxy, and tert-butoxy. Examples of $C_1$ to $C_3$ alkoxy groups include methoxy, ethoxy n-propyoxy and iso-propoxy. Typically, a $C_1$ to $C_3$ alkoxy group is a $C_1$ to $C_2$ alkoxy group such as a methoxy or ethoxy group. For the avoidance of doubt, where two alkoxy groups are present, the alkoxy groups may be the same or different.

As used herein, a $C_2$ to $C_4$ alkenyl group is a linear or branched alkenyl group containing from 2 to 4 carbon atoms and having one or more, e.g. one or two, typically one double bonds. Typically a $C_2$ to $C_4$ alkenyl group is a $C_2$ to $C_3$ alkenyl group. Examples of $C_2$ to $C_4$ alkenyl groups include ethenyl, propenyl and butenyl. For the avoidance of doubt, where two alkenyl groups are present, the alkenyl groups may be the same or different.

As used herein, a $C_2$ to $C_4$ alkynyl group is a linear or branched alkynyl group containing from 2 to 4 carbon atoms and having one or more, e.g. one or two, typically one triple bonds. Typically a $C_2$ to $C_4$ alkynyl group is a $C_2$ to $C_3$ alkynyl group. Examples of $C_2$ to $C_4$ alkynyl groups include ethynyl, propynyl and butynyl. For the avoidance of doubt, where two alkynyl groups are present, the alkynyl groups may be the same or different.

Unless otherwise stated, an alkyl, alkoxy, alkenyl or alkynyl group as defined herein may be unsubstituted or substituted as provided herein. The substituents on a substituted alkyl, alkenyl, alkynyl or alkoxy group are typically themselves unsubstituted. Where more than one substituent is present, these may be the same or different.

As used herein, a halogen is typically chlorine, fluorine, bromine or iodine and is preferably chlorine, bromine or fluorine, especially chorine or fluorine.

A 4- to 10- membered carbocyclic group is a cyclic hydrocarbon containing from 4 to 10 carbon atoms. A carbocyclic group may be saturated or partially unsaturated, but is typically saturated. A 4- to 10- membered carbocyclic group may be a fused bicyclic group or a spiro bicyclic group, as defined herein. A 4- to 10- membered carbocyclic group may be a saturated 4- to 6-membered, preferably 5- or 6- membered carbocyclic group. Examples of 4- to 6- membered saturated carbocyclic groups include cyclobutyl, cyclopentyl and cyclohexyl groups.

A 4- to 10- membered heterocyclic group is a cyclic group containing from 4 to 10 atoms selected from C, O, N and S in the ring, including at least one heteroatom, and typically one or two heteroatoms. The heteroatom or heteroatoms are typically selected from O, N, and S, most typically from O and N, especially N. A heterocyclic group may be saturated or partially unsaturated, but is typically saturated. A 4- to 10- membered heterocyclic group may be a fused bicyclic group or a spiro bicyclic group, as defined herein. A 4- to 10- membered heterocyclic group may be a saturated 4- to 6-membered, preferably 5- or 6- membered heterocyclic group.

References herein to heterocyclic group(s) include quaternised derivatives thereof, as defined herein. Preferred nitrogen-containing heterocyclic groups include azetidine, morpholine, 1,4-oxazepane, octahydropyrrolo[3,4-c]pyrrole, piperazine, piperidine, and pyrrolidine, including quaternised derivatives thereof, as defined herein.

As used herein, a $C_6$ to $C_{10}$ aryl group is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 10- membered heteroaryl group is a substituted or unsubstituted monocyclic or fused polycyclic aromatic group containing from 5 to 10 atoms in the ring portion, including at least one heteroatom, for example 1, 2 or 3 heteroatoms, typically selected from O, S and N. A heteroaryl group is typically a 5- or 6-membered heteroaryl group or a 9- or 10- membered heteroaryl group. Preferably, the heteroaryl group comprises 1, 2 or 3, preferably 1 or 2 nitrogen atoms. References herein to heteroaryl group(s) include quaternised derivatives thereof, as defined herein. Preferred nitrogen-containing heteroaryl groups include imidazole, pyridine, pyrimidine and pyrazine, including quaternised derivatives thereof, as defined herein.

As used herein, a fused bicyclic group is a group comprising two cyclic moieties sharing a common bond between two atoms. A spiro bicyclic group is a group comprising two cyclic moieties sharing a common atom.

A carbocyclic, heterocyclic, aryl or heteroaryl group may be unsubstituted or substituted as described herein. The substituents on a substituted carbocyclic, heterocyclic, aryl or heteroaryl group are typically themselves unsubstituted, unless otherwise stated.

A number of the compounds described herein comprise heterocyclic or heteroaryl groups comprising at least one nitrogen atom. In such compounds, said nitrogen atom(s) are independently selected from secondary, tertiary and quaternary nitrogen atom(s). A quaternary nitrogen atom is present when the compound comprises a quaternised derivative of one or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, palmitic, benzoic, acetic, triphenylacetic, methanesulphonic, ethanesulphonic, 1-hydroxy-2-naphthenoic, isethionic, benzenesulphonic or p-toluenesulphonic acid. Pharmaceutically acceptable bases include alkali metal (e.g. sodium or potassium), alkali earth metal (e.g. calcium or magnesium) and zinc bases, for example hydroxides, carbonates, and bicarbonates, and organic bases such as alkyl amines, aralkyl (i.e. aryl-substituted alkyl; e.g. benzyl) amines and heterocyclic amines.

Where the compound of Formula (I) contains a positively charged nitrogen atom, the compound may exist as a zwitterion, where $R^1$ is $O^-$, thus leaving a $COO^-$ group. Such compounds may also be provided in the form of a pharmaceutically acceptable salt. Suitable salts include those formed with pharmaceutically acceptable acids, which provide a proton to the $COO^-$ group, and a counterion to balance the positive charge on the quaternary nitrogen atom. Suitable pharmaceutically acceptable acids include hydrochloric acid, sulphonic acids including methanesulphonic acid and toluene sulphonic acid, ascorbic acid and citric acid. Hydrochloric acid and sulphonic acids are preferred, in particular hydrochloric acid. Alternatively, zwitterions can be combined with pharmaceutically acceptable bases as mentioned above, for example, alkali metal (e.g. sodium or potassium) and alkali earth metal (e.g. calcium or magnesium) hydroxides.

In Formula (I), the stereochemistry is not limited. In particular, compounds of Formula (I) containing one or more stereocentre (e.g. one or more chiral centre) may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound according to Formula (I) which is enantiomerically or diasteriomerically pure. Thus the compound is preferably substantially ontically pure

*Compounds of the Invention*

Preferably, $R^1$ is selected from OH, NHOH and $OR^{1a}$, or where the compound of Formula (I) contains a positively charged nitrogen atom, $R^1$ may be $O^-$, such that the compound forms a zwitterion. $R^{1a}$ is typically an unsubstituted $C_1$ to C4 alkyl group, such as an unsubstituted $C_1$ to $C_2$ alkyl group. More preferably, $R^{1a}$ is methyl or t-butyl.

More preferably, $R^1$ is OH or NHOH, or where the compound of Formula (I) contains a positively charged nitrogen atom, $R^1$ may be $O^-$, such that the compound forms a zwitterion. Still more preferably, $R^1$ is OH, or where the compound of Formula (I) contains a positively charged nitrogen atom, $R^1$ may be $O^-$, such that the compound forms a zwitterion.

Preferably, $R^2$ is selected from H and $C_1$ to $C_2$ alkyl, preferably $R^2$ is selected from H and methyl. More preferably, $R^2$ is H. Preferably, in the invention, $R^4$ is selected from H and methyl. More preferably, $R^4$ is H. Still more preferably, $R^2$ and $R^4$ are independently H or methyl, most preferably they are both H.

Preferably, therefore, in the invention, $R^1$ is selected from OH, NHOH and $OR^{1a}$; or where the compound of Formula (I) contains a positively charged nitrogen atom, $R^1$ may be $O^-$, such that the compound forms a zwitterion; $R^2$ is selected from H and unsubstituted $C_1$ to $C_2$ alkyl; and $R^4$ is H.

Each $R^3$ group is typically independently selected from halogen; and -OH; and $-NH_2$. More preferably, each $R^3$ group is independently selected from halogen (e.g. fluorine or chlorine) and-OH. Yet more preferably each $R^3$ group is halogen (e.g. fluorine or chlorine), most preferably fluorine. Typically, *n* is an integer from 0 to 2; more preferably *n* is 0 or 2; most preferably *n* is 0. Preferably, where more than one $R^3$ group is present, each $R^3$ is the same.

Preferably, therefore, in the invention:

- $R^1$ is OH or NHOH, or where the compound of Formula (I) contains a positively charged nitrogen atom, $R^1$ may be $O^-$, such that the compound forms a zwitterion;
- $R^2$ is selected from H and methyl;

- each $R^3$ group is independently selected from halogen (e.g. fluorine or chlorine) and -OH;
- *n* is an integer from 0 to 2; and
- $R^4$ is selected from H and methyl.

More preferably,

- $R^1$ is OH, or where the compound of Formula (I) contains a positively charged nitrogen atom, $R^1$ may be $O^-$, such that the compound forms a zwitterion;
- $R^2$ is H;
- each $R^3$ group is independently a halogen group (e.g. fluorine or chlorine);
- *n* is 0 or 2; and
- $R^4$ is H.

Preferably, in the invention, L is an unsubstituted $C_1$ alkylene group (i.e. a $-CH_2-$ group). Preferably, M is selected from a bond and an unsubstituted $C_1$ alkylene ($-CH_2-$) group; more preferably M is a bond. Most preferably, therefore, L is an unsubstituted $C_1$ alkylene group and M is a bond.

Preferably, in the invention, *p* is 0. However, in embodiments of the invention when *p* is 1, $R^5$ is preferably selected from halogen, -OH, -CN, methyl and -OMe. More preferably, $R^5$ is selected from halogen, -OH, -CN, and methyl. Still more preferably, $R^5$ is selected from halogen, -CN, and methyl; most preferably $R^5$ is methyl.

Preferably, in the invention, Ⓐ is selected from phenyl and 5- to 6-membered heteroaryl and heterocyclic groups, wherein when Ⓐ is a heterocyclic or heteroaryl group it comprises at least one nitrogen atom, said nitrogen atom(s) being independently selected from secondary, tertiary and quaternary nitrogen atom(s). Preferably, Ⓐ is aromatic. Preferably, Ⓐ is selected from benzene, pyridine, pyrimidine, pyrazine, pyrazole, triazole, dihydropyridine, piperazine and piperidine. More preferably, Ⓐ is selected from benzene, pyridine, pyrimidine, pyrazine, pyrazole, and triazole. Still more preferably, Ⓐ is selected from benzene, pyridine, and pyrazole. Most preferably, Ⓐ is benzene (phenyl).

Preferably, therefore, in the invention:

- L is an unsubstituted $C_1$ alkylene group;
- M is selected from a bond and an unsubstituted $C_1$ alkylene group;
- *p* is 0; or *p* is 1 and $R^5$ is selected from halogen, -CN, and methyl;
- Ⓐ is selected from phenyl and 5- to 6-membered heteroaryl and heterocyclic groups, wherein when Ⓐ is a heterocyclic or heteroaryl group it comprises at least one nitrogen atom, said nitrogen atom(s) being independently selected from secondary, tertiary and quaternary nitrogen atom(s).

More preferably,

- L is an unsubstituted $C_1$ alkylene group;
- M is selected from a bond and an unsubstituted $C_1$ alkylene group; preferably M is a bond;
- *p* is 0; or *p* is 1 and $R^5$ is methyl; preferably *p* is 0
- Ⓐ is selected from benzene, pyridine, pyrimidine, pyrazine, pyrazole, triazole, dihydropyridine, piperazine and piperidine; preferably Ⓐ is benzene (phenyl).

In the invention, *q* is preferably 1 or 2. In one preferred embodiment, *q* is 1. In another preferred embodiment, *q* is 2.

In the invention, each $R^6$ is preferably independently selected from:

◦ halogen, -OH;
◦ oxo, providing that Ⓐ is a carbocyclic or heterocyclic group;
◦ $-R^{6a}$, $-OR^{6a}$, $NR^{20}R^{6a}$, $-C(O)NR^{20}R^{6a}$, $-NR^{20}C(O)R^{6a}$, $-SO_2R^{6a}$, $-SO_2NR^{20}R^{6a}$, and $-NR^{20}SO_2R^{6a}$; wherein
each $R^{6a}$ is independently selected from $C_1$ to $C_3$ alkyl and $C_2$ to $C_3$ alkynyl, and wherein each $R^{6a}$ is independently unsubstituted or is substituted with one or two groups selected from -OH; $-NR^{20}R^{21}$; $-N^+R^{20}R^{21}R^{22}$; $-NR^{20}C(NR^{21})NR^{22}R^{23}$; $-NR^{20}C(N^+R^{21}R^{22})NR^{23}R^{24}$; $-NR^{20}C(NR^{21})R^{22}$; $-NR^{21}C(N^+R^{21}R^{22})R^{23}$; $-C(NR^{20})NR^{21}R^{22}$; and $-C(N^+R^{20}R^{21})NR^{22}R^{23}$; or with one, two or three halogen groups
◦ $-R^7-(Het^1)_v-R^9-R^{R1}$ and $-R^7-R^{R1}-Het^2-R^{R2}$; wherein
$R^{R1}$ and $R^{R2}$ are each independently a 5- to 6- membered heteroaryl or 4- to 8-membered heterocyclic group comprising at least one nitrogen atom, and said nitrogen atom(s) are independently selected from secondary, tertiary and quaternary nitrogen atom(s); and wherein each $R^{R1}$ and $R^{R2}$ is independently unsubstituted or is substituted

with one or two groups independently selected from

- oxo, providing that said $R^{R1}$ or $R^{R2}$ group is a heterocyclic group;
- $-R^{20}$, $-R^7-OR^{20}$; $-R^7-NR^{20}R^{21}$; $-R^7-N^+R^{20}R^{21}R^{22}$; $-R^7-NR^{20}C(NR^{21})NR^{22}R^{23}$; $-R^7-NR^{20}C(N^+R^{21}R^{22})NR^{23}R^{24}$; $-R^7-NR^{20}C(NR^{21})R^{22}$; $-R^7-NR^{20}C(N^+R^{21}R^2)R^{23}$; $-R^7-C(NR^{20})NR^{21}R^{22}$; $-R^7-C(N^+R^{20}R^{21})NR^{22}R^{23}$; $-R^7-C(NR^{20})R^{21}$; $-R^7(N^+R^{20}R^{21})R^{22}$; $-R^7-C(O)-R^9-NR^{20}R^{21}$; $R^7-C(O)-R^9-N^+R^{20}R^{21}R^{22}$; and $R^7-C(O)-R^{20}$; or
- with one, two or three halogen groups;

More preferably, each $R^6$ is independently selected from:

- ◦ halogen (e.g. fluorine or chlorine), and -OH;
- ◦ oxo, providing that Ⓐ is a carbocyclic or heterocyclic group;
- ◦ $-R^{6a}$, $-OR^{6a}$, $-C(O)NR^{20}R^{6a}$, $-NR^{20}C(O)R^{6a}$, and $-SO_2NR^{20}R^{6a}$; wherein each $R^{6a}$ is independently selected from $C_1$ to $C_3$ alkyl and $C_2$ to $C_3$ alkynyl, and is independently unsubstituted or is substituted with one group selected from -OH; $-NR^{20}R^{21}$;$-N^+R^{20}R^{21}R^{22}$; $-NR^{20}C(NR^{21})NR^{22}R^{23}$; and $-NR^{20}C(NR^{21})R^{22}$; or with three halogen (e.g. fluorine) groups; and
- ◦ $-R^7-(Het^1)_v-R^9-R^{R1}$ and $-R^7-R^{R1}-Het^2-R^{R2}$.

In embodiments of the invention in which $R^6$ is other than $-R^7-(Het^1)_v-R^9-R^{R1}$ or $-R^7-R^{R1}-Het^2-R^{R2}$, $R^6$ is more preferably selected from halogen (preferably chlorine); -OH; $-OCF_3$; $-(CH_2)_z-H$;$-(CH_2)_z-OH$; $-(CH_2)_z-N(R^X)_2$; $-(CH_2)_z-N^+(R^X)_3$, $-O-(CH_2)_z-H$; $-O-(CH_2)_z-OH$; $-O-(CH_2)_z-N(R^X)_2$; $-O-(CH_2)_z-W(R^X)_3$; $-O-(CH_2)_z-N(R^X)-C(NR^X)-CH_3$; $-O-(CH_2)_z-N(R^X)-C(NR^X)-N(R^X)_2$; $-C(O)NR^X-(CH_2)_z-N(R^X)_2$; $-C(O)NR^X-(CH_2)_z-N^+(R^X)_3$; $-NR^XC(O)-(CH_2)_z-N(R^X)_2$; $-NR^XC(O)-(CH_2)_z-N^+(R^X)_3$; $-SO_2-(CH_2)_z-H$; $-SO_2-NR^X-(CH_2)_z-N(R^X)_2$; $-SO_2-NR^X-(CH_2)_z-N^+(R^X)_3$; $-C\equiv C-CH_2-N(R^X)_2$ and$-C\equiv C-CH_2-N^+(R^X)_3$; wherein $R^X$ is H or Me and z is 1, 2 or 3;

In embodiments of the invention in which $R^6$ is selected from $-R^7-(Het^1)_v-R^9-R^{R1}$ and $-R^7-R^{R1}-Het^2-R^{R2}$:

- $R^{R1}$ and $R^{R2}$ are preferably each independently selected from piperazine; azetidine; morpholine; piperidine; pyrrolidine; octahydropyrrolo[3,4-c]pyrrole; imidazole; pyridine; and 1,4-oxazepane; preferably $R^{R1}$ and $R^{R2}$ are each independently selected from piperazine; morpholine and piperidine; wherein the nitrogen atom(s) in rings $R^{R1}$ and $R^{R2}$ are independently selected from secondary, tertiary and quaternary nitrogen atom(s); and wherein each $R^{R1}$ and $R^{R2}$ is independently unsubstituted or is substituted with one or two groups independently selected from

  - oxo, providing that said $R^{R1}$ or $R^{R2}$ group is a heterocyclic group; or
  - $-R^{20}$, $-R^7-OR^{20}$, $-R^7-NR^{20}R^{21}$; $-R^7-N^+R^{20}R^{21}R^{22}$; $-R^7-C(NR^{20})NR^{21}R^{22}$; $-R^7-C(NR^{20})R^{21}$; $-R^7-C(O)-R^9-NR^{20}R^{21}$; and $R^7-C(O)-R^{20}$;

- $Het^1$ is preferably selected from -O-, -C(O)-, $-SO_2-$, $-NR^{20}C(O)-$, and $-O-R^8-C(O)-$; and
- $Het^2$ is preferably selected from -C(O)- and $-R^8-C(O)-$.

In embodiments of the invention in which $R^6$ is selected from $-R^7-(Het^1)_v-R^9-R^{R1}$ and $-R^7-R^{R1}-Het^2-R^{R2}$; each $R^6$ is more preferably independently selected from $-R^{R1}$; $-(CH_2)_z-RR^1$; $-O-(CH_2)_z-R^{R1}$;$-C(O)-R^{R1}$; $-(CH_2)_z-C(O)-R^{R1}$; $-N(R^X)-C(O)-R^{R1}$; $-N(R^X)-C(O)-(CH_2)_z-R^{R1}$; $-O-(CH_2)_z-C(O)-R^{R1}$;$-SO_2-R^{R1}$; $R^{R1}-C(O)-R^{R2}$; $-(CH_2)_z-R^{R1}-C(O)-R^{R2}$; $-R^{R1}-(CH_2)_z-C(O)-R^{R2}$; and each $R^{R1}$ and $R^{R2}$ is independently unsubstituted or is substituted with one or two groups selected from $-CH_3$; $-N(R^X)_2$;$-N^+(R^X)_3$; $-(CH_2)_z-N(R^X)_2$; $-(CH_2)_z-N^+(R^X)_3$; $-OCH_3$; oxo; $-C(O)-CH_3$; $-(CO)-N(R^X)_2$; $-(CO)-(CH_2)_z-N(R^X)_2$; $-C(NR^X)-N(R^X)_2$ and $-C(NR^X)-CH_3$; wherein $R^X$ is H or Me and z is 1, 2 or 3. In such embodiments, $R^{R1}$ and $R^{R2}$ are preferably each independently selected from piperazine; azetidine; morpholine; piperidine; pyrrolidine; octahydropyrrolo[3,4-c]pyrrole; imidazole; pyridine; and 1,4-oxazepane; preferably $R^{R1}$ and $R^{R2}$ are each independently selected from piperazine; morpholine and piperidine

In embodiments of the invention in which $R^6$ is selected from $-R^7-(Het^1)_v-R^9-R^{R1}$ and $-R^7-R^{R1}-Het^2-R^{R2}$; each $R^6$ is preferably a $-R^7-(Het^1)_v-R^9-R^{R1}$ group.

Preferably, therefore, in the invention, *q* is 1 or 2; and each $R^6$ is independently selected from:

A:

- ◦ halogen (e.g. fluorine or chlorine), and -OH;
- ◦ oxo, providing that Ⓐ is a carbocyclic or heterocyclic group;
- ◦ $-R^{6a}$, $-OR^{6a}$, $-C(O)NR^{20}R^{6a}$, $-NR^{20}C(O)R^{6a}$, and $-SO_2NR^{20}R^{6a}$; wherein each $R^{6a}$ is independently selected

from $C_1$ to $C_3$ alkyl and $C_2$ to $C_3$ alkynyl, and is independently unsubstituted or is substituted with one group selected from -OH; $-NR^{20}R^{21}$; $-N^+R^{20}R^{21}R^{22}$; $-NR^{20}C(NR^{21})NR^{22}R^{23}$; and $-NR^{20}C(NR^{21})R^{22}$; or with three halogen (e.g. fluorine) groups; and

B:
◦ $-R^7-(Het^1)_v-R^9-R^{R1}$ and $-R^7-R^{R1}-Het^2-R^{R2}$; wherein

- $R^{R1}$ and $R^{R2}$ are each independently selected from piperazine; azetidine; morpholine; piperidine; pyrrolidine; octahydropyrrolo[3,4-c]pyrrole; imidazole; pyridine; and 1,4-oxazepane; preferably $R^{R1}$ and $R^{R2}$ are each independently selected from piperazine; morpholine and piperidine; wherein the nitrogen atom(s) in rings $R^{R1}$ and $R^{R2}$ are independently selected from secondary, tertiary and quaternary nitrogen atom(s); and wherein each $R^{R1}$ and $R^{R2}$ is independently unsubstituted or is substituted with one or two groups independently selected from
  - oxo, providing that said $R^{R1}$ or $R^{R2}$ group is a heterocyclic group; or
  - $-R^{20}$, $-R^7-OR^{20}$; $-R^7-NR^{20}R^{21}$; $-R^7-N^+R^2R^{21}R^{22}$; $-R^7-C(NR^{20})NR^{21}R^{22}$; $-R^7-C(NR^{20})R^{21}$; $-R^7-C(O)-R^9-NR^{20}R^{21}$; and $R^7-C(O)-R^{20}$;
- $Het^1$ is selected from -O-, -C(O)-, $-SO_2-$, $-NR^{20}C(O)-$, and $-O-R^8-C(O)-$; and
- $Het^2$ is selected from -C(O)- and $-R^8-C(O)-$.

More preferably, *q* is 1 or 2; and each $R^6$ is independently selected from:

- halogen (preferably chlorine); -OH; $-OCF_3$; $-(CH_2)_z-H$; $-(CH_2)_z-OH$; $-(CH_2)_z-N(R^X)_2$;$-(CH_2)_z-N^+(R^X)_3$,$-O-(CH_2)_z-H$; $-O-(CH_2)_z-OH$; $-O-(CH_2)_z-N(R^X)_2$; $-O-(CH_2)_z-N^+(O^X)_3$; $-O-(CH_2)_z-N(R^X)-C(NR^X)-CH_3$; $-O-(CH_2)_z-N(R^X)-C(NR^X)-N(R^X)_2$; $-C(O)NR^X-(CH_2)_z-N(R^X)_2$; $-C(O)NR^X-(CH_2)_z-N^+(R^X)_3$; $-NR^XC(O)-(CH_2)_z-N(R^X)_2$; $-NR^XC(O)-(CH_2)_z-N^+(R^X)_3$; $-SO_2-(CH_2)_z-H$; $-SO_2-NR^X-(CH_2)_z-N(R^X)_2$; $-SO_2-NR^X-(CH_2)_z-N^+(R^X)_3$; $-OC{\equiv}CH_2-N(R^X)_2$ and $-C{\equiv}C-CH_2-N^+(R^X)_3$; wherein $R^X$ is H or Me and z is 1, 2 or 3; and
- $-R^{R1}$; $-(CH_2)_z-R^{R1}$; $-O-(CH_2)_z-R^{R1}$; $-C(O)-R^{R1}$; $-(CH_2)_z-C(O)-R^{R1}$; $-N(R^X)-C(O)-R^{R1}$;$-N(R^X)-C(O)-(CH_2)_z-R^{R1}$; $-O-(CH_2)_z-C(O)-R^{R1}$; $-SO_2-R^{R1}$; $R^{R1}-C(O)-R^{R2}$; $-(CH_2)_z-R^{R1}-C(O)-R^{R2}$; $-R^{R1}-(CH_2)_z-C(O)-R^{R2}$; and each $R^{R1}$ and $R^{R2}$ is independently unsubstituted or is substituted with one or two groups selected from $-CH_3$; $-N(R^X)_2$; $-N^+(R^X)_3$; $-(CH_2)_z-N(R^X)_2$; $-(CH_2)_z-N^+(R^X)_3$; $-OCH_3$; oxo; $-C(O)-CH_3$; $-(CO)-N(R^X)_2$; $-(CO)-(CH_2)_z-N(R^X)_2$; $-C(NR^X)-N(R^X)_2$ and $-C(NR^X)-CH_3$; wherein $R^X$ is H or Me and z is 1, 2 or 3; and wherein $R^{R1}$ and $R^{R2}$ are each independently selected from piperazine; azetidine; morpholine; piperidine; pyrrolidine; octahydropyrrolo[3,4-c]pyrrole; imidazole; pyridine; and 1,4-oxazepane; preferably from piperazine; morpholine and piperidine.

In particularly preferred compounds of the invention, therefore:

- $R^1$ is OH or NHOH, or where the compound of Formula (I) contains a positively charged nitrogen atom, $R^1$ may be $O^-$, such that the compound forms a zwitterion;
- $R^2$ is selected from H and methyl;
- each $R^3$ group is independently selected from halogen (e.g. fluorine or chlorine) and -OH;
- *n* is an integer from 0 to 2;
- $R^4$ is selected from H and methyl;
- L is an unsubstituted $C_1$ alkylene group;
- M is selected from a bond and an unsubstituted $C_1$ alkylene group;
- *p* is 0; or *p* is 1 and $R^5$ is selected from halogen, -CN, and methyl;
- Ⓐ is selected from phenyl and 5- to 6-membered heteroaryl and heterocyclic groups, wherein when Ⓐ is a heterocyclic or heteroaryl group it comprises at least one nitrogen atom, said nitrogen atom(s) being independently selected from secondary, tertiary and quaternary nitrogen atom(s);
- *q* is 1 or 2; and each $R^6$ is independently selected from:

**A:**

◦ halogen (e.g. fluorine or chlorine), and -OH;
◦ oxo, providing that Ⓐ is a carbocyclic or heterocyclic group;
◦ $-R^{6a}$, $-OR^{6a}$, $-C(O)NR^{20}R^{6a}$, $-NR^{20}C(O)R^{6a}$, and $-SO_2NR^{20}R^{6a}$; wherein each $R^{6a}$ is independently se-

lected from $C_1$ to $C_3$ alkyl and $C_2$ to $C_3$ alkynyl, and is independently unsubstituted or is substituted with one group selected from -OH;-$NR^{20}R^{21}$; -$N^+R^{20}R^{21}R^{22}$; -$NR^{20}C(NR^{21})NR^{22}R^{23}$; and -$NR^{20}C(NR^{21})R^{22}$; or with three halogen (e.g. fluorine) groups; and

**B:**

◦ -$R^7$-$(Het^1)_v$-$R^9$-$R^{R1}$ and -$R^7$-$R^{R1}$-$Het^2$-$R^{R2}$; wherein

- $R^{R1}$ and $R^{R2}$ are each independently selected from piperazine; azetidine; morpholine; piperidine; pyrrolidine; octahydropyrrolo[3,4-c]pyrrole; imidazole; pyridine; and 1,4-oxazepane; preferably $R^{R1}$ and $R^{R2}$ are each independently selected from piperazine; morpholine and piperidine; wherein the nitrogen atom(s) in rings $R^{R1}$ and $R^{R2}$ are independently selected from secondary, tertiary and quaternary nitrogen atom(s); and wherein each $R^{R1}$ and $R^{R2}$ is independently unsubstituted or is substituted with one or two groups independently selected from

  - oxo, providing that said $R^{R1}$ or $R^{R2}$ group is a heterocyclic group; or
  - -$R^{20}$, -$R^7$-$OR^{20}$; -$R^7$-$NR^{20}R^{21}$; -$R^7$-$N^+R^{20}R^{21}R^{22}$; -$R^7$-$C(NR^{20})NR^{21}R^{22}$; -$R^7$-$C(NR^{20})R^{21}$; -$R^7$-C(O)-$R^9$-$NR^{20}R^{21}$; and $R^7$-C(O)-$R^{20}$;

- $Het^1$ is selected from -O-, -C(O)-, -$SO_2$-, -$NR^{20}C(O)$-, and -O-$R^8$-C(O)-; and
- $Het^2$ is selected from -C(O)- and -$R^8$-C(O)-.

In still more particularly preferred compounds of the invention:

- $R^1$ is OH, or where the compound of Formula (I) contains a positively charged nitrogen atom, $R^1$ may be $O^-$, such that the compound forms a zwitterion;
- $R^2$ is H;
- each $R^3$ group is independently a halogen group (e.g. fluorine or chlorine);
- *n* is 0 or 2;
- $R^4$ is H;
- L is an unsubstituted $C_1$ alkylene group;
- M is selected from a bond and an unsubstituted $C_1$ alkylene group; preferably M is a bond;
- *p* is 0; or *p* is 1 and $R^5$ is methyl; preferably *p* is 0
- Ⓐ is selected from benzene, pyridine, pyrimidine, pyrazine, pyrazole, triazole, dihydropyridine, piperazine and piperidine; preferably Ⓐ is benzene (phenyl);
- *q* is 1 or 2; and each $R^6$ is independently selected from:

  ◦ halogen (preferably chlorine); -OH; -$OCF_3$; -$(CH_2)_z$-H; -$(CH_2)_z$-OH; -$(CH_2)_z$-$N(R^X)_2$; -$(CH_2)_z$-$N^+(R^X)_3$; -O-$(CH_2)_z$-H; -O-$(CH_2)_z$-OH; -O-$(CH_2)_z$-$N(R^X)_2$; -O-$(CH_2)_z$-$N^+(R^X)_3$; -O-$(CH_2)_z$-$N(R^X)$-$C(NR^X)$-$CH_3$; -O-$(CH_2)_z$-$N(R^X)$-$C(NR^X)$-$N(R^X)_2$; -$C(O)NR^X$-$(CH_2)_z$-$N(R^X)_2$; -$C(O)NR^X$-$(CH_2)_z$-$N^+(R^X)_3$; -$NR^XC(O)$-$(CH_2)_z$-$N(R^X)_2$; -$NR^XC(O)$-$(CH_2)_z$-$N^+(R^X)_3$; -$SO_2$-$(CH_2)_z$-H; -$SO_2$-$NR^X$-$(CH_2)_z$-$N(R^X)_2$;-$SO_2$-$NR^X$-$(CH_2)_z$-$N^+(R^X)_3$; -C≡C-$CH_2$-$N(R^X)_2$ and -C≡C-$CH_2$-$N^+(R^X)_3$; wherein $R^X$ is H or Me and z is 1, 2 or 3;

  and

  ◦ -$R^{R1}$; -$(CH_2)_z$-$R^{R1}$; -O-$(CH_2)_z$-$R^{R1}$; -C(O)-$R^{R1}$; -$(CH_2)_z$-C(O)-$R^{R1}$; -$N(R^X)$-C(O)-$R^{R1}$; -$N(R^X)$-C(O)-$(CH_2)_z$-$R^{R1}$; -O-$(CH_2)_z$-C(O)-$R^{R1}$; -$SO_2$-$R^{R1}$; $R^{R1}$-C(O)-$R^{R2}$;-$(CH_2)_z$-$R^{R1}$-C(O)-$R^{R2}$; -$R^{R1}$-$(CH_2)_z$-C(O)-$R^{R2}$; and each $R^{R1}$ and $R^{R2}$ is independently unsubstituted or is substituted with one or two groups selected from -$CH_3$; -$N(R^X)_2$; -$N^+(R^X)_3$; -$(CH_2)_z$-$N(R^X)_2$; -$(CH_2)_z$-$N^+(R^X)_3$; -$OCH_3$; oxo; -C(O)-$CH_3$; -(CO)-$N(R^X)_2$; -(CO)-$(CH_2)_z$-$N(R^X)_2$; -$C(NR^X)$-$N(R^X)_2$ and -$C(NR^X)$-$CH_3$; wherein $R^X$ is H or Me and z is 1, 2 or 3; and wherein $R^{R1}$ and $R^{R2}$ are each independently selected from piperazine; azetidine; morpholine; piperidine; pyrrolidine; octahydropyrrolo[3,4-c]pyrrole; imidazole; pyridine; and 1,4-oxazepane; preferably from piperazine; morpholine and piperidine.

Preferably, in the invention, $R^7$ is selected from a bond and unsubstituted $C_1$ alkylene; more preferably $R^7$ is a bond. Preferably, in the invention, $R^8$ is unsubstituted $C_1$ alkylene. Preferably, in the invention, $R^9$ is selected from a bond and unsubstituted $C_1$ to $C_2$ alkylene; more preferably $R^9$ is unsubstituted $C_1$ to $C_2$ alkylene; preferably $C_1$ alkylene.

Preferably, in the invention, $R^{20}$, $R^{21}$, $R^{22}$, $R^{23}$ and $R^{24}$ are each independently selected from H and unsubstituted $C_1$ alkyl.

Preferred compounds of the invention are provided in the Examples.

More preferred compounds of the invention are selected from: 2-[2-[(4-phenylthiazol-2-yl)methylcarbamoyl]indan-2-yl]acetic acid; 2-[5,6-difluoro-2-[(4-phenylthiazol-2-yl)methylcarbamoyl]indan-2-yl]acetic acid; 2-[2-[[4-(3-methoxyphenyl)thiazol-2-yl]methylcarbamoyl]indan-2-yl]acetic acid; 2-[2-[[4-(4-methoxyphenyl)thiazol-2-yl]methylcarbamoyl]indan-2-yl]acetic acid; 2-[2-[[4-(4-chlorophenyl)thiazol-2-yl]methylcarbamoyl]indan-2-yl]acetic acid; 2-[2-[[4-(3-pyridyl)thiazol-2-yl]methylcarbamoyl]indan-2-yl]acetic acid; 2-[2-[[4-(4-hydroxyphenyl)thiazol-2-yl]methylcarbamoyl]indan-2-yl]acetic acid; 2-[2-[[4-(3-hydroxyphenyl)thiazol-2-yl]methylcarbamoyl]indan-2-yl]acetic acid; 2-[2-[[4-[2-(2-hydroxyethoxy)phenyl]thiazol-2-yl]methylcarbamoyl]indan-2-yl]acetic acid; 2-[2-[[4-[4-(4-methylpiperazin-1-yl)phenyl]thiazol-2-yl]methylcarbamoyl]indan-2-yl]acetic acid; 2-[2-[[4-[4-(4-methylpiperazine-1-carbonyl)phenyl]thiazol-2-yl]methylcarbamoyl]indan-2-yl]acetic acid; 2-[2-[[4-[2-methoxy-4-(4-methylpiperazin-1-yl)phenyl]thiazol-2-yl]methylcarbamoyl]indan-2-yl]acetic acid; 2-[2-[[4-[4-[3-(dimethylamino)azetidin-1-yl]-2-methoxy-phenyl]thiazol-2-yl]methylcarbamoyl]indan-2-yl]acetic acid; 2-[2-[[4-[6-(4-methylpiperazin-1-yl)-3-pyridyl]thiazol-2-yl]methylcarbamoyl]indan-2-yl]acetic acid; 2-[2-[[4-[6-[3-(dimethylamino)azetidin-1-yl]-3-pyridyl]thiazol-2-yl]methylcarbamoyl]indan-2-yl]acetic acid; 2-[2-[[4-[4-[4-[2-(4-methylpiperazin-1-yl)-2-oxoethyl]piperazin-1-yl]phenyl]thiazol-2-yl]methylcarbamoyl]indan-2-yl]acetic acid; 2-[2-[[4-[4-[3-(dimethylamino)prop-1-ynyl]phenyl]thiazol-2-yl]methylcarbamoyl]indan-2-yl]acetic acid; 2-[2-[[4-[6-[2-(4-methylpiperazin-1-yl)-2-oxo-ethoxy]-3-pyridyl]thiazol-2-yl]methylcarbamoyl]indan-2-yl]acetic acid; 2-[2-[[4-[4-[[2-(dimethylamino)acetyl]amino]phenyl]thiazol-2-yl]methylcarbamoyl]indan-2-yl]acetic acid; 2-[2-[[4-[4-[(4-methylpiperazine-1-carbonyl)amino]phenyl]thiazol-2-yl]methylcarbamoyl]indan-2-yl]acetic acid; 2-[2-[[4-[4-[[2-(4-methylpiperazin-1-yl)acetyl]amino]phenyl]thiazol-2-yl]methylcarbamoyl]indan-2-yl]acetic acid; 2-[2-[[4-[4-[2-(4-methylpiperazin-1-yl)-2-oxo-ethoxy]phenyl]thiazol-2-yl]methylcarbamoyl]indan-2-yl]acetic acid; 2-[2-[[4-[4-(2-morpholinoethoxy)phenyl]thiazol-2-yl]methylcarbamoyl]indan-2-yl]acetic acid; 2-[2-[[4-[4-[(1-methylpyridin-1-ium-4-yl)methoxylphenyl]thiazol-2-yl]methylcarbamoyl]indan-2-yl]acetate; 2-[2-[[4-[4-(3-morpholinopropoxy)phenyl]thiazol-2-yl]methylcarbamoyl]indan-2-yl]acetic acid; 2-[2-[[4-[4-[3-(4-methylmorpholin-4-ium-4-yl)propoxy]phenyl]thiazol-2-yl]methylcarbamoyl]indan-2-yl]acetate; 2-[2-[[4-[4-[3-(4-methylpiperazin-1-yl)propoxy]phenyl]thiazol-2-yl]methylcarbamoyl]indan-2-yl]acetic acid; 2-[2-[[4-[4-(3-pyrrolidin-1-ylpropoxy)phenyl]thiazol-2-yl]methylcarbamoyl]indan-2-yl]acetic acid; 2-[2-[[4-[4-(3-pyridin-1-ium-1-ylpropoxy)phenyl]thiazol-2-yl]methylcarbamoyl]indan-2-yl]acetate; 2-[2-[[4-[3-methylsulfonyl-4-(3-morpholinopropoxy)phenyl]thiazol-2-yl]methylcarbamoyl]indan-2-yl]acetic acid; 2-[2-[[4-[4-[3-(4-methylmorpholin-4-ium-4-yl)propoxy]-3-methylsulfonyl-phenyl]thiazol-2-yl]methylcarbamoyl]indan-2-yl]acetate; 2-[5,6-difluoro-2-[[4-[4-[3-(4-methylmorpholin-4-ium-4-yl)propoxy]-3-methylsulfonyl-phenyl]thiazol-2-yl]methylcarbamoyl]indan-2-yl]acetate; 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid; 2-[5,6-difluoro-2-[[4-[2-methoxy-4-[2-oxo-2-[3-(trimethylammonio)azetidin-1-yl]ethoxy]phenyl]thiazol-2-yl]methylcarbamoyl]indan-2-yl]acetate; 2-[2-[[4-[2-methoxy-4-[2-(2-methyl-1,3,3a,4,6,6a-hexahydropyrrolo[3,4-c]pyrrol-5-yl)-2-oxo-ethoxy]phenyl]thiazol-2-yl]methylcarbamoyl]indan-2-yl]acetic acid; 2-[2-[[4-[2-methoxy-4-[2-(4-methylmorpholin-4-ium-4-yl)ethoxy]phenyl]thiazol-2-yl]methylcarbamoyl]indan-2-yl]acetate; 2-[5,6-difluoro-2-[[4-[2-methoxy-4-[2-(4-methylmorpholin-4-ium-4-yl)ethoxy]phenyl]thiazol-2-yl]methylcarbamoyl]indan-2-yl]acetate; 2-[5,6-difluoro-2-[[4-[2-methoxy-4-[3-(4-methylmorpholin-4-ium-4-yl)propoxy]phenyl]thiazol-2-yl]methylcarbamoyl]indan-2-yl]acetate; 2-[2-[[4-[2-methoxy-4-(3-morpholinopropoxy)phenyl]thiazol-2-yl]methylcarbamoyl]indan-2-yl]acetic acid; 2-[5,6-difluoro-2-[[4-[2-methoxy-4-(3-pyrrolidin-1-ylpropoxy)phenyl]thiazol-2-yl]methylcarbamoyl]indan-2-yl]acetic acid; 2-[5,6-difluoro-2-[[4-[2-methoxy-4-[3-(1-methylpyrrolidin-1-ium-1-yl)propoxy]phenyl]thiazol-2-yl]methylcarbamoyl]indan-2-yl]acetate; 2-[2-[[4-[2-methoxy-4-(4-pyridylmethoxy)phenyl]thiazol-2-yl]methylcarbamoyl]indan-2-yl]acetic acid; 2-[2-[[4-[2-methoxy-4-[(1-methylpyridin-1-ium-4-yl)methoxy]phenyl]thiazol-2-yl]methylcarbamoyl]indan-2-yl]acetate-, 2-[2-[[4-[2-methoxy-4-(2-morpholinoethoxy)phenyl]thiazol-2-yl]methylcarbamoyl]indan-2-yl]acetic acid; 2-[2-[[4-[2-methoxy-4-(3-pyridylmethoxy)phenyl]thiazol-2-yl]methylcarbamoyl]indan-2-yl]acetic acid; 2-[2-[[4-[4-[2-(4,4-dimethylpiperazin-4-ium-1-yl)-2-oxo-ethoxy]-2-methoxy-phenyl]thiazol-2-yl]methylcarbamoyl]indan-2-yl]acetate; 2-[2-[[4-[4-[3-(dimethylamino)propoxy]-2-methoxy-phenyl]thiazol-2-yl]methylcarbamoyl]-5,6-difluoro-indan-2-yl]acetic acid; 2-[5,6-difluoro-2-[[4-[2-methoxy-4-[3-(trimethylammonio)propoxy]phenyl]thiazol-2-yl]methylcarbamoyl]indan-2-yl]acetate; 2-[2-[[4-[4-[2-(4,4-dimethylpiperazin-4-ium-1-yl)-2-oxo-ethoxy]-2-methoxy-phenyl]thiazol-2-yl]methylcarbamoyl]-5,6-difluoro-indan-2-yl]acetate; 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid; 2-[2-[[4-[4-[2-(1,4-dimethyl-3-oxo-piperazin-1-ium-1-yl)ethoxy]-2-methoxy-phenyl]thiazol-2-yl]methylcarbamoyl]indan-2-yl]acetate; 2-[2-[[4-[2-methoxy-4-[2-(1-methylimidazol-2-yl)ethoxy]phenyl]thiazol-2-yl]methylcarbamoyl]indan-2-yl]acetic acid; 2-[2-[[4-[4-[2-(1,3-dimethylimidazol-1-ium-2-yl)ethoxy]-2-methoxy-phenyl]thiazol-2-yl]methylcarbamoyl]-5,6-difluoro-indan-2-yl]acetate; 2-[2-[[4-[4-[2-(4-acetylpiperazin-1-yl)ethoxy]-2-methoxy-phenyl]thiazol-2-yl]methylcarbamoyl]indan-2-yl]acetic acid; 2-[2-[[4-(1-methylpyrazol-4-yl)thiazol-2-yl]methylcarbamoyl]indan-2-yl]acetic acid; 2-[2-[[4-(1,5-dimethylpyrazol-4-yl)thiazol-2-yl]methylcarbamoyl]indan-2-yl]acetic acid; 2-[2-[[4-(2-methylpyrazol-3-yl)thiazol-2-yl]methylcarbamoyl]indan-2-yl]acetic acid; 2-[2-[[4-(1,3-dimethylpyrazol-4-yl)thiazol-2-yl]methylcarbamoyl]indan-2-yl]acetic acid; 2-[2-[[4-[1-(2-morpholinoethyl)pyrazol-4-yl]thiazol-2-yl]methylcarbamoyl]indan-2-yl]acetic acid; 2-[2-[[4-[1-(3-morpholinopropyl)pyrazol-4-yl]thiazol-2-yl]methylcarbamoyl]indan-2-yl]acetic acid; 2-[2-[[4-[1-[1-(4-methylpiperazine-1-carbonyl)-4-piperidyl]pyrazol-4-yl]thiazol-2-yl]methylcarbamoyl]indan-2-yl]acetic acid; 2-[2-[[4-[1-[1-[2-(4-methylpiperazin-1-yl)-2-oxo-ethyl]-4-piperidyl]pyrazol-4-yl]thiazol-2-yl]methylcarbamoyl]indan-2-yl]acetic acid; 2-[2-[[4-[1-[3-(4-acetylpiperazin-1-yl)propyl]pyrazol-4-yl]thiazol-2-yl]methylcarbamoyl]indan-2-yl]acetic acid; 2-[2-[[4-[1-[3-(4-methylpiperazin-1-yl)propyl]pyrazol-4-yl]thiazol-2-yl]methylcarbamoyl]indan-2-yl]acetic acid; 2-[2-[[4-[1-[3-(1,4-oxazepan-4-yl)propyl]pyrazol-4-yl]thiazol-2-yl]methylcarbamoyl]indan-2-yl]acetic acid; 2-[2-[[4-[1-[3-(4-methoxy-1-piperidyl)propyl]pyrazol-4-yl]thiazol-2-yl]methylcarbamoyl]indan-2-yl]acetic acid; 2-[2-[[4-[1-[3-[4-(4-methylpiperazine-1-carbonyl)piperazin-1-yl]propyl]pyrazol-4-yl]thiazol-2-yl]methylcarbamoyl]indan-2-yl]acetic acid; 2-[2-[[4-[1-[3-[4-(1-methylpiperidine-4-carbonyl)piperazin-1-yl]propyl]pyrazol-4-yl]thiazol-2-yl]methylcarbamoyl]indan-2-yl]acetic acid; 2-[2-[[4-[1-[3-(4-methyl-3-oxo-piperazin-1-yl)propyl]pyrazol-4-yl]thiazol-2-yl]methylcarbamoyl]indan-2-yl]acetic acid; 2-[2-[[4-[1-[2-(4-acetylpiperazin-1-yl)ethyl]pyrazol-4-yl]thiazol-2-yl]methylcarbamoyl]indan-2-yl]acetic acid; 2-[2-[[4-[1-[(1-methylpyridin-1-ium-4-yl)methyl]pyrazol-4-yl]thiazol-2-yl]methylcarbamoyl]indan-2-yl]acetate; 2-[2-[[4-[3-[[4-(dimethylamino)-1-piperidyl]sulfonyl]-4-methoxy-phenyl]thiazol-2-yl]methylcarbamoyl]indan-2-yl]acetic acid; 2-[2-[[4-[3-[[4-(dimethylamino)-1-piperidyl]sulfonyl]-4-methoxy-phenyl]thiazol-2-yl]methylcarbamoyl]indan-2-yl]acetic acid; 2-[2-[[4-[3-[[4-(dimethylamino)-1-piperidyl]sulfonyl]-4-methoxy-phenyl]thiazol-2-yl]methylcarbamoyl]-5,6-difluoro-indan-2-yl]acetic acid; 2-[5,6-difluoro-2-[[4-[4-methoxy-3-[[4-(trimethylammonio)-1-piperidyl]sulfonyl]phenyl]thiazol-2-yl]methylcarbamoyl]indan-2-yl]acetate; 2-[5,6-difluoro-2-[[4-[2-methoxy-5-[[4-(trimethylammonio)-1-piperidyl]sulfonyl]phenyl]thiazol-2-yl]methylcarbamoyl]indan-2-yl]acetate; 2-[2-[[4-[3-(4-methylpiperazin-1-yl)sulfonylphenyl]thiazol-2-yl]methylcarbamoyl]indan-2-yl]acetic acid; 2-[2-[[4-[4-(4-methylpiperazin-1-yl)sulfonylphenyl]thiazol-2-yl]methylcarbamoyl]indan-2-yl]acetic acid; 2-[2-[[4-[3-(4-methylpiperazin-1-yl)sulfonyl-4-(2-morpholinoethoxy)phenyl]thiazol-2-yl]methylcarbamoyl]indan-2-yl]acetic acid; 2-[2-[[4-[3-(4-methylpiperazin-1-yl)sulfonyl-4-(3-morpholinopropoxy)phenyl]thiazol-2-yl]methylcarbamoyl]indan-2-yl]acetic acid; 2-[2-[[4-[3-[[4-(dimethylamino)-1-piperidyl]sulfonyl]-4-(2-morpholinoethoxy)phenyl]thiazol-2-yl]methylcarbamoyl]-5,6-difluoro-indan-2-yl]acetic acid; 2-[2-[[4-[3-methylsulfonyl-4-(2-morpholinoethoxy)phenyl]thiazol-2-yl]methylcarbamoyl]indan-2-yl]acetic acid; 2-[2-[[4-[4-[2-(4-methylmorpholin-4-ium-4-yl)ethoxy]-3-methylsulfonyl-phenyl]thiazol-2-yl]methylcarbamoyl]indan-2-yl]acetate; 2-[2-[[4-[6-[2-(4-methylmorpholin-4-ium-4-yl)ethyl]-3-pyridyl]thiazol-2-yl]methylcarbamoyl]indan-2-yl]acetate; 2-[2-[[4-[6-(4-pyridylmethoxy)-3-pyridyl]thiazol-2-yl]methylcarbamoyl]indan-2-yl]acetic acid; 2-[2-[[4-[2-(trifluoromethoxy)phenyl]thiazol-2-yl]methylcarbamoyl]indan-2-yl]acetic acid; 2-[2-[[4-(2-methoxyphenyl)thiazol-2-yl]methylcarbamoyl]indan-2-yl]acetic acid; 2-[2-[[4-(2-ethylphenyl)thiazol-2-yl]methylcarbamoyl]indan-2-yl]acetic acid; 2-[2-[[4-[2-(hydroxymethyl)phenyl]thiazol-2-yl]methylcarbamoyl]indan-2-yl]acetic acid; 2-[2-[[4-[3-(hydroxymethyl)phenyl]thiazol-2-yl]methylcarbamoyl]indan-2-yl]acetic acid; 2-[2-[[4-(2-methoxy-3-pyridyl)thiazol-2-yl]methylcarbamoyl]indan-2-yl]acetic acid; 2-[2-[[4-(4-methoxy-3-pyridyl)thiazol-2-yl]methylcarbamoyl]indan-2-yl]acetic acid; 2-[2-[[4-[1-(4-methylpiperazine-1-carbonyl)-4-piperidyl]thiazol-2-yl]methylcarbamoyl]indan-2-yl]acetic acid; 2-[2-[[4-[1-(4-methylpiperazin-1-yl)sulfonyl-3-piperidyl]thiazol-2-yl]methylcarbamoyl]indan-2-yl]acetic acid; 2-[2-[[4-[4-(4-ethanimidoylpiperazin-1-yl)phenyl]thiazol-2-yl]methylcarbamoyl]indan-2-yl]acetic acid; 2-[2-(hydroxyamino)-2-oxo-ethyl]-N-[(4-phenylthiazol-2-yl)methyl]indane-2-carboxamide; 2-[2-(hydroxyamino)-2-oxo-ethyl]-N-[[4-[1-(2-morpholinoethyl)pyrazol-4-yl]thiazol-2-yl]methyl]indane-2-carboxamide; 2-[2-(hydroxyamino)-2-oxo-ethyl]-N-[[4-[1-[2-(4-methylpiperazin-1-yl)-2-oxo-ethyl]pyrazol-4-yl]thiazol-2-yl]methyl]indane-2-carboxamide; 2-[2-[[4-[4-[2-(ethanimidoylamino)ethoxy]phenyl]thiazol-2-yl]methylcarbamoyl]indan-2-yl]acetic acid; and 2-[2-[[4-[4-(2-guanidinoethoxy)phenyl]thiazol-2-yl]methylcarbamoyl]indan-2-yl]acetic acid; or a pharmaceutically acceptable salt thereof.

Yet more preferred compounds of the invention are selected from: 2-[2-[(4-phenylthiazol-2-yl)methylcarbamoyl]indan-2-yl]acetic acid; 2-[5,6-difluoro-2-[(4-phenylthiazol-2-yl)methylcarbamoyl]indan-2-yl]acetic acid; 2-[2-[[4-(4-methoxyphenyl)thiazol-2-yl]methylcarbamoyl]indan-2-yl]acetic acid; 2-[2-[[4-[4-[3-(dimethylamino)azetidin-1-yl]-2-methoxy-phenyl]thiazol-2-yl]methylcarbamoyl]indan-2-yl]acetic acid; 2-[2-[[4-[4-[4-[2-(4-methylpiperazin-1-yl)-2-oxo-ethyl]piperazin-1-yl]phenyl]thiazol-2-yl]methylcarbamoyl]indan-2-yl]acetic acid; 2-[2-[[4-[4-[2-(4-methylpiperazin-1-yl)-2-oxo-ethoxy]phenyl]thiazol-2-yl]methylcarbamoyl]indan-2-yl]acetic acid; 2-[2-[[4-[3-methylsulfonyl-4-(3-morpholinopropoxy)phenyl]thiazol-2-yl]methylcarbamoyl]indan-2-yl]acetic acid; 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid; 2-[2-[[4-[2-methoxy-4-(3-morpholinopropoxy)phenyl]thiazol-2-yl]methylcarbamoyl]indan-2-yl]acetic acid; 2-[5,6-difluoro-2-[[4-[2-methoxy-4-(3-pyrrolidin-1-ylpropoxy)phenyl]thiazol-2-yl]methylcarbamoyl]indan-2-yl]acetic acid; 2-[2-[[4-[2-methoxy-4-(4-pyridylmethoxy)phenyl]thiazol-2-yl]methylcarbamoyl]indan-2-yl]acetic acid; 2-[2-[[4-[2-methoxy-4-(2-morpholinoethoxy)phenyl]thiazol-2-yl]methylcarbamoyl]indan-2-yl]acetic acid; 2-[2-[[4-[2-methoxy-4-(3-pyridylmethoxy)phenyl]thiazol-2-yl]methylcarbamoyl]indan-2-yl]acetic acid; 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid; 2-[2-[[4-[4-[2-(4-acetylpiperazin-1-yl)ethoxy]-2-methoxy-phenyl]thiazol-2-yl]methylcarbamoyl]indan-2-yl]acetic acid; 2-[2-[[4-(1,5-dimethylpyrazol-4-yl)thiazol-2-yl]methylcarbamoyl]indan-2-yl]acetic acid; 2-[2-[[4-[3-[[4-(dimethylamino)-1-piperidyl]sulfonyl]-4-methoxy-phenyl]thiazol-2-yl]methylcarbamoyl]indan-2-yl]acetic acid; 2-[2-[[4-[3-[[4-(dimethylamino)- 1-piperidyl]sulfonyl]-4-methoxy-phenyl]thiazol-2-yl]methylcarbamoyl]-5,6-difluoro-indan-2-yl]acetic acid; 2-[2-[[4-[3-(4-methylpiperazin-1-yl)sulfonylphenyl]thiazol-2-yl]methylcarbamoyl]indan-2-yl]acetic acid; 2-[2-[[4-[3-(4-methylpiperazin-1-yl)sulfonyl-4-(2-morpholinoethoxy)phenyl]thiazol-2-yl]methylcarbamoyl]indan-2-yl]acetic acid; 2-[2-[[4-[3-(4-methylpiperazin-1-yl)sulfonyl-4-(3-morpholinopropoxy)phenyl]thiazol-2-yl]methylcarbamoyl]indan-2-yl]acetic acid; 2-[2-[[4-[3-methylsulfonyl-4-(2-morpholinoethoxy)phenyl]thiazol-2-yl]methylcarbamoyl]indan-2-yl]acetic acid; 2-[2-[[4-[6-(4-pyridylmethoxy)-3-pyridyl]thiazol-2-yl]methylcarbamoyl]indan-2-yl]acetic

acid; 2-[2-[[4-(4-methoxy-3-pyridyl)thiazol-2-yl]methylcarbamoyl]indan-2-yl]acetic acid; 2-[2-(hydroxyamino)-2-oxo-ethyl]-N-[(4-phenylthiazol-2-yl)methyl]indane-2-carboxamide; 2-[2-(hydroxyamino)-2-oxo-ethyl]-N-[[4-[1-(2-morpholinoethyl)pyrazol-4-yl]thiazol-2-yl]methyl]indane-2-carboxamide; and 2-[2-(hydroxyamino)-2-oxo-ethyl]-N-[[4-[1-[2-(4-methylpiperazin-1-yl)-2-oxo-ethyl]pyrazol-4-yl]thiazol-2-yl]methyl]indane-2-carboxamide; or a pharmaceutically acceptable salt thereof.

Most preferred compounds of the invention are selected from: 2-[5,6-difluoro-2-[(4-phenylthiazol-2-yl)methylcarbamoyl]indan-2-yl]acetic acid; 2-[2-[[4-[4-[4-[2-(4-methylpiperazin-1-yl)-2-oxoethyl]piperazin-1-yl]phenyl]thiazol-2-yl]methylcarbamoyl]indan-2-yl]acetic acid; 2-[2-[[4-[2-methoxy-4-(3-morpholinopropoxy)phenyl]thiazol-2-yl]methylcarbamoyl]indan-2-yl]acetic acid; 2-[2-[[4-[2-methoxy-4-(4-pyridylmethoxy)phenyl]thiazol-2-yl]methylcarbamoyl]indan-2-yl]acetic acid; 2-[2-[[4-[2-methoxy-4-(2-morpholinoethoxy)phenyl]thiazol-2-yl]methylcarbamoyl]indan-2-yl]acetic acid; 2-[2-[[4-[2-methoxy-4-(3-pyridylmethoxy)phenyl]thiazol-2-yl]methylcarbamoyl]indan-2-yl]acetic acid; 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid; 2-[2-[[4-[4-[2-(4-acetylpiperazin-1-yl)ethoxy]-2-methoxy-phenyl]thiazol-2-yl]methylcarbamoyl]indan-2-yl]acetic acid; 2-[2-[[4-[3-(4-methylpiperazin-1-yl)sulfonyl-4-(2-morpholinoethoxy)phenyl]thiazol-2-yl]methylcarbamoyl]indan-2-yl]acetic acid; 2-[2-[[4-[3-methylsulfonyl-4-(2-morpholinoethoxy)phenyl]thiazol-2-yl]methylcarbamoyl]indan-2-yl]acetic acid; and 2-[2-[[4-[6-(4-pyridylmethoxy)-3-pyridyl]thiazol-2-yl]methylcarbamoyl]indan-2-yl]acetic acid; or a pharmaceutically acceptable salt thereof.

*Synthesis*

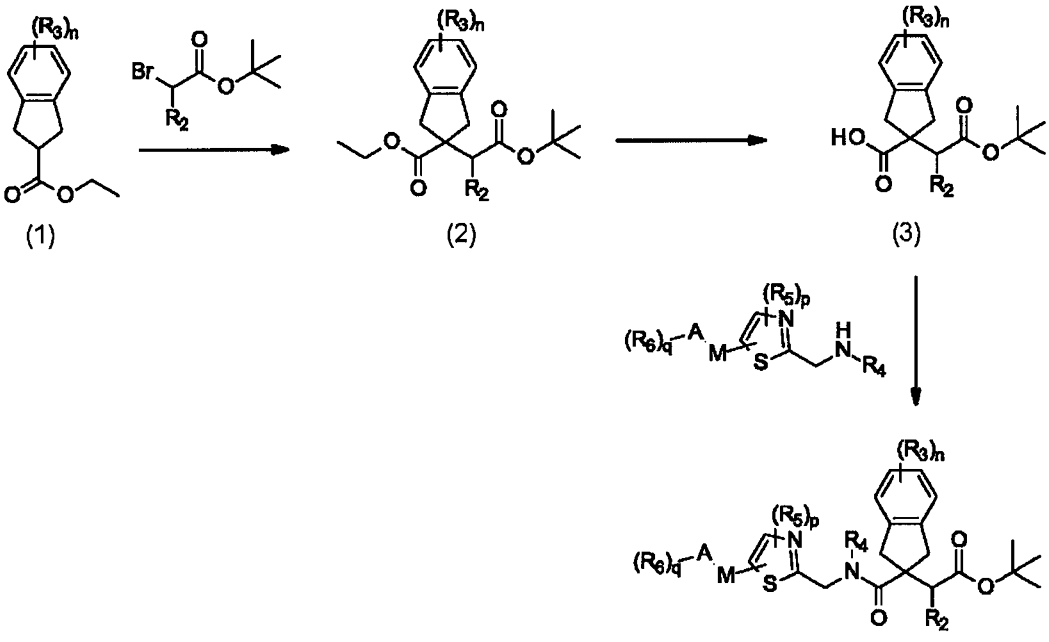

(1) (2) (3) (4)

The compounds of the invention can be prepared by any suitable method. For example, as described in more detail below, deprotonation of commercially available ethyl esters (1) with strong base (such as sodium hexamethyldisilazide) then alkylation of the anion with *tert*-butyl bromoacetates gives diester (2) (Bell, I.M. and Stump, C.A., WO2006/29153; Robinson, R.P. et al, Bioorganic and Medicinal Chemistry Letters, 1996, 1719). Basic hydrolysis of the ethyl ester in the presence of the *tert*-butyl ester gives (3). Amide formation with a suitable 2-aminomethyl thiazole (4) followed by treatment with TFA to remove the *tert*-butyl ester then affords the desired acids.

The acids can be converted to esters ($R^1 = OR^{1a}$) or other produrug forms ($R^1 = OCH_2OC(O)R^{1a}$) by techniques known to the skilled person.

Examples of suitable protocols for formation of amino-methyl thiazoles are provided below. For example, amino-methyl thiazole (4) can be synthesized from commercially available starting materials. In a preferred method, bromoketone is commercially available or is synthesized by routine methods from the ketone precursor. Reaction with commercially available thioamide, such as the Boc derivative depicted in the reaction scheme below, yields target amino-methyl

thiazole (4) via a Hantzsch synthesis reaction (Synthesis, 2017, 22, 3535-3541)

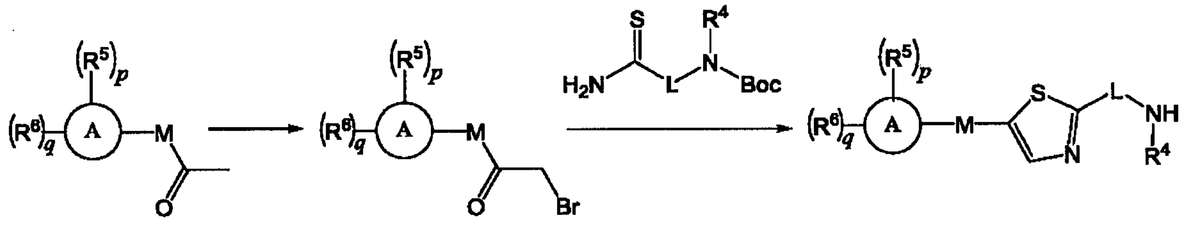

(4)

There are numerous ways of accessing hydroxamic acids (for a review see Ganeshpurkar, A., et al, Current Organic Syntheses, 2018, 15, 154-165) but a very reliable procedure is to couple acids with O-(oxan-2-yl)hydroxylamine using peptide coupling conditions to give protected hydroxamates then deprotect with TFA to generate the hydroxamic acids (see for example Ding, C., et al, Bioorg. Med. Chem. Lett, 2017, 25, 27-37).

*Compositions and Combinations*

The present invention also provides a pharmaceutical composition, the pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

The composition of the invention may be provided as a kit comprising instructions to enable the kit to be used in the methods described herein or details regarding which subjects the method may be used for.

As explained above, the compounds of the invention are useful in treating or preventing bacterial infection. In particular, they are useful as inhibitors of LasB, in particular LasB of *Pseudomonas aeruginosa* (PA). The compounds may be used alone or they may be used in combination therapies with antibiotic agents, to enhance the action of the antibiotic agent.

The present invention therefore also provides a combination comprising (i) a compound of the invention as described herein and (ii) an antibiotic agent. The combination may further comprise one or more additional active agents. The compound of the invention and the antibiotic agent may be provided in a single formulation, or they may be separately formulated. Where separately formulated, the two agents may be administered simultaneously or separately. They may be provided in the form of a kit, optionally together with instructions for their administration.

Where formulated together, the two active agents may be provided as a pharmaceutical composition comprising (i) a compound of the invention as described herein and (ii) a further antibacterial compound; and (iii) a pharmaceutically acceptable carrier or diluent.

Preferably, the antibiotic agent is efficacious against *Pseudomonas* infection. Most preferably, the antibiotic is tobramycin, neomycin, streptomycin, gentamycin, ceftazidime, ticarcillin, piperacillin, tazobactam, imipenem, meropenem, rifampicin, ciprofloxacin, amikacin, colistin, aztreonam or levofloxacin.

The compound or combination of the invention may be administered in a variety of dosage forms. Thus, they can be administered orally, for example as tablets, troches, lozenges, aqueous or oily suspensions, dispersible powders or granules. They may also be administered parenterally, whether subcutaneously, intravenously, intramuscularly, intrasternally, transdermally or by infusion techniques. The compound or combination may also be administered as a suppository. Preferably, the compound or combination may be administered via inhaled (aerosolised) or intravenous administration, most preferably by inhaled (aerosolised) administration.

The compound or combination of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

The compound or combination of the invention may be formulated for inhaled (aerosolised) administration as

a solution or suspension. The compound or combination of the invention may be administered by a metered dose inhaler (MDI) or a nebulizer such as an electronic or jet nebulizer. Alternatively, the compound or combination of the invention may be formulated for inhaled administration as a powdered drug, such formulations may be administered from a dry powder inhaler (DPI). When formulated for inhaled administration, the compound or combination of the invention may be delivered in the form of particles which have a mass median aerodynamic diameter (MMAD) of from 1 to 100 $\mu$m, preferably from 1 to 50 $\mu$m, more preferably from 1 to 20 $\mu$m such as from 3 to 10 $\mu$m, e.g. from 4 to 6 $\mu$m. When the compound or combination of the invention is delivered as a nebulized aerosol, the reference to particle diameters defines the MMAD of the droplets of the aerosol. The MMAD can be measured by any suitable technique such as laser diffraction.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

*Therapeutic Efficacy*

The compounds, compositions and combinations of the present invention are therapeutically useful. The present invention therefore provides compounds, compositions and combinations as described herein, for use in medicine. The present invention provides compounds as described herein, for use in treating the human or animal body. For the avoidance of doubt, the agent may comprise a compound of the invention in the form of a solvate.

The compounds, compositions and combinations of the invention are useful in treating or preventing bacterial infection. The present invention therefore provides a compound, combination or composition as described herein for use in a method of treating or preventing bacterial infection in a subject in need thereof. Also provided is a method for treating or preventing bacterial infection in a subject in need thereof, which method comprises administering to said subject an effective amount of a compound, combination or composition as described herein. Further provided is the use of a compound, combination or composition as described herein in the manufacture of a medicament for use in treating or preventing bacterial infection in a subject.

The compounds described herein are useful as inhibitors of LasB, in particular LasB of *Pseudomonas aeruginosa* (PA). The inhibition of LasB in the bacteria prevents LasB secreted by bacteria from hydrolysing host tissue and host immune-response proteins, thereby supporting the subject in its natural response to bacterial infection and inflammation. The compounds described herein are therefore useful as standalone adjuncts in antibacterial therapy, for example in chemotherapy regimes. Further, the compounds are useful in inhibiting biofilm formation, and/or in disrupting a biofilm. This activity in preventing biofilm formation or disrupting established biofilms facilitates antibiotic agents in eradication of bacterial infection. It also facilitates the host's own immune system in attacking the bacterial infection. The compounds may therefore be used as stand alone antibacterial agents.

Alternatively, the compounds described herein may be used in combination with antibiotic agents to enhance the action of the antibiotic agent. Therefore, further provided is a compound of the invention as described herein for use in a method of treating or preventing bacterial infection by co-administration with an antibiotic agent. Also provided is a method for treating or preventing bacterial infection in a subject in need thereof, which method comprises co-administering to said subject an effective amount of a compound as described herein and an antibiotic agent. Also provided is the use of a compound as described herein in the manufacture of a medicament for use in treating or preventing bacterial infection by co-administration with an antibiotic agent.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

The compounds, compositions and combinations described herein are useful in the treatment of bacterial infection which occurs after a relapse following an antibiotic treatment. The compounds and combinations can therefore be used in the treatment of a patient who has previously received antibiotic treatment for the (same episode of) bacterial infection.

The bacterium causing the infection may be any bacterium expressing LasB or an analogue thereof. Typically the bacterium causing the infection expresses LasB. The bacterium may, for instance, be any bacterium that can form a biofilm. The bacterium may be Gram-positive or Gram-negative. In a preferred instance the bacterium is Gram-negative. The bacterium may in particular be a pathogenic bacterium.

The bacterial infection may be caused by *Bacillus, Pseudomonas, Staphylococcus, Streptococcus, Listeria, Escherichia* or *Burkholderia.* For example, the bacterium may be one selected from *Staphylococcus aureus, Haemophilus influenza, Pseudomonas aeruginosa* and *Burkholderia cepacia.*

In one preferred instance, the bacterium may be one selected from a bacterium of the family Pseudomonadaceae. For example, the bacterium may be selected from one of the following genera: *Pseudomonas, Azomonas, Azomonotrichon, Azorhizophilus, Azotobacter, Cellvibrio, Mesophilobacter, Rhizobacter, Rugamonas* and *Serpens.* Preferably the bacterium is a *Pseudomonas,* particularly where the condition to be treated is pneumonia. The bacterium may be an opportunistic pathogen. The bacterium may be selected from *Pseudomonas aeruginosa, Pseudomonas oryzihabitans,* and *Pseudomonas plecoglossicida,* and most preferably, the bacterium is *Pseudomonas aeruginosa* (PA).

The compound, composition or combination of the invention may be used to treat or prevent infections and conditions caused by any one or a combination of the above-mentioned bacteria. In particular, the compound or combination of the invention may be used in the treatment or prevention of pneumonia. The compound or combination may also be used in the treatment of septic shock, urinary tract infection, and infections of the gastrointestinal tract, skin or soft tissue.

The compounds and combinations are particularly useful in the treatment of patients suffering from cystic fibrosis. Preferably, the compound or combination of the invention may be used in the treatment or prevention of pneumonia in a subject suffering from cystic fibrosis. For example, the subject may have any of the six CFTR mutation classes, and/or may be infected by or chronically colonised by PA. The compounds and combinations of the invention may also be used in the treatment of neutropenic patients.

A compound or combination of the invention can be administered to the subject in order to prevent the onset or reoccurrence of one or more symptoms of the bacterial infection. This is prophylaxis. In this embodiment, the subject can be asymptomatic. The subject is typically one that has been exposed to the bacterium. A prophylactically effective amount of the agent or formulation is administered to such a subject. A prophylactically effective amount is an amount which prevents the onset of one or more symptoms of the bacterial infection.

A compound or combination of the invention can be administered to the subject in order to treat one or more symptoms of the bacterial infection. In this embodiment, the subject is typically symptomatic. A therapeutically effective amount of the agent or formulation is administered to such a subject. A therapeutically effective amount is an amount effective to ameliorate one or more symptoms of the disorder.

A therapeutically or prophylactically effective amount of the compound of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 5 mg to 2 g.

*Other Uses*

The antibacterial properties of the compounds described herein mean that they are also useful in the treatment of bacterial infection *in vitro,* i.e. other than by the treatment of human or animal subjects. Thus, also described herein is a cleaning composition comprising a indane derivative of Formula (I) or a salt thereof. The cleaning composition may further comprise, for example, a detergent, a surfactant (including ionic and non-ionic surfactants), a diluent, a bleach (including a hypochlorite such as sodium hypochlorite or calcium hypochlorite, chlorine, chlorine dioxide, hydrogen peroxide or an adduct thereof, sodium perborate, and sodium percarbonate), an alcohol (such as ethanol or isopropanol), or a disinfectant. Typically, the disinfectant may be selected from benzyl-4-chlorophenol, amylphenol, phenylphenol, glutaraldehyde, alkyl dimethyl benzyl ammonium chloride, alkyl dimethyl ethylbenzyl ammonium chloride, iodine, peracetic acid and chlorine dioxide. Typically, the detergent may be an alkaline detergent such as sodium hydroxide, sodium metasilicate, or sodium carbonate, or an acid detergent such as hydrochloric acid, nitric acid, sulfuric acid, phosphoric acid, citric acid, or tartaric acid.

Also described herein is the use of the indane derivative of Formula (I) as described herein for the prevention or treatment of bacterial contamination *in vitro.* Such use may be an *in vitro* method for the prevention or treatment of bacterial infection which comprises a step of treatment of an object with a compound or combination of the invention. Such use is a non-therapeutic use and may involve, for example, prevention or treatment of bacterial contamination on a surface, such as a surface of an indwelling medical device, or an object used in a clinical setting. The surface may be the surface of a catheter, a nebulizer, a ventilator, or a face mask. Typically, the bacterial contamination is caused by any bacteria described herein. Preferably, the bacteria is *Pseudomonas aeruginosa.*

The following Examples illustrate the invention. They do not however, limit the invention in any way. In this

regard, it is important to understand that the particular assay used in the Examples section is designed only to provide an indication of biological activity. There are many assays available to determine biological activity, and a negative result in any one particular assay is therefore not determinative.

## Experimental Details

*General synthetic methodology*

As described below, there are two synthetic methodologies to synthesize the compounds of the invention.

Method A. Regiospecific synthesis of key intermediate (3)

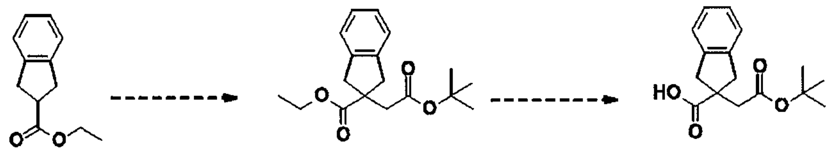

(1) (2) (3)

Scheme 1

Deprotonation of commercially available ethyl ester (1) with strong base (such as sodium hexamethyldisilazide) then alkylation of the anion with *tert*-butyl bromoacetate gives known diester (2) (Bell, I.M. and Stump, C.A., WO2006/29153; Robinson, R.P. et al, Bioorganic and Medicinal Chemistry Letters, 1996, 1719). Basic hydrolysis of the ethyl ester in the presence of the *tert*-butyl ester gives (3) where R = *tert*-butyl. Amide formation with a suitable 2-aminomethyl benzothiazole followed by treatment with TFA to remove the *tert*-butyl ester then affords the desired acids.

This methodology can be adapted to substituents on the indane ring.

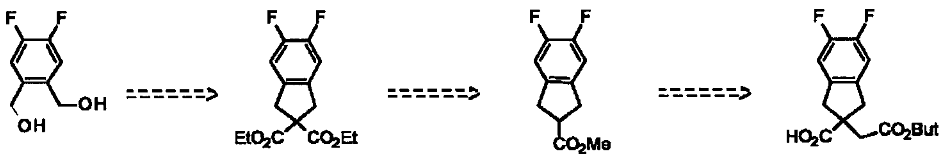

(4) (5) (6) (7)

Scheme 2

For example commercially available diol [4,5- difluoro- 2- (hydroxymethyl)phenyl]methanol (4) can be converted into the bis bromomethyl analogue with either HBr (WO2008/151211) or phosphorus tribromide (US2006/223830) which can further be reacted with diethyl malonate to give indane (5), (Scheme 2). Standard hydrolysis of both esters followed by mono decarboxylation affords the mono acid (WO2006/125511) which can be esterified to give (6), the difluoro analogue of (1). Using the same methodology as applied to (1) then affords key acid (7), the difluoro analogue of intermediate (3). Similar chemistry can be applied to the corresponding dichloro analogues.

Method B. Synthesis of phenylthiazole analogues (14) from bromomethyl ketone (9)

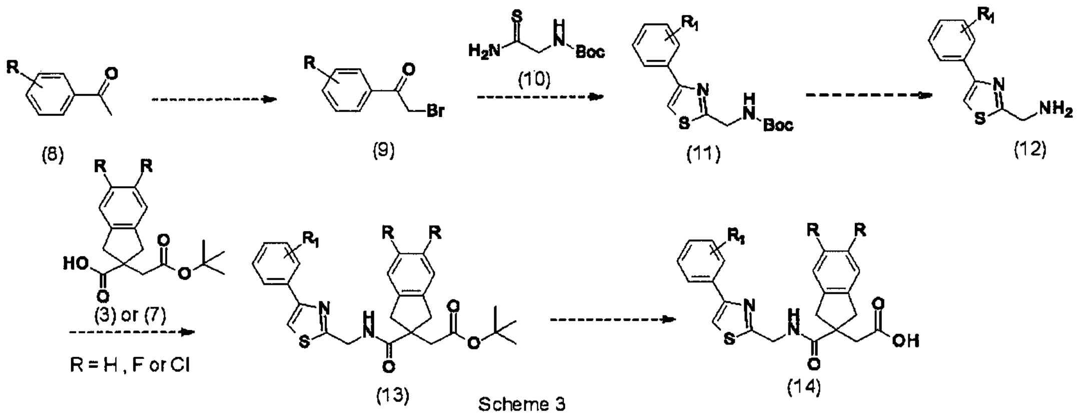


Scheme 3

Treatment of commercial bromomethyl ketone (9) with commercial Boc-protected thioamide glycine derivative (10) followed by Boc deprotection affords primary amine (12) in a Hantzsch thiazole synthesis (Merritt, E., et al, Synthesis, 2017, 22, 3535-3541). Amide formation and treatment with TFA to remove the tert-butyl ester then affords the desired acid (14).

Method B-1. Coupling reaction to access amide-substituted analogues (21)

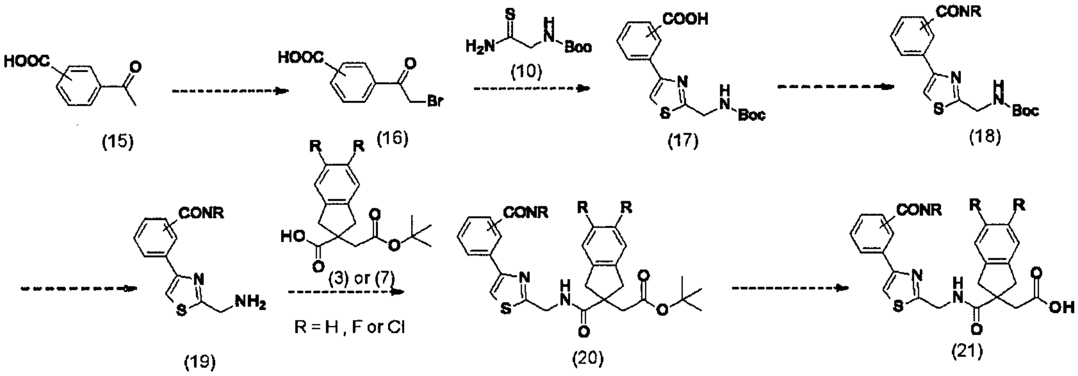


Scheme 4

Method B applied to 3- or 4-(2-bromoacetyl)benzoic acid (15) afforded corresponding carboxylic acids (17), which are coupled in peptidic coupling conditions to primary or secondary amines to give (18). Final compounds (21) are then obtained following Method B.

Method B-2. Coupling reaction to access amino-substituted analogues (28)

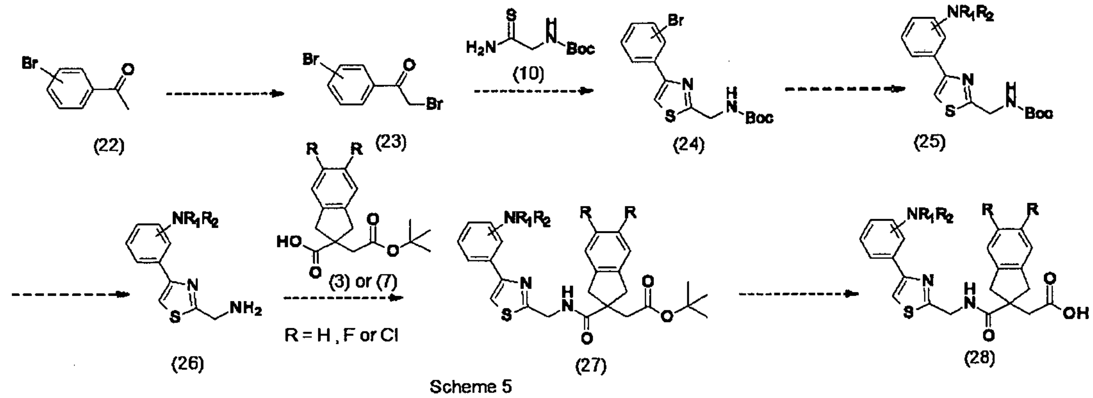


Scheme 5

Method B applied to 2,3'-dibromoacetophenone or 2-4'-dibromoacetophenone (22) affords corresponding brominated derivatives (24), which are coupled using palladium catalysed Buchwald conditions (Buchwald, S.L., Chem. Rev. 2016, 116, 12564-12649) to primary or secondary amines to give (25). Final compounds (28) are then obtained following Method B.

Method B-3. Coupling reaction to access reversed amide-substituted analogues (35)

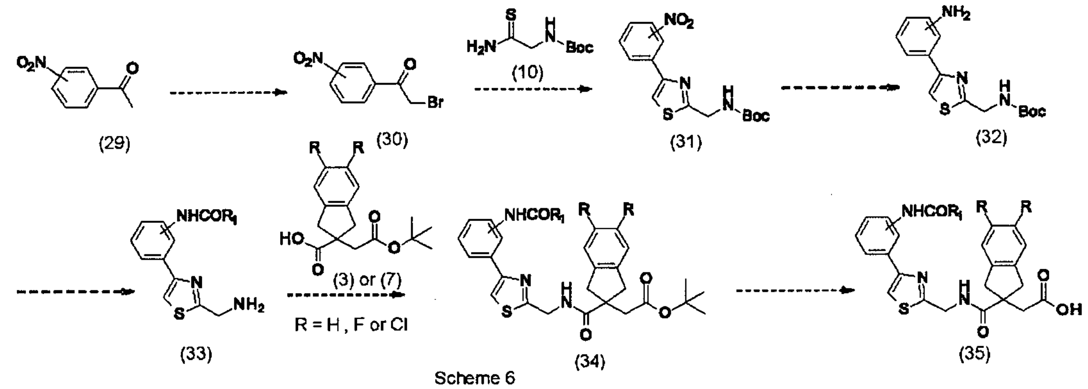


Scheme 6

Method B applied to acetophenones with a nitro substituent (29) affords the corresponding phenacyl bromides (30) then nitroarylthiazoles (31). Reduction of nitro gives anilines (32) (for a recent review of nitro reduction to amines see Orlandi, M et al, Organic Process Research and Development, 2018, 22, 430-445) then standard amide formation provides amides (23). Completion of the syntheses via (33) and (34) to final compounds (35) is achieved using the processes described in Method B.

Method B-4. Coupling reaction to access O-substituted analogues (41)

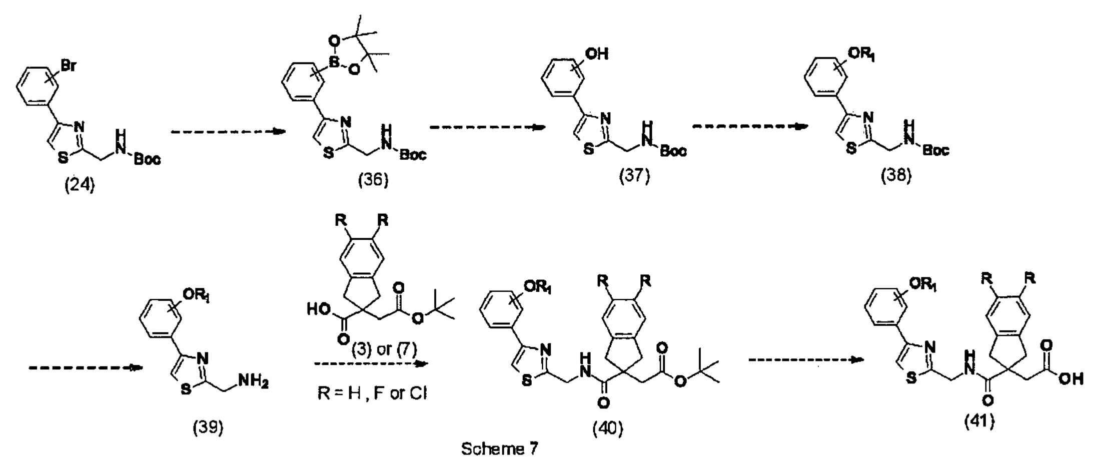

(24) (36) (37) (38)
(39) (40) (41)

Scheme 7

Bromophenyl thiazoles (24) are prepared by the standard Hantzsch synthesis then the bromo substituent is replaced by boron to give (36) in a palladium-catalysed Miyaura reaction (for a review, see Molander, G.A, JACS, 2012, 134, 11667-11673). Oxidation with hydrogen peroxide (Cao, S. et al, Chemistry - A European Journal, 2013, 19, 9050-9058) then reveals phenols (37). Derivatization of the phenols using standard methodology generates (38), which is followed by the chemistry of Method B to give (39) and finally (40).

Method C. Synthesis of phenylthiazole analogues (48) from bromothiazole intermediate (42) through coupling with boronate esters (39) or corresponding boronic acids (44)

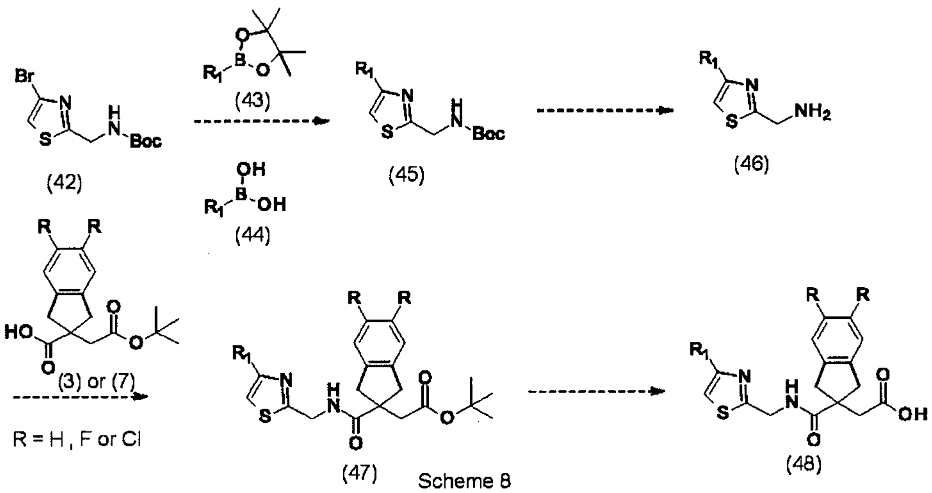

(42) (43) (44) (45) (46)
(47) (48)

Scheme 8

Coupling of commercially-available bromothiazole (42) with boronate ester (43) or acids (44) is achieved through Suzuki chemistry (Suzuki, A., Chem. Rev., 1995, 95, 2457-2483) to give biaryl analogues (46) which can be transformed into compounds (48) using the chemistry of Method B.

Method D. Synthesis of phenylthiazole analogues (56) from benzyl protected glycine thioamide intermediate (50).

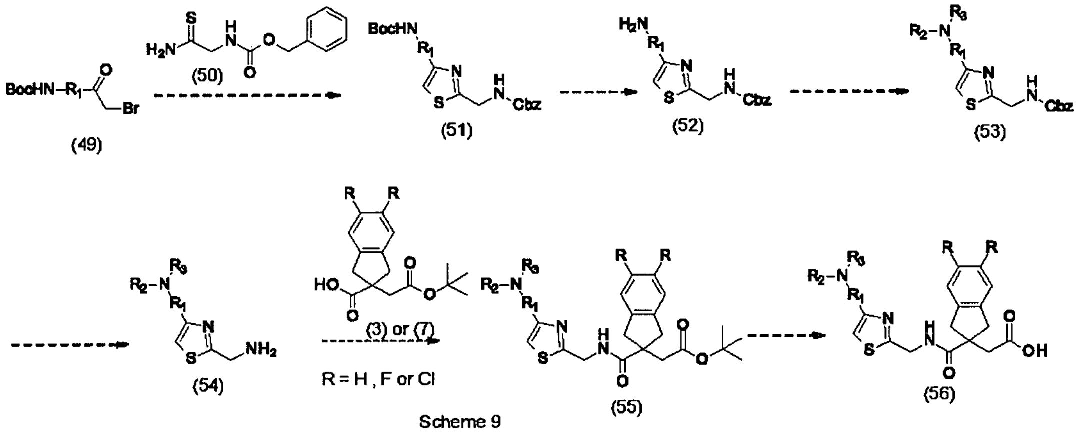


Scheme 9

This alternative protecting group strategy is preferably used when it is desired to introduce a protected amine at the thiazole stage such as in (51). Deprotection of this amine by removal of the t-butyloxycarbonyl (BOC) group gives (52) and further derivatisation of (52) gives (53). In cases where derivatisation of (52) gives a secondary amine (eg R2 = alkyl) then reprotection is typically performed (R3 = BOC). The benzyloxycarbonyl group offers orthogonal protection so removal by hydrogenation reveals amine (54) then the processes of Method B give (55) and (56), where if a BOC group is present it is removed concomitantly with the t-butyl ester.

Method E. Synthesis of phenylthiazole analogues (63) from other amino-protected intermediate (58)

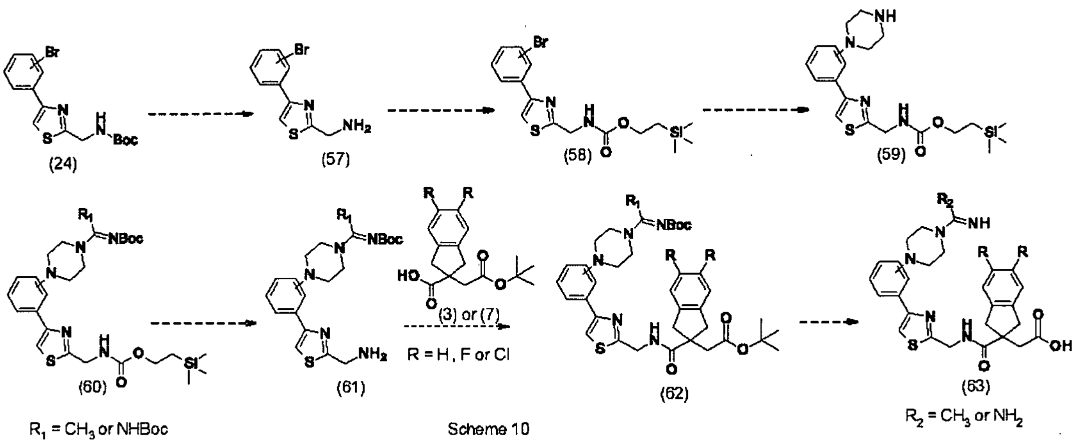


$R_1 = CH_3$ or NHBoc

Scheme 10

$R_2 = CH_3$ or $NH_2$

Method E is a variation of Method D and addresses the same issues of orthogonal protection. In this case the (2-trimethylsilyl)ethoxycarbonyloxy protecting group (Teoc group; Carpino, L.A., et al, JCS Chemical Commun., 1978, 358). is used in place of the benzyloxycarbonyl protecting group. Buchwald reaction on (58) affords unprotected piperazine (59) which is further functionalised as BOC-protected amidine or guanidine groups as in (60). Removal of the Teoc group is accomplished with fluoride anion then the remainder of he chemistry follows the processes in Method B, agan with concomitant removal by TFA of both the BOC group and the t-butyl ester.

Method F. Synthesis of hydroxamic acid analogues (66)

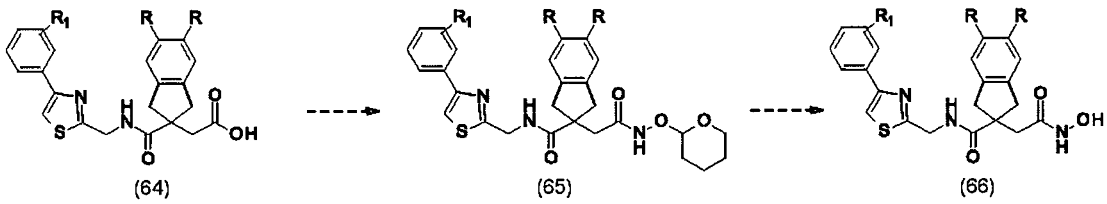

(64) (65) (66)

R = H , F or Cl

Scheme 11

There are numerous ways of accessing hydroxamic acids (for a review see Ganeshpurkar, A., et al, Current Organic Syntheses, 2018, 15, 154-165) but a very reliable procedure is to couple acids (64) with O- (oxan- 2- yl)hydroxylamine using peptide coupling conditions to give protected hydroxamates (65) then deprotect with TFA to generate the hydroxamic acids (66), (see for example Ding, C., et al, Bioorg. Med. Chem. Lett, 2017, 25, 27-37).

## Examples

1H NMR spectra are reported at 300, 400 or 500 MHz in DMSO-d6 solutions (δ in ppm), using DMSO-$d_5$ as reference standard (2.50 ppm), or $CDCl_3$ solutions using chloroform as the reference standard (7.26 ppm). When peak multiplicities are reported, the following abbreviations are used: s (singlet), d (doublet), t (triplet), m (multiplet), bs (broadened singlet), bd (broadened doublet), dd (doublet of doublets), dt (doublet of triplets), q (quartet). Coupling constants, when given, are reported in hertz (Hz).

The term "purified by prep hplc (MDAP)" refers compound purification using a mass-directed auto purification system on an Agilent 1260 infinity machine with an XSelect CHS Prep C18 column, eluting with 0.1% FA in water/ACN and detection with a Quadrupole LC/MS.

*Abbreviations*

| | |
|---|---|
| ACN | Acetonitrile |
| AcOH | Acetic acid |
| AIBN | Azobisisobutyronitrile |
| aq. | Aqueous |
| Bpin | Bis(pinacolato)diboron |
| $CaCl_2$ | Calcium chloride |
| $Cs_2CO_3$ | Cesium carbonate |
| cfu | Colony forming unit |
| COMU | (1-Cyano-2-ethoxy-2-oxoethylidenaminooxy)dimethylamino-morpholino-carbenium hexafluorophosphate |
| $Cu(OAc)_2$ | Copper(II) acetate |
| CuO | Copper oxide |
| DCM | Dichloromethane |
| DEA | Diethylamine |
| DIAD | Diisopropyl azodicarboxylate |
| DIBAL-H | Diisobutylaluminium hydride |
| DIPEA | N,N-Diisopropylethylamine |
| DMAP | 4-Dimethylaminopyridine |
| DMF | N,N-Dimethylformamide |
| DMSO | Dimethyl sulfoxide |
| dppf | 1,1'-Bis(diphenylphosphino)ferrocene |
| EDC.HCl | N-(3-Dimethylaminopropyl)-N'-ethylcarbodiimide hydrochloride |
| $Et_2O$ | Diethyl ether |
| EtOAc | Ethyl acetate |
| EtOH | Ethanol |
| $Et_3N$ | Triethylamine |
| Ex | Excitation |

| | |
|---|---|
| FA | Formic acid |
| FCC | Flash column chromatography purification on silica |
| h | Hour(s) |
| $H_2$ | Hydrogen |
| HATU | 1-[Bis(dimethylamino)methylene]-1H-1,2,3-triazolo[4,5-b]pyridmium 3-oxid hexafluorophosphate |
| HCl | Hydrochloric acid/hydrochloride salt |
| HOBt | Hydroxybenzotriazole |
| $H_2SO_4$ | Sulfuric Acid |
| IPA | *Iso*-propyl alcohol |
| Km | Michaelis constant |
| KOH | Potassium hydroxide |
| LiAlH4 | Lithium aluminium hydride |
| MeCN | Acetonitrile |
| MeI | Methyl iodide |
| MeOH | Methanol |
| min | Minute(s) |
| $MgSO_4$ | Magnesium sulfate |
| $N_2$ | Nitrogen |
| NBS | N-bromo succinimide |
| $Na_2CO_3$ | Sodium carbonate |
| $NaHCO_3$ | Sodium bicarbonate |
| $Na_2SO_4$ | Sodium sulfate |
| $Pd_2(dba)_3$ | Tris(dibenzylideneacetone)dipalladium(0) |
| $PdCl_2(PPh_3)_2$ | Bis(triphenylphosphine)palladium(II) dichloride |
| $PdCl_2$(dppf) | [1,1'-Bis(diphenylphosphino)ferrocene]dichloropalladium(II) |
| RT | Room temperature |
| SCX-2 | Strong cation exchange resin (silica-propyl sulfonic acid) |
| T%B | Time, % solvent B |
| TES | Triethylsilane |
| TMSOTf | Trimethylsilyl trifluoromethanesulfonate |
| TFA | Trifluoroacetic acid |
| THF | Tetrahydrofuran |
| $T_3P$ | Propylphosphinic anhydride |

The chemical formula below depicts the structure of (RuPhos) Palladium (II) phenethylamine chloride (1:1 MTBE adduct) used for Buchwald coupling steps (RuPhos Pd G1 complex).

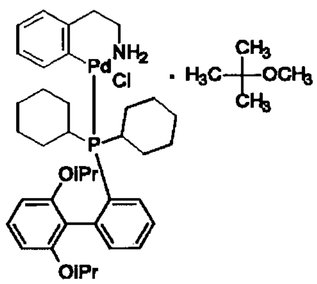

**Example 1**

**2-[2-[(4-phenylthiazol-2-yl)methylcarbamoyl]indan-2-yl]acetic acid**

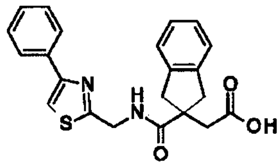

Method B was used as described above.

a. Tert-butyl N-[(4-phenylthiazol-2-yl)methyl]carbamate

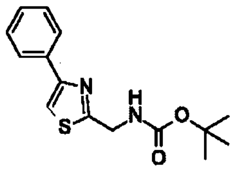

A solution of 2-bromoacetophenone (1.99 g, 10 mmol) and tert-butyl (2-amino 2-thioxoethyl)carbamate (1.90 g, 10 mmol) in EtOH (50 mL) was stirred at room temperature for 16 h. The solution was evaporated and the resulting residue diluted in water (25 mL) and extracted with EtOAc (2 x 25 mL). The combined extracts were dried and evaporated affording a pale yellow solid. Recrystallisation from MeCN (13 mL) led to colourless solid (2.03 g, 70%). M/z 235 (M+H-tBu)$^+$.

b. (4-phenylthiazol-2-yl)methanamine trifluoroacetate

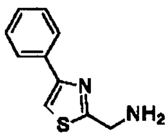

TFA (5 mL) was added to a solution of tert-butyl N-[(4-phenylthiazol-2-yl)methyl]carbamate (2.03 g, 6.99 mmol) in DCM (10 mL) at room temperature. The resulting solution was heated to reflux for 1 h. The cooled solution was evaporated, redissolved in MeOH (5 mL) and applied to SCX-2 cartridge (20 g). The cartridge was washed with MeOH (50 mL) then the product eluted with 2M $NH_3$ in MeOH (50 mL). The solution was evaporated affording a white solid (1.33 g, 100%). M/z 174 (M+H-$NH_3$)$^+$.

c. Methyl indane-2-carboxylate

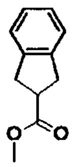

To a stirred solution of 2,3-dihydro-1H-indene-2-carboxylic acid (20 g, 123 mmol) in methanol (200 mL) was added con. $H_2SO_4$ (10 mL, 185 mmol) drop wise at room temperature and stirred at 80 °C for 16 h. The reaction mixture was evaporated to get residue. The residue was dissolved in water (100 mL) and extracted with EtOAc (2x100 mL). The organic layer was washed with sat. sodium bicarbonate, brine and evaporated affording a light brown liquid (20 g, 92%). M/z 177.1 (M+H)$^+$.

d. Methyl 2-(2-tert-butoxy-2-oxo-ethyl)indane-2-carboxylate

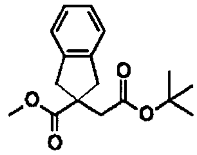

To a solution of methyl 2,3-dihydro-1H-indene-2-carboxylate (5 g, 28.3 mmol) in THF (100 mL) was added NaHMDS (21 mL, 42.5 mmol, 2M in THF) at -78 °C under argon and stirred at -78 °C for 1 h. Then tert-butyl 2-bromoacetate solution (6.4 mL, 42.5 mmol) in THF (30 mL) was added drop wise for 15 minutes at -78 °C and stirred at same temperature for 2 h. The reaction mixture was quenched with sat. ammonium chloride solution (50 mL) at -78 °C and allowed to stir at room temperature for 30 minutes. The organic layer was separated, aqueous layer was extracted with EtOAc (2x100 mL), and the combined organic layer was evaporated to get crude compound. The crude compound was triturated with n-pentane (50 mL) at -78°C and stirred at same temperature for 15 minutes. The resulting solid was filtered and dried under vacuum affording an off white (3.7 g, 45%). M/z = 313.0 $(M+Na)^+$.

e. 2-(2-tert-butoxy-2-oxo-ethyl)indane-2-carboxylic acid

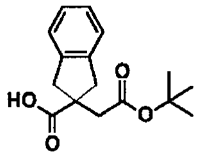

To a stirred solution of methyl 2-(2-(tert-butoxy)-2-oxoethyl)-2,3-dihydro-1H-indene-2-carboxylate (430 g, 1.48 mol) in THF (2.15 L) and ethanol (2.15 L) was added 0.5 M $LiOH.H_2O$ (6.8 L, 2.96 mol) drop wise at room temperature and stirred at same temperature for 2 h. The reaction mixture was evaporated and the residue was diluted with $H_2O$ (1 L) and extracted with diethyl ether. The aqueous layer was acidified with 1N HCl to pH 3-4. The resulting precipitate was filtered, washed with water, n-pentane and dried under vacuum affording a white solid (254.5 g, 62%). M/z 275.2 $(M-H)^-$. $^1H$ NMR (300 MHz, DMSO-$d_6$): $\delta$ 12.4 (1H, bs), 7.18-7.10 (4H, m), 3.39 (2H, d, J = 16.2 Hz), 2.92 (2H, d, J = 16.2 Hz), 2.64 (2H, s), 1.37 (9H, s).

f. Tert-butyl 2-[2-[(4-phenylthiazol-2-yl)methylcarbamoyl]indan-2-yl]acetate

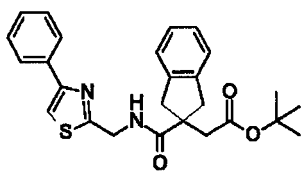

HATU (160 mg, 0.420 mmol) and DIPEA (0.18 mL, 1.05 mmol) were added to a solution of (4-phenylthiazol-2-yl)methanamine (83 mg, 0.438 mmol) and 2-(2-(tert-butoxy)-2-oxoethyl)-2,3-dihydro-1H-indene-2-carboxylic acid (97 mg, 0.35 mmol) in DMF (2 mL) at room temperature. The resulting mixture was stirred for 2 h, diluted with water (3 mL) and saturated aqueous $NaHCO_3$ solution (3 mL) and extracted with DCM (6 mL). The combined extracts were dried and evaporated. The resulting pale yellow oil was chromatographed on silica eluting with 5-30% EtOAc in hexane affording a white solid (75 mg, 48%). M/z 449 $(M+H)^+$.

g. 2-[2-[(4-phenylthiazol-2-yl)methylcarbamoyl]indan-2-yl]acetic acid

TFA (1 mL) was added to a solution of *tert*-butyl 2-[2-[(4-phenylthiazol-2-yl)methylcarbamoyl]indan-2-yl]acetate

(215 mg, 0.479 mmol) in DCM (2 mL) at room temperature. The reaction mixture was stitrred at room temperature for 2.5 h, cooled, evaporated and azeotroped with toluene (2 x 5 mL). The residue was purified by preparative hplc (MDAP) affording the title product as a white solid (137 mg, 73%). M/z 393.2 $(M+H)^+$. $^1H$ NMR (d6-DMSO) δ 12.1 (1H, bs), 8.71 (1H, bs), 7.99 (3H, m), 7.49-7.11 (7H, m), 4.60 (2H, d, *J* = 6 Hz), 3.48 (2H, d, *J* = 16 Hz), 2.98 (2H, d, *J* = 16 Hz), 2.72 (2H, s).

**Example 2**

**2-[2-[(5-phenylthiazol-2-yl)methylcarbamoyl]indan-2-yl]acetic acid**

Method B was used as described above.

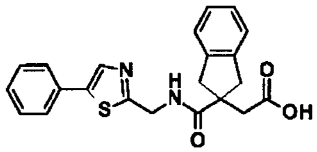

The title product was prepared following the procedure described in example 1 (step-c and -d) using 2-(aminomethyl)-5-phenylthiazole as amine in step-c. M/z 393.3 $(M+H)^+$. $^1H$ NMR (d6-DMSO) δ 12.13 (1H, bs), 8.67 (1H, s), 8.03 (1H, s), 7.59 (2H, m), 7.42 (2H, m), 7.35 (1H, m), 7.20 (2H, m), 7.12 (2H, m), 4.52 (2H, d, *J* = 6 Hz), 3.45 (2H, d, *J* = 16 Hz), 2.99 (2H, d, *J* = 16 Hz), 2.71 (2H, s).

**Example 3**

**2-[5,6-difluoro-2-[(4-phenylthiazol-2-yl)methylcarbamoyl]indan-2-yl]acetic acid**

Method B was used as described above.

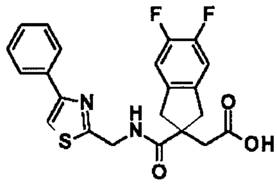

a. Dimethyl 4,5-difluorophthalate

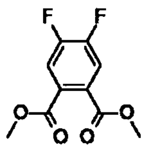

To an ice-cooled solution of 4,5-difluorophthalic acid (11.9 g, 58.9 mmol) in MeOH (250 mL) was added concentrated $H_2SO_4$ (40 mL, 0.75 mol) keeping the temperature <20 °C. The mixture was stirred at 65 °C for 4 h. The cooled reaction mixture was concentrated in vacuo, then the residue was cautiously added to EtOAc and aq. $NaHCO_3$. The aq. phase was extracted with EtOAc and the combined organic extracts were washed with aq. $NaHCO_3$, then brine, dried ($Na_2SO_4$), filtered and concentrated in vacuo to yield the title compound as a colourless oil (12.98 g, 96%). $^1H$ NMR ($CDCl_3$) δ 7.56 (2H, t, *J* = 8.7 Hz), 3.91 (6H, s).

b. (4,5-Difluoro-1,2-phenylene)dimethanol

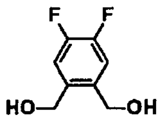

To an ice-cooled solution of lithium aluminium hydride (1M in THF, 226 mL, 0.226 mol) was added a solution of dimethyl 4,5-difluorophthalate (12.98 g, 56.4 mmol) in THF (100 mL) over 30 min keeping the temperature below 12 °C. The mixture was stirred in the ice bath for 30 min, then at RT for 1 h. The reaction mixture was cooled to 0 °C then, cautiously, water (8.5 mL), 15% aq. NaOH (8.5 mL) and water (26 mL) were added successively, keeping the temperature below 15 °C. Celite was added and the mixture stirred at RT for 1 h, then filtered through a celite pad, washing through with more THF. The filtrate was concentrated in vacuo to yield the title compound as a white solid (9.52 g, 97%). $^1$H NMR (d6-DMSO) δ 7.36 (2H, t, *J* = 10.1 Hz), 5.29 (2H, t, *J* = 5.5 Hz), 4.47 (4H, d, *J* = 5.4 Hz).

c. 1,2-Bis(bromomethyl)-4,5-difluorobenzene

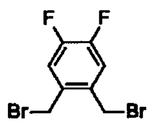

A mixture of (4,5-difluoro-1,2-phenylene)dimethanol (9.52 g, 54.7 mmol) and 48% hydrobromic acid (68.5 mL) was stirred at 110 °C for 1 h. The cooled reaction mixture was diluted with water and then extracted with $Et_2O$. The aq. phase was extracted with $Et_2O$ and the combined organic extracts were washed with water, then brine, dried ($Na_2SO_4$), filtered and concentrated in vacuo to leave a residue. FCC (1-10% EtOAc in hexane) to yield the title compound as a colourless oil (15.2 g, 93%). $^1$H NMR ($CDCl_3$) δ 7.20 (2H, t, *J* = 9.1 Hz), 4.55 (4H, s).

d. Diethyl 5,6-difluoro-1,3-dihydro-2*H*-indene-2,2-dicarboxylate

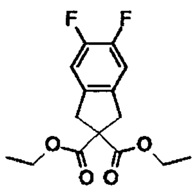

Sodium hydride (60% in oil, 4.46 g, 112 mmol) was added over 15 min to a mixture of 1,2-bis(bromomethyl)-4,5-difluorobenzene (15.2 g, 50.7 mmol) and diethyl malonate (9.74 g, 60.8 mmol) in THF (200 mL) keeping the temperature below 20 °C. The mixture was stirred at RT for 4 h, then saturated aqueous ammonium chloride solution was added. The mixture was concentrated in vacuo and then extracted twice with EtOAc. The combined organic extracts were washed with brine, dried ($Na_2SO_4$), filtered and concentrated in vacuo to leave a residue. FCC (5-25% EtOAc in hexane) yielded the title compound as a colourless oil (9.95 g, 66%). $^1$H NMR ($CDCl_3$) δ 6.97 (2H, t, *J* = 8.7 Hz), 4.21 (4H, q, *J* = 7.1 Hz), 3.52 (4H, s), 1.26 (6H, t, *J* = 7.1 Hz).

e. 5,6-Difluoro-2,3-dihydro-1*H*-indene-2-carboxylic acid

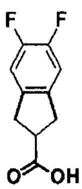

To a solution of diethyl 5,6-difluoro-1,3-dihydro-2*H*-indene-2,2-dicarboxylate (9.94g, 33.3 mmol) in dioxane (130 mL) was added water (130 mL) and concentrated HCl (140 mL). The mixture was refluxed for 23 h. The cooled reaction mixture was diluted with water and extracted with $Et_2O$ (×3). The combined organic extracts were washed with water, then brine, dried ($Na_2SO_4$), filtered and concentrated in vacuo to yield the title compound as a colourless solid (6.6 g, quant.). M/z 197 $(M-H)^-$.

f. Methyl 5,6-difluoro-2,3-dihydro-1*H*-indene-2-carboxylate

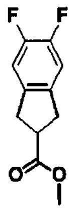

To an ice-cooled solution of 5,6-difluoro-2,3-dihydro-1*H*-indene-2-carboxylic acid (6.6 g, 33.3 mmol) in MeOH (200 mL) was added concentrated $H_2SO_4$ (40 mL, 0.75 mol) keeping the temperature <20 °C. The mixture was stirred at 65 °C for 1 h. The cooled reaction mixture was concentrated in vacuo, then the residue was cautiously added to EtOAc and aq. $NaHCO_3$. The aq. phase was extracted with more EtOAc and the combined organic extracts were washed with brine, dried ($Na_2SO_4$), filtered and concentrated in vacuo to leave a residue. FCC (5-25% EtOAc in hexane) yielded the title compound as a pale yellow solid (5.97 g, 84%). $^1$H NMR ($CDCl_3$) δ 6.98 (2H, t, *J* = 8.8 Hz), 3.73 (3H, s), 3.39 (1H, m), 3.24-3.12 (4H, m).

g. Methyl 2-(2-(*tert*-butoxy)-2-oxoethyl)-5,6-difluoro-2,3-dihydro-1H-indene-2-carboxylate

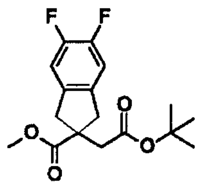

To a solution of methyl 5,6-difluoro-2,3-dihydro-1*H*-indene-2-carboxylate (5.97 g, 28.2 mmol) in THF (120 mL), cooled to -78 °C, was added sodium bis(trimethylsilyl)amide (1M in THF, 42.2 mL, 42.2 mol) over 15 min. The mixture was stirred at -78 °C for 45 min then a solution of *tert*-butyl bromoacetate (8.24 g, 42.2 mmol) in THF (15 mL) was added over 10 min. The reaction mixture was allowed to warm to -10 °C over 1 h. Saturated aqueous ammonium chloride solution was added, the mixture was concentrated under reduced pressure. The residue was extracted twice with EtOAc and the combined organic extracts were washed with brine, dried ($Na_2SO_4$), filtered and concentrated in vacuo to leave a residue. FCC (5-20% EtOAc in hexane) yielded the title compound as a pale yellow gum (8.78 g, 96%). $^1$H NMR ($CDCl_3$) δ 6.96 (2H, t, *J* = 8.9 Hz), 3.72 (3H, s), 3.47 (2H, d, *J* = 16.2 Hz), 2.90 (2H, d, *J* = 16.2 Hz), 2.71 (2H, s), 1.42 (9H, s).

h. 2- [(tert- butoxy)carbonyl]- 5,6- difluoro- 2,3- dihydro- 1H- indene- 2- carboxylic acid

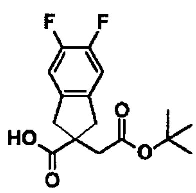

To a solution of methyl 2-(2-(*tert*-butoxy)-2-oxoethyl)-5,6-difluoro-2,3-dihydro-1*H*-indene-2-carboxylate (0.834

g, 2.56 mmol) in THF (25 mL) and MeOH (10 mL) was added lithium hydroxide (0.5M in water, 10.2 mL, 5.1 mmol). The mixture was stirred at RT for 2.5 h, then concentrated in vacuo. The residual solution was layered with EtOAc and acidified by addition of 6M HCl. The aq. phase was extracted with more EtOAc and the combined organic extracts were washed with brine, dried ($Na_2SO_4$), filtered and concentrated in vacuo to leave a residue. FCC (2-6% MeOH in DCM) yielded the title compound as a cream solid (0.59 g, 74%). $^1$H NMR (d6-DMSO) δ 12.47 (1H, bs), 7.26 (2H, t, *J* = 9.2 Hz), 3.33 (2H, d, *J* = 16.4 Hz), 2.91 (2H, d, *J* = 16.4 Hz), 2.67 (2H, s), 1.37 (9H, s). M/z 311 (M-H)$^-$.

i. 2-(2-((benzo[d]thiazol-2-ylmethyl)carbamoyl)-5,6-difluoro-2,3-dihydro-1H-inden-2-yl)acetic acid

The title product was prepared as a white solid following the procedure described in example 1, using 1,3-benzothiazol-2-ylmethanamine and 2-(2-(*tert*-butoxy)-2-oxoethyl)-5,6-difluoro-2,3-dihydro-1*H*-indene-2-carboxylic acid. M/z 425 (M+Na)$^+$. $^1$H NMR (d6-DMSO) δ 12.20 (1H, bs), 8.99 (1H, bs), 8.02 (1H, d, J = 8.0 Hz), 7.92 (1H, d, J = 8.0 Hz), 7.94 (1H, dt, J = 7.7, 1.3 Hz), 7.40 (1H, dt, J = 7.6, 1.1 Hz), 7.28 (2H, t, J = 9.2 Hz), 4.65 (2H, d, J = 5.7 Hz), 3.43 (2H, d, J = 16.4 Hz), 2.99 (2H, d, J = 16.4 Hz), 2.76 (2H, s).

**Example 4**

**2-(2-((benzo[d]thiazol-2-ylmethyl)carbamoyl)-5,6-dichloro-2,3-dihydro-1H-inden-2-yl)acetic acid**

Method B was used as described above.

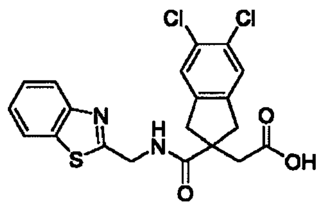

The title product was prepared using the procedures described for example 3, but using 4,5-dichlorophthalic acid. M/z 435.1 (M+H)$^+$. 1H NMR (d6-DMSO) δ 12.66 (1H, br s), 8.04 (1H, d, J = 8.0 Hz), 7.92 (1H, d, J = 8.0 Hz), 7.50 - 7.46 (1H, m), 7.44 (2H, s), 7.42 - 7.38 (1H, m), 4.67 (2H, d, J = 5.7 Hz), 3.39 (2H, d, J = 16.4 Hz), 2.92 (2H, d, J = 16.4 Hz), 2.39 (2H, s).

Other compounds prepared by Method B from commercial bromo-methyl ketone reagents and purified in a similar manner by preparative HPLC are shown in the Table below, wherein R is the moiety:

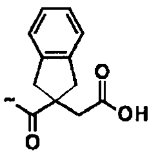

| Example | Structure | Name, MS and NMR data |
|---|---|---|
| **5** | 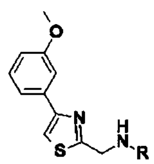 | 2-[2-[[4-(3-methoxyphenyl)thiazol-2-yl]methylcarbamoyl]indan-2-yl]acetic acid<br>M/z 423.3 (M+H)$^+$<br>$^1$H NMR (d6-DMSO) δ 12.13 (1H, bs), 8.63 (1H, bs), 8.00 (1H, s), 7.49 (2H, m), 7.31 (1H, m), 7.20 (1H, m), 7.11 (2H, m), 6.89 (1H, m), 4.54 (2H, d, *J* = 6 Hz), 3.59 (3H, s), 3.45 (2H, d, *J* = 16 Hz), 2.99 (2H, d, *J* = 16 Hz), 2.71 (2H, s). |
| **6** | 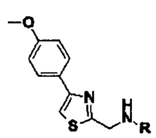 | 2-[2-[[4-(4-methoxyphenyl)thiazol-2-yl]methylcarbamoyl]indan-2-yl]acetic acid<br>M/z 423.3 (M+H)$^+$<br>$^1$H NMR (d6-DMSO) δ 12.11 (1H, bs), 8.65 (1H, bs), 7.73 (2H, m), 7.61 (1H, s), 7.20 (1H, m), 7.11 (2H, m), 6.91 (1H, m), 4.55 (2H, d, *J* = 6 Hz), 3.58 (3H, s), 3.45 (2H, d, *J* = 16 Hz), 3.00 (2H, d, *J* = 16 Hz), 2.73 (2H, s). |

(continued)

| Example | Structure | Name, MS and NMR data |
|---|---|---|
| **7** | 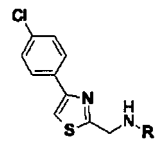 | 2-[2-[[4-(4-chlorophenyl)thiazol-2-yl]methylcarbamoyl]indan-2-yl]acetic acid<br>M/z 427.3 $(M+H)^+$<br>$^1$H NMR (d6-DMSO) $\delta$ 12.12 (1H, bs), 8.71 (1H, bs), 8.03 (1H, s), 7.91 (2H, m), 7.49 (1H, s), 7.20 (1H, m), 7.11 (2H, m), 4.57 (2H, d, *J* = 6 Hz), 3.45 (2H, d, *J* = 16 Hz), 2.98 (2H, d, *J* = 16 Hz), 2.71 (2H, s). |
| **8** | 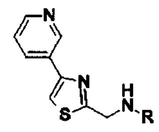 | 2-[2-[[4-(3-pyridyl)thiazol-2-yl]methylcarbamoyl]indan-2-yl]acetic acid<br>M/z 394.4 $(M+H)^+$<br>$^1$H NMR (d6-DMSO) $\delta$ 9.13 (1H, bs), 8.91 (1H, bs), 8.52 (1H, m), 8.25 (1H, m), 8.15 (1H, s), 7.46 (1H, m), 7.20 (1H, m), 7.11 (2H, m), 4.59 (2H, d, *J* = 6 Hz), 3.45 (2H, d, *J* = 16 Hz), 2.97 (2H, d, *J* = 16 Hz), 2.72 (2H, s). |
| **9** | 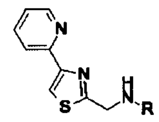 | 2-[2-[[4-(2-pyridyl)thiazol-2-yl]methylcarbamoyl]indan-2-yl]acetic acid<br>M/z 394.4 $(M+H)^+$<br>$^1$H NMR (d6-DMSO) $\delta$ 12.01 (1H, bs), 8.69 (1H, m), 8.61 (1H, m), 8.21 (1H, s), 8.09 (1H, m), 7.95 (1H, m), 7.39 (1H, m), 7.20 (1H, m), 7.11 (2H, m), 4.51 (2H, *d, J* = 6 Hz), 3.48 (2H, d, *J* = 16 Hz), 3.00 (2H, d, *J* = 16 Hz), 2.71 (2H, s). |
| **10** | 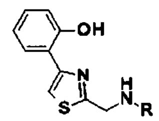 | 2-[2-[[4-(2-hydroxyphenyl)thiazol-2-yl]methylcarbamoyl]indan-2-yl]acetic acid<br>M/z 409.3 $(M+H)^+$<br>$^1$H NMR (d6-DMSO) $\delta$ 12.15 (1H, bs), 10.72 (1H, s), 8.79 (1H, bs), 8.02 (1H, s), 7.91 (1H, m), 7.24-7.11 (5H, m), 6.95-6.86 (2H, m), 4.61 (2H, d, *J* = 6 Hz), 3.48 (2H, d, *J* = 16 Hz), 2.99 (2H, d, *J* = 16 Hz), 2.71 (2H, s). |
| **11** | 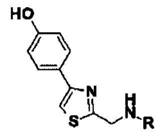 | 2-[2-[[4-(4-hydroxyphenyl)thiazol-2-yl]methylcarbamoyl]indan-2-yl]acetic acid<br>M/z 409.3 $(M+H)^+$<br>$^1$H NMR (d6-DMSO) $\delta$ 12.10 (1H, bs), 9.56 (1H, s), 8.89 (1H, bs), 7.71 (2H, m), 7.69 (1H, s), 7.20 (1H, m), 7.11 (2H, m), 6.79 (2H, m), 4.52 (2H, d, *J* = 6 Hz), 3.46 (2H, d, J = 16 Hz), 2.99 (2H, d, *J* = 16 Hz), 2.70 (2H, s). |
| **12** | 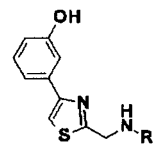 | 2-[2-[[4-(3-hydroxyphenyl)thiazol-2-yl]methylcarbamoyl]indan-2-yl]acetic acid<br>M/z 409.3 $(M+H)^+$<br>$^1$H NMR (d6-DMSO) $\delta$ 9.32 (1H, bs), 7.86 (1H, s), 7.32 (2H, m), 7.22-7.10 (5H, m), 6.71 (1H, m), 4.51 (2H, d, *J* = 6 Hz), 3.49 (2H, d, *J* = 16 Hz), 2.99 (2H, d, *J* = 16 Hz), 2.71 (2H, s). |

**Example 13**

**2-[2-[[4-[2-(2-hydroxyethoxy)phenyl]thiazol-2-yl]methylcarbamoyl]indan-2-yl]acetic acid**

Method B was used as described above.

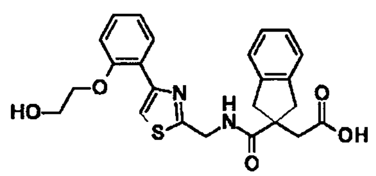

a. Methyl 2-[2-[2-[(tert-butoxycarbonylamino)methyl]thiazol-4-yl]phenoxy]acetate

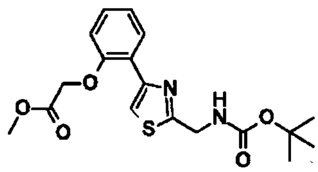

$K_2CO_3$ (964 mg, 6.98 mmol) and then methyl bromoacetate (0.53 mL, 5.58 mmol) were added to a solution of tert-butyl N-[[4-(2-hydroxyphenyl)thiazol-2-yl]methyl]carbamate (1.425 g, 4.65 mmol) in DMF (11 mL) at room temperature. The reaction mixture was stirred at room temperature for 4 h, and then partitioned between water (20 mL) and EtOAc (20 mL). The combined extracts were washed with water (10 mL), brine (10 mL), dried and evaporated. The resulting crude material was chromatographed on silica eluting with 5-25% EtOAc in hexane affording a white solid (1.53 g, 82%). M/z 379 $(M+H)^+$.

b. Tert-butyl N-[[4-[2-(2-hydroxyethoxy)phenyl]thiazol-2-yl]methyl]carbamate

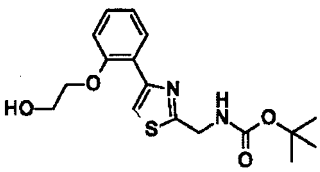

A solution of 1M $LiAlH_4$ in $Et_2O$ (2.64 mL) was added dropwise over 5 min to a solution of methyl 2-[2-[2-[(tert-butoxycarbonylamino)methyl]thiazol-4-yl]phenoxy]acetate (500mg, 1.32 mmol) in THF (10 mL) at -10°C. The reaction mixture was stirred at -10°C for 15 min and quenched at the same temperature with water (0.3 mL) and 15N aqueous NaOH solution (0.1 mL). The reaction mixture was filtered through celite and the filtrate evaporated. The resulting crude material was chromatographed on silica eluting with 40-70% EtOAc in hexane affording a white solid (0.49 g, 100%). M/z 351 $(M+H)^+$.

c. 2-[2-[[4-[2-(2-hydroxyethoxy)phenyl]thiazol-2-yl]methylcarbamoyl]indan-2-yl]acetic acid

The title product (29.3 mg, 47%) was obtained as white solid following the procedure described in example 1 step-b to step-d. M/z 453.3 $(M+H)^+$. $^1$H NMR (d6-DMSO) $\delta$ 12.11 (1H, bs), 8.64 (1H, m), 8.13 (2H, m), 7.29-7.00 (7H, m), 4.60 (2H, d, *J* = 6 Hz), 4.14 (2H, t), 3.81 (2H, t), 3.47 (2H, d, *J* = 16 Hz), 3.00 (2H, d, *J* = 16 Hz), 2.75 (2H, s).

**Example 14**

**2-[2-[[4-[2-(2-aminoethoxy)phenyl]thiazol-2-yl]methylcarbamoyl]indan-2-yl]acetic acid**

Method B was used as described above.

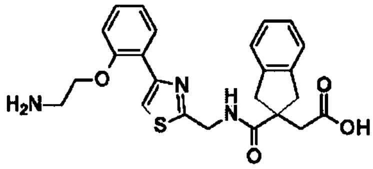

a. Tert-butyl 2-[2-[[4-[2-(2-methylsulfonyloxyethoxy)phenyl]thiazol-2-yl]methylcarbamoyl]indan-2-yl]acetate

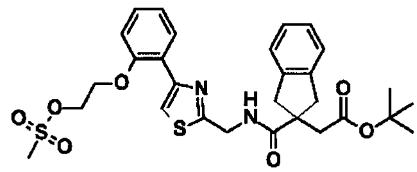

TEA (0.082 mL, 0.59 mmol) and methanesulfonyl chloride (0.037 mL, 0.472 mmol) were added successively to a solution of tert-butyl 2-[2-[[4-[2-(2-hydroxyethoxy)phenyl]thiazol-2-yl]methylcarbamoyl]indan-2-yl]acetate (200 mg, 0.393 mmol) in DCM (7.5 mL) at 0°C. The reaction mixture was stirred at room temperature for 30 min, diluted with DCM (10 mL), washed with 0.1N HCl (10 mL) and water (10 mL), dried and evaporated affording a gum (200mg, 100%) which was used directly in the next step.

b. Tert-butyl 2-[2-[[4-[2-(2-azidoethoxy)phenyl]thiazol-2-yl]methylcarbamoyl]indan-2-yl]acetate

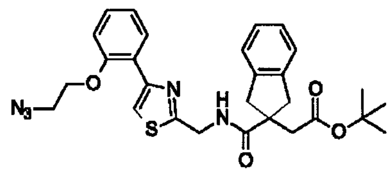

$NaN_3$ (28 mg, 0.433 mmol) was added to a solution of tert-butyl 2-[2-[[4-[2-(2-methylsulfonyloxyethoxy)phenyl]thiazol-2-yl]methylcarbamoyl]indan-2-yl]acetate (231 mg, 0.393 mmol) in DMF (1.5 mL) at room temperature. The reaction mixture was heated at 50°C and stirred overnight. The cooled reaction mixture was partitioned between water (3 mL) and EtOAc (3 mL), and then extracted with EtOAc (2x 3 mL). Combined organic extratcs were washed with water (3 mL) and brine (3 mL), dried and evaporated. The resulting crude material was chromatographed on silica eluting with 20-50% EtOAc in hexane affording a gum (179 mg, 86%). M/z 534 $(M+H)^+$.

c. Tert-butyl 2-[2-[[4-[2-(2-aminoethoxy)phenyl]thiazol-2-yl]methylcarbamoyl]indan-2-yl]acetate

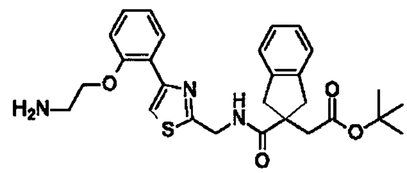

$PPh_3$ (132 mg, 0.505 mmol) was added to a solution of tert-butyl 2-[2-[[4-[2-(2-azidoethoxy)phenyl]thiazol-2-yl]methylcarbamoyl]indan-2-yl]acetate (231 mg, 0.393 mmol) in THF (3 mL) at room temperature. The reaction mixture was stirred at room temperature for 1 h. Then water (0.061 mL, 3.39 mmol) was added. The reaction mixture was stirred overnight and evaporated. The resulting crude material was chromatographed on silica eluting with 2M $NH_3$/MeOH in DCM affording a gum (140 mg, 82%). M/z 508 $(M+H)^+$.

d. 2-[2-[[4-[2-(2-aminoethoxy)phenyl]thiazol-2-yl]methylcarbamoyl]indan-2-yl]acetic acid

The title product (39.5 mg, 73%) was obtained as white solid following the procedure described in example 1 step-d. M/z 452.3 $(M+H)^+$. $^1H$ NMR (d6-DMSO) $\delta$ 10.2 (1H, bs), 8.09 (1H, m), 8.01 (1H, s), 7.31 (1H, m), 7.20 (2H, m), 7.11 (2H, m), 7.05 (1H, m), 4.60 (2H, d, $J$ = 6 Hz), 4.13 (2H, t), 3.47 (2H, d, $J$ = 16 Hz), 3.09 (2H, t), 3.00 (2H, d, $J$ = 16 Hz), 2.75 (2H, s).

**Example 15**

**2-[2-[[4-[4-[2-(dimethylamino)ethylcarbamoyl]phenyl]thiazol-2-yl]methylcarbamoyl]indan-2-yl]acetic acid**

Method B-1 was used as described above.

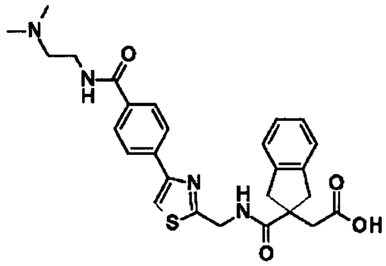

a. 4-[2-[(tert-butoxycarbonylamino)methyl]thiazol-4-yl]benzoic acid

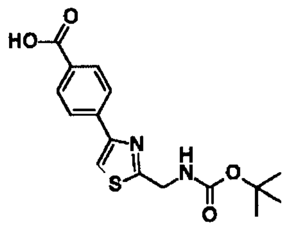

A white solid (376 mg, 56%) was obtained following the procedure described in example 1 step-a from 4-(2-bromoacetyl)benzoic acid. M/z 279 $(M+H-tBu)^+$.

b. Tert-butyl N-[[4-[4-[2-(dimethylamino)ethylcarbamoyl]phenyl]thiazol-2-yl]methyl]carbamate

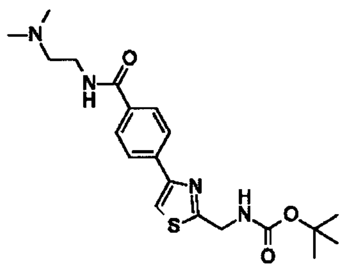

HATU (321 mg, 0.843 mmol), TEA (0.24 mL, 1.69 mmol) and N,N-dimethylethylenediamine (0.074 mL, 0.675 mmol) were added to a solution of 4-[2-[(tert-butoxycarbonylamino)methyl]thiazol-4-yl]benzoic acid (188 mg, 0.562 mmol) in DMF (2 mL) at room temperature. The reaction mixture was stirred for 3 h and partitioned between water (5 mL) and DCM (5 mL). The organic extract was dried and evaporated. The crude product was purified by chromatography on silica eluting with 0-12% 2M $NH_3$/MeOH in DCM affording a white solid (182 mg, 80% yield). M/z 405 $(M+H)^+$.

c. 2-[2-[[4-[4-[2-(dimethylamino)ethylcarbamoyl]phenyl]thiazol-2-yl]methylcarbamoyl]indan-2-yl]acetic acid

The title product was obtained following the procedure described in example 1 step-b to step-d as white solid after purification by preparative hplc (MDAP) (19 mg, 17%). M/z 507.4 $(M+H)^+$. $^1H$ NMR (d6-DMSO) $\delta$ 8.71 (1H, m), 8.42 (1H, m), 8.11 (1H, s), 8.00 (2H, d), 7.89 (2H, d), 7.20 (2H, m), 7.11 (2H, m), 4.60 (2H, d, $J$ = 6 Hz), 3.48 (2H, d, $J$ = 16 Hz), 3.35 (2H, m), 2.98 (2H, d, $J$ = 16 Hz), 2.72 (2H, s), 2.49 (2H, m), 2.21 (6H, s).

Other compounds were prepared following the method B-1 described for Example 15 using other commercial amine reagents in step-b and starting with from 4-(2-bromoacetyl)benzoic acid, methyl 4-(2-bromoacetyl)benzoate or methyl 2-(2-bromoacetyl)benzoate, purified in a similar manner by preparative HPLC are shown in the Table below, wherein R is the moiety:

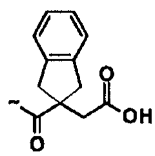

| Example | Structure | Name, MS and NMR data |
|---|---|---|
| **16** | 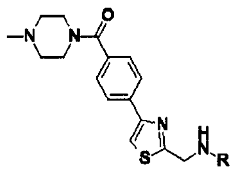 | 2-[2-[[4-[4-(4-methylpiperazine-1-carbonyl)phenyl]thiazol-2-yl] methylcarbamoyl]indan-2-yl]acetic acid<br>M/z 519 (M+H)$^+$<br>$^1$H NMR (d6-DMSO) δ 8.68 (1H, m), 8.09 (1H, s), 7.99 (2H, d), 7.42 (2H, d), 7.21 (2H, m), 7.12 (2H, m), 4.60 (2H, d, *J* = 6 Hz), 3.46 (2H, d, *J* = 16 Hz), 3.32 (4H, m), 2.99 (2H, d, *J* = 16 Hz), 2.71 (2H, s), 2.38 (4H, m), 2.23 (3H, s). |
| **17** | 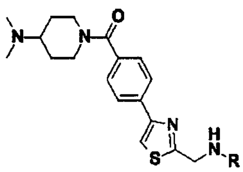 | 2-[2-[[4-[4-[4-(dimethylamino)piperidine-1-carbonyl]phenyl]thiazol-2-yl] methylcarbamoyl]indan-2-yl] acetic acid<br>M/z 547.2 (M+H)$^+$<br>$^1$H NMR (500 MHz, DMSO-$d_6$): δ 8.69 (1H, t, J = 5.5 Hz), 8.06 (1H, s), 7.98 (2H, d, J = 8 Hz), 7.45 (2H, d, J = 8 Hz), 7.22-7.20 (2H, m), 7.14-7.12 (2H, m), 4.60 (2H, d, J = 6 Hz), 4.50-4.48 (1H, m), 3.69-3.66 (1H, m), 3.46 (2H, d, J = 16.5 Hz), 3.13-3.11 (1H, m), 2.99 (2H, d, J = 16.5 Hz), 2.87-2.85 (1H, m), 2.75 (2H, s), 2.65-2.63 (1H, m), 2.34 (6H, s), 1.90-1.75 (2H, m), 1.42-1.40 (2H, m). |
| **18** | 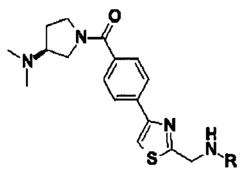 | 2-[2-[[4-[4-[(3S)-3-(dimethylamino)pyrrolidine-1-carbonyl]phenyl]thiazol-2-yl]methylcarbamoyl]indan-2-yl]acetic acid<br>M/z 533.2 (M+H)$^+$<br>$^1$H NMR (500 MHz, DMSO-$d_6$): δ 12.11 (1H, bs), 8.66 (1H, t, J = 5.5 Hz), 8.08 (1H, s), 7.98 (2H, d, J = 8 Hz), 7.59-757 (2H, m), 7.22-7.20 (2H, m), 7.14-7.12 (2H, m), 4.60 (2H, d, J = 6 Hz), 3.80-3.53 (4H, m), 3.46 (2H, d, J = 16.5 Hz), 3.44-3.40 (1H, bs), 2.99 (2H, d, J = 16.5 Hz), 2.75 (2H, s), 2.45-2.05 (7H, m), 1.90-1.75 (1H, m). |
| **19** | 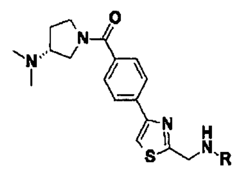 | 2-[2-[[4-[4-[(3R)-3-(dimethylamino)pyrrolidine-1-carbonyl]phenyl]thiazol-2-yl]methylcarbamoyl]indan-2-yl]acetic acid<br>M/z 533.2 (M+H)$^+$<br>$^1$H NMR (500 MHz, DMSO-$d_6$): δ 12.11 (1H, bs), 9.81 (1H, bs), 8.66 (1H, t, J = 5.5 Hz), 8.09 (1H, s), 7.99 (2H, d, J = 8 Hz), 7.59 (2H, d, J = 8 Hz), 7.22-7.20 (2H, m), 7.14-7.12 (2H, m), 4.60 (2H, d, J = 5.5 Hz), 3.92-3.85 (2H, m), 3.75-3.53 (3H, m), 3.46 (2H, d, J = 16.5 Hz), 2.99 (2H, d, J = 16.5 Hz), 2.98-2.78 (6H, bs), 2.75 (2H, s), 2.36-2.31 (1H, m), 2.15-2.06 (1H, m). |
| **20** | 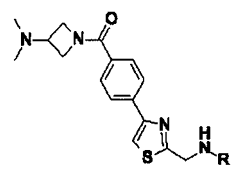 | 2-[2-[[4-[4-[3-(dimethylamino)azetidine-1-carbonyl]phenyl]thiazol-2-yl] methylcarbamoyl]indan-2-yl]acetic acid<br>M/z 519.2 (M+H)$^+$<br>$^1$H NMR (500 MHz, DMSO-$d_6$): δ 9.15 (1H, bs), 8.10 (1H, s), 7.99 (2H, d, J = 8.5 Hz), 7.70 (2H, d, J = 8.5 Hz), 7.21-7.19 (2H, m), 7.14-7.12 (2H, m), 4.60 (2H, d, J = 5.5 Hz), 4.33-4.30 (1H, m), 4.11-4.04 (2H, m), 3.84-3.81 (1H, m), 3.49 (2H, d, J = 16.5 Hz), 3.12-3.05 (1H, m), 2.99 (2H, d, J = 16.5 Hz), 2.70 (2H, s), 2.08 (6H, s). |

(continued)

| Example | Structure | Name, MS and NMR data |
|---|---|---|
| **21** | 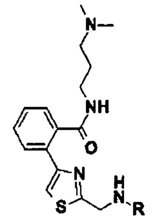 | 2-[2-[[4-[2-[3-(dimethylamino)propylcarbamoyl]phenyl]thiazol-2-yl] methylcarbamoyl]indan-2-yl]acetic acid<br>M/z 521.3 $(M+H)^+$<br>$^1H$ NMR (500 MHz, DMSO-$d_6$): δ 9.42 (1H, bs), 8.15 (1H, t, J = 5 Hz), 7.74 (1H, d, J = 7.5 Hz), 7.55 (1H, s), 7.47 (1H, t, J = 7.5 Hz), 7.39 (1H, t, J = 7.5 Hz), 7.33 (1H, d, J = 7.5 Hz), 7.20-7.18 (2H, m), 7.13-7.11 (2H, m), 4.57 (2H, d, J = 6 Hz), 3.44 (2H, d, J = 16 Hz), 3.16-3.12 (2H, m), 2.96 (2H, d, J = 16 Hz), 2.62 (2H, s), 2.36 (2H, t, J = 7.5 Hz), 2.24 (6H, s), 1.60-1.54 (2H, m). |

**Example 22**

**2-[2-[[4-[4-(4,4-dimethylpiperazin-4-ium-1-carbonyl)phenyl]thiazol-2-yl]methylcarbamoyl]indan-2-yl]acetate**

Method B-1 was used as described above.

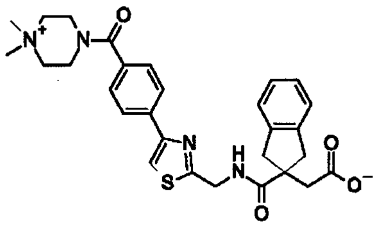

a. Tert-butyl 2-[2-[[4-[4-(4,4-dimethylpiperazin-4-ium-1-carbonyl)phenyl]thiazol-2-yl]methylcarbamoyl]indan-2-yl]acetate

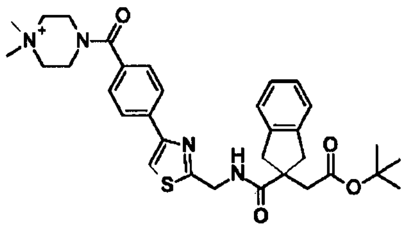

MeI (425 mg, 3 mmol) was added to a solution of tert-butyl 2-[2-[[4-[4-(4-methylpiperazine-1-carbonyl)phenyl]thiazol-2-yl]methylcarbamoyl]indan-2-yl]acetate (172 mg, 0.3 mmol) in THF (3 mL) at room temperature. The reaction was stirred overnight and evaporated. The resulting residue was triturated with $Et_2O$ affording a yellow solid (241 mg, crude) which was used in the next step without further purification. M/z 589 $(M)^+$.

b. 2-[2-[[4-[4-(4,4-dimethylpiperazin-4-ium-1-carbonyl)phenyl]thiazol-2-yl]methylcarbamoyl]indan-2-yl] acetate

TFA (2 mL) was added to a solution of tert-butyl 2-[2-[[4-[4-(4,4-dimethylpiperazin-4-ium-1-carbonyl)phenyl]thiazol-2-yl]methylcarbamoyl]indan-2-yl]acetate (215 mg, 0.3 mmol) in DCM (2 mL) at room temperature. The reaction mixture was stirred at for 45 min, evaporated and azeotroped with toluene. The residue was purified by preparative hplc (MDAP) affording a white solid (94 mg, 59%). M/z 533.3 $(M+H)^+$. $^1H$ NMR (d6-DMSO) δ 8.40 (1H, bs), 8.11 (1H, s), 8.00 (2H, d), 7.51 (2H, d), 7.20 (2H, m), 7.11 (2H, m), 4.61 (2H, d, *J* = 6 Hz), 3.98-3.47 (8H, m), 3.42 (2H, d, *J* = 16 Hz), 3.21 (6H, s), 3.00 (2H, d, *J* = 16 Hz), 2.51 (2H, s).

**Example 23**

**2-[2-[[4-[3-(4-methylpiperazine-1-carbonyl)phenyl]thiazol-2-yl]methylcarbamoyl]indan-2-yl]acetic acid**

Method B-1 was used as described above.

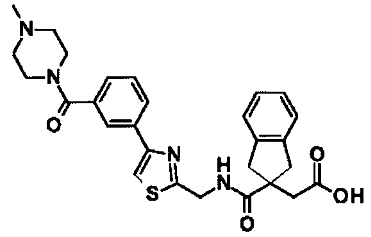

The title product was obtained following the procedure described in example 15, using 3-(2-bromoacetyl)benzoic acid in step-a and N-methyl-piperazine in step-b. The crude product was purified by preparative hplc (MDAP) affording a white solid (63 mg, 53%). M/z 519 $(M+H)^+$. $^1H$ NMR (d6-DMSO) $\delta$ 8.65 (1H, m), 8.08 (1H, s), 7.99 (1H, m), 7.91 (1H, m), 7.50 (1H, m), 7.32 (1H, m), 7.20 (2H, m), 7.12 (2H, m), 4.59 (2H, d, $J$ = 6 Hz), 3.61 (4H, m), 3.42 (2H, d, $J$ = 16 Hz), 2.99 (2H, d, $J$ = 16 Hz), 2.71 (2H, s), 2.35 (4H, m), 2.23 (3H, s).

**Example 24**

**2-[2-[[4-[5-[2-(dimethylamino)ethylcarbamoyl]-2-methoxy-phenyl]thiazol-2-yl]methylcarbamoyl]indan-2-yl]acetic acid**

Method B-1 was used as described above.

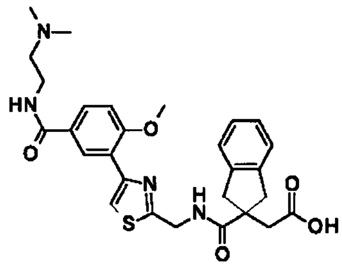

a. Methyl 3-acetyl-4-methoxy-benzoate

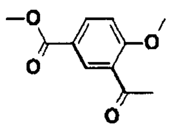

To a stirred solution of 3-acetyl-4-hydroxybenzoic acid (2 g, 11.1 mmol) in acetone (20 mL) was added $K_2CO_3$ (3 g, 22.2 mmol), MeI (3.4 mL, 55.5 mmol) at room temperature and stirred for 24 h. The reaction mixture was evaporated and resulting residue was diluted with water (20 mL) and extracted with EtOAc (2 x 50 mL). The combined organic layer was dried, filtered and evaporated. The crude was chromatographed on silica eluting with 10-20% EtOAc in petroleum ether affording an off white solid (1.5 g, 65%). M/z 209.1 $(M+H)^+$.

b. Methyl 3-(2-bromoacetyl)-4-methoxy-benzoate

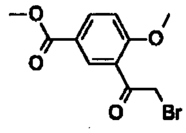

To a stirred solution of methyl 3-acetyl-4-methoxy-benzoate (0.7 g, 3.36 mmol) in DCM (15 mL) was added PTSA (64 mg, 0.33 mmol) and NBS (600 mg, 3.36 mmol) at 0 °C. Then the reaction mixture was allowed to stir at room temperature for 8 h. The reaction mixture was quenched with saturate sodium bicarbonate, extracted with DCM (2 x 30 mL) and combined organic layer was dried, filtered and evaporated. The crude was chromatographed on silica eluting with 1-2% MeOH in DCM affording an off white solid (620 mg, 64%). M/z 287.0 $(M+H)^+$.

c. Methyl 3-[2-[(tert-butoxycarbonylamino)methyl]thiazol-4-yl]-4-methoxy-benzoate

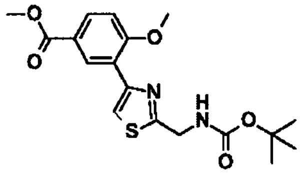

To a stirred solution of methyl 3-(2-bromoacetyl)-4-methoxy-benzoate (600 mg, 2.09 mmol) in EtOH (15 mL) was added tert-butyl (2-amino-2-thioxoethyl)carbamate (402 mg, 2.09 mmol) at room temperature and stirred for 3 h. The reaction mixture was evaporated and resulting residue was chromatographed on silica eluting with 10-20% EtOAc in petroleum ether affording an off white solid (530 mg, 66%). M/z 379.1 $(M+H)^+$.

d. 3-[2-[(tert-butoxycarbonylamino)methyl]thiazol-4-yl]-4-methoxy-benzoic acid

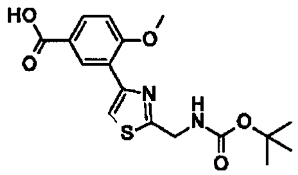

To a stirred solution of methyl 3-[2-[(tert-butoxycarbonylamino)methyl]thiazol-4-yl]-4-methoxybenzoate (550 mg, 1.45 mmol) in THF/$H_2O$ (7.5 mL, 2:1) was added IN NaOH (14.5 mL, 14.5 mmol) at room temperature and stirred for 16 h. The reaction mixture was concentrated and the resulting residue was diluted with water (10 mL) and acidified to pH ~5 with saturated aqueous citric acid solution. The resulting precipitate was filtered, washed with water (5 mL) and dried in vacuum affording an off white solid (480 mg, 90%). M/z 365.1 $(M+H)^+$.

e. 2-[2-[[4-[5-[2-(dimethylamino)ethylcarbamoyl]-2-methoxy-phenyl]thiazol-2-yl]methylcarbamoyl]indan-2-yl]acetic acid

The title product was obtained following the procedure described in example 15, using N,N-dimethylethane-1, 2-diamine in step-b. The crude compound was purified by MDAP affording an off white solid (57 mg, 48%). M/z 537.2 $(M+H)^+$. $^1H$ NMR (500 MHz, DMSO-$d_6$): δ 9.70 (1H, bs), 8.70 (1H, s), 8.58 (1H, s), 7.98 (1H, s), 7.83-7.81 (1H, m), 7.20-7.18 (3H, m), 7.12-7.10 (2H, m), 4.62 (2H, d, J = 5.5 Hz), 3.97 (3H, s), 3.51 (2H, d, J = 16 Hz), 3.38-3.35 (2H, bs), 2.99 (2H, d, J = 16 Hz), 2.64 (2H, s), 2.47-2.44 (2H, m), 2.21 (6H, s).

**Example 25**

**2-[2-[[4-[2-methoxy-5-(4-methylpiperazine-1-carbonyl)phenyl)thiazol-2-yl]methylcarbamoyl]indan-2-yl]acetic acid**

Method B-1 was used as described above.

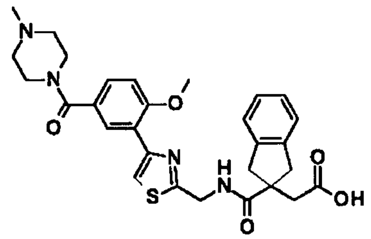

The title product was obtained following the procedure described in example 24, using N-methyl-piperazine. The crude compound was purified by MDAP affording an off white solid (57 mg, 48%). M/z 549.2 $(M+H)^+$. $^1H$ NMR (500 MHz, DMSO-$d_6$): δ 8.19 (1H, d, J = 2 Hz), 8.00 (1H, s), 7.37 (1H, dd, J = 8.5 Hz, J = 2 Hz), 7.20-7.17 (3H, m), 7.13-7.11 (2H, m), 4.59 (2H, d, J = 6 Hz), 3.96 (3H, s), 3.50-3.43 (4H, m), 3.45 (2H, d, J = 16.5 Hz), 2.98 (2H, d, J = 16.5 Hz), 2.67-2.63 (2H, m), 2.35-2.25 (4H, m), 2.19 (3H, s).

**Example 26**

**2-[2-[[4-[4-(4-methylpiperazin-1-yl)phenyl]thiazol-2-yl]methylcarbamoyl]indan-2-yl]acetic acid**

Method B-2 was used as described above.

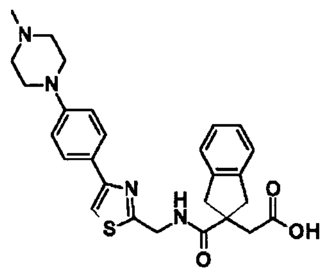

a. Tert-butyl N-[[4-(4-bromophenyl)thiazol-2-yl]methyl]carbamate

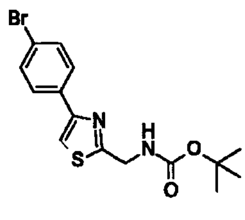

A white solid (931 mg, 84%) was obtained following the procedure described in example 1 step-a from 2,4'-dibromoacetophenone. M/z 313 $(M+H-tBu)^+$.

b. Tert-butyl N-[[4-[4-(4-methylpiperazin-1-yl)phenyl]thiazol-2-yl]methyl]carbamate

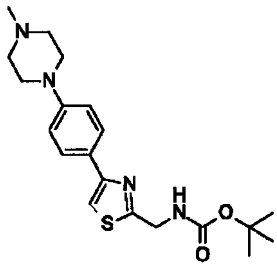

1-methylpiperazine (120 mg, 1.2 mmol), RuPhos Pd G3 (84 mg, 0.1 mmol), RuPhos (47 mg, 0.1 mmol) and NaO*t*Bu (115 mg, 1.2 mmol) were added to a solution of tert-butyl N-[[4-(4-bromophenyl)thiazol-2-yl]methyl]carbamate (369 mg, 1 mmol) in THF (2 mL) at room temperature. The vial was sealed, flashed with N2, and stirred at 80°C overnight. The reaction mixture was cooled and partitioned between EtOAc (5 mL) and water (5 mL). the organic extract was washed with brine (3 mL), dried and evaporated. The crude product was purified by chromatography on silica eluting with 4-10% MeOH in DCM affording a beige solid (200 mg, 51% yield). M/z 332 $(M+H-tBu)^+$.

c. 2-[2-[[4-[4-(4-methylpiperazin-1-yl)phenyl]thiazol-2-yl]methylcarbamoyl]indan-2-yl]acetic acid

The title product was obtained following the procedure described in example 1 step-b to step-d as white solid after purification by preparative hplc (MDAP) (61 mg, 34%). M/z 491.2 $(M+H)^+$. $^1H$ NMR (d6-DMSO) $\delta$ 8.75 (1H, bs), 7.76 (2H, d), 7.71 (1H, s), 7.20 (2H, m), 7.11 (2H, m), 6.96 (2H, d), 4.57 (2H, d, *J* = 6 Hz), 3.49 (2H, d, *J* = 16 Hz), 3.22 (4H, m), 2.98 (2H, d, *J* = 16 Hz), 2.72 (2H, s), 2.46 (4H, m), 2.21 (3H, s).

**Example 27**

**2-[2-[[4-[4-(4-methylpiperazine-1-carbonyl)phenyl]thiazol-2-yl]methylcarbamoyl]indan-2-yl]acetic acid**

Method B-2 was used as described above.

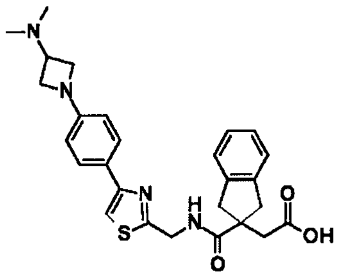

The title product was obtained following the procedure described in example 26, using N,N-dimethylazetidin-3-amine in step-b. The crude product was purified by preparative hplc (MDAP) affording a white solid (106 mg, 60%). M/z 491.3 $(M+H)^+$. $^1H$ NMR (d6-DMSO) $\delta$ 8.62 (1H, m), 7.74 (2H, d), 7.63 (1H, s), 7.20 (2H, m), 7.11 (2H, m), 6.45 (2H, d), 4.56 (2H, d, *J* = 6 Hz), 3.95 (2H, m), 3.57 (2H, m), 3.47 (2H, d, *J* = 16 Hz), 3.27 (1H, m), 3.00 (2H, d, *J* = 16 Hz), 2.73 (2H, s), 2.12 (6H, s).

**Example 28**

**2-[2-[[4-[3-(4-methylpiperazin-1-yl)phenyl]thiazol-2-yl]methylcarbamoyl]indan-2-yl]acetic acid**

Method B-2 was used as described above.

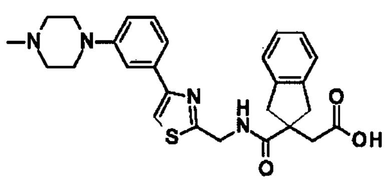

The title product was obtained following the procedure described in example 26, using 2,3'-dibromoacetophenone in step-a. The crude product was purified by preparative hplc (MDAP) affording a white solid (42 mg, 45%). M/z 491.2 $(M+H)^+$. $^1H$ NMR (d6-DMSO) δ 8.75 (1H, bs), 7.93 (1H, s), 7.49 (1H, m), 7.33 (1H, m), 7.29-7.19 (3H, m), 7.11 (2H, m), 6.89 (1H, m), 4.59 (2H, d, *J* = 6 Hz), 3.48 (2H, d, *J* = 16 Hz), 3.20 (4H, m), 3.00 (2H, d, *J* = 16 Hz), 2.71 (2H, s), 2.48 (4H, m), 2.22 (3H, s).

**Example 29**

**2-[2-[[4-[3-[3-(dimethylamino)azetidin-1-yl]phenyl]thiazol-2-yl]methylcarbamoyl]indan-2-yl]acetic acid**

Method B-2 was used as described above.

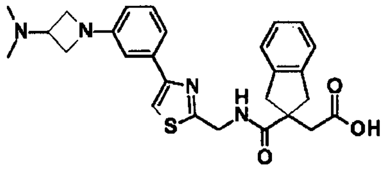

The title product was obtained following the procedure described in example 26, using 2,3'-dibromoacetophenone in step-a and N,N-dimethylazetidin-3-amine in step-b. The crude product was purified by preparative hplc (MDAP) affording a white solid (50 mg, 54%). M/z 491.2 $(M+H)^+$. $^1H$ NMR (d6-DMSO) δ 8.72 (1H, bs), 7.89 (1H, s), 7.22-7.15 (4H, m), 7.13 (2H, m), 6.96 (1H, m), 6.39 (1H, m), 4.59 (2H, d, *J* = 6 Hz), 3.92 (2H, t), 3.57 (2H, t), 3.49 (2H, d, *J* = 16 Hz), 3.20 (1H, m), 3.00 (2H, d, *J* = 16 Hz), 2.71 (2H, s), 2.11 (6H, s).

**Example 30**

**2-[2-[[4-[4-[(3aS,6aR)-2-methyl-1,3,3a,4,6,6a-hexahydropyrrolo[3,4-c]pyrrol-5-yl]phenyl]thiazol-2-yl]methylcarbamoyl]indan-2-yl]acetic acid**

Method B-2 was used as described above.

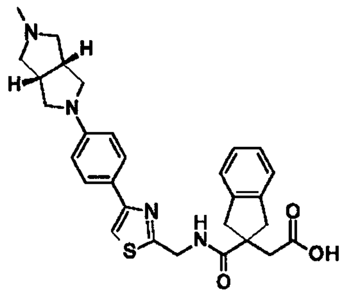

The title product was obtained following the procedure described in example 26, using (3aS,6aR)-5-methyl-2,3,3a,4,6,6a-hexahydro-1H-pyrrolo[3,4-c]pyrrole in step-b. The crude product was purified by preparative hplc (MDAP) affording a white solid (62 mg, 35%). M/z 517.3 $(M+H)^+$. $^1H$ NMR (d6-DMSO) δ 8.79 (1H, bs), 7.72 (2H, m), 7.61 (1H, s), 7.20 (2H, m), 7.11 (2H, m), 6.64 (2H, m), 4.55 (2H, d, *J* = 6 Hz), 3.49 (2H, d, *J* = 16 Hz), 3.3 7 (2H, m), 3.12 (2H, m), 3.00 (2H, d, *J* = 16 Hz), 2.89 (2H, m), 2.71 (2H, s), 2.58 (2H, m), 2.42 (2H, m), 2.21 (3H, s).

**Example 31**

**2-[2-[[4-[2-methoxy-4-(4-methylpiperazin-1-yl)phenyl]thiazol-2-yl]methylcarbamoyl]indan-2-yl]acetic acid**

Method B-2 was used as described above.

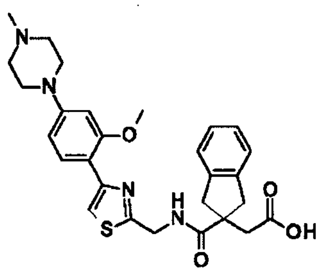

a. 2-bromo-1-(4-bromo-2-methoxy-phenyl)ethenone

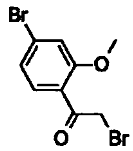

Trimethylphenylammonium tribromide (376 mg, 1 mmol) was added to a solution of 1-(4-bromo-2-methoxy-phenyl)ethanone (229 mg, 1 mmol) in THF (5 mL) at room temperature. The reaction mixture was stirred at room temperature overnight, filtered and the filtrate was evaporated. The residue was used in the next step without further purification (308 mg, crude).

b. Tert-butyl N-[[4-(4-bromo-2-methoxy-phenyl)thiazol-2-yl]methyl]carbamate

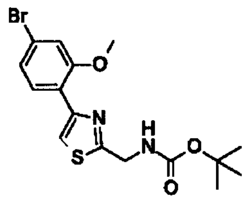

A solution of 2-bromo-1-(4-bromo-2-methoxy-phenyl)ethanone (308 g, 1 mmol) and tert-butyl (2-amino 2-thioxoethyl)carbamate (190 mg, 1 mmol) in EtOH (5 mL) was stirred at room temperature for 5 h. The solution was evaporated and the resulting residue purified by chromatography on silica eluting with 0-40% EtOAc in hexane affording a pale yellow solid (187 mg, 47% over 2 steps). M/z 343 $(M+H-tBu)^+$.

c. Tert-butyl N-[[4-[2-methoxy-4-(4-methylpiperazin-1-yl)phenyl]thiazol-2-yl]methyl]carbamate

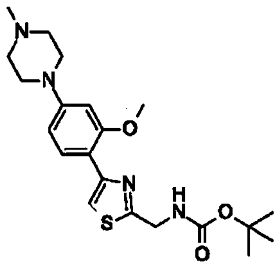

An orange solid (194 mg, 46%) was obtained following the procedure described in example 26 step-b and purification by chromatography on silica eluting with 1-10% MeOH in DCM M/z 419 $(M+H)^+$.

d. 2-[2-[[4-[2-methoxy-4-(4-methylpiperazin-1-yl)phenyl]thiazol-2-yl]methylcarbamoyl]indan-2-yl]acetic acid

The title product was obtained following the procedure described in example 1 step-b to step-d as white solid after purification by preparative hplc (MDAP) (61 mg, 33%). M/z 521.4 $(M+H)^+$ $^1H$ NMR (d6-DMSO) $\delta$ 8.69 (1H, bs), 7.95 (1H, d), 7.69 (1H, s), 7.20 (2H, m), 7.11 (2H, m), 6.59 (2H, m), 4.57 (2H, d, *J* = 6 Hz), 3.89 (3H, s), 3.49 (2H, d, *J* = 16 Hz), 3.22 (4H, m), 2.98 (2H, d, *J* = 16 Hz), 2.72 (2H, s), 2.49 (4H, m), 2.22 (3H, s).

**Example 32**

**2-[2-[[4-[4-[3-(dimethylamino)azetidin-1-yl]-2-methoxy-phenyl]thiazol-2-yl]methylcarbamoyl]indan-2-yl]acetic acid**

Method B-2 was used as described above.

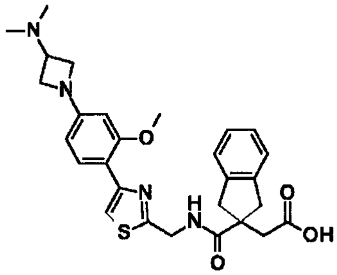

The title product was obtained following the procedure described in example 31, using N,N-dimethylazetidin-3-amine in step-c. The crude product was purified by preparative hplc (MDAP) affording a white solid (96 mg, 53%). M/z 521.4 $(M+H)^+$. $^1H$ NMR (d6-DMSO) $\delta$ 8.68 (1H, m), 7.92 (1H, d), 7.61 (1H, s), 7.20 (2H, m), 7.11 (2H, m), 6.19 (2H, d), 4.55 (2H, d, *J* = 6 Hz), 3.95 (2H, m), 3.87 (3H, s), 3.61 (2H, m), 3.50 (2H, d, *J* = 16 Hz), 3.19 (1H, m), 3.00 (2H, d, *J* = 16 Hz), 2.73 (2H, s), 2.11 (6H, s).

**Example 33**

**2-[5,6-difluoro-2-[[4-[2-methoxy-4-[4-(trimethylammonio)-1-piperidyl]phenyl]thiazol-2-yl]methylcarbamoyl]indan-2-yl]acetate**

Method B-2 was used as described above.

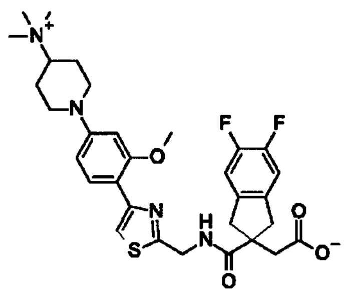

a. Tert-butyl 2-[2-[[4-[4-[4-(dimethylamino)-1-piperidyl]-2-methoxy-phenyl]thiazol-2-yl]methylcarbamoyl]-5,6-difluoro-indan-2-yl]acetate

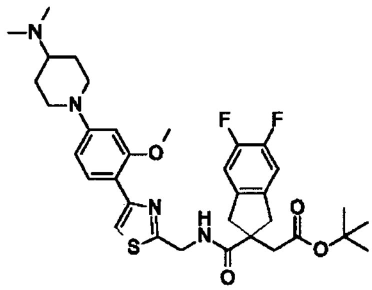

A white solid (107 mg, 70%) was obtained following the procedure described in example 31 using N'N-dimethylpiperidin-4-amine in step-c and 2-(2-(tert-butoxy)-2-oxoethyl)-5,6-difluoro-2,3-dihydro- 1H-indene-2-carboxylic acid in step-e. The crude product was purified by chromatography on silica eluting with 1-10% (2M NH3 in MeOH) in DCM. M/z 641 $(M+H)^+$.

b. [1-[4-[2-[[[2-(2-tert-butoxy-2-oxo-ethyl)-5,6-difluoro-indane-2-carbonyl]amino]methyl]thiazol-4-yl]-3-methoxy-phenyl]-4-piperidyl]-trimethylammonium

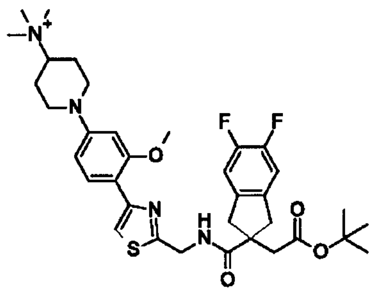

Mel (0.052 mL, 0.835 mmol) was added to a solution of tert-butyl 2-[2-[[4-[4-[4-(dimethylamino)-1-piperidyl]-2-methoxy-phenyl]thiazol-2-yl]methylcarbamoyl]-5,6-difluoro-indan-2-yl]acetate (107 mg, 0.167 mmol) in THF (2 mL) at room temperature. The reaction mixture was stiired at room temperature for 24 h, evaporated and the residue was used in the next step without further purification. M/z 782 $(M)^+$.

c. 2-[5,6-difluoro-2-[[4-[2-methoxy-4-[4-(trimethylammonio)-1-piperidyl]phenyl]thiazol-2-yl]methylcarbamoyl]indan-2-yl]acetate

Water (0.077 mL, 4.28 mmol) and TFA (0.77 mL, 10.02 mmol) were added to a solution of [1-[4-[2-[[[2-(2-tert-butoxy-2-oxo-ethyl)-5,6-difluoro-indane-2-carbonyl]amino]methyl]thiazol-4-yl]-3-methoxy-phenyl]-4-piperidyl]-trimethyl-ammonium (131 mg, 0.167 mmol) in DCM (2 mL) at room temperature. The reaction mixture was stirred at room temperature for 2 h, evaporated and azeotroped with toluene. The crude product was purified by preparative hplc (MDAP) (58 mg, 58% over 2 steps). M/z 599.4 $(M+H)^+$. $^1H$ NMR (d6-DMSO) $\delta$ 12.97 (1H, bs), 7.99 (1H, m), 7.70 (1H, s), 7.20 (2H, m), 6.63 (2H, m), 4.53 (2H, d, *J* = 6 Hz), 4.03 (2H, m), 3.90 (3H, s), 3.52 (1H, m), 3.35 (2H, m), 3.02 (9H, s), 2.86 (2H, d, *J* = 16 Hz), 2.80 (2H, m), 2.32 (2H, s), 2.18 (2H, m), 1.75 (2H, m).

**Example 34**

**2-[2-[[4-[6-(4-methylpiperazin-1-yl)-3-pyridyl]thiazol-2-yl]methylcarbamoyl]indan-2-yl]acetic acid**

Method B-2 was used as described above.

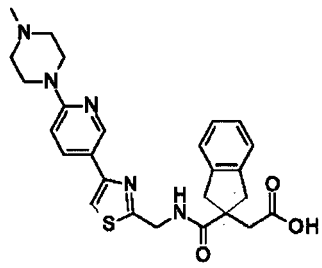

a. 2-bromo-1-(6-bromo-3-pyridyl)ethanone

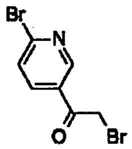

Aqueous hydrogen bromide (48%, 1.9 mL, 17.1 mmol) was added dropwise to a solution of 5-acetyl-2-bromo pyridine (500 mg, 2.5 mmol) in acetic acid (3 mL) at 0°C. Bromine (0.128 mL, 2.5 mmol) was then added dropwise (over 3 min) to the reaction mixture at 0°C. the reaction mixture was stirred at 0°C for 15 min and at room temperature overnight. Aqueous 10% $NaHCO_3$ was then added until basic pH and the product extracted with DCM (2x 5 mL). Combined organic extracts were dried and evaporated. The residue was triturated with $Et_2O$ affording a yellow solid which was used in the next step without further purification (484 mg, 89%). Trimethylphenylammonium tribromide (376 mg, 1 mmol) was added to a solution of 1-(4-bromo-2-methoxyphenyl)ethanone (229 mg, 1 mmol) in THF (5 mL) at room temperature. The reaction mixture was stirred at room temperature overnight, filtered and the filtrate was evaporated. The residue was used in the next step without further purification (308 mg, crude). M/z 279 $(M+H)^+$.

b. Tert-butyl N-[[4-(6-bromo-3-pyridyl)thiazol-2-yl]methyl]carbamate

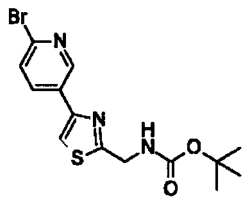

$K_2CO_3$ (100 mg, 0.724 mmol) was added to a solution of 2-bromo-1-(6-bromo-3-pyridyl)ethanone (200 mg, 0.717 mmol) and tert-butyl (2-amino 2-thioxoethyl)carbamate (137 mg, 0.720 mmol) in EtOH (3 mL) at room temperature. The reaction mixture was stirred at room temperature overnight and evaporated. The residue was dissolved in EtOAc (5 mL), washed with aqueous citric acid (10%, 3 mL) and water (3 mL), dried and evaporated. the resulting residue purified by chromatography on silica eluting with 0-100% EtOAc in hexane affording a pale yellow solid (43 mg, 16% over 2 steps). M/z 372 $(M+H)^+$.

c. Tert-butyl N-[[4-[6-(4-methylpiperazin-1-yl)-3-pyridyl]thiazol-2-yl]methyl]carbamate

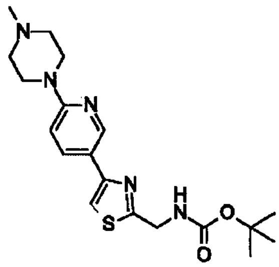

1-methylpiperazine (44 mg, 0.437 mmol), RuPhos Pd G1 (11 mg, 0.0142 mmol) and $Cs_2CO_3$ (238 mg, 0.730 mmol) were added to a solution of tert-butyl N-[[4-(6-bromo-3-pyridyl)thiazol-2-yl]methyl]carbamate (107 mg, 0.289 mmol) in THF (6 mL) at room temperature. The vial was sealed, flashed with N2, and stirred at 95°C overnight. The reaction mixture was cooled, evaporated, and partitioned between EtOAc (5 mL) and water (5 mL). the organic extract was washed with brine (3 mL), dried and evaporated. The crude product was purified by chromatography on silica eluting with 0-10% MeOH in DCM affording a yellow solid (29 mg, 26% yield). M/z 390 $(M+H)^+$.

d. 2-[2-[[4-[6-(4-methylpiperazin-1-yl)-3-pyridyl]thiazol-2-yl]methylcarbamoyl]indan-2-yl]acetic acid

The title product was obtained following the procedure described in example 1 step-b to step-d as white solid after purification by preparative hplc (MDAP) (7.4 mg, 39%). M/z 492.2 $(M+H)^+$. $^1H$ NMR (d6-DMSO) $\delta$ 8.65 (2H, m), 8.00 (1H, m), 7.76 (1H, s), 7.20 (2H, m), 7.11 (2H, m), 6.88 (1H, m), 4.57 (2H, d, $J$ = 6 Hz), 3.49 (2H, d, $J$ = 16 Hz), 3.22 (4H, m), 3.00 (2H, d, $J$ = 16 Hz), 2.72 (2H, s), 2.46 (4H, m), 2.21 (3H, s).

**Example 35**

**2-[2-[[4-[6-[3-(dimethylamino)azetidin-1-yl]-3-pyridyl]thiazol-2-yl]methylcarbamoyl]indan-2-yl]acetic acid**

Method B-2 was used as described above.

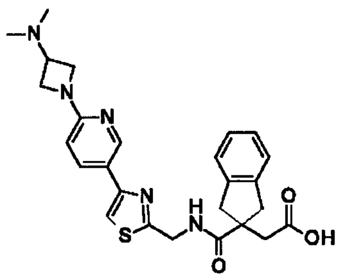

The title product was obtained following the procedure described in example 34, using N,N-dimethylazetidin-3-amine in step-c. The crude product was purified by preparative hplc (MDAP) affording a white solid (55 mg, 51%). M/z 492.3 $(M+H)^+$. $^1H$ NMR (d6-DMSO) $\delta$ 12.1 (1H, bs), 8.62 (1H, m), 8.00 (1H, d), 7.76 (1H, s), 7.20 (2H, m), 7.11 (2H, m), 6.46 (2H, d), 4.57 (2H, d, $J$ = 6 Hz), 4.06 (2H, m), 3.80 (2H, s), 3.46 (2H, d, $J$ = 16 Hz), 3.31 (1H, m), 3.00 (2H, d, $J$ = 16 Hz), 2.71 (2H, s), 2.18 (6H, s).

**Example 36**

**2-[2-[[4-[4-[4-[2-(4-methylpiperazin-1-yl)-2-oxo-ethyl]piperazin-1-yl]phenyl]thiazol-2-yl]methylcarbamoyl]indan-2-yl]acetic acid**

Method B-2 was used as described above.

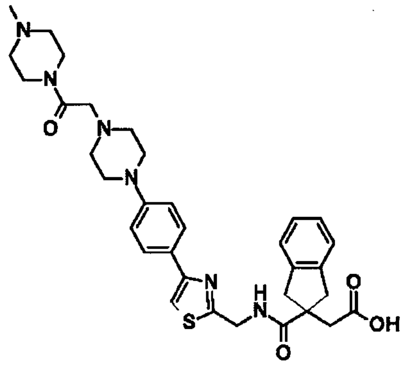

a. Tert-butyl N-[[4-[4-(4-methylpiperazin-1-yl)phenyl]thiazol-2-yl]methyl]carbamate

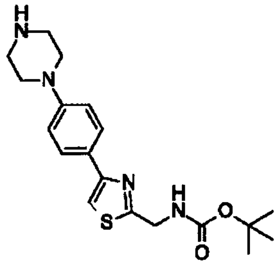

A beige solid (413 mg, 73%) was obtained following the procedure described in example 26 step-b using piperazine and tert-butyl N-[[4-(4-bromophenyl)thiazol-2-yl]methyl]carbamate. M/z 375 $(M+H)^+$.

b. Tert-butyl N-[[4-[4-[4-[2-(4-methylpiperazin-1-yl)-2-oxo-ethyl]piperazin-1-yl]phenyl]thiazol-2-yl]methyl]carbamate

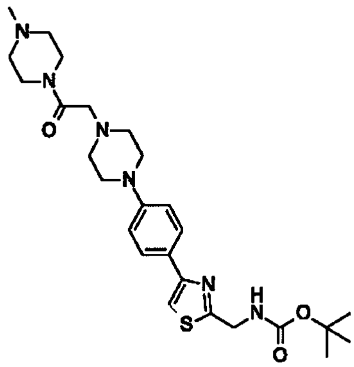

TEA (0.23 mL, 1.66 mmol) and 1-(chloroacetyl)-4-methylpiperazine hydrochloride (130 mg, 0.608 mmol) were added to a solution of tert-butyl N-[[4-[4-(4-methylpiperazin-1-yl)phenyl]thiazol-2-yl]methyl]carbamate (207 mg, 0.563 mmol) in DCM (2 mL) at room temperature. The reaction mixture was stirred at room temperature for 2 days and partitioned between DCM (5 mL) and water (5 mL). the organic extract was dried and evaporated, and the residue was used in the next step without further purification. M/z 515 $(M+H)^+$.

c. 2-[2-[[4-[4-(4-ethanimidoylpiperazin-1-yl)phenyl]thiazol-2-yl]methylcarbamoyl]indan-2-yl]acetic acid

The title product was obtained following the procedure described in example 1 step-b to step-d. The crude product was purified by preparative hplc (MDAP) affording a white solid (37 mg, 54%). M/z 617.3 $(M+H)^+$. $^1H$ NMR (d6-DMSO) $\delta$ 12.71 (1H, bs), 8.78 (2H, m), 8.69 (1H, s), 7.18 (2H, m), 7.09 (2H, m), 6.96 (2H, m), 4.60 (2H, d, $J$ = 6 Hz),

3.55 (2H, m), 3.48-3.29 (4H, m), 3.20 (6H, m), 2.89 (2H, d, *J* = 16 Hz), 2.55 (4H, m), 2.36 (2H, s), 2.34-2.21 (4H, m), 2.18 (3H, s).

**Example 37**

**2-[2-[[4-[4-[3-(dimethylamino)prop-1-ynyl]phenyl]thiazol-2-yl]methylcarbamoyl]indan-2-yl]acetic acid**

Method B-2 was used as described above.

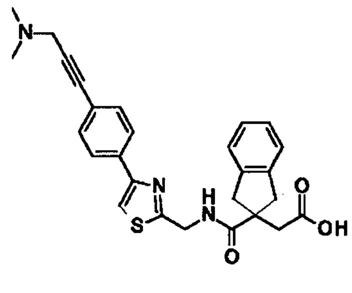

a. Tert-butyl N-[[4-[4-[3-(dimethylamino)prop-1-ynyl]phenyl]thiazol-2-yl]methyl]carbamate

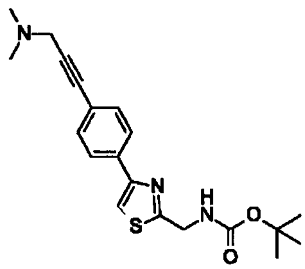

3-dimethylamino-1-propyne (0.127 mL, 1.18 mmol), $Pd(PPh_3)_4$ (160 mg, 0.138 mmol), CuI (20 mg, 0.105 mmol) and TEA (0.98 mL, 7.03 mmol) were added to a solution of tert-butyl N-[[4-(4-bromophenyl)thiazol-2-yl]methyl]carbamate () in acetonitrile (20 mL) at room temperature. The reaction mixture was degassed, flushed with argon, heated at 100°C under microwave conditions for 1.5 h and evaporated. The residue was partitioned between DCM (10 mL) and water (10 mL). The organic extract was dired and evaporated. The crude product was purified by chromatography on silica eluting with 0-10% MeOH in DCM affording a beige solid (96 mg, 24% yield). M/z 372 $(M+H)^+$.

b. 2-[2-[[4-[4-[3-(dimethylamino)prop-1-ynyl]phenyl]thiazol-2-yl]methylcarbamoyl]indan-2-yl]acetic acid

The title product was obtained following the procedure described in example 1 step-b to step-d. The crude product was purified by preparative hplc (MDAP) affording a white solid (67 mg, 39%). M/z 474.0 $(M+H)^+$. $^1H$ NMR (d6-DMSO) $\delta$ 12.62 (1H, bs), 8.03 (1H, s), 7.92 (2H, m), 7.49 (2H, m), 7.17 (2H, m), 7.10 (2H, m), 4.60 (2H, d, *J* = 6 Hz), 3.48 (2H, s), 3.45 (2H, d, *J* = 16 Hz), 2.90 (2H, d, *J* = 16 Hz), 2.36 (2H, s), 2.24 (6H, s).

**Example 38**

**2-[2-[[4-[6-[2-(4-methylpiperazin-1-yl)-2-oxo-ethoxy]-3-pyridyl]thiazol-2-yl]methylcarbamoyl]indan-2-yl]acetic acid**

Method B was used as described above.

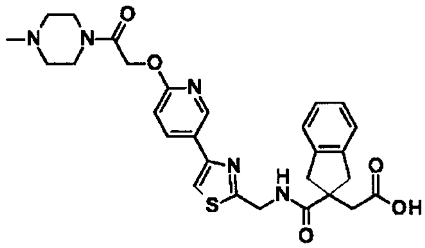

a. Ethyl 2-[(5-acetyl-2-pyridyl)oxy]acetate

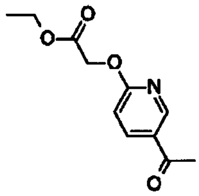

NaH 60% (160mg, 4 mmol) was added to a solution of ethyl glycolate (0.379 mL, 4 mmol) in THF (10 mL) at room temperature. The reaction mixture was stirred at room temperature for 1 h, and a solution of 1-(6-fluoropyridin-3-yl)ethanone (500 mg, 3.59 mmol) in THF (10 mL) was added dropwise. The reaction mixture was stirred at room temperature for 2 h, diluted with EtOAc (20 mL) and washed with water (20 mL) and brine (20 mL). The organic extract was dried and evaporated affording a yellow gum which was used in the next step without further purification (730 mg, crude). M/z 223.9 $(M+H)^+$.

b. 2-[[5-(2-bromoacetyl)-2-pyridyl]oxy]acetic acid

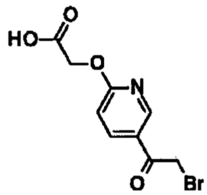

A yellow solid (758 mg, crude) was obtained following the procedure described in example 34 step-a and trituration in $Et_2O$. M/z 273/275 $(M+H)^+$.

c. 2-[[5-[2-[(tert-butoxycarbonylamino)methyl]thiazol-4-yl]-2-pyridyl]oxy]acetic acid

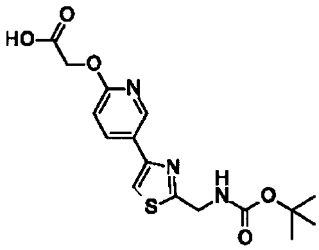

A yellow solid (81 mg, 40%) was obtained following the procedure described in example 34 step-b and purification by chromatography on silica eluting with 0-10% MeOH in DCM M/z 365.9 $(M+H)^+$.

d. Tert-butyl N-[[4-[6-[2-(4-methylpiperazin-1-yl)-2-oxo-ethoxy]-3-pyridyl]thiazol-2-yl]methyl]carbamate

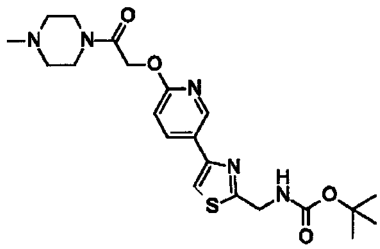

HATU (101 mg, 0.265 mmol), DIPEA (0.115 mL, 0.660 mmol) and 1-methylpiperazine (0.050 mL, 0.451 mmol) were added to a solution of 2-[[5-[2-[(tert-butoxycarbonylamino)methyl]thiazol-4-yl]-2-pyridyl]oxy]acetic acid (81 mg, 0.222 mmol) in DCM (4 mL) at room temperature. The reaction mixture was stirred at room temperature overnight, diluted with DCM (10 mL) and washed with water (10 mL) and brine (10 mL). The organic extract was dried and evaporated. The crude product was purified by chromatography on silica eluting with 0-10% MeOH in DCM affording a beige solid (76 mg, 76% yield). M/z 448 $(M+H)^+$.

e. 2-[2-[[4-[6-[2-(4-methylpiperazin-1-yl)-2-oxo-ethoxy]-3-pyridyl]thiazol-2-yl]methylcarbamoyl]indan-2-yl]acetic acid

The title product was obtained following the procedure described in example 1 step-b to step-d as white solid after purification by preparative hplc (MDAP) (30.3 mg, 53%). M/z 550.3 $(M+H)^+$. $^1H$ NMR (d6-DMSO) $\delta$ 12.01 (1H, bs), 8.65 (2H, m), 8.20 (1H, m), 7.93 (1H, s), 7.20 (2H, m), 7.11 (2H, m), 6.94 (1H, m), 5.09 (2H, s), 4.59 (2H, d, *J* = 6 Hz), 3.50-3.21 (6H, m), 3.00 (2H, d, *J* = 16 Hz), 2.70 (2H, s), 2.39-2.25 (4H, m), 2.21 (3H, s).

**Example 39**

**2-[2-[[4-[4-[[2-(dimethylamino)acetyl]amino]phenyl]thiazol-2-yl]methylcarbamoyl]indan-2-yl]acetic acid**

Method B-3 was used as described above.

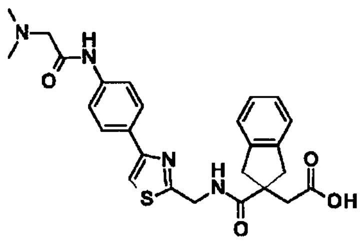

a. Tert-butyl N-[[4-(4-nitrophenyl)thiazol-2-yl]methyl]carbamate

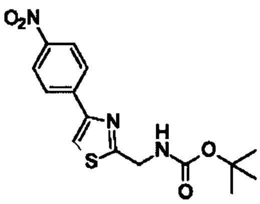

An orange solid (661 mg, 48%) was obtained from 2-bromo-4'-nitroacetophenone following the procedure described in example 34 step-b and purification by chromatography on silica eluting with 0-100% EtOAc in hexane. M/z 336 $(M+H)^+$.

b. Tert-butyl N-[[4-(4-aminophenyl)thiazol-2-yl]methyl]carbamate

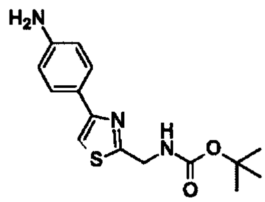

Pd/C (10%, 30 mg) was added to a solution of tert-butyl N-[[4-(4-nitrophenyl)thiazol-2-yl]methyl]carbamate (100 mg, 0.298 mmol) in EtOH (10 mL) at room temperature. The reaction mixture was purged with $H_2$, stirred under $H_2$ atmosphere for 3 h, filtered through celite and the filtrate was evaporated affording a yellow oil (915 mg, 100%). M/z 306 $(M+H)^+$.

c. Tert-butyl N-[[4-[4-[[2-(dimethylamino)acetyl]amino]phenyl]thiazol-2-yl]methyl]carbamate

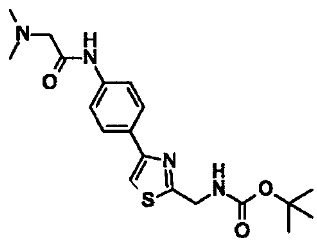

A solution of HATU (136 mg, 0.358 mmol) in DMF (1 mL) was added slowly to a solution of tert-butyl N-[[4-(4-aminophenyl)thiazol-2-yl]methyl]carbamate (91 mg, 0.298 mmol), N,N-dimethylglycine (34 mg, 0.330 mmol) and DIEPA (0.104 mL, 0.597 mmol) in DMF (4 mL) at room temperature. The reaction mixture was stirred at room temperature for 3 h, evaporated. The residue was diluted with EtOAc (5 mL), washed with water (5 mL) and saline (5 mL), dried and evaporated. The crude product was purified by chromatography on silica eluting with 0-10% MeOH in DCM affording a white solid (50 mg, 45% yield). M/z 391 $(M+H)^+$.

d. 2-[2-[[4-[4-[[2-(dimethylamino)acetyl]amino]phenyl]thiazol-2-yl]methylcarbamoyl]indan-2-yl]acetic acid

The title product was obtained following the procedure described in example 1 step-b to step-d as white solid after purification by preparative hplc (MDAP) (136 mg, 60%). M/z 493.2 $(M+H)^+$. $^1$H NMR (d6-DMSO) $\delta$ 10.1 (1H, bs), 8.66 (1H, m), 7.88 (3H, m), 7.49 (2H, m), 7.20 (2H, m), 7.11 (2H, m), 4.56 (2H, d, *J* = 6 Hz), 3.55-3.45 (4H, m), 3.00 (2H, d, *J* = 16 Hz), 2.75 (2H, s), 2.50 (6H, s).

**Example 40**

**2-[2-[[4-[4-[[2-(trimethylammonio)acetyl]amino]phenyl]thiazol-2-yl]methylcarbamoyl]indan-2-yl] acetate**

Method B-3 was used as described above.

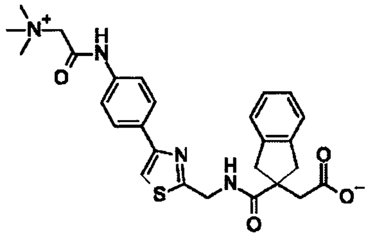

a. [2-[4-[2-[[[2-(2-tert-butoxy-2-oxo-ethyl)indane-2-carbonyl]amino]methyl]thiazol-4-yl]anilino]-2-oxo-ethyl]-trimethyl-ammonium

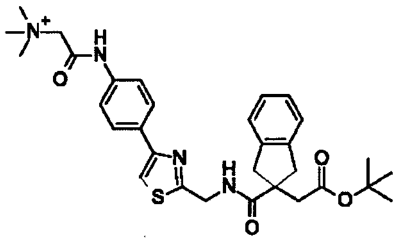

MeI (0.100 mL, 1.61 mmol) was added to a solution of tert-butyl 2-[2-[[4-[4-[[2-(dimethylamino)acetyl]amino]phenyl]thiazol-2-yl]methylcarbamoyl]indan-2-yl]acetate (250 mg, 0.456 mmol) and silver carbonate (150mg, 0.544 mmol) in THF (3 mL) at room temperature. The reaction mixture was stirred at 50°C for 3 h, filtered through celite and concentrated. The residue was triturated with $Et_2O$ affording a brown solid which was used in the next step without further purification (250mg, 97%). M/z 563 $(M)^+$.

b. 2-[2-[[4-[4-[[2-(trimethylammonio)acetyl]amino]phenyl]thiazol-2-yl]methylcarbamoyl]indan-2-yl]acetate

The title product was obtained following the procedure described in example 1 step-d as white solid after purification by preparative hplc (MDAP) (76 mg, 34%). M/z 507.2 $(M+H)^+$. $^1H$ NMR (d6-DMSO) $\delta$ 11.6 (1H, bs), 9.91 (1H, bs), 7.89 (2H, m), 7.84 (1H, s), 7.66 (2H, m), 7.20 (2H, m), 7.11 (2H, m), 4.61 (2H, d, $J$ = 6 Hz), 4.48 (2H, s), 3.45 (2H, d, $J$ = 16 Hz), 3.31 (9H, s), 2.97 (2H, d, $J$ = 16 Hz), 2.71 (2H, s).

Other compounds were prepared following the method B-3 described for Example 39 and 40 using other commercial carboxylic acid reagents in step-c of example 39, and starting with either 2-bromo-4'-nitroacetophenone or 2-bromo-3'-nitroacetophenone purified in a similar manner by preparative HPLC are shown in the Table below, wherein R is the moiety:

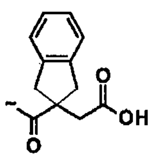

| Example | Structure | Name, MS and NMR data |
|---|---|---|
| **41** | 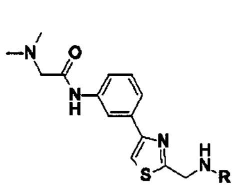 | 2-[2-[[4-[3-[[2-(dimethylamino)acetyl]amino]phenyl]thiazol-2-yl] methylcarbamoyl]indan-2-yl]acetic acid<br>M/z 493.3 $(M+H)^+$<br>$^1$H NMR (d6-DMSO) δ 12.0 (1H, bs), 9.99 (1H, bs), 8.69 (1H, m), 8.20 (1H, m), 7.90 (1H, s), 7.62 (2H, m), 7.36 (1H, m), 7.20 (2H, m), 7.11 (2H, m), 4.59 (2H, d, *J* = 6 Hz), 3.47 (2H, d, *J* = 16 Hz), 3.31 (2H, m), 3.00 (2H, d, *J* = 16 Hz), 2.69 (2H, s), 2.43 (6H, s). |
| 42 | 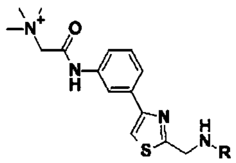 | 2-[2-[[4-[3-[[2-(trimethylammonio)acetyl]amino]phenyl]thiazol-2-yl] methylcarbamoyl]indan-2-yl] acetate<br>M/z 507.2 $(M+H)^+$<br>$^1$H NMR (d6-DMSO) δ 11.28 (1H, bs), 10.21 (1H, bs), 8.22 (1H, m), 7.91 (1H, s), 7.64 (2H, m), 7.39 (1H, m), 7.20 (2H, m), 7.11 (2H, m), 4.60 (2H, d, *J* = 6 Hz), 4.49 (2H, s), 3.45 (2H, d, *J* = 16 Hz), 3.32 (9H, s), 2.98 (2H, d, *J* = 16 Hz), 2.60 (2H, s). |
| **43** | 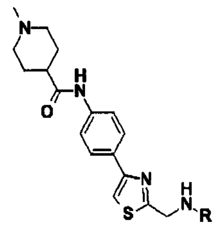 | 2-[2-[[4-[4-[(1-methylpiperidine-4-carbonyl)amino]phenyl]thiazol-2-yl] methylcarbamoyl]indan-2-yl]acetic acid<br>M/z 533.2 $(M+H)^+$<br>$^1$H NMR (500 MHz, DMSO-$d_6$): δ 9.95 (1H, s), 8.77 (1H, bs), 7.83 (2H, d, J = 8.5 Hz), 7.83 (1H, s), 7.65 (2H, d, J = 8.5 Hz), 7.21-7.20 (2H, m), 7.14-7.12 (2H, m), 4.57 (2H, d, J = 5.5 Hz), 3.45 (2H, d, J = 16.5 Hz), 2.98 (2H, d, J = 16.5 Hz), 2.86-2.84 (2H, m), 2.73 (2H, s), 2.36-2.27 (1H, m), 2.19 (3H, s), 1.93 (2H, t, J = 6.5 Hz), 1.77-1.74 (2H, m), 1.70-1.63 (2H, m). |
| **44** | 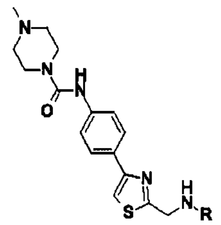 | 2-[2-[[4-[4-[(4-methylpiperazine-1-carbonyl)amino] phenyl]thiazol-2-yl] methylcarbamoyl]indan-2-yl]acetic acid<br>M/z 534.2 $(M+H)^+$<br>$^1$H NMR (500 MHz, DMSO-$d_6$): δ 9.67 (1H, bs), 8.60 (1H, s), 7.78 (2H, d, J = 8.5 Hz), 7.77 (1H, s), 7.52 (2H, d, J = 8.5 Hz), 7.20-7.18 (2H, m), 7.13-7.11 (2H, m), 4.57 (2H, d, J = 6.0 Hz), 3.45-3.48-3.43 (6H, m), 2.96 (2H, d, J = 16.5 Hz), 2.64 (2H, s), 2.32-2.30 (4H, m), 2.20 (3H, s). |
| **45** | 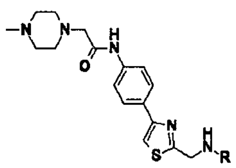 | 2-[2-[[4-[4-[[2-(4-methylpiperazin-1-yl)acetyl]amino]phenyl]thiazol-2-yl] methylcarbamoyl]indan-2-yl]acetic acid<br>M/z 548.2 $(M+H)^+$<br>$^1$H NMR (500 MHz, DMSO-$d_6$): δ 9.82 (1H, bs), 8.65 (1H, t, J = 5.5 Hz), 7.86 (2H, d, J = 8.0 Hz), 7.85 (1H, s), 7.68 (2H, d, J = 8.0 Hz), 7.21-7.20 (2H, m), 7.14-7.12 (2H, m), 4.57 (2H, d, J = 6.0 Hz), 3.46 (2H, d, J = 16.5 Hz), 3.20 (2H, s), 2.99 (2H, d, J = 16.5 Hz), 2.90-2.78 (4H, m), 2.75 (2H, s), 2.74-2.60 (4H, m), 2.49 (3H, bs). |

**Example 46**

**2-[2-[[4-[4-[2-(4-methylpiperazin-1-yl)-2-oxo-ethoxy]phenyl]thiazol-2-yl]methylcarbamoyl]indan-2-yl]acetic acid**

Method B-4 was used as described above.

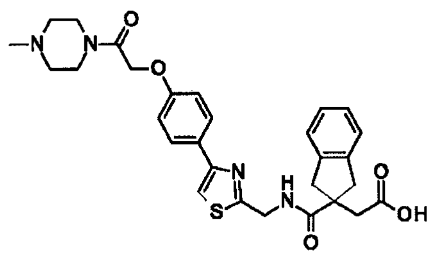

a. Tert-butyl N-[[4-(4-hydroxyphenyl)thiazol-2-yl]methyl]carbamate

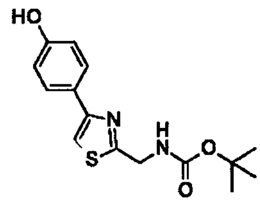

To a solution of 2-bromo-1-(4-hydroxyphenyl)ethan-1-one (1.5 g, 6.97 mmol) in EtOH (30 mL) was added tert-butyl (2-amino-2-thioxoethyl)carbamate (1.3 g, 6.97 mmol) at room temperature. The mixture was stirred 12 h then concentrated under reduced pressure. The residue was purified by silica gel chromatography eluting with 30-40% EtOAc in petroleum ether affording an off white solid (900 mg, 42%). M/z 307.1 $(M+H)^+$.

b. Tert-butyl N-[[4-[4-[2-(4-methylpiperazin-1-yl)-2-oxo-ethoxy]phenyl]thiazol-2-yl]methyl]carbamate

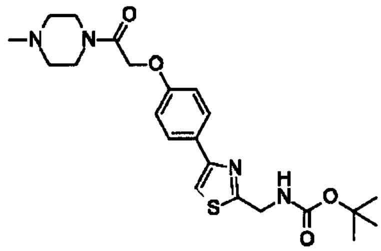

To a solution of tert-butyl N-[[4-(4-hydroxyphenyl)thiazol-2-yl]methyl]carbamate (250 mg, 0.81 mmol) in acetone (10 mL) was added potassium carbonate (281 mg, 2.04 mmol) and 2-chloro-1-(4-methylpiperazin-1-yl)ethan-1-one hydrochloride (192 mg, 0.89 mmol) at room temperature. The mixture was heated at 65 °C for 12 h, then filtered through celite pad which was washed with EtOAc (10 mL). The filtrate was concentrated and the residue was purified by silica gel chromatography eluting with 5-15% MeOH in DCM affording an off white solid (260 mg, 72%). M/z 447.2 $(M+H)^+$.

c. 2-[4-[2-(aminomethyl)thiazol-4-yl]phenoxy]-1-(4-methylpiperazin-1-yl)ethenone hydrochloride

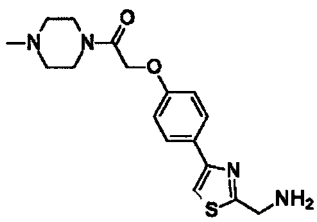

To a solution of tert-butyl N-[[4-[4-[2-(4-methylpiperazin-1-yl)-2-oxo-ethoxy]phenyl]thiazol-2-yl]methyl]carbamate (240 mg, 0.53 mmol) in dioxane (5 mL) was added 4M HCl in dioxane (2 mL) at room temperature. The mixture was stirred at room temperature for 6 h and concentrated under reduced pressure. The residue was triturated with diethyl ether (15 mL) affording an off white solid (280 mg, crude). M/z 346.9 $(M+H)^+$.

d. Tert-butyl 2-[2-[[4-[4-[2-(4-methylpiperazin- 1-yl)-2-oxo-ethoxy]phenyl]thiazol-2-yl]methylcarbamoyl]indan-2-yl]acetate

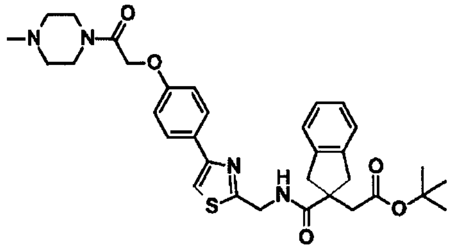

To a solution of 2-[4-[2-(aminomethyl)thiazol-4-yl]phenoxy]-1-(4-methylpiperazin-1-yl)ethenone hydrochloride (275 mg, 0.72 mmol) in DMF (5 mL) was added DIPEA (0.4 mL, 2.1 mmol) and stirred for 10 minutes. Then 2-(2-(tert-butoxy)-2-oxoethyl)-2,3-dihydro-1H-indene-2-carboxylic acid (219 mg, 0.79 mmol), EDC. HCl (206 mg, 1.08 mmol) and HOBt (98 mg, 0.72 mmol) was added at room temperature. The mixture was stirred for 12 h and diluted with cold water (15 mL) and stirred for 30 minutes. The resulting precipitate was filtered, washed with water (5 mL) and dried in vacuum affording a pale yellow solid (220 mg, 76%). M/z 605.3 $(M+H)^+$.

e. 2-[2-[[4-[4-[2-(4-methylpiperazin-1-yl)-2-oxo-ethoxy]phenyl]thiazol-2-yl]methylcarbamoyl]indan-2-yl]acetic acid

To a solution of tert-butyl 2-[2-[[4-[4-[2-(4-methylpiperazin-1-yl)-2-oxo-ethoxy]phenyl]thiazol-2-yl]methylcarbamoyl]indan-2-yl]acetate (120 mg, 0.19 mmol) in DCM (5 mL) was added TFA (2 mL) at 0 °C and stirred at room temperature for 4 h. The mixture was evaporated and the residue was triturated with diethyl ether (10 mL). The crude compound was purified by preparative HPLC [X-BRIDGE-C18 (150*30), 5 u, Mobile phase: A: 0.1% Formic Acid in $H_2O$, B: MeCN] affording the title compound as an off white solid (45 mg, 42%). M/z 549.2 $(M+H)^+$. $^1H$ NMR (500 MHz, DMSO-$d_6$): δ 9.01 (1H, bs), 7.82 (2H, d, J = 8.5 Hz), 7.77 (1H, s), 7.21-7.19 (2H, m), 7.14-7.12 (2H, m), 6.96 (2H, d, J = 8.5 Hz), 4.84 (2H, s), 4.58 (2H, d, J = 5.5 Hz), 3.46-3.43 (6H, m), 2.97 (2H, d, J = 16.5 Hz), 2.71 (2H, s), 2.33 (2H, bs), 2.26 (2H, bs), 2.18 (3H, s).

**Example 47**

**2-[2-[[4-[4-[2-(4,4-dimethylpiperazin-4-ium-1-yl)-2-oxo-ethoxy]phenyl]thiazol-2-yl]methylcarbamoyl]indan-2-yl]acetate**

Method B-4 was used as described above.

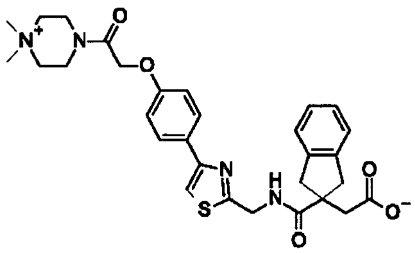

a. Tert-butyl 2-[2-[[4-[4-[2-(4,4-dimethylpiperazin-4-ium-1-yl)-2-oxo-ethoxy]phenyl]thiazol-2-yl]methylcarbamoyl]indan-2-yl]acetate

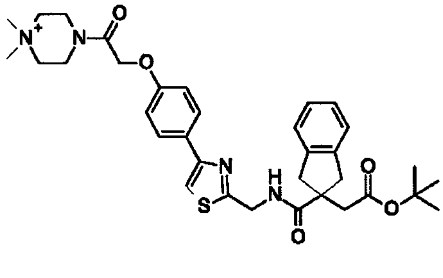

To a solution of tert-butyl 2-[2-[[4-[4-[2-(4-methylpiperazin-1-yl)-2-oxo-ethoxy]phenyl]thiazol-2-yl]methylcarbamoyl]indan-2-yl]acetate (70 mg, 0.11 mmol) in acetonitrile (4 mL) was added MeI (25 mg, 0.17 mmol) at 0 °C and stirred at room temperature for 4 h. The mixture was evaporated and the residue was triturated with diethyl ether (10 mL) affording a pale brown solid (90 mg, crude). M/z 619.3 $(M)^+$.

b. 2-[2-[[4-[4-[2-(4,4-dimethylpiperazin-4-ium-1-yl)-2-oxo-ethoxy]phenyl]thiazol-2-**yl]methylcarbamoyl]indan-2-yl]acetate**

A solution of Tert-butyl 2-[2-[[4-[4-[2-(4,4-dimethylpiperazin-4-ium-1-yl)-2-oxo-ethoxy]phenyl]thiazol-2-yl]methylcarbamoyl]indan-2-yl]acetate (90 mg, 0.14 mmol) in DCM (5 mL) was treated with TFA (1.5 mL) at 0 °C and stirred at room temperature for 4 h. The mixture was evaporated and the residue was triturated with diethyl ether (10 mL). The crude compound was purified by preparative HPLC [X-BRIDGE-C18 (150*30), 5 u, Mobile phase: A: 0.1% Formic Acid in $H_2O$, B: MeCN] affording the title compound as an off white solid (30 mg, 46% from two steps). M/z 563.2 $(M+H)^+$. $^1H$ NMR (500 MHz, DMSO-$d_6$): δ 12.59 (1H, bs), 7.86 (2H, d, J = 8.5 Hz), 7.80 (1H, s), 7.15-7.10 (4H, m), 7.00 (2H, d, J = 8.5 Hz), 4.93 (2H, s), 4.58 (2H, d, J = 5.0 Hz), 3.88-3.79 (4H, m), 3.48 (2H, m), 3.40-3.35 (4H, obs), 3.17 (6H, s), 2.88 (2H, d, J = 16.0 Hz), 2.37 (2H, s).

Other compounds were prepared following the method B-4 described for Example 46 and 47 starting with either 2-bromo-1-(4-hydroxyphenyl)ethan-1-one or 2-bromo-1-(3-hydroxyphenyl)ethanone using other commercial chlorinated reagents in example 46 step-b and purified in a similar manner by preparative HPLC are shown in the Table below, wherein R is the moiety:

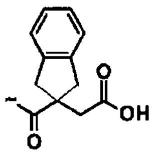

| Example | Structure | Name, MS and NMR data |
|---|---|---|
| **48** | 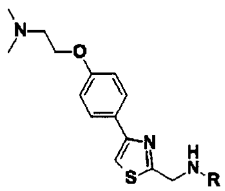 | 2-[2-[[4-[4-[2-(dimethylamino)ethoxy]phenyl]thiazol-2-yl]methylcarbamoyl]indan-2-yl]acetic acid<br>M/z 480.2 $(M+H)^+$<br>$^1H$ NMR (500 MHz, DMSO-$d_6$): δ 8.82 (1H, bs), 7.83 (2H, d, J = 8.5 Hz), 7.78 (1H, s), 7.21-7.20 (2H, m), 7.14-7.12 (2H, m), 6.98 (2H, d, J = 8.5 Hz), 4.56 (2H, d, J = 6.0 Hz), 4.08 (2H, t, J = 5.5 Hz), 3.46 (2H, d, J = 16.0 Hz), 2.98 (2H, d, J = 16.0 Hz), 2.73 (2H, s), 2.64 (2H, t, J = 5.5 Hz), 2.22 (6H, s). |
| **49** | 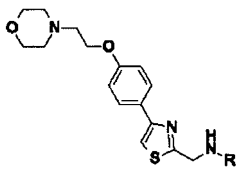 | 2-[2-[[4-[4-(2-morpholinoethoxy)phenyl]thiazol-2-yl]methylcarbamoyl]indan-2-yl]acetic acid<br>M/z 522.1 $(M+H)^+$<br>$^1H$ NMR (500 MHz, MeOD): δ 7.81 (2H, d, J = 8.5 Hz), 7.51 (1H, s), 7.21-7.20 (2H, m), 7.15-7.14 (2H, m), 7.01 (2H, d, J = 8.5 Hz), 4.70 (2H, s), 4.27 (2H, t, J = 5.0 Hz), 3.82-3.80 (4H, m), 3.52 (2H, d, J = 16.0 Hz), 3.15 (2H, t, J = 5.0 Hz), 3.10 (2H, d, J = 16.0 Hz), 2.98-2.92 (4H, m), 2.82 (2H, s). |
| **50** | 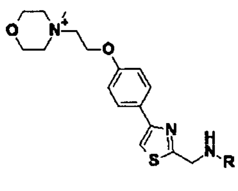 | 2-[2-[[4-[4-[2-(4-methylmorpholin-4-ium-4-yl)ethoxy]phenyl]thiazol-2-yl]methylcarbamoyl]indan-2-yl]acetate<br>M/z 536.1 $(M+H)^+$<br>$^1H$ NMR (500 MHz, DMSO-$d_6$): δ 10.47 (1H, bs), 7.90 (2H, d, J = 9.0 Hz), 7.83 (1H, s), 7.19-7.17 (2H, m), 7.12-7.11 (2H, m), 7.07 (2H, d, J = 9.0 Hz), 4.56 (2H, d, J = 5.5 Hz), 4.53 (2H, t, J = 5.0 Hz), 3.97-3.95 (6H, m), 3.61-3.57 (2H, m), 3.55-3.51 (2H, m), 3.43 (2H, d, J = 16.0 Hz), 3.28 (3H, s), 2.94 (2H, d, J = 16.0 Hz), 2.58 (2H, s). |

(continued)

| Example | Structure | Name, MS and NMR data |
|---|---|---|
| **51** | 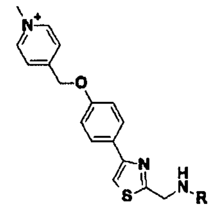 | 2-[2-[[4-[4-[(1-methylpyridin-1-ium-4-yl)methoxy]phenyl]thiazol-2-yl] methylcarbamoyl]indan-2-yl]acetate<br>M/z 514.1 (M+H)+<br>$^1$H NMR (500 MHz, MeOD): δ 8.87 (2H, d, J = 6.5 Hz), 8.15 (2H, d, J = 6.5 Hz), 7.85-7.83 (2H, m), 7.54 (1H, s), 7.20-7.17 (2H, m), 7.14-7.12 (2H, m), 7.10-7.08 (2H, m), 5.49 (2H, s), 4.71 (2H, s), 4.39 (3H, s), 3.52 (2H, d, J = 16.0 Hz), 3.08 (2H, d, J = 16.0 Hz), 2.77 (2H, s). |
| **52** | 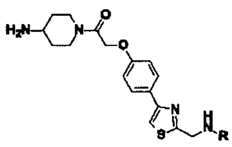 | 2-[2-[[4-[4-[2-(4-amino-1-piperidyl)-2-oxo-ethoxy]phenyl]thiazol-2-yl] methylcarbamoyl]indan -2-yl]acetic acid<br>M/z 549.2 (M+H)+<br>$^1$H NMR (500 MHz, DMSO-$d_6$): δ 9.47 (1H, bs), 7.83 (2H, d, J = 9.0 Hz), 7.79 (1H, s), 7.20-7.18 (2H, m), 7.13-7.11 (2H, m), 6.95 (2H, d, J = 9.0 Hz), 4.89 (1H, d, J = 14.5 Hz), 4.80 (1H, d, J = 14.5 Hz), 4.57 (2H, d, J = 5.5 Hz), 4.29-4.27 (1H, m), 3.88-3.84 (1H, m), 3.44 (2H, d, J = 16.5 Hz), 3.13-3.08 (2H, m), 2.99 (2H, d, J = 16.5 Hz), 2.70-2.67 (1H, bs), 2.66 (2H, s), 1.89-1.83 (2H, m), 1.45-1.43 (1H, m), 1.28-1.24 (1H, m). |
| **53** | 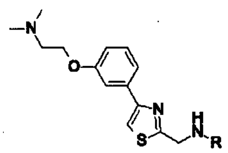 | 2-[2-[[4-[3-[2-(dimethylamino)ethoxy]phenyl]thiazol-2-yl]methylcarbamoyl] indan-2-yl]acetic acid<br>M/z 480.2 (M+H)+<br>$^1$H NMR (500 MHz, DMSO-$d_6$): δ 8.96 (1H, bs), 8.00 (1H, s), 7.50 (1H, d, J = 8 Hz), 7.49 (1H, s), 7.32 (1H, t, J = 8 Hz), 7.21-7.19 (2H, m), 7.14-7.13 (2H, m), 6.90 (1H, d, J = 8 Hz), 4.58 (2H, d, J = 5.5 Hz), 4.10 (2H, t, J = 5.5 Hz), 3.45 (2H, d, J = 16.5 Hz), 3.00 (2H, d, J = 16.5 Hz), 2.72 (2H, s), 2.65 (2H, t, J = 6 Hz), 2.23 (6H, s). |
| **54** | 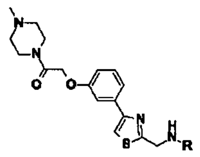 | 2-[2-[[4-[3-[2-(4-methylpiperazin-1-yl)-2-oxo-ethoxy]phenyl]thiazol-2-yl] methylcarbamoyl]indan-2-yl]acetic acid<br>M/z 547.2 (M+H)+<br>$^1$H NMR (500 MHz, MeOD): δ 7.68 (1H, s), 7.51 (1H, d, J = 2.0 Hz), 7.49 (1H, d, J = 7.5 Hz), 7.33 (1H, dd, J = 8 Hz, J = 7.5 Hz), 7.22-7.20 (2H, m), 7.16-7.14 (2H, m), 6.96-6.94 (1H, dd, J = 7.5 Hz, J = 2.0 Hz), 4.89 (2H, s), 4.71 (2H, s), 3.79 (4H, bs), 3.52 (2H, d, J = 16.5Hz), 3.10 (2H, d, J = 16.5Hz), 3.10-2.95 (4H, m), 2.83 (2H, s), 2.71 (3H, s). |

**Example 55**

**2-[2-[[4-[4-(3-morpholinopropoxy)phenyl]thiazol-2-yl]methylcarbamoyl]indan-2-yl]acetic acid**

Method B-4 was used as described above.

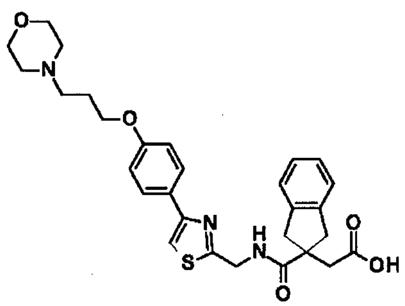

a. Tert-butyl N-[[4-[4-(3-bromopropoxy)phenyl]thiazol-2-yl]methyl]carbamate

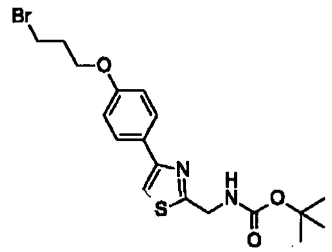

To a solution of tert-butyl N-[[4-(4-hydroxyphenyl)thiazol-2-yl]methyl]carbamate (1.0 g, 3.26 mmol) in acetonitrile (20 mL) was added $K_2CO_3$ (0.9 g, 6.50 mmol) at room temperature and stirred for 10 minutes. Then, 1,3-dibromopropane (1.0 g, 4.90 mmol) was added at room temperature and refluxed for 16 h. The reaction mixture was filtered through celite pad, which was washed with EtOAc (20 mL) and filtrate was evaporated. The residue was purified by silica gel chromatography eluting with 15-20% EtOAc in petroleum ether affording a pale yellow solid (600 mg, 43%). M/z 427.1 $(M+H)^+$.

b. Tert-butyl N-[[4-[4-(3-morpholinopropoxy)phenyl]thiazol-2-yl]methyl]carbamate

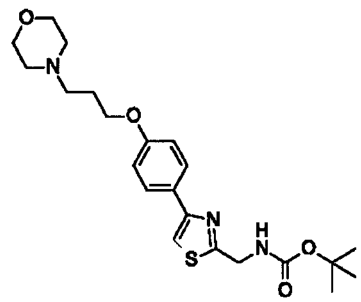

To a solution of tert-butyl N-[[4-[4-(3-bromopropoxy)phenyl]thiazol-2-yl]methyl]carbamate (300 mg, 0.74 mmol) in acetonitrile (15 mL) was added $K_2CO_3$ (145 mg, 1.05 mmol) and morpholine (73.5 mg, 0.84 mmol) at room temperature. The mixture was refluxed for 16 h. Then filtered through celite pad which was washed with DCM (10 mL). The filtrate was concentrated and the residue was purified by silica gel chromatography eluting with 10-15% MeOH in DCM affording an off white solid (210 mg, 70%). M/z 434.2 $(M+H)^+$.

c. 2-[2-[[4-[4-[2-(4-methylpiperazin-1-yl)-2-oxo-ethoxy]phenyl]thiazol-2-yl]methylcarbamoyl]indan-2-yl]acetic acid

The title product was obtained following the procedure described in example 46 step-c to step-e. The crude compound was purified by preparative HPLC [SYMMETRY-C 18 (300*19), 7 u, Mobile phase: A: 0.1% Formic Acid in $H_2O$, B: MeCN] affording an off white solid (67 mg, 67%). M/z 536.1 $(M+H)^+$. $^1$HNMR (500 MHz, MeOD): $\delta$ 7.78 (2H, d, J = 8.5 Hz), 7.49 (1H, s), 7.21-7.20 (2H, m), 7.15-7.13 (2H, m), 6.96 (2H, d, J = 8.5 Hz), 4.70 (2H, s), 4.11 (2H, t, J = 5.5 Hz), 3.81 (4H, bs), 3.52 (2H, d, J = 16.5 Hz), 3.08 (2H, d, J = 16.5 Hz), 3.00-2.81 (6H, m), 2.82 (2H, s), 2.14-2.11 (2H, m).

**Example 56**

**2-[2-[[4-[4-[3-(4-methylmorpholin-4-ium-4-yl)propoxy]phenyl]thiazol-2-yl]methylcarbarmoyl]indan-2-yl]acetate**

Method B-4 was used as described above.

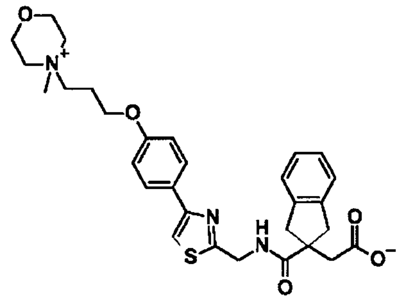

The title product was obtained following the procedure described in example 47. The crude compound was purified by preparative HPLC [X-BRIDGE-C18 (150*30), 5 u, Mobile phase: A: 0.1% Formic Acid in $H_2O$, B: MeCN] affording an off white solid (47 mg, 47%). M/z 550.2 $(M+H)^+$. $^1$HNMR (500 MHz, DMSO-$d_6$): δ 7.88 (2H, d, J = 8.5 Hz), 7.80 (1H, s), 7.18-7.17 (2H, m), 7.13-7.10 (2H, m), 7.01 (2H, d, J = 8.5 Hz), 4.57 (2H, d, J = 6.0 Hz), 4.12 (2H, t, J = 5.5 Hz), 3.93-3.90 (4H, m), 3.66-3.63 (2H, m), 3.47 (4H, bs), 3.41 (2H, d, J = 16.0 Hz), 3.17 (3H, s), 3.92 (2H, d, J = 16.0 Hz), 2.52 (2H, s), 2.24-2.20 (2H, m).

Other compounds were prepared following the method B-4 described for Example 55 and 56 using other commercial amine reagents or pyridine in example 55 step-b and starting from either tert-butyl N-[[4-(4-hydroxyphenyl)thiazol-2-yl]methyl]carbamate or tert-butyl N-[[4-(4-hydroxy-3-methylsulfonyl-phenyl)thiazol-2-yl]methyl]carbamate purified in a similar manner by preparative HPLC are shown in the Table below, wherein R is the moiety:

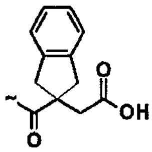

| Example | Structure | Name, MS and NMR data |
|---|---|---|
| **57** | 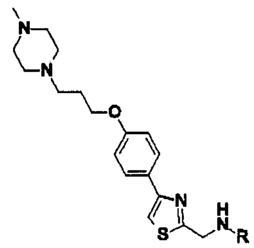 | 2-[2-[[4-[4-[3-(4-methylpiperazin-1-yl)propoxy]phenyl]thiazol-2-yl] methylcarbamoyl]indan-2-yl]acetic acid<br>M/z 549.2 $(M+H)^+$<br>$^1$H NMR (500 MHz, MeOD): δ 7.77 (2H, d, J = 8.5 Hz), 7.49 (1H, s), 7.19-7.18 (2H, m), 7.13-7.12 (2H, m), 6.94 (2H, d, J = 8.5 Hz), 4.71 (2H, s), 4.07 (2H, t, J = 6.0 Hz), 3.52 (2H, d, J = 16.0 Hz), 3.08 (2H, d, J = 16.0 Hz), 2.87-2.77 (10H, bs), 2.68 (2H, t, J = 7.5 Hz), 2.51 (3H, s), 2.02-2.00 (2H, m). |
| **58** | 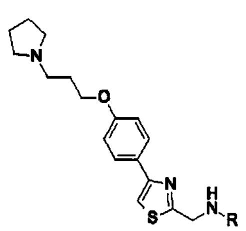 | 2-[2-[[4-[4-(3-pyrrolidin-1-ylpropoxy)phenyl]thiazol-2-yl]methylcarbamoyl] indan-2-yl]acetic acid<br>M/z 520.2 $(M+H)^+$<br>$^1$H NMR (400 MHz, DMSO-$d_6$): δ 8.85 (1H, bs), 7.83 (2H, d, J = 8.8 Hz), 7.78 (1H, s), 7.21-7.19 (2H, m), 7.15-7.12 (2H, m), 6.97 (2H, d, J = 8.8 Hz), 4.57 (2H, d, J = 6.0 Hz), 4.04 (2H, t, J = 6.4 Hz), 3.45 (2H, d, J = 16 Hz), 2.98 (2H, d, J = 16 Hz), 2.72 (2H, s), 2.61 (2H, t, J = 6.8 Hz), 2.54-2.50 (4H, bs), 1.93-1.85 (2H, m), 1.72-1.68 (4H, m). |
| **59** | 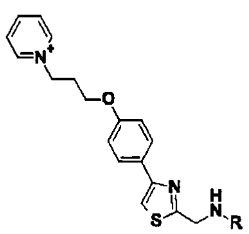 | 2-[2-[[4-[4-(3-pyridm-1-ium-1-ylpropoxy)phenyl]thiazol-2-yl] methylcarbamoyl]indan-2-yl]acetate<br>M/z 528.1 $(M+H)^+$<br>$^1$H NMR (500 MHz, DMSO-$d_6$): δ 9.14 (2H, d, J = 5.5 Hz), 8.62 (1H, t, J = 8 Hz), 8.16 (2H, t, J = 6.5 Hz), 7.83 (2H, d, J = 9 Hz), 7.78 (1H, s), 7.16-7.14 (2H, m), 7.11-7.09 (2H, m), 6.81 (2H, d, J = 9 Hz), 4.81 (2H, t, J = 6.5 Hz), 4.57 (2H, d, J = 6 Hz), 4.14 (2H, t, J = 6 Hz), 3.39 (2H, d, J = 16 Hz), 2.88 (2H, d, J = 16 Hz), 2.45-2.40 (2H, m), 2.38 (2H, s). |

(continued)

| Example | Structure | Name, MS and NMR data |
|---|---|---|
| 60 | 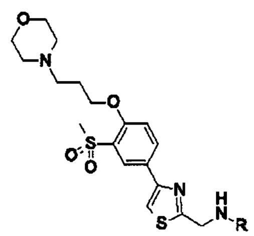 | 2-[2-[[4-[3-methylsulfonyl-4-(3-morpholinopropoxy)phenyl]thiazol-2-yl] methylcarbamoyl]indan-2-yl]acetic acid<br>M/z 614.1 (M+H)+<br>$^1$H NMR (500 MHz, DMSO-$d_6$): δ 12.14 (1H, bs), 9.61 (1H, bs), 8.67 (1H, t, J = 6.0 Hz), 8.38 (1H, d, J = 2 Hz), 8.21 (1H, d, J = 8.5 Hz), 8.01 (1H, s), 7.38 (1H, d, J = 8.5 Hz), 7.22-7.20 (2H, m), 7.15-7.12 (2H, m), 4.59 (2H, d, J = 6 Hz), 4.34-4.27 (2H, bs), 4.08-3.97 (2H, bs), 3.75-3.55 (4H, bs), 3.45 (2H, d, J = 16.5 Hz), 3.32 (7H, bs), 3.15-3.05 (2H, bs), 2.99 (2H, d, J = 16.5 Hz), 2.25-2.18 (2H, bs). |
| 61 | 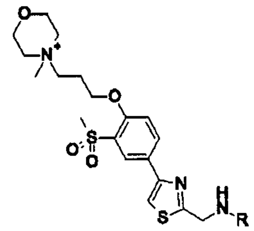 | 2-[2-[[4-[4-[3-(4-methylmorpholin-4-ium-4-yl)propoxy]-3-methylsulfonyl-phenyl]thiazol-2-yl]methylcarbamoyl]indan-2-yl]acetate<br>M/z 628.1 (M+H)+<br>$^1$H NMR (500 MHz, DMSO-$d_6$): δ 12.25 (1H, bs), 8.38 (1H, d, J = 2.5 Hz), 8.24 (1H, dd, J = 8.5 Hz, J = 2.5 Hz), 8.02 (1H, s), 7.38 (1H, d, J = 8.5 Hz), 7.20-7.19 (2H, m), 7.14-7.11 (2H, m), 4.59 (2H, d, J = 5.5 Hz), 4.33 (2H, t, J = 5.5 Hz), 3.96-3.93 (4H, m), 3.69-3.66 (2H, m), 3.49-3.42 (6H, m), 3.41 (3H, s), 3.18 (3H, s), 2.95 (2H, d, J = 16 Hz), 2.63 (2H, s), 2.32-2.27 (2H, m). |

**Example 62**

**2-[5,6-difluoro-2-[[4-[4-[3-(4-methylmorpholin-4-ium-4-yl)propoxy]-3-methylsulfonyl-phenyl]thiazol-2-yl]methylcarbamoyl]indan-2-yl]acetate**

Method B-4 was used as described above.

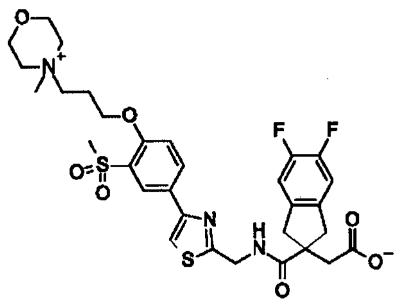

The title product was obtained following the procedure described in example 56 starting from tert-butyl N-[[4-(4-hydroxy-3-methylsulfonyl-phenyl)thiazol-2-yl]methyl]carbamate and using 2-[(tert-butoxy)carbonyl]-5,6-difluoro-2,3-dihydro-1H-indene-2-carboxylic acid in the peptide coupling step. The crude compound was purified by preparative HPLC [X-BRIDGE-C18 (150*30), 5 u, Mobile phase: A: 0.1% Formic Acid in $H_2O$, B: MeCN] affording an off white solid. M/z 664.1 (M+H)+. $^1$H NMR (500 MHz, DMSO-$d_6$): δ 11.01 (1H, bs), 8.39 (1H, d, J = 2 Hz), 8.24 (1H, dd, J = 8.5 Hz, J = 2 Hz), 8.02 (1H, s), 7.39 (1H, d, J = 8.5 Hz), 7.24 (2H, t, J = 9 Hz), 4.58 (2H, d, J = 6 Hz), 4.33 (2H, t, J = 5.5 Hz), 3.96-3.93 (4H, m), 3.70-3.66 (2H, m), 3.52-3.48 (4H, bs), 3.47-3.44 (2H, bs), 3.40 (3H, s), 3.25 (3H, s), 2.92 (2H, d, J = 16.5 Hz), 2.55 (2H, s), 2.32-2.29 (2H, m).

**Example 63**

**2-[2-[[4-[2-methoxy-4-[2-(4-methylpiperazin-1-yl)-2-oxo-ethoxy]phenyl]thiazol-2-yl]methylcarbamoyl]indan-2-yl]acetic acid**

Method B-4 was used as described above.

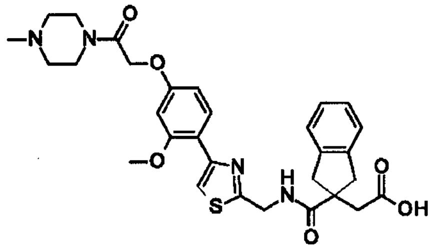

a. Tert-butyl N-[[4-[2-methoxy-4-(4,4,5,5-tetramethyl-1,3,2-dioxaborolan-2-yl)phenyl]thiazol-2-yl]methyl]carbamate

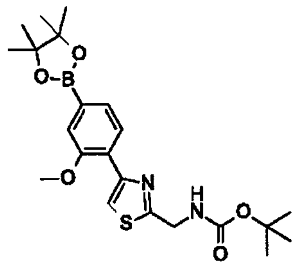

Bis(pinacolato)diboron (400 mg, 1.58 mmol), [1,1'-Bis(diphenylphosphino)ferrocene]dichloropalladium (II) complex in DCM (90 mg, 0.110 mmol) and potassium acetate (370 mg, 3.77 mmol) were added to a solution of 1-(5-bromo-2-methoxyphenyl)sulfonyl-N,N-dimethyl-piperidin-4-amine (118 mg, 0.312 mmol) in dioxane (15 mL) at room temperature. The reaction mixture was flushed with $N_2$, heated at 100°C, stirred for 1.5 h under microwave, cooled to room temperature, diluted in EtOAc (20 mL), filtered through celite, and the filtrate was evaporated. The crude product was purified by chromatography on silica eluting with 0-40% EtOAc in hexane affording a white solid (503 mg, 90% yield). M/z 447 $(M+H)^+$.

b. Tert-butyl N-[[4-(4-hydroxy-2-methoxy-phenyl)thiazol-2-yl]methyl]carbamate

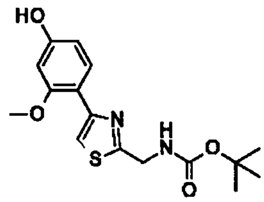

Hydrogen peroxide solution (30%, 1.8 mL, 17.8 mmol) was added to a solution of tert-butyl N-[[4-[2-methoxy-4-(4,4,5,5-tetramethyl-1,3,2-dioxaborolan-2-yl)phenyl]thiazol-2-yl]methyl]carbamate (500 mg, 1.12 mmol) in MeOH (12 mL) at room temperature. The reaction mixture was stirred at room temperature for 3 h, evaporated and the residue was dissolved in DCM (15 mL). The organic extract was washed with water (10 mL) and brine (10 mL), dried and evaporated affording a yellow solid which was used in the next step without further purification (255 mg, 68%). M/z 337.2 $(M+H)^+$.

c. Tert-butyl N-[[4-[2-methoxy-4-[2-(4-methylpiperazin-1-yl)-2-oxo-ethoxy]phenyl]thiazol-2-yl]methyl] carbamate

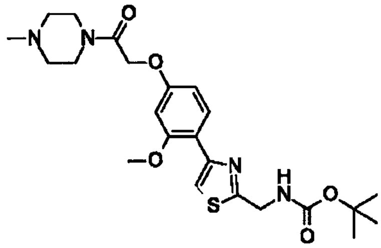

2-chloro-1-(4-methylpiperazin-1-yl)ethan-1-one hydrochloride (178 mg, 0.835 mmol) and $K_2CO_3$ (263 mg, 1.90 mmol) were added to a solution of tert-butyl N-[[4-(4-hydroxy-2-methoxyphenyl)thiazol-2-yl]methyl]carbamate (255 mg, 0.758 mmol) in DMF (6 mL) at room temperature. The reaction mixture was stirred overnight and evaporated. The residue was dissolved with EtOAc (10 mL), washed with water (10 mL) and brine (10 mL), dried and evaporated. The crude product was purified by chromatography on silica eluting with 0-10% MeOH in DCM affording a white solid (134 mg, 38% yield). M/z 447 $(M+H)^+$.

d. 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid

The title product was obtained following the procedure described in either example 1 step-b to step-d or example 46 step-c to step-e as white solid after purification by preparative hplc (MDAP) (52 mg, 48%). M/z 579.1 $(M+H)^+$. $^1H$ NMR (d6-DMSO) $\delta$ 12.1 (1H, bs), 8.60 (1H, m), 8.00 (1H, m), 7.75 (1H, s), 7.20 (2H, m), 7.11 (2H, m), 6.68 (1H, m), 6.59 (1H, m), 4.88 (2H, s), 4.59 (2H, d, $J$ = 6 Hz), 3.89 (3H, s), 3.58-3.42 (6H, d, $J$ = 16 Hz), 2.97 (2H, d, $J$ = 16 Hz), 2.70 (2H, s), 2.46-2.35 (7H, m).

**Example 64**

**2-[5,6-difluoro-2-[[4-[2-methoxy-4-[2-oxo-2-[3-(trimethylammonio)azetidin-1-yl]ethoxy]phenyl]thiazol-2-yl]methylcarbamoyl]indan-2-yl]acetate**

Method B-4 was used as described above.

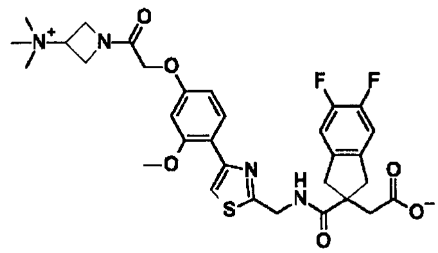

a. Tert-butyl N-[[4-(4-acetonyloxy-2-methoxy-phenyl)thiazol-2-yl]methyl]carbamate

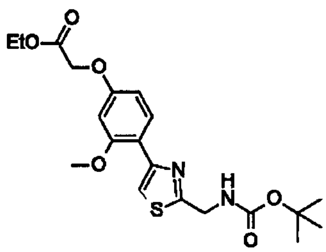

To a stirred solution of tert-butyl N-[[4-(4-hydroxy-2-methoxy-phenyl)thiazol-2-yl]methyl]carbamate (1 g, 2.97 mmol) in acetonitrile (25 mL) was added $K_2C_O3$ (616 mg, 4.46 mmol), ethyl 2-bromoacetate (546 mg, 3.27 mmol) at room temperature and refluxed for 5 h. The reaction mixture was filtered and filtrate was evaporated. The crude was

chromatographed on silica eluting with 20 % EtOAc in petroleum ether affording a pale yellow liquid (950 mg, 69%). M/z 423.1 $(M+H)^+$.

b. 2-[4-[2-[(tert-butoxycarbonylamino)methyl]thiazol-4-yl]-3-methoxy-phenoxy]acetic acid

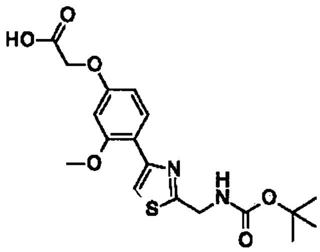

To a stirred solution of tert-butyl N-[[4-(4-acetonyloxy-2-methoxy-phenyl)thiazol-2-yl]methyl]carbamate (820 mg, 1.94 mmol) in THF:$H_2O$ (12 mL, 5:1) was added LiOH.$H_2O$ (165 mg, 3.88 mmol) at room temperature and stirred for 3 h. The reaction mixture was evaporated, diluted with $H_2O$ (50 mL) and extracted with diethyl ether (2 x 50 mL). The aqueous layer was acidified to ~ pH 5 with saturated aqueous citric acid solution and extracted with EtOAc (2 x 100 mL). The combined organic layer was dried, filtered and evaporated affording an off white solid (650 mg, 85%). M/z 395.0 $(M+H)^+$.

c. Tert-butyl N-[[4-[4-[2-[3-(dimethylamino)azetidin-1-yl]-2-oxo-ethoxy]-2-methoxyphenyl]thiazol-2-yl]methyl]carbamate

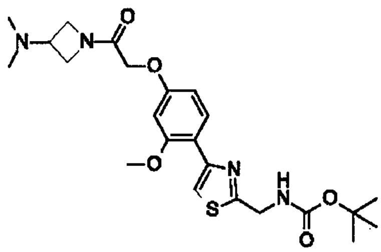

To a stirred solution of 2-[4-[2-[(tert-butoxycarbonylamino)methyl]thiazol-4-yl]-3-methoxy-phenoxy]acetic acid (400 mg, 1.01 mmol) in DMF (10 mL) was added $Et_3N$ (0.4 mL, 3.04 mmol) at room temperature and stirred for 10 minutes. Then N,N-dimethylazetidin-3-amine hydrochloride (210 mg, 1.21 mmol), T3P (1 mL, 1.52 mmol) was added and stirred for 16 h. The reaction mixture was diluted with cold water (25 mL), extracted with EtOAc (2 x 70 mL) and combined organic layer was dried, filtered and evaporated. The crude was chromatographed on silica eluting with 10 % MeOH in DCM affording a colourless solid (430 mg, 89%). M/z 477.2 $(M+H)^+$.

d. 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid

The title product was obtained following the procedure described in example 46 step-c and step-d using 2-[(tert-butoxy)carbonyl]-5,6-difluoro-2,3-dihydro-1H-indene-2-carboxylic acid and example 47. The crude compound was purified by preparative HPLC X-BRIDGE-C18 (150x30 mm), 5 u, Mobile phase: A: 0.1% Formic Acid in $H_2O$, B: MeCN] affording an off white solid (39 mg, 47%). M/z 629.2 $(M+H)^+$. $^1H$ NMR (500 MHz, DMSO-$d_6$): δ 12.45 (1H, bs), 8.06 (1H, d, J = 9 Hz), 7.78 (1H, s), 7.21 (2H, t, J = 9 Hz), 6.72 (1H, d, J = 2 Hz), 6.64 (1H, dd, J = 9.0 Hz, J = 2 Hz), 4.71-4.67 (3H, m), 4.57-4.55 (3H, m), 4.47-4.45 (1H, m), 4.40-4.37 (1H, m), 4.20-4.16 (1H, m), 3.91 (3H, s), 3.34 (2H, bs), 3.09 (9H, s), 2.86 (2H, d, J = 16 Hz), 2.38 (2H, s).

**Example 65**

**2-[2-[[4-[4-[2-[4-[3-(dimethylamino)propyl]piperazin-1-yl]-2-oxo-ethoxy]-2-methoxy-phenyl]thiazol-2-yl]methylcarbamoyl]indan-2-yl]acetic acid**

Method B-4 was used as described above.

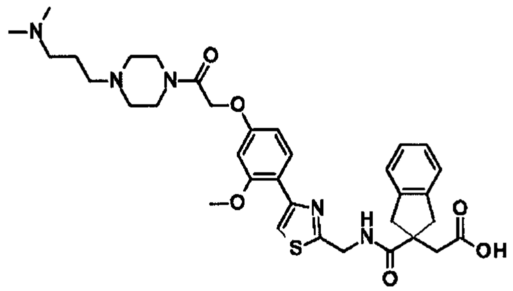

The title product was obtained following the procedure described in example 64 using N,N-dimethyl-3-piperazin-1-yl-propan-1-amine hydrochloride in step-c. The crude compound was purified by MDAP affording an off white solid. M/z 620.2 $(M+H)^+$. $^1H$ NMR (500 MHz, MeOD): δ 7.80 (2H, d, J = 8.5 Hz), 7.52 (1H, s), 7.19-7.18 (2H, m), 7.13-7.12 (2H, m), 6.98 (2H, d, J = 8.5 Hz), 4.80 (2H, s), 4.71 (2H, s), 3.60-3.58 (4H, m), 3.52 (2H, d, J = 16.0 Hz), 3.11-3.06 (4H, m), 2.81 (6H, s), 2.76 (2H, s), 2.49-2.44 (6H, m), 1.87-1.84 (2H, m).

**Example 66**

**2-[2-[[4-[2-methoxy-4-[2-(2-methyl-1,3,3a,4,6,6a-hexahydropyrrolo[3,4-c]pyrrol-5-yl)-2-oxo-ethoxy]phenyl]thiazol-2-yl]methylcarbamoyl]indan-2-yl] acetic acid**

Method B-4 was used as described above.

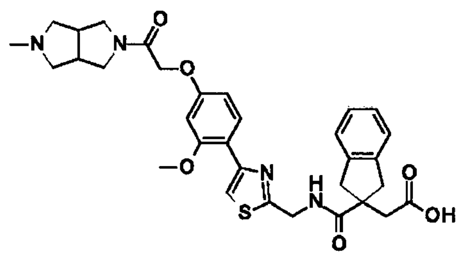

The title product was obtained following the procedure described in example 64 using 5-methyl-2,3,3a,4,6,6a-hexahydro-1H-pyrrolo[3,4-c]pyrrole hydrochloride in step-c. The crude compound was purified by MDAP affording an off white solid. M/z 575.2 $(M+H)^+$. $^1H$ NMR (500 MHz, DMSO-$d_6$): δ 9.07 (1H, bs), 7.82 (2H, dd, J = 7.0 Hz, J = 2.0 Hz), 7.79 (1H, s), 7.21-7.18 (2H, m), 7.14-7.12 (2H, m), 7.95 (2H, dd, J = 7.0 Hz, J = 2.0 Hz), 4.80-4.70 (2H, m), 4.57 (2H, d, J = 5.5 Hz), 3.72-3.69 (1H, m), 3.58-3.50 (1H, m), 3.45 (2H, d, J = 16.0 Hz), 3.40-3.20 (2H, bs), 2.98 (2H, d, J = 16.0 Hz), 2.90-2.80 (1H, m), 2.75-2.73 (1H, m), 2.71 (2H, s), 2.49-2.40 (4H, m), 2.20 (3H, s).

**Example 67**

**2-[2-[[4-[2-methoxy-4-[2-(4-methylmorpholin-4-ium-4-yl)ethoxy]phenyl]thiazol-2-yl]methylcarbamoyl] indan-2-yl] acetate**

Method B-4 was used as described above.

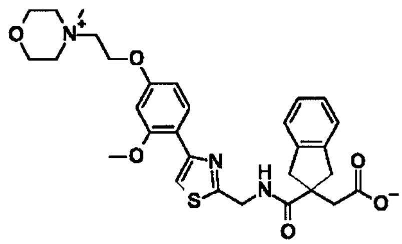

a. Tert-butyl N-[[4-[2-methoxy-4-(2-morpholinoethoxy)phenyl]thiazol-2-yl]methyl]carbamate

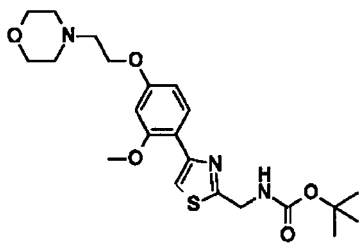

4-(2-chloroethyl)morpholine hydrochloride (205 mg, 1.10 mmol) and $Cs_2CO_3$ (977 mg, 3 mmol) were added to a solution of tert-butyl N-[[4-(4-hydroxy-2-methoxy-phenyl)thiazol-2-yl]methyl]carbamate (336 mg, 1 mmol) in acetonitrile (3 mL) at room temperature. The reaction mixture was heated at 40°C, stirred overnight cooled and partitioned between DCM (5 mL) and water (5 mL). The organic extract was dried and evpoarated. The crude product was purified by chromatography on silica eluting with 1-10% (2M $NH_3$ in MeOH) in DCM affording a white foam (409 mg, 91%). M/z 450 $(M+H)^+$.

b. Tert-butyl 2-[2-[[4-[2-methoxy-4-(2-morpholinoethoxy)phenyl]thiazol-2-yl]methylcarbamoyl]indan-2-yl]acetate

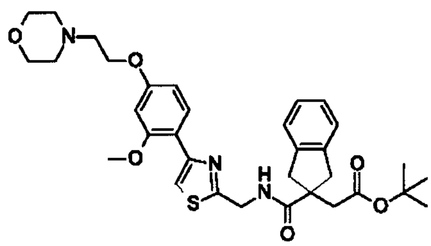

A colourless solid (249 mg, 100%) was obtained following the procedure described in example 1 step-b and step-c. The crude product was purified by chromatography on silica eluting with 1-10% (2M $NH_3$ in MeOH) in DCM. M/z 608 $(M+H)^+$.

c. Tert-butyl 2-[2-[[4-[2-methoxy-4-[2-(4-methylmorpholin-4-ium-4-yl)ethoxy]phenyl]thiazol-2-yl]methylcarbamoyl]indan-2-yl]acetate

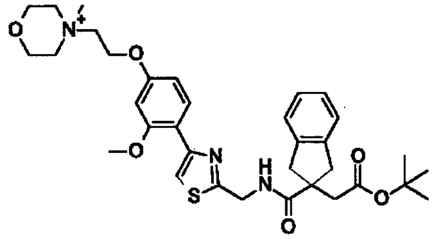

MeI (0.063 mL, 1.01 mmol) was added to a solution of tert-butyl 2-[2-[[4-[2-methoxy-4-(2-morpholinoethoxy)phenyl]thiazol-2-yl]methylcarbamoyl]indan-2-yl]acetate (123 mg, 0.202 mmol) in THF (2 mL) at room temperature. The reaction mixture was stirred for 3 h, evaporated and the resulting residue was used in the next step without further purification (151 mg, crude).

d. 2-[2-[[4-[2-methoxy-4-[2-(4-methylmorpholin-4-ium-4-yl)ethoxy]phenyl]thiazol-2-yl]methylcarbamoyl]indan-2-yl]acetate

Water (0.093 mL, 5.28 mmol) and TFA (0.93 mL, 12.12 mmol) were added to a solution of tert-butyl 2-[2-[[4-[2-methoxy-4-[2-(4-methylmorpholin-4-ium-4-yl)ethoxy]phenyl]thiazol-2-yl]methylcarbamoyl]indan-2-yl]acetate (151 mg, 0.202 mmol) in DCM (3 mL) at room temperature. The reaction mixture was stirred at for 1.5 h, evaporated and azeotroped with toluene. The residue was purified by preparative hplc (MDAP) affording a white solid (69 mg, 61% over 2 steps). M/z 566.5 $(M+H)^+$. $^1H$ NMR (d6-DMSO) δ 13.01 (1H, bs), 8.12 (1H, m), 7.80 (1H, s), 7.20 (2H, m), 7.11 (2H, m), 6.72 (2H, m), 4.58 (4H, m), 3.97 (8H, m), 3.61-3.50 (4H, m), 3.42 (6H, m), 2.89 (2H, d, *J* = 16 Hz), 2.33 (2H, m).

**Example 68**

**2-[5,6-difluoro-2-[[4-[2-methoxy-4-[2-(4-methylmorpholin-4-ium-4-yl)ethoxy]phenyl]thiazol-2-yl]methylcarbamoyl]indan-2-yl]acetate**

Method B-4 was used as described above.

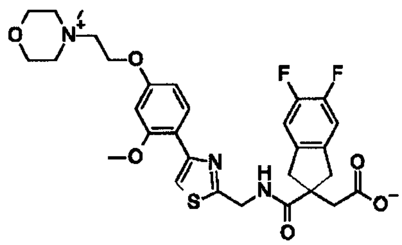

The title product was obtained following the procedure described in example 67 using 2-(2-(tert-butoxy)-2-oxoethyl)-5,6-difluoro-2,3-dihydro-1H-indene-2-carboxylic acid in step-c. The crude product was purified by preparative hplc (MDAP) affording a white solid (48 mg, 49%). M/z 602.7 $(M+H)^+$. $^1H$ NMR (d6-DMSO) δ 12.8 (1H, bs), 8.11 (1H, m), 7.80 (1H, s), 7.20 (2H, m), 6.70 (2H, m), 4.58 (4H, m), 3.98 (8H, m), 3.63-3.50 (4H, m), 3.36 (6H, m), 2.87 (2H, d, *J* = 16 Hz), 2.31 (2H, m).

**Example 69**

**2-[2-[[4-[2-methoxy-4-[3-(4-methylmorpholin-4-ium-4-yl)propoxy]phenyl]thiazol-2-yl]methylcarbamoyl]indan-2-yl]acetate**

Method B-4 was used as described above.

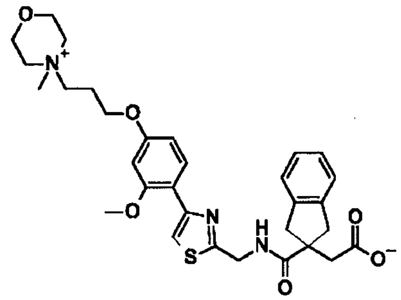

The title product was obtained following the procedure described in example 67 using 4-(3-chloropropyl)morpholine in step-a. The crude product was purified by preparative hplc (MDAP) affording a white solid (72 mg, 61%). M/z 580.5 $(M+H)^+$. $^1H$ NMR (d6-DMSO) δ 10.89 (1H, bs), 8.40 (1H, s), 7.09 (1H, m), 7.75 (1H, s), 7.20 (2H, m), 7.11 (2H, m), 6.75 (2H, m), 4.58 (2H, m), 4.18 (2H, m), 3.96 (8H, m), 3.64 (2H, m), 3.50 (2H, m), 3.42 (2H, m), 3.19 (4H, m), 2.91 (2H, d, *J* = 16 Hz), 2.50 (2H, m), 2.21 (2H, m).

**Example 70**

**2-[5,6-difluoro-2-[[4-[2-methoxy-4-[3-(4-methylmorpholin-4-ium-4-yl)propoxy]phenyl]thiazol-2-yl]methylcarbamoyl]indan-2-yl]acetate**

Method B-4 was used as described above.

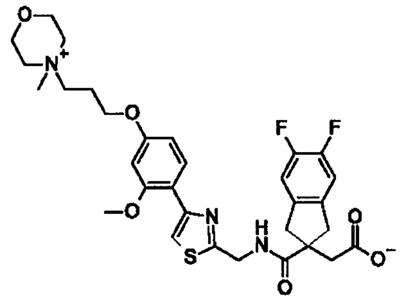

The title product was obtained following the procedure described in example 67 using 4-(3-chloropropyl)morpholine in step-a and 2-(2-(tert-butoxy)-2-oxoethyl)-5,6-difluoro-2,3-dihydro-1H-indene-2-carboxylic acid in step-c. The crude product was purified by preparative hplc (MDAP) affording a white solid (89 mg, 75%). M/z 616.5 $(M+H)^+$. $^1H$ NMR (d6-DMSO) δ 12.98 (1H, bs), 8.10 (1H, m), 7.78 (1H, s), 7.21 (2H, m), 6.63 (2H, m), 4.56 (2H, m), 4.14 (2H, m), 3.94 (6H, m), 3.64 (2H, m), 3.49 (4H, m), 3.31 (3H, m), 3.19 (3H, m), 2.84 (2H, d, *J* = 16 Hz), 2.32 (2H, m), 2.21 (2H, m).

**Example 71**

**2-[2-[[4-[2-methoxy-4-(3-morpholinopropoxy)phenyl]thiazol-2-yl]methylcarbamoyl]indan-2-yl]acetic acid**

Method B-4 was used as described above.

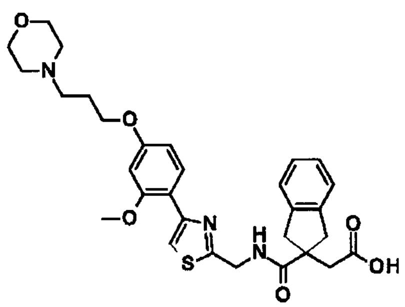

The title product was obtained following the procedure described in example 55 starting with tert-butyl N-[[4-(4-hydroxy-2-methoxy-phenyl)thiazol-2-yl]methyl]carbamate. The crude product was purified by preparative hplc (MDAP) affording a white solid. M/z 566.2 $(M+H)^+$. $^1H$ NMR (500 MHz, MeOD): δ 7.96 (1H, d, J = 8.5 Hz), 7.68 (1H, s), 7.22-7.19 (2H, m), 7.16-7.13 (2H, m), 6.63 (1H, d, J = 2 Hz), 6.60 (1H, dd, J = 8.5 Hz, J = 2 Hz), 4.70 (2H, s), 4.11 (2H, t, J = 6 Hz), 3.90 (3H, s), 3.80 (4H, t, J = 5 Hz), 3.53 (2H, d, J = 16.5 Hz), 3.10 (2H, d, J = 16.5 Hz), 2.89-2.82 (8H, m), 2.12-2.06 (2H, m).

**Example 72**

**2-[5,6-difluoro-2-[[4-[2-methoxy-4-(3-pyrrolidin-1-ylpropoxy)phenyl]thiazol-2-yl]methylcarbamoyl]indan-2-yl]acetic acid**

Method B-4 was used as described above.

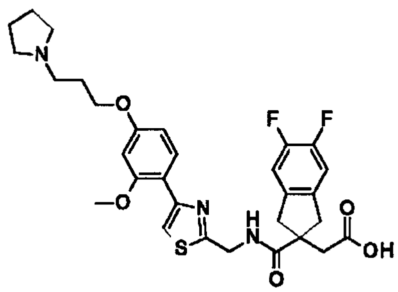

The title product was obtained following the procedure described in example 55 starting with tert-butyl N-[[4-(4-hydroxy-2-methoxy-phenyl)thiazol-2-yl]methyl]carbamate, using pyrrolidine in step-b and 2-[(tert-butoxy)carbonyl]-5,6-difluoro-2,3-dihydro-1H-indene-2-carboxylic acid in the peptide coupling step. The crude product was purified by preparative hplc (MDAP) affording a white solid. M/z 586.2 $(M+H)^+$. $^1H$ NMR (500 MHz, DMSO-$d_6$): δ 8.81 (1H, bs), 8.01 (1H, d, J = 8.5 Hz), 7.75 (1H, s), 7.27 (2H, t, J = 9.5 Hz), 6.64 (1H, d, J = 2.0 Hz), 6.60 (1H, dd, J = 8.5 Hz, J = 2.0 Hz), 4.56 (2H, d, J = 6 Hz), 4.06 (2H, t, J = 6.5 Hz), 3.89 (3H, s), 3.42 (2H, d, J = 16.5 Hz), 2.97 (2H, d, J = 16.5 Hz), 2.76 (2H, s), 2.64 (2H, t, J = 7 Hz), 2.56 (4H, bs), 1.94-1.91 (2H, m), 1.71 (4H, bs).

**Example 73**

**2-[5,6-difluoro-2-[[4-[2-methoxy-4-[3-(1-methylpyrrolidin-1-ium-1-yl)propoxy]phenyl]thiazol-2-yl]methylcarbamoyl]indan-2-yl)acetate**

Method B-4 was used as described above.

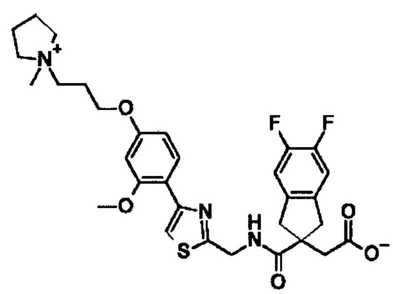

The title product was obtained following the procedure described in example 67 step-c and step-d starting with tert-butyl 2-[5,6-difluoro-2-[[4-[2-methoxy-4-(3-pyrrolidin-1-ylpropoxy)phenyl]thiazol-2-yl]methylcarbamoyl]indan-2-yl]acetate. The crude product was purified by preparative hplc (MDAP) affording a white solid. M/z 600.2 $(M+H)^+$. $^1H$ NMR (500 MHz, DMSO-$d_6$): δ 12.24 (1H, bs), 8.63 (1H, t, J = 5.5 Hz), 8.03 (1H, d, J = 8.5 Hz), 7.77 (1H, s), 7.27 (2H, t, J = 9 Hz), 6.65-6.63 (2H, m), 4.55 (2H, d, J = 5.5 Hz), 4.12 (2H, t, J = 6 Hz), 3.90 (3H, s), 3.55-3.45 (6H, m), 3.40 (2H, d, J = 16.5 Hz), 3.04 (3H, s), 2.97 (2H, d, J = 16.5 Hz), 2.79 (2H, s), 2.24-2.19 (2H, m), 2.10- 2.07 (4H, m).

**Example 74**

**2-[2-[[4-[2-methoxy-4-(4-pyridylmethoxy)phenyl]thiazol-2-yl]methylcarbamoyl]indan-2-yl]acetic acid**

Method B-4 was used as described above.

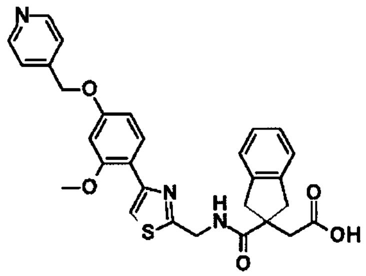

a. Tert-butyl N-[[4-[2-methoxy-4-(4-pyridylmethoxy)phenyl]thiazol-2-yl]methyl]carbamate

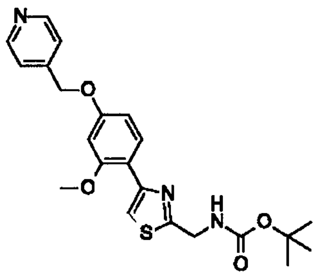

To a solution of 4-(bromomethyl)pyridine hydrobromide (361 mg, 1.42 mmol) in acetonitrile (15 mL) was added $Cs_2CO_3$ (1.1 g, 3.57 mmol) at room temperature and stirred for 10 minutes. Then tert-butyl N-[[4-(4-hydroxy-2-methoxy-phenyl)thiazol-2-yl]methyl]carbamate solution (400 mg, 1.19 mmol) in acetonitrile (5 mL) was added at room temperature and heated at 80 °C for 2 h. The reaction mixture was diluted with water (15 mL) and extracted with EtOAc (2 x 30 mL). The combined organic extract was dried, filtered and evaporated. The crude was purified by silica gel chromatography eluting with 50% EtOAc in petroleum ether affording an off white solid (280 mg, 55%). M/z 428.1 $(M+H)^+$.

b. Tert-butyl N-[[4-[2-methoxy-4-(4-pyridylmethoxy)phenyl]thiazol-2-yl]methyl]carbamate hydrochloride

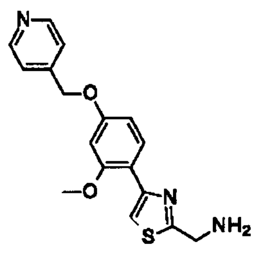

This was prepared by the same procedure as for Example 46 step-c affording an off white solid (230 mg, crude). M/Z 328.0 $(M+H)^+$.

c. Tert-butyl 2-[2-[[4-[2-methoxy-4-(4-pyridylmethoxy)phenyl]thiazol-2-yl]methylcarbamoyl]indan-2-yl]acetate

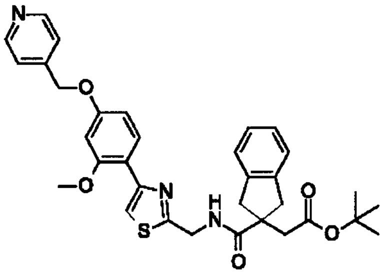

To a solution of tert-butyl N-[[4-[2-methoxy-4-(4-pyridylmethoxy)phenyl]thiazol-2-yl]methyl]carbamate hydrochloride (236 mg, 0.72 mmol) in DMF (10 mL) was added $Et_3N$ (0.35 mL, 2.1 mmol) and stirred for 10 minutes. Then 2-(2-(tert-butoxy)-2-oxoethyl)-2,3-dihydro-1H-indene-2-carboxylic acid (200 mg, 0.72 mmol) and T3P (0.64 mL, 1.0 mmol) was added at room temperature and stirred for 6 h. The reaction mixture was diluted with cold water (30 mL) and extracted with EtOAc (2 x4 0 mL). The combined organic extract was dried, filtered and evaporated. The crude was purified by silica gel chromatography eluting with 50% EtOAc in petroleum ether affording a light brown solid (200 mg, 47%). M/z 586.1 $(M+H)^+$.

d. 2-[2-[[4-[2-methoxy-4-(4-pyridylmethoxy)phenyl]thiazol-2-yl]methylcarbamoyl]indan-2-yl]acetic acid

The title product was obtained following the procedure described in either example 1 step-d or example 46 step-e. The crude compound was purified by preparative HPLC [Kromasil C18 (150*25), 10 u, Mobile phase: A: 0.1% Formic Acid in $H_2O$, B: MeCN] affording the title compound as an off white solid (29 mg, 40%). M/z 530.1 $(M+H)^+$. $^1H$ NMR (500 MHz, DMSO-$d_6$): δ 12.24 (1H, bs), 8.59 (2H, d, J = 5.5 Hz), 8.04 (1H, d, J = 8.5 Hz), 7.77 (1H, s), 7.47 (2H, d, J = 5.5 Hz), 7.20-7.18 (2H, m), 7.13-7.11 (2H, m), 6.79 (1H, s), 6.70 (1H, d, J = 8.5 Hz), 5.24 (2H, s), 4.57 (2H, d, J = 5.5 Hz), 3.90 (3H, s), 3.44 (2H, d, J = 16.5 Hz), 2.96 (2H, d, J = 16.5 Hz), 2.68 (2H, s).

**Example 75**

**2-[2-[[4-[2-methoxy-4-[(1-methylpyridin-1-ium-4-yl)methoxy]phenyl]thiazol-2-yl]methylcarbamoyl]indan-2-yl]acetate**

Method B-4 was used as described above.

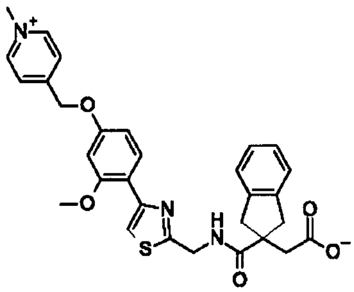

The title product was obtained following the procedure described in example 47. The crude compound was purified by preparative HPLC [YMC TRIART C18 (150*25), 10 u, Mobile phase: A: 0.1% Formic Acid in $H_2O$, B: MeCN] affording an off white solid (48 mg, 41%). M/z 544.2 $(M+H)^+$. $^1H$ NMR (500 MHz, DMSO-$d_6$): δ 8.95 (2H, d, J = 7.0 Hz), 8.15 (2H, d, J = 7.0 Hz), 8.08 (1H, d, J = 8.5 Hz), 7.80 (1H, s), 7.17-7.15 (2H, m), 7.12-7.09 (2H, m), 6.84 (1H, d, J = 2.5 Hz), 6.73-6.71 (1H, dd, J = 8.5 Hz, J = 2.5 Hz), 5.56 (2H, s), 4.57 (2H, d, J = 5.5 Hz), 4.33 (3H, s), 3.92 (3H, s), 3.00 (2H, d, J = 16 Hz), 2.88 (2H, d, J = 16 Hz), 2.41 (2H, s).

Other compounds were prepared following the method B-4 described for Example 74 and 75 using other commercial alkylating reagents in example 74 step-a, purified in a similar manner by preparative HPLC are shown in the Table below, wherein R is the moiety:

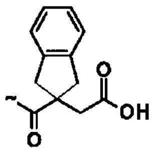

| Example | Structure | Name, MS and NMR data |
|---|---|---|
| **76** | 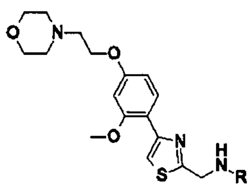 | 2-[2-[[4-[2-methoxy-4-(2-morpholinoethoxy)phenyl]thiazol-2-yl] methylcarbamoyl]indan-2-yl]acetic acid<br>M/z 552.2 $(M+H)^+$<br>$^1$H NMR (500 MHz, $CDCl_3$): δ 7.92 (1H, dd, J = 6 Hz, J = 3 Hz), 7.67 (1H, s), 7.24-7.20 (4H, m), 6.86 (1H, t, J = 5 Hz), 6.52-6.50 (2H, m), 4.69 (2H, d, J = 5 Hz), 4.30 (2H, t, J = 4.5 Hz), 3.91-3.90 (4H, m), 3.89 (3H, s), 3.48 (2H, d, J = 16.5 Hz), 3.22-3.20 (2H, m), 3.19 (2H, d, J = 16.5 Hz), 3.15-3.00 (4H, m), 2.91 (2H, s). |
| **77** | 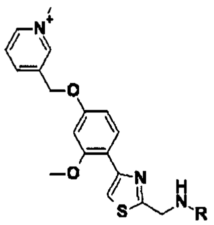 | 2-[2-[[4-[2-methoxy-4-[(1-methylpyridin-1-ium-3-yl)methoxy]phenyl]thiazol-2-yl]methylcarbamoyl]indan-2-yl]acetate<br>M/z 544.2 $(M+H)^+$<br>$^1$HNMR(500 MHz, DMSO-$d_6$): δ 11.02 (1H, bs), 9.18 (1H, s), 8.97 (1H, d, J = 6 Hz), 8.66 (1H, d, J = 8 Hz), 8.19-8.16 (1H, m), 8.11 (1H, d, J = 9 H), 7.80 (1H, s), 7.18-7.16 (2H, m), 7.12-7.10 (2H, m), 6.82 (1H, d, J = 2 Hz), 6.77 (1H, dd, J = 8.5 Hz, J = 2 Hz), 5.41 (2H, s), 4.58 (2H, d, J = 5 Hz), 4.39 (3H, s), 3.92 (3H, s), 3.41 (2H, d, J = 16 Hz), 2.92 (2H, d, J = 16 Hz). |
| **78** | 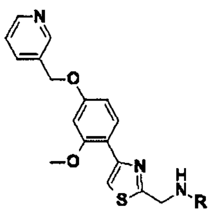 | 2-[2-[[4-[2-methoxy-4-(3-pyridylmethoxy)phenyl]thiazol-2-yl] methylcarbamoyl]indan-2-yl]acetic acid<br>M/z 530.1 $(M+H)^+$<br>$^1$H NMR (500 MHz, DMSO-$d_6$): δ 12.20 (1H, bs), 8.75 (1H, s), 8.63-8.61 (2H, m), 8.04 (1H, d, J = 8.5 Hz), 8.01 (1H, d, J = 7.5 Hz), 7.77 (1H, s), 7.53-7.51 (1H, m), 7.22-7.20 (2H, m), 7.14-7.12 (2H, m), 6.78 (1H, d, J = 2 Hz), 6.73 (1H, dd, J = 8.5 Hz, J = 2 Hz), 5.23 (2H, s), 4.57 (2H, d, J = 5.5 Hz), 3.90 (3H, s), 3.50-3.40 (2H, bs), 2.98 (2H, d, J = 16.5 Hz), 2.74 (2H, s). |
| **79** | 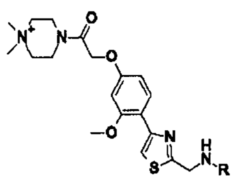 | 2-[2-[[4-[4-[2-(4,4-dimethylpiperazin-4-ium-1-yl)-2-oxo-ethoxy]-2-methoxy-phenyl]thiazol-2-yl]methylcarbamoyl]indan-2-yl]acetate<br>M/z 593.2 $(M+H)^+$<br>$^1$H NMR (500 MHz, DMSO-$d_6$): δ 10.86 (1H, bs), 8.03 (1H, d, J = 8.5 Hz), 7.77 (1H, s), 7.18-7.17 (2H, m), 7.13-7.10 (2H, m), 6.70 (1H, d, J = 2.5 Hz), 6.62 (1H, dd, J = 8.5 Hz, J = 2.5 Hz), 4.93 (2H, s), 4.57 (2H, d, J = 5.5 Hz), 3.90 (3H, s), 3.88-3.82 (4H, m), 3.48-3.40 (6H, m), 3.17 (6H, s), 2.93 (2H, d, J = 16 Hz), 2.54 (2H, s). |

Other compounds were prepared following the method B-4 described for Example 74 and 75 using other commercial alkylating reagents in example 74 step-a and 2-[(tert-butoxy)carbonyl]-5,6-difluoro-2,3-dihydro-1H-indene-2-carboxylic acid in step-c, purified in a similar manner by preparative HPLC are shown in the Table below, wherein R is the moiety:

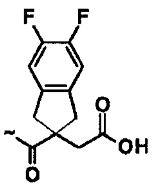

| Example | Structure | Name, MS and NMR data |
| --- | --- | --- |
| **80** | 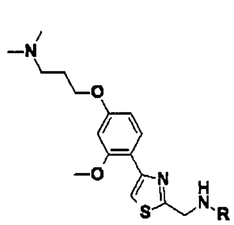 | 2-[2-[[4-[4-[3-(dimethylamino)propoxy]-2-methoxyphenyl]thiazol-2-yl] methylcarbamoyl]-5,6-difluoro-indan-2-yl]acetic acid<br>M/z 560.2 $(M+H)^+$<br>$^1$H NMR (500 MHz, DMSO-$d_6$): δ 11.70 (1H, bs), 8.68 (1H, t, J = 5.5 Hz), 8.01 (1H, d, J = 8.5 Hz), 7.75 (1H, s), 7.27 (2H, t, J = 9 Hz), 6.64 (1H, d, J = 1.5 Hz), 8.01 (1H, dd, J = 8.5 Hz, J = 1.5 Hz), 4.55 (2H, d, J = 6 Hz), 4.05 (2H, t, J = 6 Hz), 3.89 (3H, s), 3.42 (2H, d, J = 16.5 Hz), 2.97 (2H, d, J = 16.5 Hz), 2.78 (2H, s), 2.60-2.50 (2H, m), 2.29 (6H, s), 1.94-1.90 (2H, m). |
| **81** | 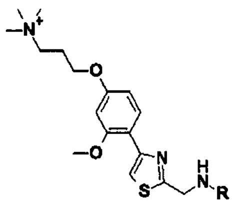 | 2-[5,6-difluoro-2-[[4-[2-methoxy-4-[3-(trimethylammonio)propoxy]phenyl] thiazol-2-yl] methylcarbamoyl] indan-2-yl] acetate<br>M/z 574.2 $(M+H)^+$<br>$^1$H NMR (500 MHz, DMSO-$d_6$): δ 9.80 (1H, bs), 8.05 (1H, d, J = 8.5 Hz), 7.77 (1H, s), 7.25 (2H, t, J = 9.5 Hz), 6.65-6.63 (2H, m), 4.55 (2H, d, J = 5.5 Hz), 4.12 (2H, t, J = 6 Hz), 3.90 (3H, s), 3.51-3.48 (2H, m), 3.40 (2H, d, J = 16.5 Hz), 3.10 (9H, s), 2.94 (2H, d, J = 16.5 Hz), 2.66 (2H, s), 2.21-2.18 (2H, m). |
| **82** | 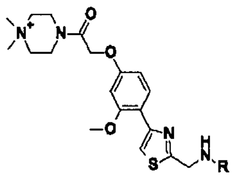 | 2-[2-[[4-[4-[2-(4,4-dimethylpiperazin-4-ium-1-yl)-2-oxo-ethoxy]-2-methoxy-phenyl]thiazol-2-yl] methylcarbamoyl]-5,6-difluoro-indan-2-yl]acetate<br>M/z 629.1 $(M+H)^+$<br>$^1$H NMR (500 MHz, DMSO-$d_6$): δ 9.88 (1H, bs), 8.02 (1H, d, J = 8.5 Hz), 7.77 (1H, s), 7.25 (2H, t, J = 9 Hz), 6.70 (1H, d, J = 2.5 Hz), 6.62 (1H, dd, J = 8.5 Hz, J = 2.5 Hz), 4.93 (2H, s), 4.56 (2H, d, J = 5.5 Hz), 3.90 (3H, s), 3.84-3.82 (4H, m), 3.48 (2H, bs), 3.40 (2H, d, J = 16.5 Hz), 3.45-3.35 (2H, obs), 3.17 (6H, s), 2.94 (2H, d, J = 16.5 Hz), 2.65 (2H, s). |

**Example 83**

**2-[2-[[4-[2-methoxy-4-[2-(4-methyl-3-oxo-piperazin-1-yl)ethoxy]phenyl]thiazol-2-yl]methylcarbamoyl]indan-2-yl]acetic acid**

Method B-4 was used as described above.

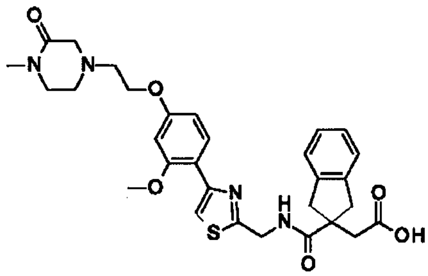

a. 4-(2-hydroxyethyl)-1-methyl-piperazin-2-one

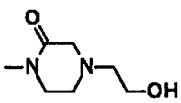

To a stirred solution of 1-Methylpiperazin-2-one (500 mg, 4.38 mmol) in THF (10 mL) was added $K_2CO_3$ (1.8 g, 13.1 mmol), 2-bromoethan-1-ol (1 g, 8.77 mmol) at room temperature and heated at 65 °C for 16 h. The reaction mixture was filtered through celite pad and washed the pad with EtOAc (10 mL). The filtrate was concentrated and the

resulting residue was chromatographed on silica eluting with 3-5% MeOH in DCM affording a pale yellow solid (300 mg, 43%). M/z 159.1 $(M+H)^+$.

b. 2-(4-methyl-3-oxo-piperazin-1-yl)ethyl methanesulfonate

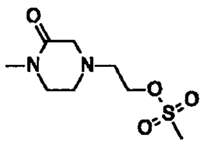

To a stirred solution of 4-(2-hydroxyethyl)-1-methyl-piperazin-2-one (230 mg, 1.45 mmol) in DCM (5 mL) was added $Et_3N$ (0.31 mL, 2.18 mmol) and mesyl chloride (0.13 mL, 1.74 mmol) at 0 °C and stirred at room temperature for 1 h. The mixture was diluted with cold water (10 mL) and adjusted pH to 7 with saturated aqueous sodium bicarbonate solution and extracted with DCM (2x20 mL). The combined organic layer was evaporated affording 2-(4-methyl-3-oxopiperazin-1-yl)ethyl methanesulfonate as a yellow solid (280 mg, crude).

c. 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid

The title product was obtained following the procedure described in example 74 step-a to step-d using 2-(4-methyl-3-oxo-piperazin-1-yl)ethyl methanesulfonate in step-a. The crude was purified by preparative HPLC [Kromasil C18 (150*25), 10 u, Mobile phase: A: 0.1% Formic Acid in $H_2O$, B: MeCN] affording an off white solid (15 mg, 27%). M/z 579.1 $(M+H)^+$. $^1H$ NMR (400 MHz, DMSO-$d_6$): δ 12.24 (1H, bs), 8.80 (1H, bs), 8.01 (1H, d, J= 8.4 Hz), 7.75 (1H, s), 7.21-7.18 (2H, m), 7.15-7.11 (2H, m), 6.67 (1H, d, J = 2.4 Hz), 6.62 (1H, dd, J = 8.4 Hz, J = 2.4 Hz), 4.57 (2H, d, J = 5.6 Hz), 4.15 (2H, t, J = 5.6 Hz), 3.89 (3H, s), 3.45 (2H, d, J = 16.4 Hz), 3.28-3.26 (2H, m), 3.11 (2H, s), 2.98 (2H, d, J = 16.4 Hz), 2.81 (3H, s), 2.79-2.75 (4H, m), 2.72 (2H, s).

## Example 84

**2-[2-[[4-[4-[2-(1,4-dimethyl-3-oxo-piperazin-1-ium-1-yl)ethoxy]-2-methoxy-phenyl]thiazol-2-yl]methylcarbamoyl]indan-2-yl]acetate**

Method B-4 was used as described above.

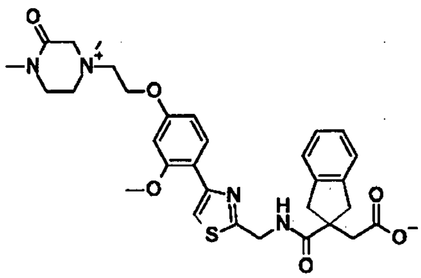

The title product was obtained following the procedure described in example 47 starting with tert-butyl 2-[2-[[4-[2-methoxy-4-[2-(4-methyl-3-oxo-piperazin-1-yl)ethoxy]phenyl]thiazol-2-yl]methylcarbamoyl]indan-2-yl]acetate. The crude compound was purified by preparative HPLC [Kromasil C18 (150*25), 10 u, Mobile phase: A: 0.1% Formic Acid in $H_2O$, B: MeCN] affording an off white solid (15 mg, 33%). M/z 593.2 $(M+H)^+$. $^1H$ NMR (500 MHz, DMSO-$d_6$): δ 12.54 (1H, bs), 8.11 (1H, d, J = 8.5 Hz), 7.80 (1H, s), 7.16-7.14 (2H, m), 7.11-7.09 (2H, m), 6.73 (1H, d, J = 2.5 Hz), 6.69 (1H, dd, J = 8.5 Hz, J = 2.5 Hz), 4.60-4.54 (4H, m), 4.30-4.20 (2H, m), 3.94-3.90 (2H, m), 3.92 (3H, s), 3.88 (2H, t, J = 5.5 Hz), 3.69 (2H, t, J = 5.5 Hz), 3.39 (2H, d, J = 16 Hz), 3.26 (3H, s), 2.93 (3H, s), 2.88 (2H, d, J = 16 Hz), 2.38 (2H, s).

**Example 85**

**2-[2-[[4-[2-methoxy-4-[2-(1-methylimidazol-2-yl)ethoxy]phenyl]thiazol-2-yl]methylcarbamoyl]indan-2-yl]acetic acid**

Method B-4 was used as described above.

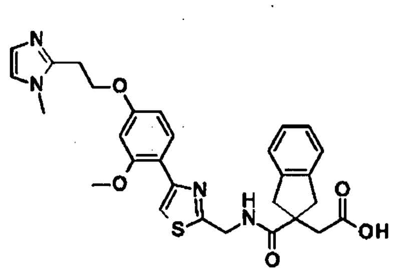

a. Tert-butyl N-[[4-[2-methoxy-4-[2-(1-methylimidazol-2-yl)ethoxy]phenyl]thiazol-2-yl]methyl]carbamate

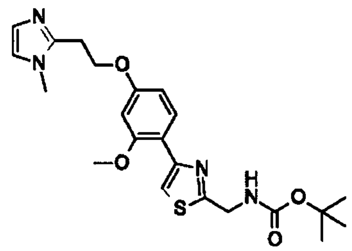

To a stirred solution of DIAD (0.73 mL, 3.71 mmol) in THF (10 mL) was added $PPh_3$ (974 mg, 3.71 mmol) at room temperature and stirred for 15 minutes. Then tert-butyl ((4-(4-hydroxy-2-methoxyphenyl)thiazol-2-yl)methyl)carbamate (500 mg, 1.48 mmol) and 2-(1-methyl-1H-imidazol-2-yl)ethan-1-ol (281 mg, 2.22 mmol) was added and stirred at room temperature for 16 h. The reaction mixture was diluted with water (10 mL), extracted with EtOAc (2 x 40 mL) and combined organic layer was dried, filtered and evaporated. The crude was chromatographed on silica eluting with 3-5% MeOH in DCM affording a pale yellow solid (140 mg, 21%). M/z 444.8 $(M+H)^+$.

b. 2-[2-[[4-[2-methoxy-4-(4-pyridylmethoxy)phenyl]thiazol-2-yl]methylcarbamoyl]indan-2-yl]acetic acid

The title product was obtained following the procedure described in example 46. The crude compound was purified by MDAP affording an off white solid. M/z 517.2 $(M+H)^+$. $^1H$ NMR (500 MHz, DMSO-$d_6$): δ 12.13 (1H, bs), 8.65 (1H, t, J = 6 Hz), 7.85 (2H, d, J = 9 Hz), 7.80 (1H, s), 7.37 (1H, bs), 7.22-7.19 (3H, m), 7.15-7.12 (2H, m), 6.99 (2H, d, J = 9 Hz), 4.57 (2H, d, J = 6 Hz), 4.36 (2H, t, J = 5.5 Hz), 3.75 (3H, s), 3.47 (2H, d, J = 16.5 Hz), 3.32 (2H, bs), 3.00 (2H, d, J = 16.5 Hz), 2.75 (2H, s).

**Example 86**

**2-[2-[[4-[4-[2-(1,3-dimethylimidazol-1-ium-2-yl)ethoxy]-2-methoxy-phenyl]thiazol-2-yl]methylcarbamoyl]-5,6-difluoro-indan-2-yl]acetate**

Method B-4 was used as described above.

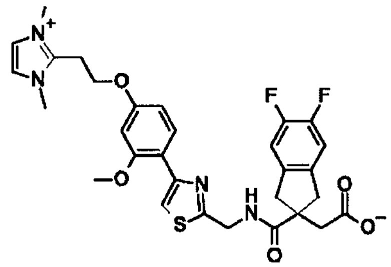

The title product was obtained from tert-butyl N-[[4-[2-methoxy-4-[2-(1-methylimidazol-2-yl)ethoxy]phenyl]thiazol-2-yl]methyl]carbamate following the procedure described in example 46 step-c and step-d using 2- [(tert- butoxy)carbonyl]- 5,6- difluoro- 2,3- dihydro- 1H- indene- 2-carboxylic acid and example 47. The crude compound was purified by preparative HPLC [SYMMETRY-C18 (300*19), 7 u, Mobile phase: A: 0.1% Formic Acid in $H_2O$, B: MeCN] affording an off white solid (24 mg, 20%). M/z 597.1 $(M+H)^+$. $^1H$ NMR (500 MHz, DMSO-$d_6$): δ 10.18 (1H, bs), 8.04 (1H, d, J = 9 Hz), 7.77 (1H, s), 7.67 (2H, s), 7.24 (2H, t, J = 9.5 Hz), 6.64 (1H, d, J = 2.5 Hz), 6.60 (1H, dd, J = 9 Hz, J = 2.5 Hz), 4.55 (2H, d, J = 5.5 Hz), 4.37 (2H, t, J = 6 Hz), 3.91 (6H, s), 3.90 (3H, s), 3.57 (2H, t, J = 6 Hz), 3.40 (2H, d, J = 16.5 Hz), 2.94 (2H, d, J = 16.5 Hz), 2.61 (2H, s).

## Example 87

### 2-[2-[[4-[4-[2-(1,3-dimethylimidazol-1-ium-2-yl)ethoxy]phenyl]thiazol-2-yl]methylcarbamoyl]indan-2-yl]acetate

Method B-4 was used as described above.

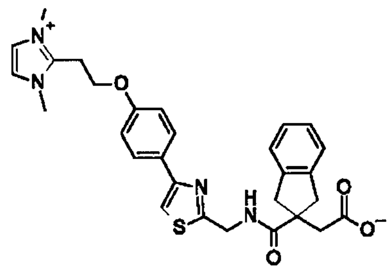

a. Tert-butyl N-[[4-[4-[2-(1-methylimidazol-2-yl)ethoxy]phenyl]thiazol-2-yl]methyl]carbamate

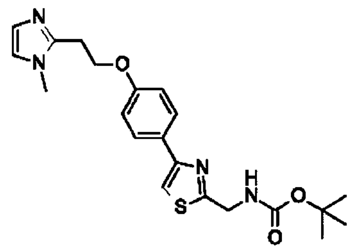

To a solution of [4-[2-[(tert-butoxycarbonylamino)methyl]thiazol-4-yl]phenyl]boronic acid (500 mg, 1.01 mmol) in DCM (10 mL) was added $Et_3N$ (0.3 mL, 2.03 mmol), $Cu(OAc)_2$ (276 mg, 1.52 mmol) and molecular sieves (1 g) at room temperature and purged with air for 10 minutes. Then 2-(1-methyl-1H-imidazol-2-yl)ethan-1-ol (192 mg, 1.52mmol) was added and purged with air for further 10 minutes. The reaction mixture was stirred at room temperature under air for 16 h. The reaction mixture was filtered through celite pad, washed the pad with DCM (20 mL) and filtrate was evaporated. The crude compound was chromatographed on silica eluting with 3-5% MeOH in DCM affording a light brown solid (100 mg, 17%). M/z = 573.2 $(M+H)^+$.

b. 2-[2-[[4-[4-[2-(1,3-dimethylimidazol-1-ium-2-yl)ethoxy]phenyl]thiazol-2-yl]methylcarbamoyl]indan-2-yl]acetate

The title product was obtained following the procedure described in example 86. The crude compound was purified by preparative HPLC [YMC TRIART C18 (150*25), 10 u, Mobile phase: A: 0.1% Formic Acid in $H_2O$, B: MeCN] affording an off white solid (65 mg, 64%). M/z 531.3 $(M+H)^+$. $^1H$ NMR (500 MHz, DMSO-$d_6$): δ 12.57 (1H, bs), 7.87 (2H, d, J = 9 Hz), 7.80 (1H, s), 7.66 (2H, s), 7.16-7.14 (2H, m), 7.10-7.09 (2H, m), 6.98 (2H, d, J = 9 Hz), 4.58 (2H, d, J = 5.5 Hz), 4.37 (2H, t, J = 6 Hz), 3.89 (6H, s), 3.58 (2H, t, J = 5.5 Hz), 3.40 (2H, d, J = 16 Hz), 2.89 (2H, d, J = 16 Hz), 2.38 (2H, s).

**Example 88**

**2-[2-[[4-[4-[2-(4-acetylpiperazin-1-yl)ethoxyl-2-methoxy-phenyl]thiazol-2-yl]methylcarbamoyl]indan-2-yl]acetic acid**

Method B-4 was used as described above.

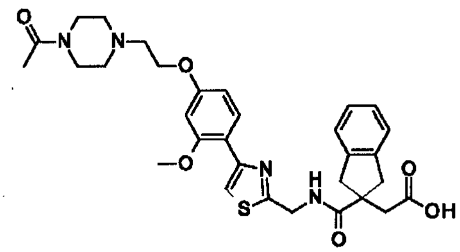

The title product was obtained following the procedure described in example 85 using 1-[4-(2-hydroxyethyl)piperazin-1-yl]ethanone in step-a. The crude compound was purified by MDAP affording an off white solid. M/z 593.2 $(M+H)^+$. $^1H$ NMR (500 MHz, DMSO-$d_6$): δ 12.30 (1H, bs), 8.66 (1H, bs), 8.01 (1H, d, J = 8.5 Hz), 7.75 (1H, s), 7.21-7.20 (2H, m), 7.15-7.12 (2H, m), 6.66 (1H, d, J = 2.0 Hz), 6.62 (1H, dd, J = 8.5 Hz, J = 2.0 Hz), 4.57 (2H, d, J = 5.5 Hz), 4.14 (2H, t, J = 5.5 Hz), 3.89 (3H, s), 3.45 (2H, d, J = 16.5 Hz), 3.45-3.41 (4H, m), 2.98 (2H, d, J = 16.5 Hz), 2.75-2.72 (4H, m), 2.63 (2H, bs), 2.44-2.43 (2H, m), 1.98 (3H, s).

**Example 89**

**2-[2-[[4-(1-methylpyrazol-4-yl)thiazol-2-yl]methylcarbamoyl]indan-2-yl]acetic acid** Method C was used as described above.

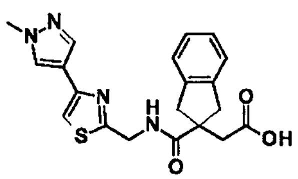

a. Tert-butylN-[[4-(1-methylpyrazol-4-yl)thiazol-2-yl]methyl]carbamate

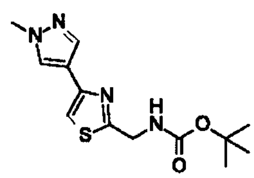

1-methylpyrazole-4-boronic acid pinacol ester (172 mg, 0.825 mmol), $Pd(PPh_3)_4$ (43 mg, 0.037 mmol) and $Na_2CO_3$ (159 mg, 1.5 mmol) were added to a solution of tert-butyl ((4-bromothiazole-2-yl)methyl)carbamate (220 mg,

0.75 mmol) in 3:1 dioxane/water (4 mL) at room temperature. The reaction mixture was flushed with $N_2$, heated at 100°C and stirred for 2 h. The mixture was cooled to room temperature, partitioned between EtOAc (5 mL) and water (5 mL). The organic extract was washed with brine (5 mL), dried and evaporated. The crude product was purified by chromatography on silica eluting with 50-100% EtOAc in hexane affording a colourless oil (211 mg, 95% yield). M/z 295 $(M+H)^+$.

b. [4-(1-methylpyrazol-4-yl)thiazol-2-yl]methanamine hydrochloride

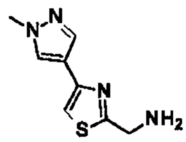

Tert-butyl N-[[4-(1-methylpyrazol-4-yl)thiazol-2-yl]methyl]carbamate (211 mg, 0.717 mmol) was dissolved in 4M aqueous HCl (3.1 mL, 12.46 mmol) at room temperature. The reaction mixture was heated at 50°C and stirred for 1 h. The cooled solution was dissolved in MeOH (5 mL) and applied to SCX-2 cartridge. The cartridge was washed with MeOH (10 mL) then the product eluted with 2M $NH_3$ in MeOH (10 mL). The solution was evaporated affording a white solid (125 mg, 90%). M/z 195 $(M+H)^+$.

c. Tert-butyl 2-[2-[[4-(1-methylpyrazol-4-yl)thiazol-2-yl]methylcarbamoyl]indan-2-yl]acetate

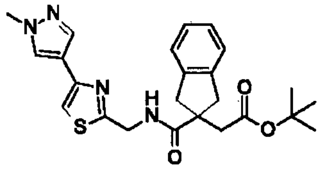

TEA (0.15 mL, 1.09 mmol) and HATU (165 mg, 0.434 mmol) were added to a solution of [4-(1-methylpyrazol-4-yl)thiazol-2-yl]methanamine hydrochloride (97 mg, 0.5 mmol) and 2-(2-tert-butoxy-2-oxo-ethyl)indane-2-carboxylic acid (100 mg, 0.362 mmol) in DCM (3 mL) at room temperature. The reaction mixture was stirred at room temperature for 1 h and partitioned between DCM (5 mL) and brine (5 mL). The organic extract was dried and evaporated. The crude product was purified by chromatography on silica eluting with 50-100% EtOAc in hexane affording a colourless oil (169 mg, 100% yield). M/z 453 $(M+H)^+$.

d. 2-[2-[[4-(1-methylpyrazol-4-yl)thiazol-2-yl]methylcarbamoyl]indan-2-yl]acetic acid

Water (0.170 mL, 9.29 mmol) and TFA (1.7 mL, 21.87 mmol) were added to a solution of tert-butyl 2-[2-[[4-(1-methylpyrazol-4-yl)thiazol-2-yl]methylcarbamoyl]indan-2-yl]acetate (164 mg, 0.362 mmol) in DCM (3 mL) at room temperature. The reaction mixture was stirred at room temperature for 3 h, evaporated and azeotroped with toluene (5 mL). The crude product was purified by preparative hplc (MDAP) affording a white solid (66 mg, 46%). M/z 397.4 $(M+H)^+$. $^1H$ NMR (d6-DMSO) $\delta$ 12.1 (1H, bs), 8.70 (1H, bs), 8.05 (1H, s), 7.77 (1H, s), 7.50 (1H, s), 7.20 (2H, m), 7.11 (2H, m), 4.54 (2H, d, $J$ = 6 Hz), 3.83 (3H, s), 3.45 (2H, d, $J$ = 16 Hz), 2.98 (2H, d, $J$ = 16 Hz), 2.72 (2H, s).

Other compounds have been prepared following the method C described in example 89 from commercial boronic acid pinacol esters reagents and purified in a similar manner by preparative HPLC are shown in the Table below, wherein R is the moiety:

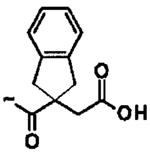

| Example | Structure | Name, MS and NMR data |
|---|---|---|
| **90** | 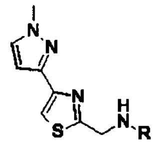 | 2-[2-[[4-(1-methylpyrazol-3-yl)thiazol-2-yl]methylcarbamoyl]indan-2-yl]acetic acid<br>M/z 397.4 $(M+H)^+$<br>$^1$H NMR (d6-DMSO) δ 12.12 (1H, bs), 8.63 (1H, m), 7.71 (1H, m), 7.63 (1H, s), 7.20 (2H, m), 7.11 (2H, m), 6.53 (1H, m), 4.53 (2H, d, *J* = 6 Hz), 3.86 (3H, s), 3.49 (2H, d, *J* = 16 Hz), 2.99 (2H, d, *J* = 16 Hz), 2.72 (2H, s). |
| **91** | 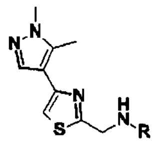 | 2-[2-[[4-(1,5-dimethylpyrazol-4-yl)thiazol-2-yl]methylcarbamoyl]indan-2-yl] acetic acid<br>M/z 411.4 $(M+H)^+$<br>$^1$H NMR (d6-DMSO) δ 12.11 (1H, bs), 8.61 (1H, m), 7.68 (1H, s), 7.40 (1H, s), 7.20 (2H, m), 7.11 (2H, m), 4.56 (2H, d, *J* = 6 Hz), 3.72 (3H, s), 3.45 (2H, d, *J* = 16 Hz), 2.99 (2H, d, *J* = 16 Hz), 2.70 (2H, s), 2.50 (3H, s). |
| **92** | 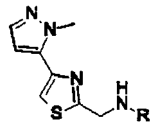 | 2-[2-[[4-(2-methylpyrazol-3-yl)thiazol-2-yl]methylcarbamoyl]indan-2-yl]acetic acid<br>M/z 397.3 $(M+H)^+$<br>$^1$H NMR (d6-DMSO) δ 12.12 (1H, bs), 8.68 (1H, m), 7.86 (1H, s), 7.42 (1H, m), 7.20 (2H, m), 7.11 (2H, m), 6.60 (1H, m), 4.59 (2H, d, *J* = 6 Hz), 4.03 (3H, s), 3.45 (2H, d, *J* = 16 Hz), 3.00 (2H, d, *J* = 16 Hz), 2.71 (2H, s). |
| **93** | 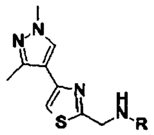 | 2-[2-[[4-(1,3-dimethylpyrazol-4-yl)thiazol-2-yl]methylcarbamoyl]indan-2-yl] acetic acid<br>M/z 411.3 $(M+H)^+$<br>$^1$H NMR (d6-DMSO) δ 12.1 (1H, bs), 8.62 (1H, m), 7.92 (1H, s), 7.42 (1H, s), 7.20 (2H, m), 7.11 (2H, m), 4.53 (2H, d, *J* = 6 Hz), 3.76 (3H, s), 3.46 (2H, d, *J* = 16 Hz), 2.97 (2H, d, *J* = 16 Hz), 2.72 (2H, s), 2.32 (3H, s). |
| **94** | 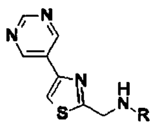 | 2-[2-[(4-pyrimidin-5-ylthiazol-2-yl)methylcarbamoyl]indan-2-yl]acetic acid<br>M/z 395.4 $(M+H)^+$<br>$^1$H NMR (d6-DMSO) δ 12.15 (1H, bs), 9.29 (2H, s), 9.14 (1H, s), 8.70 (1H, m), 8.31 (1H, s), 7.20 (2H, m), 7.11 (2H, m), 6.60 (1H, m), 4.52 (2H, d, *J* = 6 Hz), 3.48 (2H, d, *J* = 16 Hz), 3.00 (2H, d, J = 16 Hz), 2.75 (2H, s). |
| **95** | 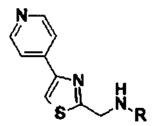 | 2-[2-[[4-(4-pyridyl)thiazol-2-yl]methylcarbamoyl]indan-2-yl]acetic acid<br>M/z 394.1 $(M+H)^+$<br>$^1$H NMR (500 MHz, DMSO-$d_6$): δ 12.24 (1H, bs), 8.78 (1H, bs), 8.61 (2H, d, J = 6 Hz), 8.32 (1H, s), 7.86 (2H, dd, J = 6 Hz, J =1.5 Hz), 7.21-7.20 (2H, m), 7.15-7.12 (2H, m), 4.60 (2H, d, J = 5.5 Hz), 3.45 (2H, d, J = 16.5 Hz), 2.99 (2H, d, J =16.5 Hz), 2.74 (2H, s). |
| **96** | 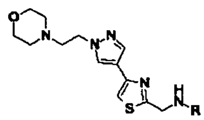 | 2-[2-[[4-[1-(2-morpholinoethyl)pyrazol-4-yl]thiazol-2-yl]methylcarbamoyl]indan-2-yl]acetic acid<br>M/z 496.2 $(M+H)^+$<br>$^1$H NMR (d6-DMSO) δ 9.00 (1H, bs), 8.11 (1H, s), 7.70 (1H, s), 7.50 (1H, s), 7.20 (2H, m), 7.11 (2H, m), 4.52 (2H, d, *J* = 6 Hz), 4.21 (2H, t), 3.52 (4H, t), 3.48 (2H, d, *J* = 16 Hz), 2.97 (2H, d, *J* = 16 Hz), 2.70 (4H, m), 2.41 (4H, m). |
| **97** | 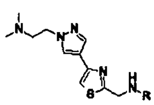 | 2-[2-[[4-[1-[2-(dimethylamino)ethyl] pyrazol-4-yl]thiazol-2-yl]methylcarbamoyl] indan-2-yl]acetic acid<br>M/z 454.2 $(M+H)^+$<br>$^1$H NMR (d6-DMSO) δ 12.2 (1H, bs), 8.71 (1H, bs), 8.18 (1H, s), 7.69 (1H, s), 7.50 (1H, s), 7.20 (2H, m), 7.11 (2H, m), 4.55 (2H, d, *J*= 6 Hz), 4.20 (2H, t), 3.47 (2H, d, *J* = 16 Hz), 2.99 (2H, d, *J* = 16 Hz), 2.71 (4H, m), 2.65 (2H, t), 2.17 (6H, s). |

**Example 98**

**2-[2-[[4-(5-methyl-1H-pyrazol-3-yl)thiazol-2-yl]methylcarbamoyl]indan-2-yl] acetic acid**

Method C was used as described above.

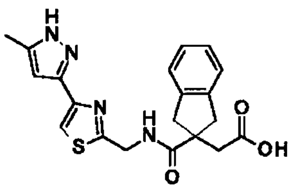

a. Tert-butyl N-[[4-(5-methyl-1-tetrahydropyran-2-yl-pyrazol-3-yl)thiazol-2-yl]methyl]carbamate

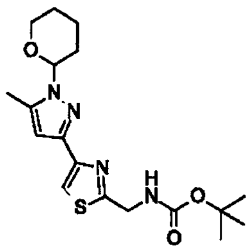

To a stirred solution of tert-butyl ((4-bromothiazol-2-yl) methyl) carbamate (200 mg, 0.68 mmol) in dioxane: $H_2O$ (5 mL, 5:1) was added $Na_2CO_3$ (213 mg, 2.0 mmol), 5-methyl-1-(tetrahydro-2H-pyran-2-yl)-3-(4,4,5,5-tetramethyl-1,3,2-dioxaborolan-2-yl)-1H-pyrazole (240 mg, 0.82 mmol) at room temperature and purged with argon for 15 minutes. Then $Pd(PPh_3)_4$ (79 mg, 0.06 mmol) was added, purged with argon for further 5 minutes and heated at 90 °C for 6 h. The reaction mixture was filtered through celite pad, washed the pad with EtOAc. The filtrate was washed with water, dried, filtered and evaporated. The crude compound was chromatographed on silica eluting with 25% EtOAc in petroleum ether affording a pale yellow solid (400 mg, crude). M/z 379.2 $(M+H)^+$.

b. 2-[2-[[4-(5-methyl-1H-pyrazol-3-yl)thiazol-2-yl]methylcarbamoyl]indan-2-yl]acetic acid

The title product was obtained following the procedure described in example 89. The crude product was purified by preparative hplc (MDAP) affording a white solid. M/z 397.1 $(M+H)^+$. $^1H$ NMR (500 MHz, DMSO-$d_6$): $\delta$ 12.50-12.36 (2H, bs), 8.65 (1H, bs), 7.64 (1H, s), 7.21-7.19 (2H, m), 7.14-7.12 (2H, m), 6.33 (1H, s), 4.55 (2H, d, J = 6.5 Hz), 3.45 (2H, d, J = 16.5 Hz), 2.98 (2H, d, J = 16.5 Hz), 2.74 (2H, s), 2.23 (3H, s).

**Example 99**

**2-[2-[[4-[1-(3-morpholinopropyl)pyrazol-4-yl]thiazol-2-yl]methylcarbamoyl]indan-2-yl]acetic acid**

Method C was used as described above.

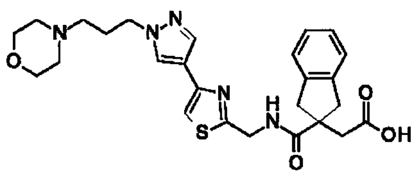

a. 4-[3-[4-(4,4,5,5-tetramethyl-1,3,2-dioxaborolan-2-yl)pyrazol-1-yl]propyl]morpholine

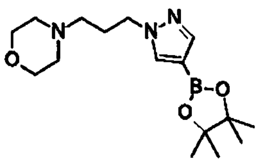

4-(3-chloropropyl)morpholine (164 mg, 1 mmol) and $Cs_2CO_3$ (179 mg, 0.55 mmol) were added to a solution of 4-pyrazoleboronic acid pinacol ester (194 mg, 1 mmol) in DMF (2 mL) at room temperature. The reaction mixture was heated at 80°C and stirred for 1 day. The cooled suspension was evaporated, suspended in water (5 mL) and extracted with EtOAc (2 x 5 mL). The combined organic extracts were dried and evaporated affording an orange oil which was used in the next step without further purification (232 mg, 72%). M/z 322 $(M+H)^+$.

b. 2-[2-[[4-[1-(3-morpholinopropyl)pyrazol-4-yl]thiazol-2-yl]methylcarbamoyl]indan-2-yl]acetic acid

The title product was obtained following the procedure described in example 89 from 4-[3-[4-(4,4,5,5-tetramethyl-1,3,2-dioxaborolan-2-yl)pyrazol-1-yl]propyl]morpholine. The crude product was purified by preparative hplc (MDAP) affording a white solid (27.4 mg, 31%). M/z 510.3 $(M+H)^+$. $^1H$ NMR(d6-DMSO) δ 12.1 (1H,bs), 8.62 (1H, m), 8.09 (1H, s), 7.80 (1H, s), 7.50 (1H, s), 7.20 (2H, m), 7.11 (2H, m), 4.52 (2H, d, *J* = 6 Hz), 4.16 (2H, t), 3.60 (4H, m), 3.47 (2H, d, *J* = 16 Hz), 3.00 (2H, d, *J* = 16 Hz), 2.70 (2H, s), 2.40-2.22 (6H, m), 1.98 (2H, m).

**Example 100**

**2-[2-[[4-[1-[3-(4-methylmorpholin-4-ium-4-yl)propyl]pyrazol-4-yl]thiazol-2-yl]methylcarbamoyl]indan-2-yl]acetate**

Method C was used as described above.

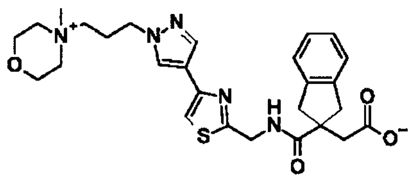

The title product was obtained following the procedure described in example 22 from tert-butyl 2-[2-[[4-[1-(3-morpholinopropyl)pyrazol-4-yl]thiazol-2-yl]methylcarbamoyl]indan-2-yl]acetate. The crude product was purified by preparative hplc (MDAP) affording a yellow solid (19 mg, 29%). M/z 524.2 $(M+H)^+$. $^1H$ NMR (d6-DMSO) δ 11.73 (1H, bs), 8.46 (1H, bs), 8.19 (1H, s), 7.90 (1H, s), 7.51 (1H, s), 7.20 (2H, m), 7.11 (2H, m), 4.52 (2H, d, *J* = 6 Hz), 4.23 (2H, t), 3.89 (4H, m), 3.45 (8H, m), 3.11 (3H, s), 2.91 (2H, d, *J* = 16 Hz), 2.45 (2H, s), 2.30 (2H, m).

**Example 101**

**2-[2-[[4-[1-(3-morpholinopropyl)pyrazol-4-yl]thiazol-2-yl]methylcarbainoyl]indan-2-yl]acetic acid**

Method C was used as described above.

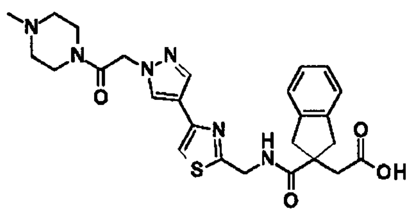

a. 2-[4-[2-[(tert-butoxycarbonylamino)methyl]thiazol-4-yl]pyrazol-1-yl]acetic acid

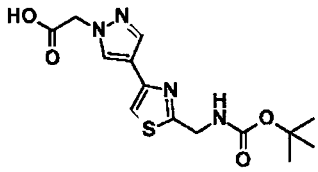

1-(ethoxycarbonylmethyl)-1H-pyrazole-5-boronic acid pinacol ester (231 mg, 0.825 mmol), $Pd(PPh_3)_4$ (43 mg, 0.037 mmol) and $Na_2CO_3$ (238 mg, 2.25 mmol) were added to a solution of tert-butyl (4-bromothiazole-2-yl)(methyl)carbamate (220 mg, 0.75 mmol) in 3:1 dioxane/water (4 mL) at room temperature. The reaction mixture was flushed with $N_2$, heated at 100°C and stirred for 1 h. The mixture was cooled to room temperature, diluted with water (10 mL) and washed with EtOAc (10 mL). the aqueous fraction was acidified to pH ~ 4 with 1M aqueous HCl and extracted with EtOAc (2 x 5 mL). The combined organic extracts were dried and evaporated affording a white solid (195 mg, 77%). M/z 337 $(M-H)^-$.

b. 2-[2-[[4-[1-(3-morpholinopropyl)pyrazol-4-yl]thiazol-2-yl]methylcarbamoyl]indan-2-yl]acetic acid

The title product was obtained following the procedure described in example 15 step-b to step-e using N-methylpiperazine in step-b. The crude product was purified by preparative hplc (MDAP) affording a white solid (125 mg, 71%). M/z 523.3 $(M+H)^+$. $^1H$ NMR (d6-DMSO) $\delta$ 8.85 (1H, bs), 8.00 (1H, s), 7.77 (1H, s), 7.52 (1H, s), 7.20 (2H, m), 7.11 (2H, m), 5.12 (2H, s), 4.52 (2H, d, *J* = 6 Hz), 3.49 (6H, m), 2.98 (2H, d, *J* = 16 Hz), 2.70 (2H, s), 2.36-2.24 (4H, m), 2.19 (3H, s).

**Example 102**

**2-[2-[[4-[1-(1-methyl-4-piperidyl)pyrazol-4-yl]thiazol-2-yl]methylcarbamoyl]indan-2-yl]acetic acid**

Method C was used as described above.

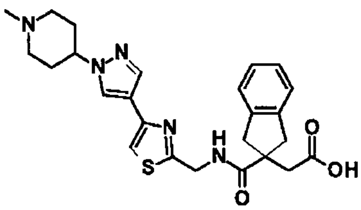

a. Tert-butyl 4-[4-(4,4,5,5-tetramethyl-1,3,2-dioxaborolan-2-yl)pyrazol-1-yl]piperidine-1-carboxylate

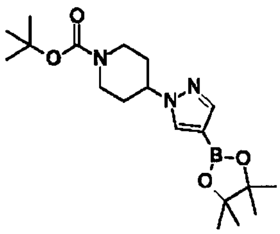

Tert-butyl 4-((methylsulfonyl)oxy)piperidine-1-carboxylate (1.73 g, 4.2 mmol) and $Cs_2CO_3$ (1.63 g, 5 mmol) were added to a solution of 4-pyrazoleboronic acid pinacol ester (776 mg, 4 mmol) in DMF (10 mL) at room temperature. The reaction mixture was heated at 100°C and stirred for 18 h. The cooled solution was evaporated, suspended in water (20 mL) and extracted with EtOAc (2 x 20 mL). The combined organic extracts were dried and evaporated. The crude product was purified by chromatography on silica eluting with 0-70% EtOAc in hexane affording a colourless oil (720 mg, 48% yield). M/z 322 $(M+H-tBu)^+$.

b. 4-[4-(4,4,5,5-tetramethyl-1,3,2-dioxaborolan-2-yl)pyrazol-1-yl]piperidine

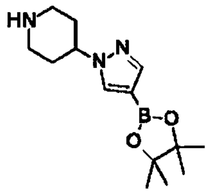

TFA (5 mL) was added to a solution of tert-butyl 4-[4-(4,4,5,5-tetramethyl-1,3,2-dioxaborolan-2-yl)pyrazol-1-yl]piperidine-1-carboxylate (555 mg, 1.47 mmol) in DCM (5 mL) at room temperature. The reaction mixture was heated at 40°C, stirred for 15 min, evaporated and azeotroped with toluene affording a brown oil which was used in the next step without further purification (48 mg, 100%). M/z 278 $(M+H)^+$.

c. Tert-butyl N-[[4-[1-(4-piperidyl)pyrazol-4-yl]thiazol-2-yl]methyl]carbamate

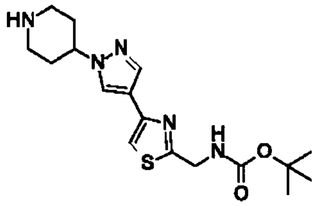

A yellow oil (868 mg, crude) was obtained following the procedure described in example 89 step-a and was used in the next step without further purification. M/z 364 $(M+H)^+$.

d. Tert-butyl N-[[4-[1-(1-methyl-4-piperidyl)pyrazol-4-yl]thiazol-2-yl]methyl]carbamate

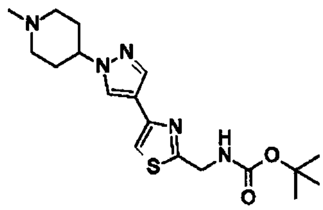

Formaldehyde (37%, 0.044 mL, 0.588 mmol) and formic acid (0.023 mL, 0.617 mmol) were added to a solution of 4-[4-(4,4,5,5-tetramethyl-1,3,2-dioxaborolan-2-yl)pyrazol-1-yl]piperidine (107 mg, 0.294 mmol) in THF (2 mL) at room temperature. The reaction mixture was heated at reflux and stirred for 6 h, cooled, and water (5 mL) was added. The aqueous layer was washed with EtOAc (5 mL), 1M NaOH aqueous solution (1 mL) and extracted with EtOAc (2 x 5 mL). The combined organic extracts were dried and evaporated . The crude product was purified by chromatography on silica eluting with 2-10% (2M $NH_3$ in MeOH) in DCM affording a colourless oil (50 mg, 30%). M/z 378 $(M+H)^+$.

e. [4-[1-(1-methyl-4-piperidyl)pyrazol-4-yl]thiazol-2-yl]methanamine trifluoroacetate

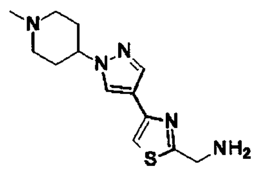

TFA (0.5 mL) was added to a solution of tert-butyl N-[[4-[1-(1-methyl-4-piperidyl)pyrazol-4-yl]thiazol-2-yl]methyl]carbamate (50 mg, 0.132 mmol) in DCM (1 mL) at room temperature. The reaction mixture was heated at 45°C, stirred for 1 h, cooled, evaporated and azeotroped with toluene. The residue was dissolved in MeOH (5 mL) and applied to SCX-2 cartridge. The cartridge was washed with MeOH (10 mL) then the product eluted with 2M $NH_3$ in MeOH (10 mL). The solution was evaporated affording a colourless oil (37 mg, 100%). M/z 278 $(M+H)^+$.

f. 2-[2-[[4-[1-(1-methyl-4-piperidyl)pyrazol-4-yl]thiazol-2-yl]methylcarbamoyl]indan-2-yl]acetic acid

The title product was obtained following the procedure described in example 89 step-c and step-d. The crude product was purified by preparative hplc (MDAP) affording a white solid (16 mg, 52%). M/z 480.3 $(M+H)^+$. $^1$H NMR (d6-DMSO) δ 9.02 (1H, bs), 8.12 (1H, s), 7.80 (1H, s), 7.49 (1H, s), 7.20 (2H, m), 7.11 (2H, m), 4.50 (2H, d, *J* = 6 Hz), 4.11 (1H, m), 3.46 (2H, d, *J* = 16 Hz), 2.96 (2H, d, *J* = 16 Hz), 2.83 (2H, m), 2.70 (2H, s), 2.19 (3H, s), 2.10-1.89 (6H, m).

**Example 103**

**2-[2-[[4-[1-[1-[2-(dimethylamino)acetyl]-4-piperidyl]pyrazol-4-yl]thiazol-2-yl]methylcarbamoyl]indan-2-yl]acetic acid**

Method C was used as described above.

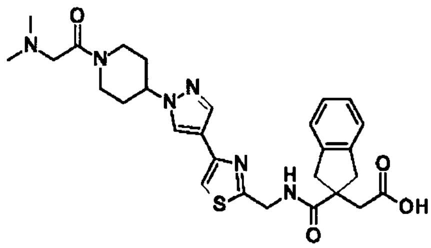

a. Tert-butyl N-[[4-[1-[1-[2-(dimethylamino)acetyl]-4-piperidyl]pyrazol-4-yl]thiazol-2-yl]methyl]carbamate

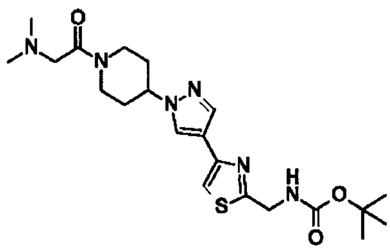

HATU (105 mg, 0.275 mmol) and DIPEA (0.13 mL, 0.750 mmol) were added to a solution of N,N-dimethylglycine (19 mg, 0.184 mmol) and tert-butyl N-[[4-[1-(4-piperidyl)pyrazol-4-yl]thiazol-2-yl]methyl]carbamate (91 mg, 0.25 mmol) in DCM (4 mL) at room temperature. The reaction mixture was stirred for 1 h, quenched with 1:1 water/saturated $NaHCO_3$ aqeuous solution (4 mL) and extracted with DCM (2 x 5 mL). The combined organic extracts were dried and evaporated. The crude product was purified by chromatography on silica eluting with 1-7% (2M NH3 in MeOH) in DCM affording a white solid (78 mg, 70%). M/z 449 $(M+H)^+$.

b. 2-[2-[[4-[1-[1-[2-(dimethylamino)acetyl]-4-piperidyl]pyrazol-4-yl]thiazol-2-yl]methylcarbamoyl]indan-2-yl]acetic acid

The title product was obtained following the procedure described in example 102 step-e and step-f. The crude product was purified by preparative hplc (MDAP) affording a white solid (63 mg, 53%). M/z 551.3 $(M+H)^+$. $^1H$ NMR (d6-DMSO) δ 8.66 (1H, m), 8.15 (1H, s), 7.81 (1H, s), 7.50 (1H, s), 7.20 (2H, m), 7.11 (2H, m), 4.55 (2H, d, *J* = 6 Hz), 4.42 (2H, m), 4.13 (1H, m), 3.48 (2H, d, *J* = 16 Hz), 3.29-3.11 (2H, m), 3.00 (2H, d, *J* = 16 Hz), 2.70 (3H, m), 2.24 (6H, s), 2.05 (2H, m), 1.90 (1H, m), 1.75 (1H, m).

**Example 104**

**2-[2-[[4-[1-[1-(4-methylpiperazine-1-carbonyl)-4-piperidyl]pyrazol-4-yl]thiazol-2-yl]methylcarbamoyl]indan-2-yl]acetic acid**

Method C was used as described above.

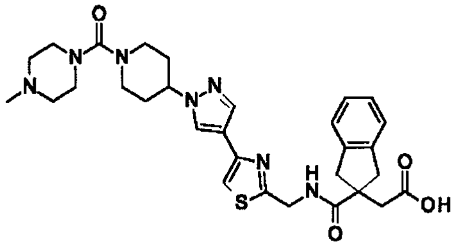

a. Tert-butyl N-[[4-[1-[1-[2-(dimethylamino)acetyl]-4-piperidyl]pyrazol-4-yl]thiazol-2-yl]methyl]carbamate

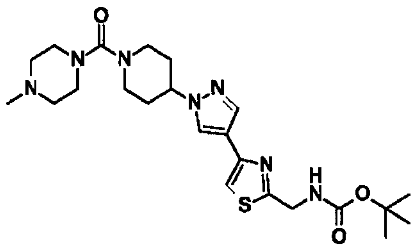

TEA (0.12 mL, 0.882 mmol) and 4-methyl-1-piperazinecarbonyl chloride hydrochloride (53 mg, 0.323 mmol) were added to a solution of tert-butyl N-[[4-[1-(4-piperidyl)pyrazol-4-yl]thiazol-2-yl]methyl]carbamate (107 mg, 0.294 mmol) in DCM (4 mL) at room temperature. The reaction mixture was stirred for 1 h, quenched with water (5 mL) and extracted with DCM (2 x 5 mL). The combined organic extracts were dried and evaporated. The crude product was purified by chromatography on silica eluting with 2-10% (2M $NH_3$ in MeOH) in DCM affording a white solid (90 mg, 42%). M/z 490 $(M+H)^+$.

b. 2-[2-[[4-[1-[1-(4-methylpiperazine-1-carbonyl)-4-piperidyl]pyrazol-4-yl]thiazol-2-yl]methylcarbamoyl]indan-2-yl]acetic acid

The title product was obtained following the procedure described in example 102 step-e and step-f. The crude product was purified by preparative hplc (MDAP) affording a white solid (55.8 mg, 42%). M/z 592.4 $(M+H)^+$. $^1H$ NMR (d6-DMSO) δ 8.67 (1H, m), 8.15 (1H, s), 7.80 (1H, s), 7.50 (1H, s), 7.20 (2H, m), 7.11 (2H, m), 4.53 (2H, d, *J* = 6 Hz), 4.36 (1H, m), 3.65 (2H, m), 3.48 (2H, d, *J* = 16 Hz), 3.17 (3H, m), 3.00 (2H, d, *J* = 16 Hz), 2.90 (2H, m), 2.70 (3H, m), 2.30 (4H, m), 2.19 (3H, s), 2.00 (2H, m), 1.89 (2H, m).

**Example 105**

**2-[2-[[4-[1-[1-[2-(4-methylpiperazin-1-yl)-2-oxo-ethyl]-4-piperidyl]pyrazol-4-yl]thiazol-2-yl]methylcarbamoyl]indan-2-yl]acetic acid**

Method C was used as described above.

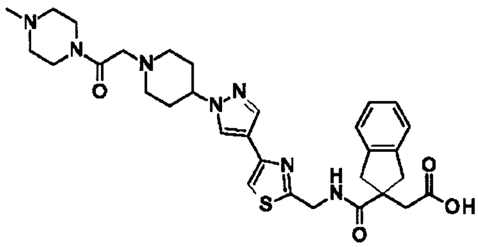

The title product was obtained following the procedure described in example 104 using 1-(2-chloroacetyl)4-methyl-piperazine hydrochloride in step-a. The crude product was purified by preparative hplc (MDAP) affording a white solid (12.4 mg, 50%). M/z 606.4 $(M+H)^+$. $^1H$ NMR (d6-DMSO) $\delta$ 12.1 (1H, bs), 9.89 (1H, bs), 8.13 (1H, s), 7.80 (1H, s), 7.50 (1H, s), 7.20 (2H, m), 7.11 (2H, m), 4.52 (2H, d, $J$ = 6 Hz), 4.15 (1H, m), 3.55 (2H, m), 3.44 (4H, m), 3.19 (2H, s), 3.00-2.86 (4H, m), 2.70 (2H, s), 2.31 (2H, m), 2.22 (7H, m), 2.05-1.88 (4H, m).

**Example 106**

**2-[2-[[4-[1-[3-(4-acetylpiperazin-1-yl)propyl]pyrazol-4-yl]thiazol-2-yl]methylcarbamoyl]indan-2-yl]acetic acid**

Method C was used as described above.

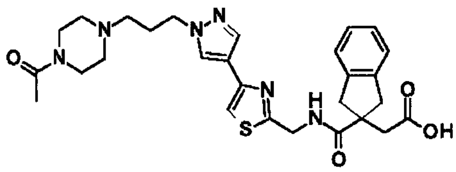

a. Tert-butyl N-[[4-(1H-pyrazol-4-yl)thiazol-2-yl]methyl]carbamate

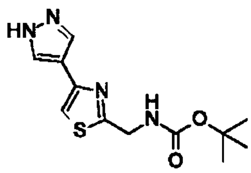

To a solution of tert-butyl ((4-bromothiazol-2-yl)methyl)carbamate (3 g, 10.2 mmol) in DMF:$H_2O$ (30 mL, 4:1) was added 4-(4,4,5,5-tetramethyl-1,3,2-dioxaborolan-2-yl)-1H-pyrazole (2.4 g, 12.3 mmol) and $Na_2CO_3$ (3.2 g, 30.8 mmol) at room temperature and the reaction mixture was purged with argon for 10 minutes. Then $PdCl_2$(dppf).DCM (420 mg, 0.51 mmol) was added to reaction mixture and purged with argon for further 5 minutes. The mixture was heated at 110 °C for 10 h. The reaction mixture was filtered through celite pad and washed the pad with EtOAc (50 mL). The filtrate was washed with ice cold water (2 x 30 mL) and evaporated. The residue was purified by silica gel chromatography eluting with 80% EtOAc in petroleum ether affording a yellow solid (820 mg, 29%). M/z 281.1 $(M+H)^+$.

b. Tert-butyl N-[[4-[1-(3-bromopropyl)pyrazol-4-yl]thiazol-2-yl]methyl]carbamate

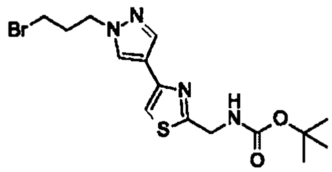

To a solution of tert-butyl N-[[4-(1H-pyrazol-4-yl)thiazol-2-yl]methyl]carbamate (820 mg, 2.92 mmol) in acetonitrile (5 mL) was added $Cs_2CO_3$ (2.3 g, 7.32 mmol), 1,3-dibromopropane (887 mg, 4.39 mmol) at room temperature and stirred at same temperature for 4 h. The reaction mixture was filtered through celite pad, washed the pad with EtOAc (20 mL) and evaporated. The residue was purified by silica gel chromatography eluting with 30% EtOAc in petroleum ether affording a pale yellow solid (720 mg, 65%). M/z 401.1 $(M+H)^+$.

c. Tert-butyl N-[[4-[1-[3-(4-acetylpiperazin-1-yl)propyl]pyrazol-4-yl]thiazol-2-yl]methyl]carbamate

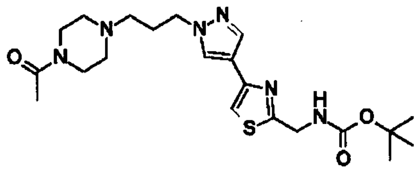

To a solution of tert-butyl N-[[4-[1-(3-bromopropyl)pyrazol-4-yl]thiazol-2-yl]methyl]carbamate (300 mg, 0.74 mmol) in acetonitrile (8 mL) was added $K_2CO_3$ (155 mg, 1.12 mmol), 1-(piperazin-1-yl)ethanone (115 mg, 0.89 mmol) at room temperature and stirred at same temperature for 16 h. The reaction mixture was filtered through celite pad, which was washed with EtOAc (10 mL) and the filtrate was evaporated. The residue was purified by silica gel chromatography eluting with 10-15% MeOH in DCM affording a pale yellow solid (180 mg, 54%). M/z 449.2 $(M+H)^+$.

d. 1-[4-[3-[4-[2-(aminomethyl)thiazol-4-yl]pyrazol-1-yl]propyl]piperazin-1-yl]ethenone hydrochloride

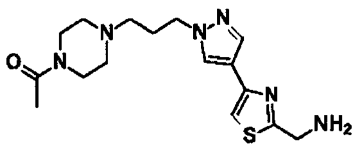

To a solution of tert-butyl N-[[4-[1-[3-(4-acetylpiperazin-1-yl)propyl]pyrazol-4-yl]thiazol-2-yl]methyl]carbamate (180 mg, 0.40 mmol) in dioxane (4 mL) was added 4M HCl in dioxane (4 mL) at room temperature and stirred for 6 h. The reaction mixture was evaporated and the resulting residue was triturated with diethyl ether (10 mL) affording an off white solid (175 mg). M/z 349.2 $(M+H)^+$.

e. Tert-butyl 2-[2-[[4-[1-[3-(4-acetylpiperazin-1-yl)propyl]pyrazol-4-yl]thiazol-2-yl]methylcarbamoyl]indan-2-yl]acetate

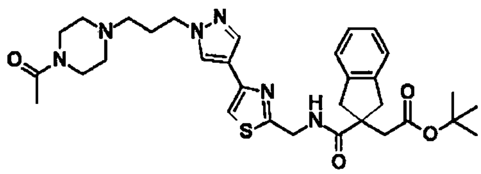

To a solution of 1-[4-[3-[4-[2-(aminomethyl)thiazol-4-yl]pyrazol-1-yl]propyl]piperazin-1-yl]ethenone hydrochloride (155 mg, 0.40 mmol) in DMF (5 mL) was added $Et_3N$ (0.17 mL, 1.2 mmol) and stirred for 10 minutes. Then 2-(2-(tert-butoxy)-2-oxoethyl)-2,3-dihydro-1H-indene-2-carboxylic acid (133 mg, 0.48 mmol), EDC. HCl (115 mg, 0.60 mmol) and HOBt (54 mg, 0.40 mmol) was added at room temperature and stirred for 16 h. The reaction mixture was diluted with cold water (10 mL) and extracted with EtOAc (2 x 20 mL). Combined organic extracts were dried, filtered and evaporated. The crude was purified by silica gel chromatography eluting with 10-15% MeOH in DCM affording a pale yellow solid (120 mg, 68%). M/z 607.3 $(M+H)^+$.

f. 2-[2-[[4-[1-[3-(4-acetylpiperazin-1-yl)propyl]pyrazol-4-yl]thiazol-2-yl]methylcarbamoyl]indan-2-yl]acetic acid

To a solution of tert-butyl 2-[2-[[4-[1-[3-(4-acetylpiperazin-1-yl)propyl]pyrazol-4-yl]thiazol-2-yl]methylcarbamoyl]indan-2-yl]acetate (70 mg, 0.11 mmol) in DCM (4 mL) was added TFA (1.5 mL) at 0 °C and stirred at room temperature for 4 h. The mixture was evaporated and the residue was triturated with diethyl ether (10 mL). The crude compound was purified by preparative HPLC [YMC TRIART C18 (150*25), 10 u, Mobile phase: A: 0.1% Formic Acid in $H_2O$, B: MeCN] affording the title compound as an off white solid (37 mg, 59%). M/z 551.2 $(M+H)^+$. $^1H$ NMR (500 MHz, DMSO-$d_6$): δ 10.04 (1H, bs), 8.09 (1H, s), 7.80 (1H, s), 7.49 (1H, s), 7.19-7.17 (2H, m), 7.12-7.10 (2H, m), 4.55 (2H, d, J = 5.0 Hz), 4.14 (2H, t, J = 7.0 Hz), 3.44-3.38 (6H, bs), 2.95 (2H, d, J = 16.0 Hz), 2.59 (2H, s), 2.32 (2H, t, J = 5.0 Hz), 2.27-2.23 (4H, m), 1.96 (3H, s), 1.96-1.94 (2H, m).

**Example 107**

**2-[2-[[4-[1-[3-(4-acetyl-1-methyl-piperazin-1-ium-1-yl)propyl]pyrazol-4-yl]thiazol-2-yl]methylcarbamoyl]indan-2-yl]acetate**

Method C was used as described above.

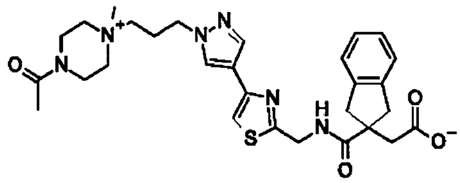

a. Tert-butyl2-[2-[[4-[1-[3-(4-acetyl-1-methyl-piperazin-1-ium-1-yl)propyl]pyrazol-4-yl]thiazol-2-yl]methylcarbamoyl]indan-2-yl]acetate

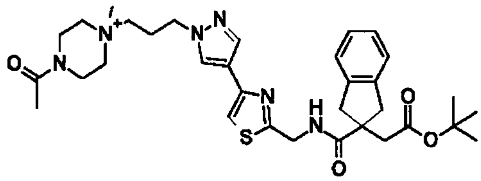

To a solution of tert-butyl 2-[2-[[4-[1-[3-(4-acetylpiperazin-1-yl)propyl]pyrazol-4-yl]thiazol-2-yl]methylcarbamoyl]indan-2-yl]acetate (40 mg, 0.06 mmol) in acetonitrile (4 mL) was added MeI (18.7 mg, 0.13 mmol) at 0 °C and stirred at room temperature for 16 h. The mixture was evaporated and the residue was triturated with diethyl ether (10 mL) affording a pale brown solid (50 mg, crude). M/z 621.3 $(M)^+$.

b. 2-[2-[[4-[1-[3-(4-acetyl-1-methyl-piperazin-1-ium-1-yl)propyl]pyrazol-4-yl]thiazol-2-yl]methylcarbamoyl]indan-2-yl] acetate

A solution of tert-butyl 2-[2-[[4-[1-[3-(4-acetyl-1-methyl-piperazin-1-ium-1-yl)propyl]pyrazol-4-yl]thiazol-2-yl]methylcarbamoyl]indan-2-yl]acetate (45 mg, 0.07 mmol) in DCM (3 mL) was treated with TFA (1.0 mL) at 0 °C and stirred at room temperature for 4 h. The mixture was evaporated and the residue was triturated with diethyl ether (10 mL). The crude compound was purified by preparative HPLC [X-BRIDGE-C18 (150*30), 5 u, Mobile phase: A: 0.1% Formic Acid in $H_2O$, B: MeCN] affording the title compound as an off white solid (17.5 mg, 43%). M/z 565.2 $(M+H)^+$. $^1H$ NMR (500 MHz, MeOD): δ 8.05 (1H, s), 7.91 (1H, s), 7.39 (1H, s), 7.19-7.17 (2H, m), 7.13-7.10 (2H, m), 4.69 (2H, s), 4.32 (2H, t, J = 6.5 Hz), 4.07-4.04 (1H, m), 3.93-3.81 (2H, m), 3.75-3.71 (1H, m), 3.52-3.46 (8H, m), 3.16 (3H, s), 3.07

(2H, d, J = 16.0 Hz), 2.74 (2H, s), 2.43-2.40 (2H, m), 2.13 (3H, s).

Other compounds prepared by the method C described for Example 106 and 107 using commercial amine reagents in step-c example 106 and purified in a similar manner by preparative HPLC are shown in the Table below, wherein R is the moiety:

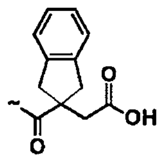

| Example | Structure | Name, MS and NMR data |
|---|---|---|
| **108** | 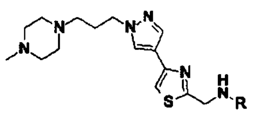 | 2-[2-[[4-[1-[3-(4-methylpiperazin-1-yl)propyl] pyrazol-4-yl]thiazol-2-yl]methylcarbamoyl]indan-2-yl]acetic acid<br>M/z 523.2 $(M+H)^+$<br>$^1$H NMR (500 MHz, DMSO-$d_6$): δ 12.14 (1H, bs), 8.63 (1H, t, J = 6.0 Hz), 8.09 (1H, s), 7.82 (1H, s), 7.50 (1H, s), 7.21-7.19 (2H, m), 7.15-7.13 (2H, m), 4.54 (2H, d, J = 5.5 Hz), 4.16 (2H, t, J = 7.0 Hz), 3.46-3.43 (6H, bs), 3.10-2.90 (4H, bs), 2.99 (2H, d, J = 16.0 Hz), 2.78-2.65 (2H, bs), 2.74 (3H, s), 2.55-2.45 (2H, bs), 1.99 (2H, bs). |
| **109** | 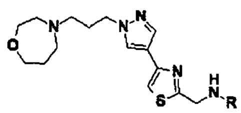 | 2-[2-[[4-[1-[3-(1,4-oxazepan-4-yl)propyl]pyrazol-4-yl]thiazol-2-yl] methylcarbamoyl]indan-2-yl]acetic acid<br>M/z 524.2 $(M+H)^+$<br>$^1$H NMR (500 MHz, DMSO-$d_6$): δ 9.05 (1H, bs), 8.07 (1H, s), 7.80 (1H, s), 7.49 (1H, s), 7.20-7.19 (2H, m), 7.13-7.11 (2H, m), 4.54 (2H, d, J = 6.0 Hz), 4.14 (2H, t, J = 6.5 Hz), 3.65 (2H, t, J = 6.0 Hz), 3.59-3.57 (2H, m), 3.44 (2H, d, J = 16.5 Hz), 2.97 (2H, d, J = 16.5 Hz), 2.69 (2H, s), 2.60-2.56 (4H, m), 2.39 (2H, t, J = 7.0 Hz), 1.92-1.85 (2H, m), 1.80-1.70 (2H, m). |
| **110** | 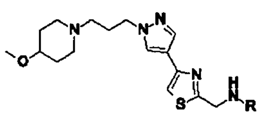 | 2-[2-[[4-[1-[3-(4-methoxy-1-piperidyl)propyl]pyrazol-4-yl]thiazol-2-yl] methylcarbamoyl]indan-2-yl]acetic acid<br>M/z 538.2 $(M+H)^+$<br>$^1$H NMR (500 MHz, DMSO-$d_6$): δ 8.70 (1H, bs), 8.06 (1H, s), 7.79 (1H, s), 7.49 (1H, s), 7.21-7.19 (2H, m), 7.14-7.12 (2H, m), 4.54 (2H, d, J = 5.5 Hz), 4.12 (2H, t, J = 7.0 Hz), 3.44 (2H, d, J = 16.0 Hz), 3.20 (3H, s), 3.16-3.12 (1H, m), 2.98 (2H, d, J = 16.0 Hz), 2.73 (2H, s), 2.65-2.63 (2H, m), 2.22 (2H, t, J = 6.5 Hz), 2.04-2.02 (2H, m), 1.95-1.88 (2H, m), 1.81-1.79 (2H, m), 1.43-1.39 (2H, m). |
| **111** | 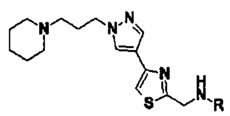 | 2-[2-[[4-[1-[3-(1-piperidyl)propyl]pyrazol-4-yl]thiazol-2-yl] methylcarbamoyl]indan-2-yl]acetic acid<br>M/z 508.2 $(M+H)^+$<br>$^1$H NMR (500 MHz, DMSO-$d_6$): δ 8.86 (1H, bs), 8.06 (1H, s), 7.79 (1H, s), 7.49 (1H, s), 7.21-7.18 (2H, m), 7.14-7.11 (2H, m), 4.54 (2H, d, J = 5.5 Hz), 4.12 (2H, t, J = 7.0 Hz), 3.45 (2H, d, J = 16.5 Hz), 2.97 (2H, d, J = 16.5 Hz), 2.71 (2H, s), 2.32-2.26 (4H, m), 2.21 (2H, t, J = 7.0 Hz), 1.96-1.90 (2H, m), 1.51-1.46 (4H, m), 1.40-1.30 (2H, m). |

(continued)

| Example | Structure | Name, MS and NMR data |
|---|---|---|
| **112** | 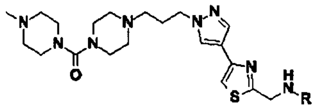 | 2-[2-[[4-[1-[3-[4-(4-methylpiperazine-1-carbonyl)piperazin-1-yl]propyl]pyrazol-4-yl]thiazol-2-yl]methylcarbamoyl]indan-2-yl]acetic acid<br>M/z 635.3 $(M+H)^+$<br>$^1$H NMR (500 MHz, DMSO-$d_6$): δ 8.08 (1H, s), 7.80 (1H, s), 7.49 (1H, s), 7.21-7.19 (2H, m), 7.13-7.12 (2H, m), 4.54 (2H, d, J = 6.0 Hz), 4.13 (2H, t, J = 7.0 Hz), 3.44 (2H, d, J = 16.0 Hz), 3.13-3.10 (8H, m), 2.97 (2H, d, J = 16.0 Hz), 2.69 (2H, s), 2.30-2.23 (10H, m), 2.15 (3H, s), 1.94-1.90 (2H, m). |
| **113** | 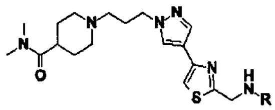 | 2-[2-[[4-[1-[3-[4-(dimethylcarbamoyl)-1-piperidyl]propyl]pyrazol-4-yl]thiazol-2-yl]methylcarbamoyl]indan-2-yl]acetic acid<br>M/z 579.2 $(M+H)^+$<br>$^1$H NMR (500 MHz, DMSO-$d_6$): δ 12.15 (1H, bs), 8.63 (1H, t, J = 6.0 Hz), 8.13 (1H, s), 7.86 (1H, s), 7.52 (1H, s), 7.21-7.19 (2H, m), 7.14-7.12 (2H, m), 4.54 (2H, d, J = 6.0 Hz), 4.20 (2H, t, J = 6.5 Hz), 3.50-3.40 (2H, bs), 3.44 (2H, d, J = 16.5 Hz), 3.00 (3H, s), 2.97 (2H, d, J = 16.5 Hz), 2.96-2.80 (5H, bs), 2.80 (3H, s), 2.74 (2H, s), 2.20-2.10 (2H, m), 1.85-1.65 (4H, m). |
| **114** | 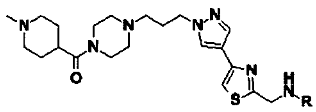 | 2-[2-[[4-[1-[3-[4-(1-methylpiperidine-4-carbonyl)piperazin-1-yl]propyl]pyrazol-4-yl]thiazol-2-yl]methylcarbamoyl]indan-2-yl]acetic acid<br>M/z 634.2 $(M+H)^+$<br>$^1$H NMR (400 MHz, DMSO-$d_6$): δ 8.66 (1H, t, J = 6.0 Hz), 8.08 (1H, s), 7.80 (1H, d, J = 0.8 Hz), 7.49 (1H, s), 7.21-7.18 (2H, m), 7.15-7.12 (2H, m), 4.54 (2H, d, J = 5.6 Hz), 4.15 (2H, t, J = 6.8 Hz), 3.46-3.42 (6H, bs), 3.05-2.94 (2H, m), 2.98 (2H, d, J = 16.0 Hz), 2.73 (2H, s), 2.67-2.61 (1H, m), 2.32-2.23 (11H, m), 1.97-1.93 (2H, m), 1.64-1.59 (4H, m). |
| **115** | 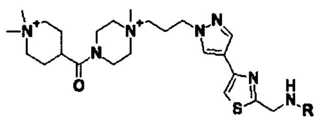 | 2-[2-[[4-[1-[3-[4-(1,1-dimethylpiperidin-1-ium-4-carbonyl)-1-methyl-piperazin-1-ium-1-yl]propyl]pyrazol-4-yl]thiazol-2-yl]methylcarbamoyl]indan-2-yl] acetate<br>M/z 662.3 $(M)^+$<br>$^1$H NMR (500 MHz, MeOD): δ 8.55 (1H, bs), 8.08 (1H, s), 7.90 (1H, s), 7.39 (1H, s), 7.18-7.16 (2H, m), 7.12-7.10 (2H, m), 4.69 (2H, s), 4.35-4.32 (2H, m), 4.05-3.96 (1H, m), 3.92-3.85 (2H, m), 3.82-3.75 (1H, m), 3.62-3.35 (12H, bs), 3.18 (3H, s), 3.17 (3H, s), 3.14 (3H, s), 3.10-3.00 (3H, m), 2.70 (2H, s), 2.43-2.40 (2H, m), 2.10-2.03 (2H, m), 1.97-1.93 (2H, m). |
| **116** | 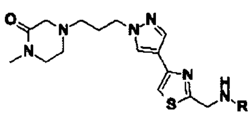 | 2-[2-[[4-[1-[3-(4-methyl-3-oxo-piperazin-1-yl)propyl]pyrazol-4-yl]thiazol-2-yl]methylcarbamoyl]indan-2-yl]acetic acid<br>M/z 537.2 $(M+H)^+$<br>$^1$H NMR (500 MHz, $CDCl_3$): δ 7.89 (1H, s), 7.76 (1H, s), 7.45-7.42 (1H, m), 7.23-7.21 (2H, m), 7.19-7.17 (2H, m), 7.08 (1H, s), 4.66 (2H, d, J = 5.0 Hz), 4.26 (2H, t, J = 6.0 Hz), 3.54 (2H, d, J = 16.5 Hz), 3.38 (2H, t, J = 5.5 Hz), 3.30 (2H, s), 3.13 (2H, d, J = 16.5 Hz), 2.96 (3H, s), 2.85 (2H, s), 2.81 (2H, t, J = 5.5 Hz), 2.54-2.47 (2H, m), 2.15-2.10 (2H, m). |

**Example 117**

**2-[2-[[4-[1-[2-(1-methylpyridin-1-ium-4-yl)ethyl]pyrazol-4-yl]thiazol-2-yl]methylcarbamoyl]indan-2-yl]acetate**

Method C was used as described above.

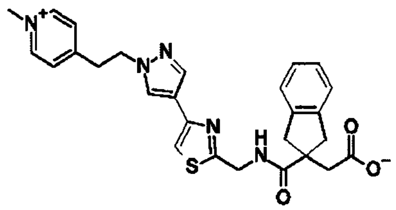

a. 2-(4-pyridyl)ethyl methanesulfonate

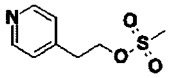

To a stirred solution of 2-(pyridin-4-yl)ethan-1-ol (1 g, 8.13 mmol) in THF (30 mL) was added $Et_3N$ (1.7 mL, 12.1 mmol) and mesyl chloride (1.1 g, 9.75 mmol) at 0 °C and stirred at same temperature for 3 h. The mixture was diluted with cold water (20 mL), adjusted pH to ~7 with saturated aqueous sodium bicarbonate solution and extracted the product with EtOAc (30 mL). The combined organic layer was dried, filtered and evaporated affording a yellow solid (1.1 g, crude). M/z 202.1 $(M+H)^+$.

b. Tert-butyl N-[[4-[1-[2-(4-pyridyl)ethyl]pyrazol-4-yl]thiazol-2-yl]methyl]carbamate

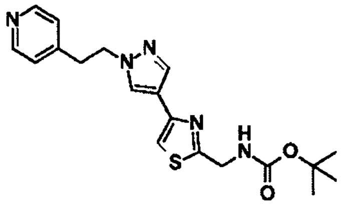

To a solution of tert-butyl ((4-(1H-pyrazol-4-yl)thiazol-2-yl)methyl)carbamate (150 mg, 0.53 mmol) in DMF (10 mL) was added $Cs_2CO_3$ (2.3 g, 7.32 mmol) and 2-(4-pyridyl)ethyl methanesulfonate (215 mg, 1.07 mmol) at room temperature. The mixture was heated at 100 °C for 16 h. The reaction mixture was filtered through celite pad and washed the pad with EtOAc (20 mL). The filtrate was concentrated and the residue was purified by reverse phase chromatography affording a brown solid (55 mg, 26%). M/z 386.1 $(M+H)^+$.

c. 2-[2-[[4-[1-[2-(1-methylpyridin-1-ium-4-yl)ethyl]pyrazol-4-yl]thiazol-2-yl]methylcarbamoyl]indan-2-yl]acetate

The title product was obtained following the procedure described in example 106 step-d and step-e and example 107. The crude compound was purified by preparative HPLC [SYMMETRY-C18 (300*19), 7 u, Mobile phase: A: 0.1% Formic Acid in $H_2O$, B: MeCN] affording an off white solid (21 mg, 46%). M/z 502.2 $(M+H)^+$. $^1H$ NMR (500 MHz, DMSO-$d_6$): δ 8.83 (2H, d, J = 6.5 Hz), 8.08 (1H, s), 7.90 (2H, d, J = 6.5 Hz), 7.80 (1H, d, J = 1.5 Hz), 7.48 (1H, s), 7.18-7.16 (2H, m), 7.12-7.10 (2H, m), 4.57 (2H, t, J = 6.5 Hz), 4.52 (2H, d, J = 6 Hz), 4.24 (3H, s), 3.47 (2H, t, J = 6.5 Hz), 3.40 (2H, d, J = 16 Hz), 2.92 (2H, d, J = 16 Hz), 2.54 (2H, s).

Other compounds prepared by the method C described for Example 117 and purified in a similar manner by preparative HPLC are shown in the Table below, wherein R is the moiety:

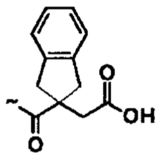

| Example | Structure | Name, MS and NMR data |
|---|---|---|
| **118** | 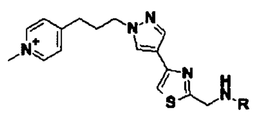 | 2-[2-[[4-[1-[3-(1-methylpyridin-1-ium-4-yl)propyl] pyrazol-4-yl]thiazol-2-yl] methylcarbamoyl]indan-2-yl]acetate<br>M/z 516.2 $(M+H)^+$<br>$^1$H NMR (500 MHz, DMSO-$d_6$): δ 8.80 (2H, d, J = 6.5 Hz), 8.05 (1H, s), 7.92 (2H, d, J = 7.0 Hz), 7.79 (1H, d, J = 0.5 Hz), 7.49 (1H, s), 7.18-7.16 (2H, m), 7.12-7.10 (2H, m), 4.55 (2H, d, J = 5.5 Hz), 4.19-4.17 (2H, m), 4.17 (3H, s), 4.41 (2H, d, J = 16.0 Hz), 2.92 (2H, d, J = 16.0 Hz), 2.86 (2H, t, J = 7.5 Hz), 2.52 (2H, s), 2.26-2.23 (2H, m). |
| **119** | 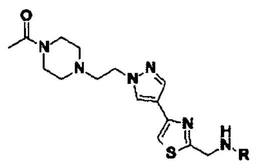 | 2-[2-[[4-[1-[2-(4-acetylpiperazin-1-yl)ethyl]pyrazol-4-yl]thiazol-2-yl] methylcarbamoyl]indan-2-yl]acetic acid<br>M/z 537.2 $(M+H)^+$<br>$^1$H NMR (500 MHz, DMSO-$d_6$): δ 12.15 (1H, bs), 8.67 (1H, bs), 8.10 (1H, s), 7.80 (1H, d, J = 0.5 Hz), 7.50 (1H, s), 7.21-7.19 (2H, m), 7.14-7.12 (2H, m), 4.54 (2H, d, J = 6.0 Hz), 4.24 (2H, t, J = 6.5 Hz), 3.44 (2H, d, J = 16.5 Hz), 3.39-3.35 (4H, m), 2.98 (2H, d, J = 16.5 Hz), 2.73-2.73 (4H, m), 2.40 (2H, t, J = 5.0 Hz), 2.36 (2H, t, J = 5.0 Hz), 1.96 (3H, s). |
| **120** | 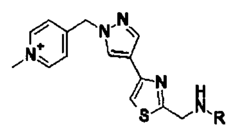 | 2-[2-[[4-[1-[(1-methylpyridin-1-ium-4-yl)methyl]pyrazol-4-yl]thiazol-2-yl] methyl carbamoyl]indan-2-yl]acetate<br>M/z 488.1 $(M+H)^+$<br>$^1$H NMR (500 MHz, MeOD): δ 8.83 (2H, d, J = 6.5 Hz), 8.21 (1H, s), 8.01 (1H, s), 7.75 (2H, d, J = 6.5 Hz), 7.45 (1H, s), 7.20-7.17 (2H, m), 7.14-7.11 (2H, m), 5.75 (2H, s), 4.69 (2H, s), 4.36 (3H, s), 3.51 (2H, d, J = 16 Hz), 3.07 (2H, d, J = 16 Hz), 2.77 (2H, s). |

**Example 121**

**2-[2-[[4-[3-[[4-(dimethylamino)-1-piperidyl]sulfonyl]-4-methoxy-phenyl]thiazol-2-yl]methylcarbamoyl]indan-2-yl]acetic acid**

Method C was used as described above.

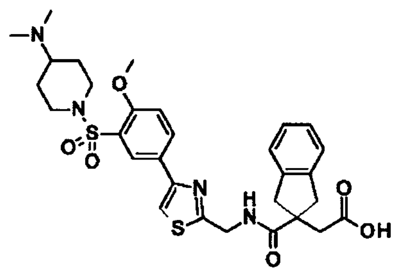

a. 1-(5-bromo-2-methoxy-phenyl)sulfonyl-N,N-dimethyl-piperidin-4-amine

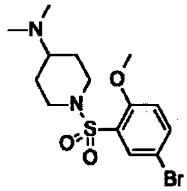

TEA (0.37 mL, 2.63 mmol) and a solution of N,N-dimethylpiperidine-4-amine (247 mg, 1.93 mmol) in DCM (1 mL) were added to a solution of 5-bromo-2-methoxybenzenesulfonyl chloride (500 mg, 1.75 mmol) in DCM (15 mL) at 0°C. The reaction mixture was stirred at 0°C for 40 min, washed with diluted $NaHCO_3$ (5 mL) and water (5 mL), dried and evaporated. The crude product was purified by chromatography on silica eluting with 2-12% (2M $NH_3$ in MeOH) in DCM affording a white solid (627 mg, 95%). M/z 377/379 $(M+H)^+$.

b. tert-butyl N-[[4-[3-[[4-(dimethylamino)-1-piperidyl]sulfonyl]-4-methoxy-phenyl]thiazol-2-yl]methyl]carbamate

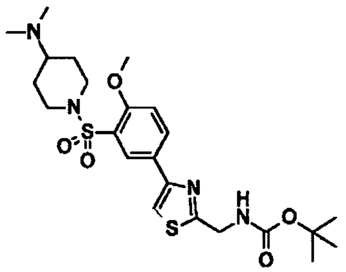

Bis(pinacolato)diboron (103 mg, 0.406 mmol), [1,1'-Bis(diphenylphosphino)ferrocene]dichloropalladium (II) complex in DCM (25 mg, 0.031 mmol) and potassium acetate (92 mg, 0.936 mmol) were added to a solution of 1-(5-bromo-2-methoxyphenyl)sulfonyl-N,N-dimethyl-piperidin-4-amine (118 mg, 0.312 mmol) in dioxane (2 mL) at room temperature. The reaction mixture was flushed with N2, heated at 110°C, stirred for 1 h, and cooled to room temperature. [1,1'-Bis(diphenylphosphino)ferrocene]dichloropalladium (II) complex in DCM (25 mg, 0.031 mmol), tert-butyl ((4-bromothiazole-2-yl)methyl)carbamate (91 mg, 0.312 mmol) and 2M $Na_2CO_3$ aqueous solution (0.31 mL, 0.624 mmol) were successively added. The reaction mixture was heated to 100°C, stirred for 4 h, cooled to room temperature, partitioned between DCM (5 mL) and water (5 mL), and extracted with DCM (5 mL). Combined organic extracts were washed with water (10 mL), dried and evaporated. The crude product was purified by chromatography on silica eluting with 2-12% (2M $NH_3$ in MeOH) in DCM affording a brown solid (106 mg, 67%). M/z 511 $(M+H)^+$.

c. 1-(5-bromo-2-methoxy-phenyl)sulfonyl-N,N-dimethyl-piperidin-4-amine

The title product was obtained following the procedure described in either example 1 step-b and step-d or example 46 step-c to step-e. The crude product was purified by preparative hplc (MDAP) affording a white solid (53.6 mg, 57%). M/z 613.3 $(M+H)^+$. $^1H$ NMR (d6-DMSO) $\delta$ 8.92 (1H, bs), 8.31 (1H, m), 8.12 (1H, m), 7.96 (1H, s), 7.31 (1H, m), 7.20 (2H, m), 7.11 (2H, m), 4.60 (2H, d, $J$ = 6 Hz), 3.91 (3H, s), 3.68 (2H, m), 3.48 (2H, d, $J$ = 16 Hz), 2.98 (2H, d, $J$ = 16 Hz), 2.71 (2H, s), 2.62 (2H, m), 2.19 (1H, m), 2.12 (6H, s), 1.73 (2H, m), 1.32 (2H, m).

**Example 122**

**2-[2-[[4-[3-[[4-(dimethylamino)-1-piperidyl]sulfonyl]-4-methoxy-phenyl]thiazol-2-yl]methylcarbamoyl]indan-2-yl]acetic acid**

Method C was used as described above.

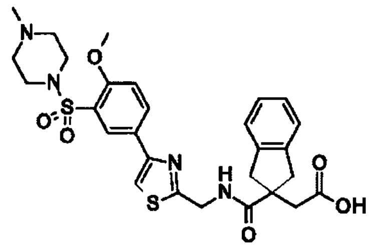

The title product was obtained following the procedure described in example 121 starting with N-methylpiperazine in step-a. The crude product was purified by preparative hplc (MDAP) affording a white solid (22.3 mg, 67%). M/z 585.1 $(M+H)^+$. $^1H$ NMR (d6-DMSO) $\delta$ 10.73 (1H, bs), 8.70 (1H, m), 8.33 (1H, m), 8.20 (1H, m), 8.00 (1H, s), 7.39 (1H, m), 7.20 (2H, m), 7.11 (2H, m), 4.59 (2H, d, $J$ = 6 Hz), 3.96 (3H, s), 3.82 (2H, m), 3.48 (4H, m), 3.11 (4H, m), 2.99 (2H, d, $J$ = 16 Hz), 2.75 (5H, m).

**Example 123**

**2-[2-[[4-[3-[[4-(dimethylamino)-1-piperidyl]sulfonyl]-4-methoxy-phenyl]thiazol-2-yl]methylcarbamoyl]-5,6-difluoro-indan-2-yl]acetic acid**

Method C was used as described above.

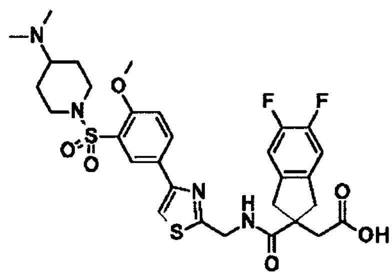

The title product was obtained following the procedure described in example 121 using 2-(2-(tert-butoxy)-2-oxoethyl)-5,6-difluoro-2,3-dihydro-1H-indene-2-carboxylic acid in step-d. The crude product was purified by preparative hplc (MDAP) affording a white solid (59.7 mg, 71%). M/z 649.2 $(M+H)^+$. $^1H$ NMR (d6-DMSO) $\delta$ 8.91 (1H, bs), 8.31 (1H, m), 8.13 (1H, m), 7.99 (1H, s), 7.29 (3H, m), 4.58 (2H, d, $J$ = 6 Hz), 3.91 (3H, s), 3.68 (2H, m), 3.48 (2H, d, $J$ = 16 Hz), 2.98 (2H, d, $J$ = 16 Hz), 2.74 (2H, s), 2.62 (2H, m), 2.19 (1H, m), 2.09 (6H, s), 1.72 (2H, m), 1.32 (2H, m).

**Example 124**

**2-[5,6-difluoro-2-[[4-[4-methoxy-3-[[4-(trimethylammonio)-1-piperidyl]sulfonyl] phenyl]thiazol-2-yl]methylcarbamoyl]indan-2-yl] acetate**

Method C was used as described above.

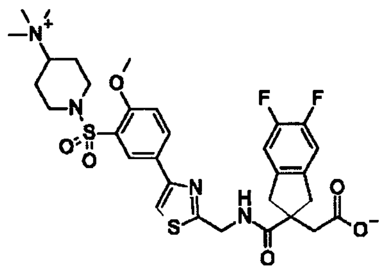

a. [1-[5-[2-[[[2-(2-tert-butoxy-2-oxo-ethyl)-5,6-difluoro-indane-2-carbonyl]amino]methyl]thiazol-4-yl]-2-methoxy-phenyl]sulfonyl-4-piperidyl]-trimethylammonium

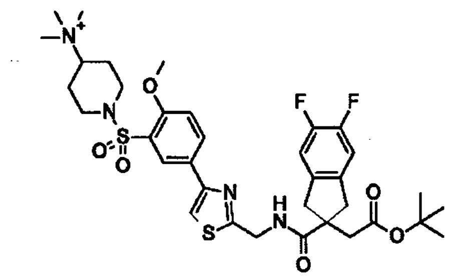

MeI (123 mg, 0.868 mmol) was added to a solution of tert-butyl 2-[2-[[4-[3-[[4-(dimethylamino)-1-piperidyl]sulfonyl]-4-methoxy-phenyl]thiazol-2-yl]methylcarbamoyl]-5,6-difluoro-indan-2-yl]acetate (122 mg, 0.174 mmol) in acetonitrile (2 mL) at 0°C. The reaction mixture was stirred at room temperature overnight and evaporated affording a white solid which was used in the next step without further purification (152 mg, crude). M/z 719 $(M)^+$.

b. 2-[5,6-difluoro-2-[[4-[4-methoxy-3-[[4-(trimethylammonio)-1-piperidyl]sulfonyl]phenyl]thiazol-2-yl]methylcarbamoyl]indan-2-yl]acetate

The title product was obtained following the procedure described in either example 1 step-d or example 46 step-e. The crude product was purified by preparative hplc (MDAP) affording a white solid (101 mg, 88% over 2 steps). M/z 663.3 $(M+H)^+$. $^1H$ NMR (d6-DMSO) δ 13.02 (1H, bs), 8.32 (1H, m), 8.19 (1H, m), 7.36 (1H, s), 7.21 (2H, m), 4.61 (2H, d, *J* = 6 Hz), 3.95 (3H, s), 3.90 (2H, m), 3.46 (1H, m), 3.38 (2H, d, *J* = 16 Hz), 3.00 (9H, s), 2.86 (2H, d, *J* = 16 Hz), 2.62 (2H, m), 2.35 (2H, s), 2.19 (2H, m), 1.68 (2H, m).

**Example 125**

**2-[2-[[4-[5-[[4-(dimethylamino)-1-piperidyl]sulfonyl]-2-methoxy-phenyl]thiazol-2-yl]methylcarbamoyl]-5,6-difluoro-indan-2-yl]acetic acid**

Method C was used as described above.

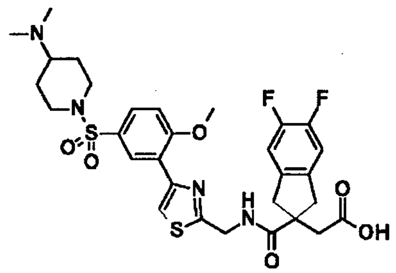

The title product was obtained following the procedure described in example 123 starting with 3-bromo-4-methoxybenzenesulfonyl chloride. The crude product was purified by preparative hplc (MDAP) affording a white solid (55 mg, 62%). M/z 649.3 $(M+H)^+$. $^1H$ NMR (d6-DMSO) δ 8.97 (1H, bs), 8.49 (1H, m), 8.11 (1H, s), 7.68 (1H, m), 7.37 (1H, m), 7.26 (2H, m), 4.61 (2H, d, *J* = 6 Hz), 4.02 (3H, s), 3.55 (2H, m), 3.41 (2H, d, *J* = 16 Hz), 2.99 (2H, d, *J* = 16 Hz), 2.72 (2H, s), 2.30 (2H, m), 2.10 (6H, s), 2.06 (1H, m), 1.72 (2H, m), 1.40 (2H, m).

**Example 126**

**2-[5,6-difluoro-2-[[4-[2-methoxy-5-[[4-(trimethylammonio)-1-piperidyl]sulfonyl]phenyl]thiazol-2-yl]methylcarbamoyl]indan-2-yl]acetate**

Method C was used as described above.

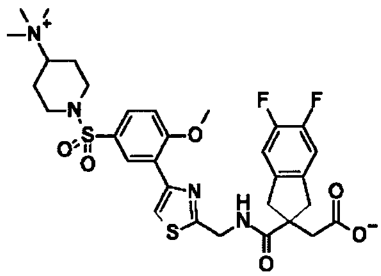

The title product was obtained following the procedure described in example 124 starting with tert-butyl 2-[2-[[4-[5-[[4-(dimethylamino)-1-piperidyl]sulfonyl]-2-methoxy-phenyl]thiazol-2-yl]methylcarbamoyl]-5,6-difluoro-indan-2-yl]acetate. The crude product was purified by preparative hplc (MDAP) affording a white solid (58.5 mg, 60%). M/z 663.3 $(M+H)^+$. $^1H$ NMR (d6-DMSO) $\delta$ 13.09 (1H, bs), 8.51 (1H, m), 8.12 (1H, s), 7.73 (1H, m), 7.40 (1H, m), 7.21 (2H, m), 4.62 (2H, d, $J$ = 6 Hz), 4.04 (3H, s), 3.81 (2H, m), 3.36 (3H, m), 2.97 (9H, s), 2.86 (2H, d, $J$ = 16 Hz), 2.35 (2H, s), 2.22 (4H, m), 1.73 (2H, m).

**Example 127**

**2-[2-[[4-[3-(4-methylpiperazin-1-yl)sulfonylphenyl]thiazol-2-yl]methylcarbamoyl]indan-2-yl]acetic acid**

Method C was used as described above.

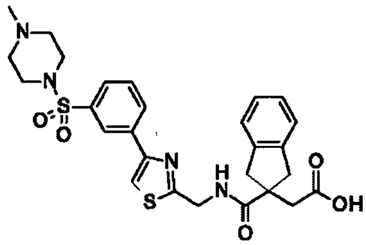

a. 1-(3-bromophenyl)sulfonyl-4-methyl-piperazine

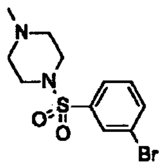

To a stirred solution of 3-bromobenzene-1-sulfonyl chloride (1.53 g, 6.0 mmol) in DCM (20 mL) was added Et3N (1 mL, 7.5 mmol) and 1-methylpiperazine (500 mg, 5.0 mmol) at 0 °C and stirred at RT for 2 h. The reaction mixture was diluted with water (80 mL), extracted with DCM (2x120 mL) and combined organic layer was dried, filtered and evaporated. The crude was purified by chromatography on neutral alumina eluting with 2% MeOH in DCM affording a pale yellow solid (1.2 g, 75%). M/z 319.0 $(M+H)^+$.

b. [3-(4-methylpiperazin-1-yl)sulfonylphenyl]boronic acid

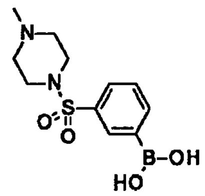

To a solution of 1-(3-bromophenyl)sulfonyl-4-methyl-piperazine (1.1 g, 3.45 mmol) in dioxane (10 mL) was added KOAc (1 g, 10.3 mmol), Bis(pinacolato)diboron (1 g, 4.15 mmol) and purged with argon for 15 minutes. Then $PdCl_2$(dppf).DCM (141 mg, 0.17 mmol) was added, purged with argon for further 5 minutes and heated at 90 °C for 4 h. The reaction mixture was filtered through celite pad, washed the pad with EtOAc (20 mL) and filtrate was evaporated. The crude was purified by reverse phase chromatography affording a white solid (200 mg, 20%). M/z 285.1 $(M+H)^+$.

c. Tert-butyl N-[[4-[3-(4-methylpiperazin-1-yl)sulfonylphenyl]thiazol-2-yl]methyl]carbamate

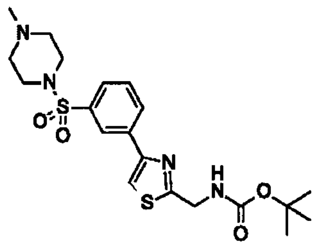

To a stirred solution of tert-butyl((4-bromothiazol-2-yl)methyl)carbamate (200 mg, 0.68 mmol) in dioxane: $H_2O$ (10 mL, 5:1) was added $Na_2CO_3$ (214 mg, 2.05 mmol) and [3-(4-methylpiperazin-1-yl)sulfonylphenyl]boronic acid (195 mg, 0.68 mmol) at room temperature and purged with argon for 15 minutes. Then $Pd(PPh_3)_4$ (80 mg, 0.06 mmol) was added, purged with argon for 5 minutes and heated at 90 °C for 8 h. The reaction mixture was filtered through celite pad and washed the pad with EtOAc (25 mL). The filtrate was washed with water, dried, filtered and evaporated. The crude was purified by preparative TLC, eluting with 5% MeOH in DCM affording a pale yellow solid (200 mg, 65%). M/z 453.2 $(M+H)^+$.

d. 2-[5,6-difluoro-2-[[4-[4-methoxy-3-[[4-(trimethylammonio)-1-piperidyl]sulfonyl]phenyl]thiazol-2-yl]methylcarbamoyl]indan-2-yl]acetate

The title product was obtained following the procedure described in either example 1 step-b to step-d or example 46 step-c to step-e. The crude compound was purified by preparative HPLC SYMMETRY-C18 (300*19), 7 u, Mobile phase: A: 0.1% Formic Acid in $H_2O$, B: MeCN] affording an off white solid (50 mg, 55%). M/z 555.2 $(M+H)^+$. $^1H$ NMR (500 MHz, DMSO-$d_6$): δ 8.69 (1H, t, J = 6 Hz), 8.26-8.24 (3H, m), 7.74-7.68 (2H, m), 7.21-7.20 (2H, m), 7.14-7.13 (2H, m), 4.60 (2H, d, J = 5.5 Hz), 3.45 (2H, d, J = 16.5 Hz), 2.99 (2H, d, J = 16.5 Hz), 2.91 (4H, bs), 2.75 (2H, s), 2.36 (4H, bs), 2.14 (3H, bs).

## Example 128

**2-[2-[[4-[3-(4,4-dimethylpiperazin-4-ium-1-yl)sulfonylphenyl]thiazol-2-yl]methylcarbamoyl]indan-2-yl]acetate**

Method C was used as described above.

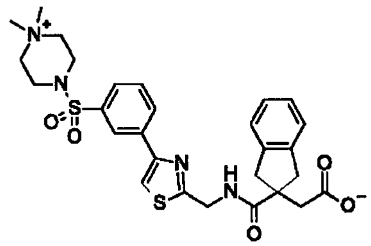

The title product was obtained following the procedure described in example 124 starting with tert-butyl 2-[2-[[4-[3-(4-methylpiperazin-1-yl)sulfonylphenyl]thiazol-2-yl]methylcarbamoyl]indan-2-yl]acetate. The crude compound was purified by preparative HPLC SYMMETRY-C8 (300*19), 7 u Mobile phase: A: 0.1% Formic Acid in $H_2O$, B: MeCN] affording an off white solid (38 mg, 42%). M/z 569.2 $(M+H)^+$. $^1H$ NMR (500 MHz, DMSO-$d_6$): δ 8.32-8.30 (2H, m), 8.24 (1H, s), 7.75-7.74 (2H, m), 7.17-7.16 (2H, m), 7.11-7.10 (2H, m), 4.62 (2H, d, J = 5.5 Hz), 3.52-3.51 (4H, m), 3.42-3.36 (6H, m), 3.05 (6H, s), 2.94 (2H, d, J = 16 Hz), 2.49 (2H, s).

Other compounds prepared by the method C described for Example 127 and 128 using commercial amine reagents in step-a example 127 and starting with either 3-bromobenzene-1-sulfonyl chloride or 4-bromobenzene-1-sulfonyl chloride purified in a similar manner by preparative HPLC are shown in the Table below, wherein R is the moiety:

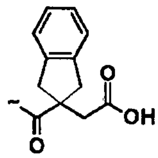

| Example | Structure | Name, MS and NMR data |
|---|---|---|
| **129** | 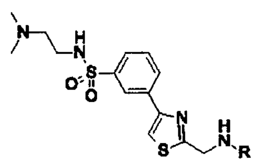 | 2-[2-[[4-[3-[2-(dimethylamino)ethylsulfamoyl]phenyl]thiazol-2-yl]methylcarbamoyl]indan-2-yl]acetic acid<br>M/z 543.2 $(M+H)^+$<br>$^1H$ NMR (500 MHz, DMSO-$d_6$): δ 9.11 (1H, bs), 8.38 (1H, s), 8.16 (1H, d, J = 7.5 Hz), 8.15 (1H, s), 7.75 (1H, d, J = 7.5 Hz), 7.65 (1H, t, J = 7.5 Hz), 7.21-7.20 (2H, m), 7.13-7.12 (2H, m), 4.61 (2H, d, J = 5.5 Hz), 3.45 (2H, d, J = 16 Hz), 2.98 (2H, d, J = 16 Hz), 2.85 (2H, t, J = 6.5 Hz), 2.70 (2H, s), 2.24 (2H, t, J = 6.5 Hz), 2.04 (6H, s). |
| **130** | 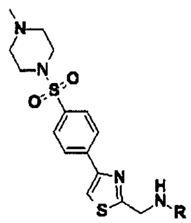 | 2-[2-[[4-[4-(4-methylpiperazin-1-yl)sulfonylphenyl]thiazol-2-yl]methylcarbamoyl]indan-2-yl]acetic acid<br>M/z 555.1 $(M+H)^+$<br>$^1H$ NMR (500 MHz, MeOD): δ 8.15 (2H, d, J = 8 Hz), 7.93 (1H, s), 7.82 (2H, d, J = 8 Hz), 7.22-7.21 (2H, m), 7.16-7.14 (2H, m), 4.72 (2H, s), 3.52 (2H, d, J = 16.5 Hz), 3.15-3.10 (4H, bs), 3.08 (2H, d, J = 16.5 Hz), 2.83 (2H, s), 2.74 (4H, bs), 2.43 (3H, s). |
| **131** | 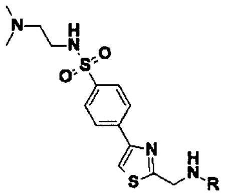 | 2-[2-[[4-[4-[2-(dimethylamino)ethylsulfamoyl]phenyl]thiazol-2-yl]methylcarbamoyl]indan-2-yl]acetic acid<br>M/z 543.1 $(M+H)^+$<br>$^1H$ NMR (500 MHz, DMSO-$d_6$): δ 8.17 (1H, s), 8.12 (2H, d, J = 8.5 Hz), 7.84 (2H, d, J = 8.5 Hz), 7.54 (1H, br s), 7.21-7.20 (2H, m), 7.14-7.12 (2H, m), 4.59 (2H, d, J = 5.5 Hz), 3.45 (2H, d, J = 16.5 Hz), 2.98 (2H, d, J = 16.5 Hz), 2.84 (2H, t, J = 7 Hz), 2.72 (2H, s), 2.23 (2H, t, J = 7 Hz), 2.04 (6H, s). |

**Example 132**

**2-[2-[[4-[3-(4-methylpiperazin-1-yl)sulfonyl-4-(2-morpholinoethoxy)phenyl]thiazol-2-yl]methylcarbamoyl]indan-2-yl]acetic acid**

Method C was used as described above.

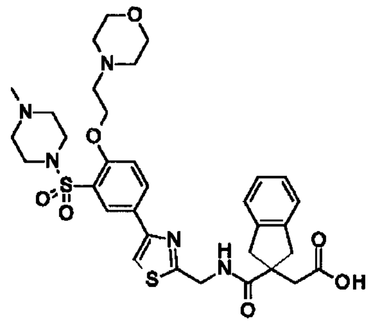

a. 4-bromo-2-(4-methylpiperazin-1-yl)sulfonyl-phenol

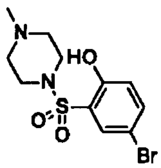

1M $BBr_3$ solution in DCM (32.2 mL, 32.2 mmol) was added dropwise to a solution of 1-(5-bromo-2-methoxyphenyl)sulfonyl-4-methyl-piperazine (750 mg, 2.15 mmol) in DCM (5 mL) at 0°C. The reaction mixture was stirred at room temperature overnight and quenched by addition into MeOH (100 mL) at 0°C. The reaction mixture was evaporated, partitioned between water (10 mL) and DCM (10 mL) and extracted with DCM (2 x 5 mL). The combined organic extracts were washed with water (10 mL), dried and evaporared. The crude product was purified by chromatography on silica eluting with 2-10% MeOH in DCM affording a white solid (200 mg, 28%). M/z 335/337 $(M+H)^+$.

b. 4-[2-[4-bromo-2-(4-methylpiperazin-1-yl)sulfonyl-phenoxy]ethyl]morpholine

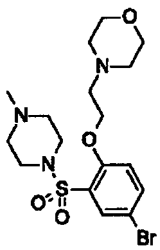

4-(2-bromoethyl)morpholine (116 mg, 0.597 mmol), NaI (45 mg, 0.298 mmol) and $K_2CO_3$ (124 mg, 0.895 mmol) were added to a solution of 4-bromo-2-(4-methylpiperazin-1-yl)sulfonyl-phenol (100 mg, 0.298 mmol) in acetone (3 mL) at room temperature. The reaction mixture was heated at 100°C, stirred under microwave conditions for 3 h, cooled to room temperature and evaporated. The residue was partitioned between, water (5 mL) and EtOAc (5 mL) and extracted with EtOAc (2 x 5 mL). The combined organic extracts were washed with brine (10 mL), dried and evaporated. The crude product was purified by chromatography on silica eluting with 2-10% (2M $NH_3$ in MeOH) in DCM affording a brown solid (91 mg, 68%). M/z 448/450 $(M+H)^+$.

c. 2-[2-[[4-[3-(4-methylpiperazin-1-yl)sulfonyl-4-(2-morpholinoethoxy)phenyl]thiazol-2-yl]methylcarbamoyl]indan-2-yl]acetic acid

The title product was obtained following the procedure described in example 121 step-b and step-c. The crude product was purified by preparative hplc (MDAP) affording a white solid (18.8 mg, 26%). M/z 684.2 $(M+H)^+$. $^1H$ NMR (d6-DMSO) $\delta$ 12.1 (1H, bs), 8.89 (1H, bs), 8.31 (1H, m), 8.12 (1H, m), 7.98 (1H, s), 7.36 (1H, m), 7.21 (2H, m), 7.12 (2H, m), 4.59 (2H, d, *J* = 6 Hz), 4.25 (2H, t), 3.57 (4H, m), 3.45 (2H, d, *J* = 16 Hz), 3.32 (4H, m), 3.15 (4H, m), 2.98 (2H, d, *J* = 16 Hz), 2.71 (4H, m), 2.31 (4H, m), 2.15 (3H, s).

**Example 133**

**2-[2-[[4-[3-(4-methylpiperazin-1-yl)sulfonyl-4-(3-morpholinopropoxy)phenyl]thiazol-2-yl]methylcarbamoyl]indan-2-yl]acetic acid**

Method C was used as described above.

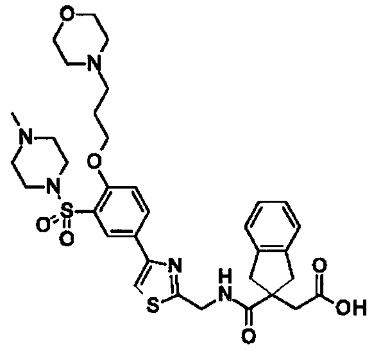

a. 4-[3-[4-bromo-2-(4-methylpiperazin-1-yl)sulfonyl-phenoxy]propyl]morpholine

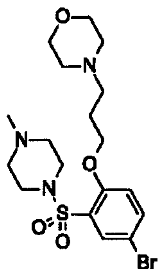

$PPh_3$ (85 mg, 0.323 mmol) and DEAD (0.051 mL, 0.323 mmol) were added to a solution of 4-bromo-2-(4-methylpiperazin-1-yl)sulfonyl-phenol (90 mg, 0.269 mmol) and 4-(3-hydroxypropyl)morpholine (0.045 mL, 0.323 mmol) in THF (2 mL) at room temperature. The reaction mixture was stirred at room temperature for 30 min and evaporated. The crude product was purified by chromatography on silica eluting with 2-12% (2M $NH_3$ in MeOH) in DCM affording a brown solid (121 mg, 97%). M/z 462/464 $(M+H)^+$.

b. 2-[2-[[4-[3-(4-methylpiperazin-1-yl)sulfonyl-4-(3-morpholinopropoxy)phenyl]thiazol-2-yl]methylcarbamoyl]indan-2-yl]acetic acid

The title product was obtained following the procedure described in example 121 step-b and step-c. The crude product was purified by preparative hplc (MDAP) affording a white solid (19.9 mg, 24%). M/z 698.3 $(M+H)^+$. $^1H$ NMR (d6-DMSO) $\delta$ 12.92 (1H, bs), 8.31 (1H, m), 8.13 (1H, m), 7.95 (1H, s), 7.31 (1H, m), 7.19 (2H, m), 7.10 (2H, m), 4.60 (2H, d, *J* = 6 Hz), 4.19 (2H, t), 3.58 (4H, m), 3.39 (2H, d, *J* = 16 Hz), 3.12 (4H, m), 2.88 (2H, d, *J* = 16 Hz), 2.49 (2H, m), 2.40-2.29 (10H, m), 2.14 (3H, s), 1.90 (2H, m).

**Example 134**

**2-[2-[[4-[3-[[4-(dimethylamino)-1-piperidyl]sulfonyl]-4-(2-morpholinoethoxy)phenyl]thiazol-2-yl]methylcarbamoyl]-5,6-difluoro-indan-2-yl]acetic acid**

Method C was used as described above.

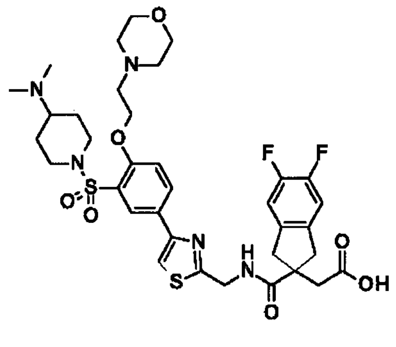

The title product was obtained following the procedure described in example 121 using N,N-dimethylpiperidine-4-amine in step-a and 2-(2-(tert-butoxy)-2-oxoethyl)-5,6-difluoro-2,3-dihydro-1H-indene-2-carboxylic acid. The crude product was purified by preparative hplc (MDAP) affording a white solid (50.6 mg, 90%). M/z 748.4 $(M+H)^+$. $^1H$ NMR (d6-DMSO) $\delta$ 8.95 (1H, bs), 8.31 (1H, m), 8.12 (1H, m), 7.98 (1H, s), 7.34 (1H, m), 7.27 (2H, m), 4.59 (2H, d, *J* = 6 Hz), 4.24 (2H, t), 3.70 (2H, m), 3.58 (4H, m), 3.42 (2H, d, *J* = 16 Hz), 2.98 (2H, d, *J* = 16 Hz), 2.79-2.61 (7H, m), 2.19 (1H, m), 2.15 (6H, s), 2.10 (3H, s), 1.72 (2H, m), 1.34 (2H, m).

**Example 135**

**2-[2-[[4-[3-methylsulfonyl-4-(2-morpholinoethoxy)phenyl]thiazol-2-yl]methylcarbamoyl]indan-2-yl]acetic acid**

Method C was used as described above.

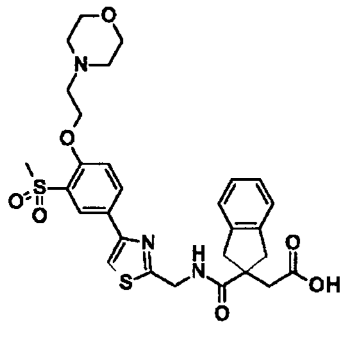

a. Tert-butyl N-[[4-(4-hydroxy-3-methylsulfonyl-phenyl)thiazol-2-yl]methyl]carbamate

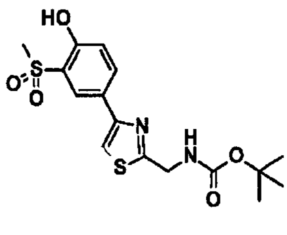

This was obtained following the procedure described in example 127 step-a and step-c starting with 4-bromo-2-methylsulfonyl-phenol. The crude product was chromatographed on silica eluting with 50% EtOAc in petroleum ether

affording a yellow solid (170 mg, 32%). M/z 383.0 (M-H)-.

b. Tert-butylN-[[4-[3-methylsulfonyl-4-(2-morpholinoethoxy)phenyl]thiazol-2-yl]methyl]carbamate

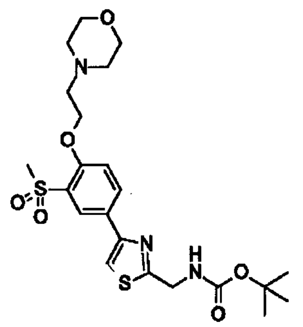

This was obtained following the procedure described in example 132 step-b. The crude product was chromatographed on silica eluting with 2-5 % MeOH in DCM affording a pale brown solid (200 mg, 90%). M/z 498.1 $(M+H)^+$.

c. 2-[2-[[4-[3-methylsulfonyl-4-(2-morpholinoethoxy)phenyl]thiazol-2-yl]methylcarbamoyl]indan-2-yl]acetic acid

The title product was obtained following the procedure described in example 46 step-b to step-e. The crude compound was purified by preparative HPLC [STMMETRY-C18 (300*19), 7 u, Mobile phase: A: 0.1% Formic Acid in $H_2O$, B: MeCN] affording a white solid (49 mg, 60%). M/z 600.1 $(M+H)^+$. $^1H$ NMR (500 MHz, $CDCl_3$): $\delta$ 8.47 (1H, d, J = 2.5 Hz), 7.96 (1H, dd, J = 8.5 Hz, J = 2.5 Hz), 7.36 (1H, s), 7.26-7.24 (2H, m), 7.23-7.19 (2H, m), 7.15-7.13 (1H, m), 7.07 (1H, d, J = 8.5 Hz), 4.70 (2H, d, J = 5.5 Hz), 4.46 (2H, bs), 3.84 (4H, bs), 3.53 (2H, d, J = 16.5 Hz), 3.28 (3H, s), 3.20-3.18 (2H, bs), 3.19 (2H, d, J = 16.5 Hz), 3.10-2.90 (4H, m), 2.92 (2H, s).

**Example 136**

**2-[2-[[4-[4-[2-(4-methylmorpholin-4-ium-4-yl)ethoxy]-3-methylsulfonyl-phenyl]thiazol-2-yl]methylcarbamoyl]indan-2-yl]acetate**

Method C was used as described above.

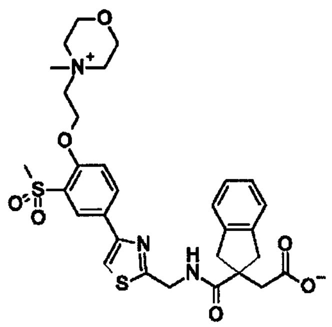

The title product was obtained following the procedure described in example 67 step-c and step-d. The crude compound was purified by preparative HPLC X-BRIDGE -C18 (150X25 mm), 5 u, Mobile phase: A: 0.1% Formic Acid in $H_2O$, B: MeCN] affording a white solid (36 mg, 65%). M/z 614.2 $(M+H)^+$. $^1H$ NMR (500 MHz, DMSO-$d_6$): $\delta$ 8.41 (1H, d, J = 2.5 Hz), 8.28 (1H, dd, J = 9.0 Hz, J = 2.5 Hz), 8.03 (1H, s), 7.42 (1H, d, J = 9.0 Hz), 7.17-7.16 (2H, m), 7.12-7.09 (2H, m), 4.74 (2H, bs), 4.60 (2H, d, J = 5.5 Hz), 4.10 (2H, bs), 3.97-3.96 (4H, m), 3.67-3.63 (2H, m), 3.57-3.55 (2H, m), 3.40 (2H, d, J = 16.0 Hz), 3.36-3.30 (6H, bs), 2.90 (2H, d, J = 16.0 Hz), 2.43 (2H, s).

**Example 137**

**2-[2-[[4-[3-[4-(dimethylamino)piperidine-1-carbonyl]-4-methoxy-phenyl]thiazol-2-yl]methylcarbamoyl]indan-2-yl]acetic acid**

Method C was used as described above.

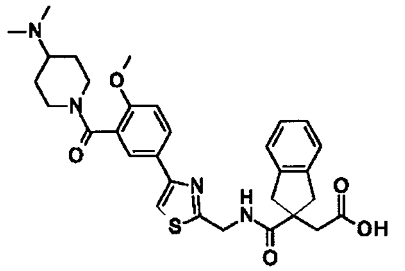

a. (5-bromo-2-methoxy-phenyl)-[4-(dimethylamino)-1-piperidyl]methanone

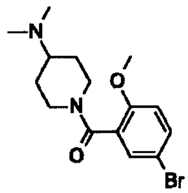

To a stirred solution of 5-bromo-2-methoxybenzoic acid (3 g, 12.9 mmol) in DMF (30 mL) was added $Et_3N$ (5.5 mL, 38.9 mmol), N,N-dimethylpiperidin-4-amine (1.82 g, 14.2 mmol) and T3P (12 mL, 19.2 mmol) at room temperature and stirred for 16 h. The reaction mixture was diluted with water (60 mL), extracted with EtOAc (2 x 100 mL) and combined organic layer was dried, filtered and evaporated. The crude was purified by chromatography on neutral alumina eluting with 10% MeOH in DCM affording a pale brown solid (3.1 g, 75%). M/z 341.1 $(M+H)^+$.

b. [4-(dimethylamino)-1-piperidyl]-[2-methoxy-5-(4,4,5,5-tetramethyl-1,3,2-dioxaborolan-2-yl)phenyl]methanone

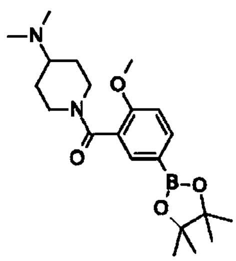

To a solution of (5-bromo-2-methoxy-phenyl)-[4-(dimethylamino)-1-piperidyl]methanone (500 mg, 1.47 mmol) in dioxane (20 mL) was added KOAc (432 mg, 4.41 mmol), bis(pinacolato)diboron (450 mg, 1.76 mmol) and purged with argon for 15 minutes. Then $PdCl_2$(dppl).DCM (60 mg, 0.07 mmol) was added, purged with argon for further 5 minutes and heated at 100 °C for 8 h. The reaction mixture was filtered through celite pad, washed the pad with EtOAc and filtrate was evaporated affording a brown solid (700 mg, crude). M/z 388.9 $(M+H)^+$.

c. Tert-butylN-[[4-[3-[4-(dimethylamino)piperidine-1-carbonyl]-4-methoxy-phenyl]thiazol-2-yl]methyl]carbamate

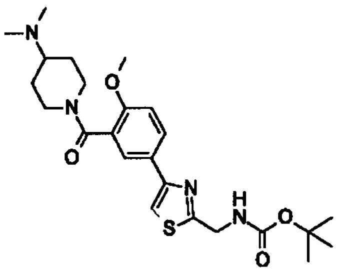

To a stirred solution of tert-butyl((4-bromothiazol-2-yl)methyl)carbamate (300 mg, 1.02 mmol) in dioxane/$H_2O$ (10 mL, 4:1) was added $Na_2CO_3$ (330 mg, 3.08 mmol), [4-(dimethylamino)-1-piperidyl]-[2-methoxy-5-(4,4,5,5-tetramethyl-1,3,2-dioxaborolan-2-yl)phenyl]methanone (600 mg, 1.54 mmol) at room temperature and purged with argon for 15 minutes. Then $PdCl_2$(dppf).DCM (42 mg, 0.05 mmol) was added and heated at 100 °C for 16 h. The reaction mixture was filtered through celite pad, washed the pad with EtOAc and filtrate was washed with water (40 mL). The organic extract was dried, filtered and evaporated. The crude was puirified by silica gel chromatography eluting with 10-20% MeOH in DCM affording a pale brown solid (350 mg, 72%). M/z 474.8 $(M+H)^+$.

d. 2-[2-[[4-[3-(4-methylpiperazin-1-yl)sulfonyl-4-(3-morpholinopropoxy)phenyl]thiazol-2-yl]methylcarbamoyl]indan-2-yl]acetic acid

The title product was obtained following the procedure described in either example 1 step-b and step-d or example 46 step-c to step-e. The crude compound was purified by preparative HPLC SYMMETRY-C18 (300*19), 7 u, Mobile phase: A: 0.1% Formic Acid in $H_2O$, B: MeCN] affording an off white solid (30 mg, 45%). LCMS 93%, M/z 577.3 $(M+H)^+$. $^1H$ NMR (500 MHz, DMSO-$d_6$): δ 9.11-8.77 (1H, m), 7.94-7.89 (2H, m), 7.80-7.70 (1H, d, J = 2 Hz), 7.20-7.19 (2H, m), 7.14-7.11 (3H, m), 4.61-4.51 (3H, m), 3.82 (3H, s), 3.48-3.33 (3H, m), 3.01-2.92 (3H, m), 2.77-2.70 (3H, m), 2.50-2.30 (1H, m), 2.20 (6H, s), 1.90-1.70 (1H, m), 1.69 (1H, m), 1.37-1.31 (2H, m).

## Example 138

**2-[2-[[4-[4-methoxy-3-[4-(trimethylammonio)piperidine-1-carbonyl]phenyl]thiazol-2-yl]methylcarbamoyl]indan-2-yl]acetate**

Method C was used as described above.

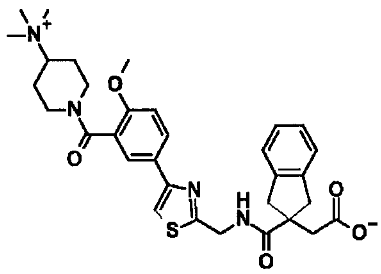

The title product was obtained following the procedure described in example 124 starting with tert-butyl 2-[2-[[4-[3-[4-(dimethylamino)piperidine-1-carbonyl]-4-methoxy-phenyl]thiazol-2-yl]methylcarbamoyl]indan-2-yl]acetate. The crude was purified by preparative HPLC KROMASIL-C18 (150X25 mm), 10 u, Mobile phase: A: 0.1% Formic Acid in $H_2O$, B: MeCN] affording a white solid (15 mg, 47%). M/z 591.3 $(M+H)^+$. $^1H$ NMR (500 MHz, DMSO-$d_6$): δ 7.97 (1H, d, J = 8.5 Hz), 7.88 (1H, s), 7.86 (1H, m), 7.18-7.10 (5H, m), 4.74-4.71 (1H, m), 4.58 (2H, d, J = 5 Hz), 3.87 (3H, s), 3.59-3.40 (2H, m), 3.40-3.30 (2H, bs), 3.04 (9H, s), 3.02-2.98 (1H, m), 2.93 (2H, d, J = 16 Hz), 2.76-2.71 (1H, m), 2.52-2.49 (2H, bs), 2.22-1.97 (2H, m), 1.75-1.40 (2H, m).

**Example 139**

**2-[2-[[4-[3-[[4-(dimethylamino)-1-piperidyl]sulfonyl]-4-(3-pyrrolidin-1-ylpropoxy)phenyl]thiazol-2-yl]methylcarbamoyl]-5,6-difluoro-indan-2-yl]acetic acid**

Method C was used as described above.

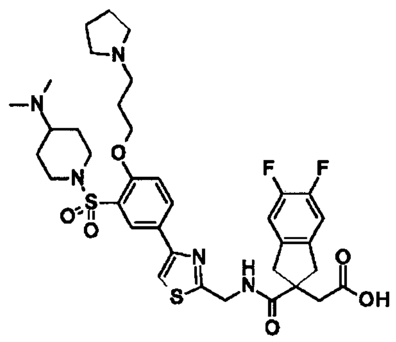

a. 1-(5-bromo-2-methoxy-phenyl)sulfonyl-N,N-dimethyl-piperidin-4-amine

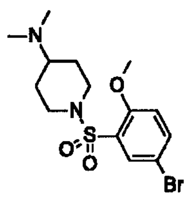

To a stirred solution of N,N-dimethylpiperidin-4-amine (2 g, 15.6 mmol) in dichloromethane (40 mL) was added $Et_3N$ (3.3 mL, 23.4 mmol) at 0 °C. Then 5-bromo-2-methoxybenzenesulfonyl chloride (4.9 g, 17.1 mmol) in DCM (10 mL) was added drop wise for 10 minutes at 0 °C and stirred at room temperature for 2 h. The reaction mixture was diluted with cold water (30 mL), extracted with DCM (2x30 mL) and combined organic layer was dried, filtered and evaporated affording a yellow solid (6 g, 93%). M/z 376.8 $(M+H)^+$.

b. 4-bromo-2-[[4-(dimethylamino)-1-piperidyl]sulfonyl]phenol

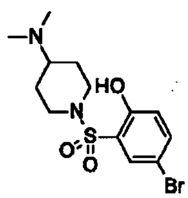

This was prepared following the procedure described in example 132 step-a. The crude was chromatographed on silica eluting with 15-20% MeOH in DCM affording an off white solid (2.4 g, 42%). M/z 362.7 $(M+H)^+$.

c. 4-bromo-2-[[4-(dimethylamino)-1-piperidyl]sulfonyl]phenol

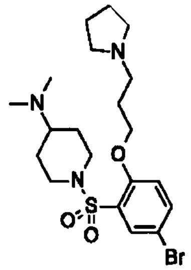

This was prepared following the procedure described in example 55 step-a and step-b using pyrrolidine. The residue was triturated with diethyl ether (2 x 20 mL) to remove solid impurity and combined diethyl ether layer was evaporated affording a colourless solid (450 mg, 74%). M/z 474.1 $(M+H)^+$.

d. 2-[2-[[4-[3-[[4-(dimethylamino)-1-piperidyl]sulfonyl]-4-(3-pyrrolidin-1-ylpropoxy)phenyl]thiazol-2-yl]methylcarbamoyl]-5,6-difluoro-indan-2-yl]acetic acid

The title product was obtained following the procedure described in example 137 step-b to step-d. The crude compound was purified by preparative HPLC [KROMASIL-C18 (150*25), 10 u, Mobile phase: A: 0.1% Formic Acid in $H_2O$, B: MeCN] affording a light brown solid (32 mg, 34%). M/z 746.2 $(M+H)^+$. $^1H$ NMR (500 MHz, DMSO-$d_6$): $\delta$ 8.87 (1H, bs), 8.31 (1H, d, J = 2.5 Hz), 8.11 (1H, dd, J = 8.5 Hz, J = 2.5 Hz), 7.97 (1H, s), 7.31-7.25 (3H, m), 4.58 (2H, d, J = 6 Hz), 4.18 (2H, t, J = 6.5 Hz), 3.69-3.66 (2H, m), 3.42 (2H, d, J = 16.5 Hz), 2.97 (2H, d, J = 16.5 Hz), 2.76 (2H, s), 2.69-2.63 (4H, m), 2.53 (4H, bs), 2.18-2.15 (1H, m), 2.13 (6H, s), 1.95-1.90 (2H, m), 1.75-1.71 (6H, m), 1.35-1.33 (2H, m).

## Example 140

**2-[2-[[4-[6-(2-morpholinoethyl)-3-pyridyl]thiazol-2-yl]methylcarbamoyl]indan-2-yl]acetic acid**

Method C was used as described above.

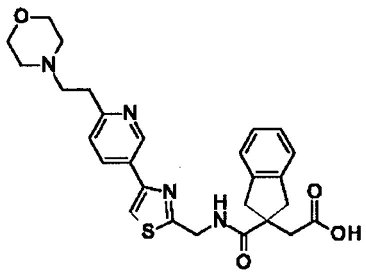

a. 2-(5-bromo-2-pyridyl)ethanol

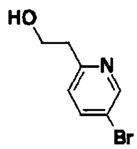

To a stirred solution of 2-(5-bromopyridin-2-yl)acetic acid (2 g, 9.25 mmol) in THF (50 mL) was added $BH_3$.THF (12 mL, 12.03 mmol, 1M in THF) drop wise at room temperature under nitrogen and stirred at same temperature for 24 h. The mixture was cooled to 0 °C, quenched with water: acetic acid (10 mL, 1:1) and stirred at room temperature for 30 minutes. The reaction mixture was evaporated and resulting residue was diluted with EtOAc (50 mL). The organic layer was washed with saturated aqueous sodium bicarbonate solution and water, dried, filtered and evaporated. The crude compound was chromatographed on silica eluting with 40% EtOAc in petroleum ether affording a yellow liquid (950 mg, 51%). M/z = 202.0 $(M+H)^+$.

b. 2-(5-bromo-2-pyridyl)ethyl methanesulfonate

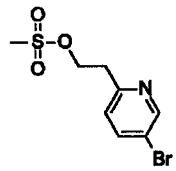

To a solution of 2-(5-bromo-2-pyridyl)ethanol (1 g, 5 mmol) in DCM (30 mL) was added $Et_3N$ (1 mL, 7.5 mmol), mesyl chloride (0.5 mL, 6 mmol) drop wise at 0 °C and allowed to stir at room temperature for 2 h. The mixture was partitioned between cold water (10 mL) and DCM (2 x 30 mL). The organic layer was separated, dried, filtered and evaporated affording as a yellow liquid (1.4 g, crude). M/z 279.7 $(M+H)^+$.

c. 4-[2-(5-bromo-2-pyridyl)ethyl]morpholine

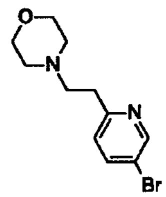

To a solution 2-(5-bromo-2-pyridyl)ethyl methanesulfonate (700 mg, 2.51 mmol) in acetonitrile (15 mL) was added $K_2CO_3$ (1.7 g, 12.58 mmol), morpholine (263 mg, 3.02 mmol) at room temperature and heated at 90 °C for 16 h. The reaction mixture was filtered through celite pad and washed the pad with DCM (20 mL). The filtrate was evaporated and resulting crude was chromatographed on silica eluting with 10% MeOH in DCM affording a yellow solid (500 mg, 73%). M/z 270.8 $(M+H)^+$.

d. 2-[2-[[4-[6-(2-morpholinoethyl)-3-pyridyl]thiazol-2-yl]methylcarbamoyl]indan-2-yl]acetic acid

The title product was obtained following the procedure described in example 127 step-b to step-d. The crude compound was purified by preparative HPLC [KROMASIL-C18 (150*25), 10 u, Mobile phase: A: 0.1% Formic Acid in $H_2O$, B: MeCN] affording an off white solid (36 mg, 40%). M/z 507.2 $(M+H)^+$. $^1H$ NMR (500 MHz, DMSO-$d_6$): δ 12.15 (1H, bs), 9.01 (1H, d, J = 2.5 Hz), 8.68 (1H, t, J = 5.5 Hz), 8.15 (1H, dd, J = 8 Hz, J = 2.5 Hz), 8.07 (1H, s), 7.36 (1H, d, J = 8 Hz), 7.22-7.19 (2H, m), 7.15-7.12 (2H, m), 4.59 (2H, d, J = 6 Hz), 3.56 (4H, t, J = 4.5 Hz), 3.45 (2H, d, J = 16 Hz), 2.99 (2H, d, J = 16 Hz), 2.92-2.90 (2H, m), 2.75 (2H, s), 2.68-2.65 (2H, m), 2.43 (2H, bs).

**Example 141**

**2-[2-[[4-[6-[2-(4-methylmorpholin-4-ium-4-yl)ethyl]-3-pyridyl]thiazol-2-yl]methylcarbamoyl]indan-2-yl]acetate**

Method C was used as described above.

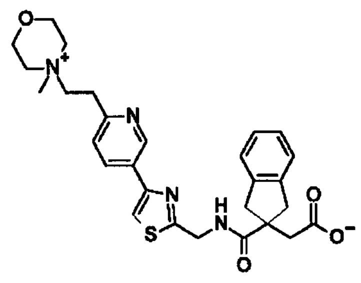

The title product was obtained following the procedure described in example 124 starting with tert-butyl 2-[2-[[4-[6-(2-morpholinoethyl)-3-pyridyl]thiazol-2-yl]methylcarbamoyl]indan-2-yl]acetate. The crude compound was purified by preparative HPLC [KROMASIL-C18 (150*25), 10 u, Mobile phase: A: 0.1% Formic Acid in $H_2O$, B: MeCN] affording an off white solid (23 mg, 36%). M/z 521.3 $(M+H)^+$. $^1H$ NMR (500 MHz, DMSO-$d_6$): δ 9.09 (1H, s), 8.29 (1H, dd, J = 8 Hz, J = 1.5 Hz), 8.14 (1H, s), 7.48 (1H, d, J = 8 Hz), 7.17-7.16 (2H, m), 7.12-7.10 (2H, m), 4.61 (2H, d, J = 5.5 Hz), 3.95 (4H, bs), 3.92-3.88 (2H, m), 3.55-3.50 (4H, m), 3.41 (2H, d, J = 16 Hz), 3.31 (2H, bs), 3.25 (3H, s), 3.92 (2H, d, J = 16 Hz), 2.51 (2H, bs).

**Example 142**

**2-[2-[[4-[6-(4-pyridylmethoxy)-3-pyridyl]thiazol-2-yl]methylcarbamoyl]indan-2-yl]acetic acid**

Method C was used as described above.

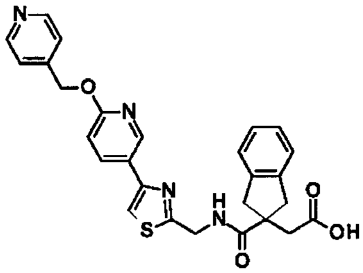

a. Tert-butyl N-[[4-(6-chloro-3-pyridyl)thiazol-2-yl]methyl]carbamate

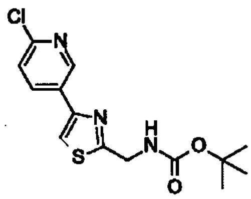

To a stirred solution of tert-butyl ((4-bromothiazol-2-yl) methyl) carbamate (500 mg, 1.71 mmol) in dioxane: $H_2O$ (10 mL, 4:1) was added $Na_2CO_3$ (534 mg, 5.13 mmol), (6-chloropyridin-3-yl) boronic acid (323 mg, 2.05 mmol) at room temperature and purged with argon for 15 minutes. Then $PdCl_2$(dppf).DCM (70 mg, 0.05mmol) was added, argon purged for further 5 minutes and heated at 100 °C for 3 h. The reaction mixture was diluted with water (50 mL), extracted with EtOAc (2 x80 mL) and combined organic layer was evaporated. The crude was chromatographed on silica eluting with 15% EtOAc in petroleum ether affording an off white solid (365 mg, 66%). M/z 326.1 $(M+H)^+$.

b. Tert-butyl N-[[4-[6-(4-pyridylmethoxy)-3-pyridyl]thiazol-2-yl]methyl]carbamate

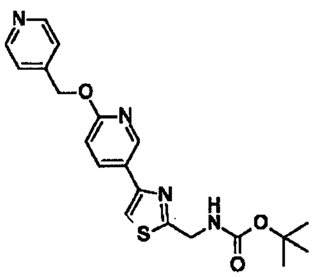

To a solution of tert-butyl N-[[4-(6-chloro-3-pyridyl)thiazol-2-yl]methyl]carbamate (20 mg, 0.06 mmol) in toluene (2 mL) was added NaOtBu (9 mg, 0.09 mmol), pyridin-4-ylmethanol (10 mg, 0.09 mmol) at room temperature and purged with argon for 15 minutes. Then $Pd_2(dba)_3$. $CHCl_3$ (3 mg, 0.003mmol), XPhos (3 mg, 0.006 mmol) was added, argon purged for further 5 minutes and heated at 100 °C for 1 h. The reaction mixture was filtered through celite pad and filtrate was evaporated. The crude was purified by preparative TLC, eluting with 3% MeOH in DCM affording an off white solid (10 mg, 41%). M/z 399.1 $(M+H)^+$.

c. 2-[2-[[4-[6-(4-pyridylmethoxy)-3-pyridyl]thiazol-2-yl]methylcarbamoyl]indan-2-yl]acetic acid

The title product was obtained following the procedure described in example 74 step-b to step-d. The crude compound was purified by preparative HPLC SYMMETRY-C8 (300*19), 7 u, Mobile phase: A: 0.1% Formic Acid in $H_2O$, B: MeCN] affording an off white solid (35 mg, 45%). M/z 501.2 $(M+H)^+$. $^1H$ NMR (500 MHz, DMSO-$d_6$): δ 12.13 (1H, bs), 8.69 (1H, dd, J = 2.5 Hz, J = 0.5 Hz), 8.65 (1H, t, J = 6 Hz), 8.58 (2H, d, J = 6.0 Hz), 8.25 (1H, dd, J = 8.5 Hz, J = 2.5 Hz), 7.96 (1H, s), 7.47 (2H, d, J = 6 Hz), 7.21-7.20 (2H, m), 7.14-7.12 (2H, m), 7.04 (1H, dd, J = 8.5 Hz, J = 0.5 Hz), 5.47 (2H, s), 4.58 (2H, d, J = 6 Hz), 3.47 (2H, d, J = 16.5 Hz), 3.00 (2H, d, J = 16.5 Hz), 2.75 (2H, s).

**Example 143**

**2-[2-[[4-[6-[(1-methylpyridin-1-ium-4-yl)methoxy]-3-pyridyl]thiazol-2-yl]methylcarbamoyl]indan-2-yl]acetate**

Method C was used as described above.

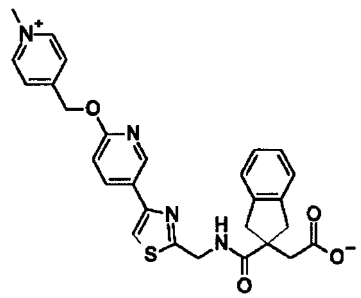

The title product was obtained following the procedure described in example 47 starting with tert-butyl 2-[2-[[4-[6-(4-pyridylmethoxy)-3-pyridyl]thiazol-2-yl]methylcarbamoyl]indan-2-yl]acetate. The crude compound was purified by preparative HPLC SYMMETRY-C8 (300*19), 7 u Mobile phase: A: 0.1% Formic Acid in $H_2O$, B: MeCN] affording an off white solid (16 mg, 18%). M/z 515.1 $(M+H)^+$. $^1H$ NMR (500 MHz, DMSO-$d_6$): δ 12.19 (1H, bs), 8.93 (2H, d, J = 6.5 Hz), 8.66 (1H, dd, J = 2.5 Hz, J = 1 Hz), 8.31 (1H, dd, J = 9 Hz, J = 2.5 Hz), 8.12 (2H, d, J = 6.5 Hz), 7.98 (1H, s), 7.20-7.18 (2H, m), 7.14-7.11 (3H, m), 5.73 (2H, s), 4.58 (2H, d, J = 6 Hz), 4.31 (3H, s), 3.45 (2H, d, J = 16 Hz), 2.98 (2H, d, J = 16 Hz), 2.66 (2H, s).

**Example 144**

**2-[2-[[4-[5-(4-methylpiperazine-1-carbonyl)-3-pyridyl]thiazol-2-yl]methylcarbamoyl]indan-2-yl]acetic acid**

Method C was used as described above.

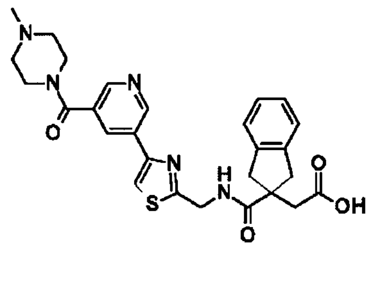

a. Methyl-5-[2-[(tert-butoxycarbonylamino)methyl]thiazol-4-yl]pyridine-3-carboxylate

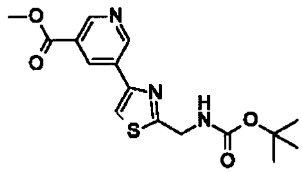

To a stirred solution of tert-butyl ((4-bromothiazol-2-yl) methyl) carbamate (500 mg, 1.71 mmol) in dioxane/water (10 mL, 5:1) was added $Na_2CO_3$ (534 mg, 5.13 mmol), (5-(methoxycarbonyl) pyridin-3-yl) boronic acid (540 mg, 2.05 mmol) at room temperature and purged with argon for 15 minutes. Then $Pd(PPh_3)_4$ (197 mg, 0.17 mmol) was added, purged with argon for further 5 minutes and heated at 90 °C for 7 h. The reaction mixture was filtered through celite pad, washed the pad with EtOAc (20 mL). The filtrate was evaporated. The crude was chromatographed on silica eluting with 30% EtOAc in petroleum ether affording a pale yellow solid (330 mg). M/z 350.1 $(M+H)^+$.

b. 2-[2-[[4-[5-(4-methylpiperazine-1-carbonyl)-3-pyridyl]thiazol-2-yl]methylcarbamoyl]indan-2-yl]acetic acid

The title product was obtained following the procedure described in example 24 step-d to step-e using N-methylpiperazine. The crude was purified by preparative HPLC [SYMMETRY-C8 (300*19), 7 u Mobile phase: A: 0.1% Formic Acid in $H_2O$, B: MeCN] affording an off white solid (13 mg, 19%). M/z 520.2 $(M+H)^+$. $^1H$ NMR (500 MHz, MeOD): $\delta$ 9.17 (1H, bs), 8.55 (1H, bs), 8.36 (1H, s), 7.99 (1H, s), 7.21-7.20 (2H, m), 7.15-7.13 (2H, m), 4.73 (2H, s), 3.83 (2H, bs), 3.55-3.50 (2H, m), 3.53 (2H, d, J = 16.5 Hz), 3.10 (2H, d, J = 16.5 Hz), 2.81 (2H, s), 2.65-2.45 (4H, m), 2.37 (3H, s).

**Example 145**

**2-[2-[[4-[2-(trifluoromethoxy)phenyl]thiazol-2-yl]methylcarbamoyl]indan-2-yl]acetic acid**

Method C was used as described above.

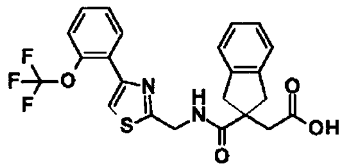

a. Tert-butyl N-[[4-[2-(trifluoromethoxy)phenyl]thiazol-2-yl]methyl]carbamate

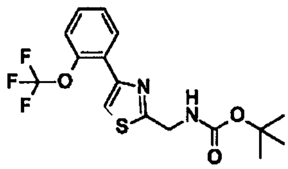

2-(trifluoromethoxy)phenylboronic acid (170 mg, 0.825 mmol), $Pd(PPh_3)_4$ (43 mg, 0.037 mmol) and $Na_2CO_3$ (159 mg, 1.5 mmol) were added to a solution of tert-butyl ((4-bromothiazole-2-yl)methyl)carbamate (220 mg, 0.75 mmol) in 3:1 dioxane/water (4 mL) at room temperature. The reaction mixture was flushed with $N_2$, heated at 100°C and stirred for 2 h. The mixture was cooled to room temperature, partitioned between EtOAc (5 mL) and water (5 mL). The organic extract was washed with brine (5 mL), dried and evaporated. The crude product was used in the next step without further purification.

b. 2-[2-[[4-[2-(trifluoromethoxy)phenyl]thiazol-2-yl]methylcarbamoyl]indan-2-yl]acetic acid

The title product was obtained following the procedure described in example 89 step-b to step-d. The crude product was purified by chromatography on silica eluting with 0-7% MeOH in DCM affording a white solid (99 mg, 58%). M/z 477.3 $(M+H)^+$. $^1H$ NMR (d6-DMSO) δ 12.16 (1H, bs), 8.71 (1H, bs), 8.09 (1H, m), 7.81 (1H, s), 7.49 (3H, m), 7.20 (2H, m), 7.11 (2H, m), 4.60 (2H, d, J = 6 Hz), 3.48 (2H, d, *J* = 16 Hz), 3.00 (2H, d, *J* = 16 Hz), 2.70 (2H, s).

Other compounds prepared by Method C from commercial boronic acid reagents and purified in a similar manner by preparative HPLC are shown in the Table below, wherein R is the moiety:

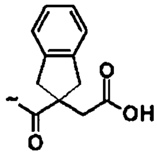

| Example | Structure | Name, MS and NMR data |
|---|---|---|
| **146** | 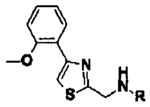 | 2-[2-[[4-(2-methoxyphenyl)thiazol-2-yl]methylcarbamoyl]indan-2-yl]acetic acid<br>M/z 423.3 $(M+H)^+$<br>$^1H$ NMR (d6-DMSO) δ 12.1 (1H, bs), 8.62 (1H, m), 8.11 (1H, m), 7.93 (1H, s), 7.32 (1H, m), 7.21 (2H, m), 7.12 (2H, m), 7.02 (1H, m), 4.60 (2H, d, *J* = 6 Hz), 3.91 (3H, s), 3.46 (2H, d, *J* = 16 Hz), 3.00 (2H, d, *J* = 16 Hz), 2.75 (2H, s). |
| **147** | 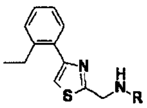 | 2-[2-[[4-(2-ethylphenyl)thiazol-2-yl]methylcarbamoyl]indan-2-yl]acetic acid<br>M/z 421.4 $(M+H)^+$<br>$^1H$ NMR (d6-DMSO) δ 12.14 (1H, bs), 8.64 (1H, m), 7.55 (1H, s), 7.41 (1H, m), 7.31 (2H, m), 7.27-7.18 (3H, m), 7.12 (2H, m), 4.59 (2H, d, *J* = 6 Hz), 3.48 (2H, d, *J* = 16 Hz), 3.00 (2H, d, *J* = 16 Hz), 2.70 (4H, m), 1.07 (3H, t). |
| **148** | 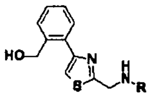 | 2-[2-[[4-[2-(hydroxymethyl)phenyl]thiazol-2-yl]methylcarbamoyl]indan-2-yl]acetic acid<br>M/z 423.3 $(M+H)^+$<br>$^1H$ NMR (d6-DMSO) δ 12.15 (1H, bs), 8.69 (1H, m), 7.75 (1H, s), 7.61 (1H, m), 7.57 (1H, m), 7.39-7.30 (2H, m), 7.20 (2H, m), 7.11 (2H, m), 5.39 (1H, bs), 4.59 (4H, m), 3.48 (2H, d, *J* = 16 Hz), 3.00 (2H, d, *J* = 16 Hz), 2.75 (2H, s). |
| **149** | 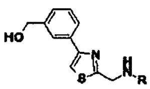 | 2-[2-[[4-[3-(hydroxymethyl)phenyl]thiazol-2-yl]methylcarbamoyl]indan-2-yl]acetic acid<br>M/z 423.3 $(M+H)^+$<br>$^1H$ NMR (d6-DMSO) δ 12.19 (1H, bs), 8.65 (1H, m), 7.90 (1H, s), 7.93 (1H, s), 7.77 (1H, m), 7.37 (1H, m), 7.26 (1H, m), 7.20 (2H, m), 7.11 (2H, m), 4.59 (2H, d, *J* = 6 Hz), 4.54 (2H, s), 3.49 (2H, d, *J* = 16 Hz), 3.00 (2H, d, *J* = 16 Hz), 2.75 (4H, s). |
| **150** | 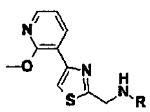 | 2-[2-[[4-(2-methoxy-3-pyridyl)thiazol-2-yl]methylcarbamoyl]indan-2-yl]acetic acid<br>M/z 424.3 $(M+H)^+$<br>$^1H$ NMR (d6-DMSO) δ 12.15 (1H, bs), 8.65 (1H, m), 8.43 (1H, m), 8.15 (1H, s), 8.02 (1H, s), 7.21 (2H, m), 7.12 (3H, m), 4.60 (2H, d, *J* = 6 Hz), 4.02 (3H, s), 3.46 (2H, d, *J* = 16 Hz), 3.00 (2H, d, *J* = 16 Hz), 2.75 (2H, s). |

The title product was obtained following the procedure described in example 89 step-c and step-d. The crude product was purified by chromatography on silica eluting with 2-10% MeOH in DCM affording a white solid (79 mg, 58%). M/z 410.2 $(M+H)^+$. $^1H$ NMR (d6-DMSO) δ 12.11 (1H, bs), 11.98 (1H, bs), 8.52 (1H, m), 8.39 (1H, s), 8.31 (1H, m), 7.41 (1H, m), 7.20 (2H, m), 7.11 (2H, m), 6.35 (1H, m), 4.56 (2H, d, *J* = 6 Hz), 3.48 (2H, d, *J* = 16 Hz), 3.00 (2H, d, *J* = 16 Hz), 2.70 (2H, s).

**Example 153**

**2-[2-[[4-[1-(4-methylpiperazine-1-carbonyl)-4-piperidyl]thiazol-2-yl]methylcarbamoyl]indan-2-yl]acetic acid**

Method B was used as described above.

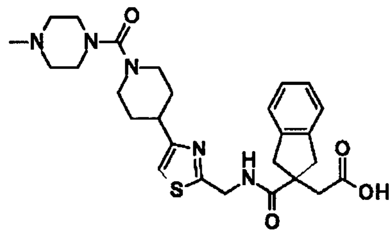

a. Tert-butyl 4-[2-(benzyloxycarbonylaminomethyl)thiazol-4-yl]piperidine-1-carboxylate

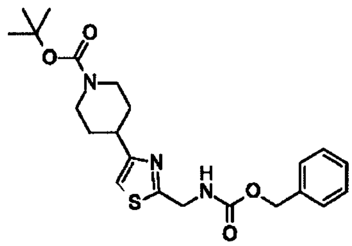

Tert-butyl 4-(2-bromoacetyl)piperidine-1-carboxylate (500 mg, 1.63 mmol) was added to a solution of N-benzyloxycarbonylglycine thioamide (366 mg, 1.63 mmol) in EtOH (8 mL) at room temperature. The reaction mixture was stirred at room temperature overnight and evaporated. The crude product was purified by chromatography on silica eluting with 0-50% EtOAc in hexane affording a white solid (331 mg, 47%). M/z 432 $(M+H)^+$.

b. Benzyl N-[[4-(4-piperidyl)thiazol-2-yl]methyl]carbamate hydrochloride

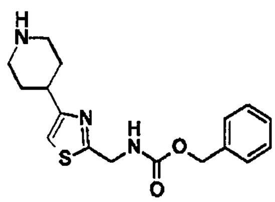

A solution of tert-butyl 4-[2-(benzyloxycarbonylaminomethyl)thiazol-4-yl]piperidine-1-carboxylate (327 mg, 0.758 mmol) in 4M HCl in dioxane (4 mL, 16 mmol) was stirred at room temperature for 2 h and evaporated. The residue was used in the next step without further purification.

c. Benzyl N-[[4-[1-(4-methylpiperazine-1-carbonyl)-4-piperidyl]thiazol-2-yl]methyl]carbamate

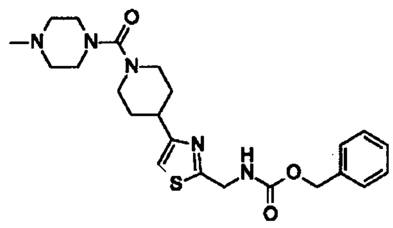

A colourless oil (253 mg, 73% over 2 steps) was obtained following the procedure described in example 104 step-a. The crude product was purified by chromatography on silica eluting with 3-10% MeOH in DCM. M/z 458 $(M+H)^+$.

d. [4-[2-(aminomethyl)thiazol-4-yl]-1-piperidyl]-(4-methylpiperazin-1-yl)methanone trifluoroacetate

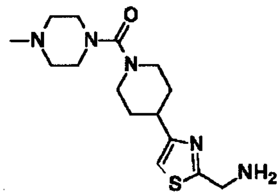

A solution of benzyl N-[[4-[1-(4-methylpiperazine-1-carbonyl)-4-piperidyl]thiazol-2-yl]methyl]carbamate (250 mg, 0.546 mmol) in TFA (2 mL, 26.12 mmol) was stirred at room temperature overnight, evaporated and azeotroped with toluene. The residue was redissolved in MeOH (2 mL) and applied to SCX-2 cartridge (5 g). The cartridge was washed with MeOH (20 mL) then the product eluted with 2M $NH_3$ in MeOH (10 mL). The solution was evaporated affording a yellow oil (167 mg, 94%). M/z 324 $(M+H)^+$.

e. [4-[2-(aminomethyl)thiazol-4-yl]-1-piperidyl]-(4-methylpiperazin-1-yl)methanone

The title product was obtained following the procedure described in example 89 step-c and step-d. The crude product was purified by preparative hplc (MDAP) affording a white solid (76 mg, 49%). M/z 526.4 $(M+H)^+$. $^1H$ NMR (d6-DMSO) $\delta$ 8.71 (1H, bs), 7.20 (2H, m), 7.11 (3H, m), 4.49 (2H, d, $J$ = 6 Hz), 3.52 (2H, m), 3.45 (2H, d, $J$ = 16 Hz), 3.12 (4H, m), 2.98 (2H, d, $J$ = 16 Hz), 2.80 (4H, m), 2.71 (2H, s), 2.27 (3H, m), 2.18 (3H, s), 1.86 (2H, m), 1.52 (2H, m).

**Example 154**

**2-[2-[[4-[1-(4-methylpiperazin-1-yl)sulfonyl-3-piperidyl]thiazol-2-yl]methylcarbamoyl]indan-2-yl]acetic acid**

Method D was used as described above.

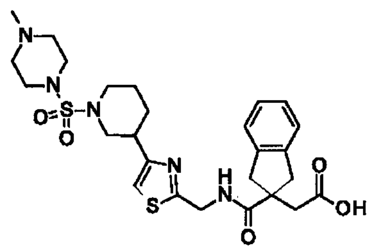

a. Tert-butyl 3-[2-(benzyloxycarbonylaminomethyl)thiazol-4-yl]piperidine-1-carboxylate

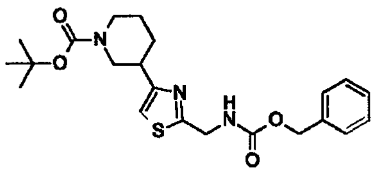

$K_2CO_3$ (228 mg, 1.65 mmol) was added to a solution of tert-butyl 3-(2-bromoacetyl)piperidine-1-carboxylate (500 mg, 1.63 mmol) and N-benzyloxycarbonylglycine thioamide (366 mg, 1.63 mmol) in EtOH (8 mL) at room temperature. The reaction mixture was stirred at room temperature overnight and evaporated. The residue was dissolved in DCM (10 mL), washed with water (10 mL) and saline (10 mL), dried and evaporated. The crude product was purified by chromatography on silica eluting with 0-100% EtOAc in hexane affording a yellow gum (558 mg, 79%). M/z 454 $(M+Na)^+$.

b. Benzyl N-[[4-(3-piperidyl)thiazol-2-yl]methyl]carbamate trifluoroacetate

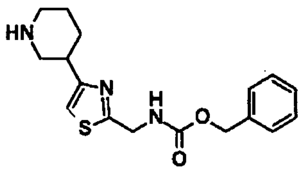

TFA (1 mL, 13.06 mmol) was added to a solution of tert-butyl 3-[2-(benzyloxycarbonylaminomethyl)thiazol-4-yl]piperidine-1-carboxylate (558 mg, 1.29 mmol) in DCM (5 mL) at room temperature. The reaction mixture was stirred for 2 h, evaporated and azeotroped with toluene. The residue was redissolved in MeOH (5 mL) and applied to SCX-2 cartridge (10 g). The cartridge was washed with MeOH (40 mL) then the product eluted with 2M $NH_3$ in MeOH (20 mL) affording a yellow gum (324 mg, 75%). M/z 332 $(M+H)^+$.

c. Benzyl N-[[4-[1-(4-methylpiperazin-1-yl)sulfonyl-3-piperidyl]thiazol-2-yl]methyl]carbamate

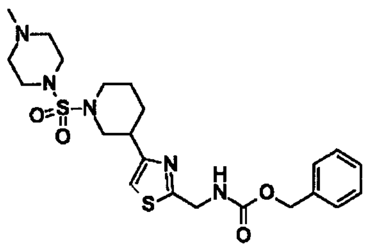

4-methylpiperazine-1-sulfonyl chloride (199 mg, 1 mmol) and TEA (0.279 mL, 2 mmol) were added to solution of benzyl N-[[4-(3-piperidyl)thiazol-2-yl]methyl]carbamate (324 mg, 0.978 mmol) in DCM (10 mL) at room temperature. The reaction mixture was stirred for 3 h and evaporated. The crude product was purified by chromatography on silica eluting with 6-10% MeOH in DCM. M/z 494 $(M+H)^+$.

d. 2-[2-[[4-[1-(4-methylpiperazin-1-yl)sulfonyl-3-piperidyl]thiazol-2-yl]methylcarbamoyl]indan-2-yl] acetic acid

The title product was obtained following the procedure described in example 153 step-d and step-e. The crude product was purified by preparative hplc (MDAP) affording a white solid (271 mg, 43%). M/z 562.2 $(M+H)^+$. $^1H$ NMR (d6-DMSO) $\delta$ 8.60 (1H, m), 7.22 (1H, m), 7.20 (2H, m), 7.11 (2H, m), 4.52 (2H, d, $J$ = 6 Hz), 3.78 (1H, m), 3.54 (1H, m), 3.45 (2H, d, $J$ = 16 Hz), 3.36-3.15 (4H, m), 2.98 (2H, d, $J$ = 16 Hz), 2.85 (4H, m), 2.71 (2H, s), 2.40 (3H, m), 2.05 (3H, s), 1.75 (2H, m), 1.60 (2H, m).

**Example 155**

**2-[2-[[4-[4-(4-carbamimidoylpiperazin-1-yl)phenyl]thiazol-2-yl]methylcarbamoyl]indan-2-yl]acetic acid**

Method E was used as described above.

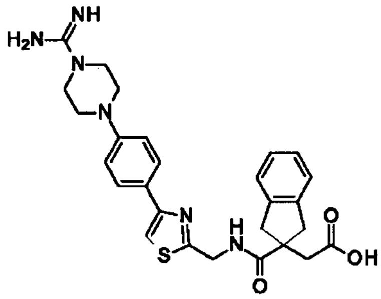

a. 2-trimethylsilylethyl N-[[4-(4-bromophenyl)thiazol-2-yl]methyl]carbamate

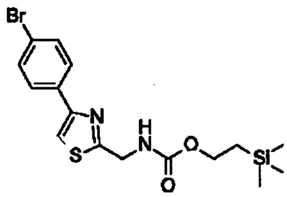

A solution of $NaHCO_3$ (625 mg, 7.44 mmol) in water (8 mL) and 1-[2-(trimethylsilyl)ethoxycarbonyloxy]pyrrolidine-2,5-dione (964 mg, 3.72 mmol) were added to a solution of [4-(4-bromophenyl)thiazol-2-yl]methanamine (1.033 g, 3.38 mmol) in dioxane (16 mL) at room temperature. The reaction mixture was stirred for 2 h and partitioned between EtOAc (20 mL) and water (20 mL). The organic extract was washed with brine (20 mL), dried and evaporated. The crude product was purified by chromatography on silica eluting with 0-50% EtOAc in hexane affording a white solid (948 mg, 68%). M/z 414 $(M+H)^+$.

b. 2-trimethylsilylethyl N-[[4-(4-piperazin-1-ylphenyl)thiazol-2-yl]methyl]carbamate

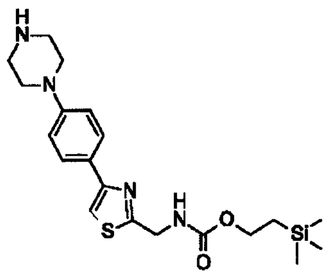

A yellow solid (459 mg, 43%) was obtained following the procedure described in example 26 step-b using piperazine. The crude product was purified by chromatography on silica eluting with 2-10% (2M $NH_3$ in MeOH) in DCM. M/z 419 $(M+H)^+$.

c. 2-trimethylsilylethyl N-[[4-[4-[4-[(E)-N,N'-bis(tert-butoxycarbonyl)carbamimidoyl]piperazin-1-yl]phenyl]thiazol-2-yl]methyl]carbamate

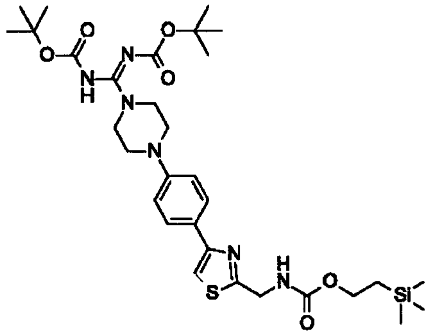

DIPEA (0.25 mL, 1.44 mmol) and N,N'-Di-Boc-1H-pyrazole-1-carboxamidine (246 mg, 0.794 mmol) were added to a solution of benzyl N-[[4-(3-piperidyl)thiazol-2-yl]methyl]carbamate (302 mg, 0.721 mmol) in 3:1 acetonitrile/DMF (4 mL) at room temperature. The reaction mixture was stirred at room temperature for 3 days and partitioned between EtOAc (5 mL) and water (5 mL). The organic extract was washed with brine (5 mL), dried and evaporated. The crude product was purified by chromatography on silica eluting with 0-50% EtOAc in hexane affording a colourless oil (478 mg, 100%). M/z 661 $(M+H)^+$.

d. Tert-butyl (NE)-N-[amino-[4-[4-[2-(aminomethyl)thiazol-4-yl]phenyl]piperazin-1-yl]methylene]carbamate

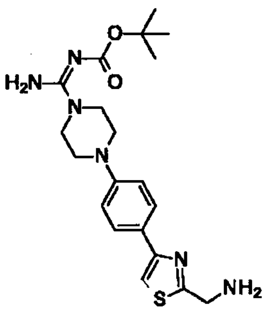

TBAF (1.4 mL, 1.44 mmol) was added to a solution of 2-trimethylsilylethyl N-[[4-[4-[4-[(E)-N,N'-bis(tert-butoxycarbonyl)carbamimidoyl]piperazin-1-yl]phenyl]thiazol-2-yl]methyl]carbamate (477 mg, 0.721 mmol) in THF (3 mL) at room temperature. The reaction mixture was heated at 60°C, stirred for 6 h and evaporated. The residue was dissolved in DCM (5 mL), washed with brine (5 mL) dried and evaporated. The crude product was purified by chromatography on silica eluting with 2-10% (2M $NH_3$ in MeOH) in DCM affording a yellow solid (129 mg, 43%). M/z 417 $(M+H)^+$.

e. 2-[2-[[4-[4-(4-carbamimidoylpiperazin-1-yl)phenyl]thiazol-2-yl]methylcarbamoyl]indan-2-yl]acetic acid

The title product was obtained following the procedure described in example 89 step-c and step-d. The crude product was purified by preparative hplc (MDAP) affording a white solid (60 mg, 47%). M/z 519.3 $(M+H)^+$. $^1H$ NMR (d6-DMSO) $\delta$ 8.69 (1H, bs), 8.31 (1H, s), 7.83 (2H, m), 7.77 (1H, s), 7.49 (1H, bs), 7.21 (2H, m), 7.12 (2H, m), 7.09 (2H, m), 4.61 (2H, s), 3.62 (4H, m), 3.49 (2H, d, $J$ = 16 Hz), 3.35 (4H, m), 2.98 (2H, d, $J$ = 16 Hz), 2.70 (2H, s).

**Example 156**

**2-[2-[[4-[4-(4-ethanimidoylpiperazin-1-yl)phenyl]thiazol-2-yl]methylcarbamoyl]indan-2-yl]acetic acid**

Method E was used as described above.

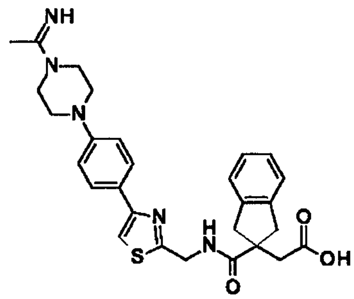

a. 2-trimethylsilylethyl N-[[4-[4-(4-ethanimidoylpiperazin-1-yl)phenyl]thiazol-2-yl]methyl]carbamate

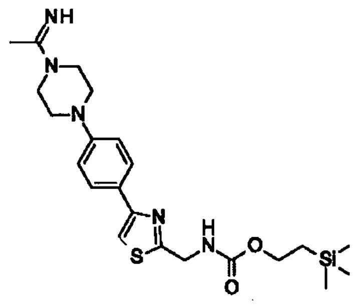

Ethyl acetimidate hydrochloride (66 mg, 0.537 mmol) and TEA (0.19 mL, 1.34 mmol) were added to a solution of 2-trimethylsilylethyl N-[[4-(4-piperazin-1-ylphenyl)thiazol-2-yl]methyl]carbamate (150 mg, 0.358 mmol) in EtOH (1.5 mL) at room temperature. The reaction mixture was heated to 90°C, stirred for 6 h and evaporated. The residue was used in the next step without further purification.

b. 2-trimethylsilylethyl N-[[4-[4-[4-[(E)-N-tert-butoxycarbonyl-C-methyl-carbonimidoyl]piperazin-1-yl]phenyl]thiazol-2-yl]methyl]carbamate

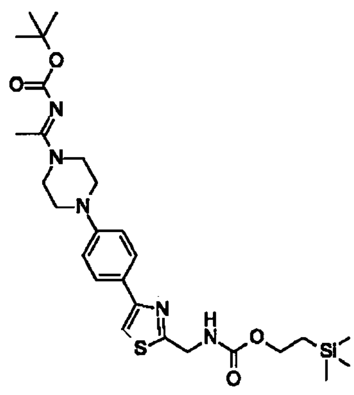

Di-tert-butyl-dicarbonate (313 mg, 1.43 mmol) and 2M NaOH (0.90 mL, 1.79 mmol) were added to a solution of 2-trimethylsilylethyl N-[[4-[4-(4-ethanimidoylpiperazin-1-yl)phenyl]thiazol-2-yl]methyl]carbamate (165 mg, 0.358 mmol) in dioxane (2 mL) at room temperature. The reaction mixture was stirred at room temperature for 2 days and partitioned between DCM (5 mL) and water (5 mL). the organic extract was dried and evaporated. The crude product was purified by chromatography on silica eluting with 0-80% EtOAc in hexane affording a white solid (100 mg, 50% over 2 steps). M/z 560 $(M+H)^+$.

c. 2-[2-[[4-[4-(4-ethanimidoylpiperazin-1-yl)phenyl]thiazol-2-yl]methylcarbamoyl]indan-2-yl]acetic acid

The title product was obtained following the procedure described in example 155 step-d and step-e. The crude product was purified by preparative hplc (MDAP) affording a white solid (53 mg, 63%). M/z 518.3 $(M+H)^+$. $^1H$ NMR (d6-DMSO) δ 9.28 (1H, bs), 8.71 (1H, bs), 8.13 (1H, s), 7.85 (2H, m), 7.76 (1H, s), 7.21 (2H, m), 7.12 (2H, m), 7.09 (2H, m), 4.61 (2H, s), 3.76 (4H, m), 3.52-3.40 (6H, m), 3.02 (2H, d, *J* = 16 Hz), 2.70 (2H, s), 2.32 (3H, s).

**Example 157**

**2-[2-(hydroxyamino)-2-oxo-ethyl]-N-[(4-phenylthiazol-2-yl)methyl]indane-2-carboxamide**

Method F was used as described above.

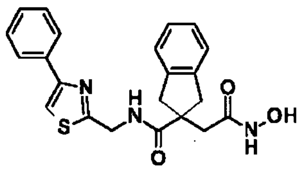

a. 2-[2-oxo-2-(tetrahydropyran-2-yloxyamino)ethyl]-N-[(4-phenylthiazol-2-yl)methyl]indane-2-carboxamide

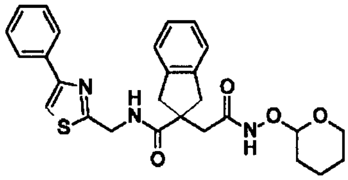

DEAD (0.089 mL, 0.510 mmol) and HATU (97 mg, 0.255 mmol) were added to a solution of 2-[2-[(4-phenylthiazol-2-yl)methylcarbamoyl]indan-2-yl]acetic acid (67 mg, 0.17 mmol) and O-(tetrahydro-2H-pyrane-2-yl)hydroxylamine (24 mg, 0.204 mmol) in DMF (1.5 mL) at room temperature. The reaction mixture was stirred at room temperature for 1 h, partitioned between EtOAc (5 mL) and water (5 mL). The organic extract was washed with water (5 mL) and brine (5 mL), dried and evaporated. The crude product was purified by chromatography on silica eluting with 0-10% MeOH in EtOAc affording a white solid (74 mg, 89%). M/z 514 $(M+Na)^+$.

b. 2-[2-(hydroxyamino)-2-oxo-ethyl]-N-[(4-phenylthiazol-2-yl)methyl]indane-2-carboxamide

TFA (0.5 mL, 6.53 mmol) was added to a solution of 2-[2-oxo-2-(tetrahydropyran-2-yloxyamino)ethyl]-N-[(4-phenylthiazol-2-yl)methyl]indane-2-carboxamide (72 mg, 0.074 mmol) in DCM (1.5 mL) at room temperature. The reaction mixture was stirred at room temperature for 2 h, evaporated and azeotroped with toluene. The crude product was purified by preparative hplc (MDAP) affording a white solid (24 mg, 40%). M/z 430.0 $(M+Na)^+$. $^1H$ NMR (d6-DMSO) δ 10.39 (1H, s), 8.77 (1H, m), 7.92 (3H, m), 7.41 (2H, m), 7.32 (1H, m), 7.20 (2H, m), 7.11 (2H, m), 4.60 (2H, d, *J* = 6 Hz), 3.48 (2H, d, *J* = 16 Hz), 2.98 (2H, d, *J* = 16 Hz), 2.50 (2H, s).

**Example 158**

**2-[2-(hydroxyamino)-2-oxo-ethyl]-N-[[4-[1-(2-morpholinoethyl)pyrazol-4-yl]thiazol-2-yl]methyl]indane-2-carboxamide**

Method F was used as described above.

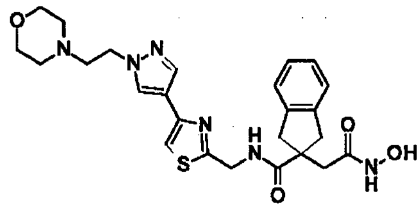

The title product was obtained following the procedure described in example 157 from 2-[2-[[4-[1-(2-morpholinoethyl)pyrazol-4-yl]thiazol-2-yl]methylcarbamoyl]indan-2-yl]acetic acid. The crude product was purified by preparative hplc (MDAP) affording a white solid (29.4 mg, 62%). M/z 511.2 $(M+H)^+$. $^1H$ NMR (d6-DMSO) δ 9.00 (1H, bs), 8.11 (1H, s), 7.70 (1H, s), 7.50 (1H, s), 7.20 (2H, m), 7.11 (2H, m), 4.52 (2H, d, *J* = 6 Hz), 3.61 (2H, m), 3.44 (6H, m), 3.00 (2H, d, *J* = 16 Hz), 2.52 (8H, m).

**Example 159**

**2-[2-(hydroxyamino)-2-oxo-ethyl]-N-[[4-[1-[2-(4-methylpiperazin-1-yl)-2-oxo-ethyl]pyrazol-4-yl]thiazol-2-yl]methyl]indane-2-carboxamide**

Method F was used as described above.

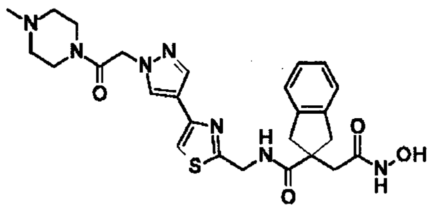

The title product was obtained following the procedure described in example 157 from 2-[2-[[4-[1-(3-morpholinopropyl)pyrazol-4-yl]thiazol-2-yl]methylcarbamoyl]indan-2-yl]acetic acid. The crude product was purified by preparative hplc (MDAP) affording a white solid (8.7 mg, 52%). M/z 538.2 $(M+H)^+$. $^1H$ NMR (d6-DMSO) δ 10.36 (1H, bs), 8.75 (1H, m), 8.12 (1H, s), 8.00 (1H, s), 7.80 (1H, s), 7.20 (2H, m), 7.11 (2H, m), 5.12 (2H, s), 4.52 (2H, d, *J* = 6 Hz), 3.40 (6H, m), 3.00 (2H, d, *J* = 16 Hz), 2.56-2.30 (6H, s), 2.19 (3H, s).

**Example 160**

**2-[2-[[4-[3-[(4-methylpiperazin-1-yl)methyl]phenyl]thiazol-2-yl]methylcarbamoyl]indan-2-yl]acetic acid**

Method B was used as described above.

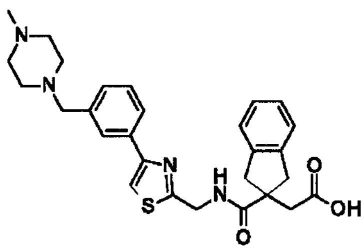

a. Methyl 3-[2-[(tert-butoxycarbonylamino)methyl]thiazol-4-yl]benzoate

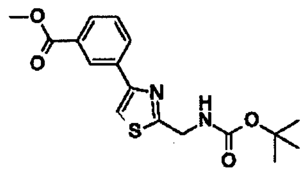

To a stirred solution of methyl 3-(2-bromoacetyl)benzoate (500 mg, 1.95 mmol) in EtOH (10 mL) was added tert-butyl (2-amino-2-thioxoethyl)carbamate (372 mg, 1.95 mmol) at room temperature and stirred for 4 h. The reaction mixture was evaporated and resulting residue was chromatographed on silica eluting with 10-20% EtOAc in petroleum ether affording an off white solid (320 mg, 48%). M/z 349.1 $(M+H)^+$.

b. Tert-butyl N-[[4-[3-(hydroxymethyl)phenyl]thiazol-2-yl]methyl]carbamate

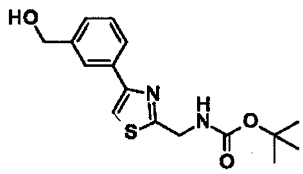

To a stirred solution of methyl 3-[2-[(tert-butoxycarbonylamino)methyl]thiazol-4-yl]benzoate (300 mg, 0.86 mmol) in THF (8 mL) was added DIBAL-H (3.5 mL, 3.44 mmol, 1M in THF) drop wise at 0 °C and stirred for 2 h at 0 °C. The reaction mixture was quenched with MeOH (5 mL) and aq. silica gel (200 mg) at 0 °C and stirred for 15 minutes. The reaction mixture was filtered, washed the cake with EtOAc (2 x 25 mL) and filtrate was evaporated. The crude was chromatographed on silica eluting with 30-40% EtOAc in petroleum ether affording an off white solid (270 mg, 98%). M/z 321.2 $(M+H)^+$.

c. Tert-butyl N-[[4-(3-formylphenyl)thiazol-2-yl]methyl]carbamate

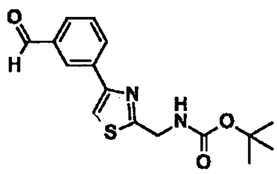

To a stirred solution of tert-butyl N-[[4-[3-(hydroxymethyl)phenyl]thiazol-2-yl]methyl]carbamate (250 mg, 0.71 mmol) in DCM (5 mL) was added Dess-Martin periodinane (497 mg, 1.17 mmol) at 0 °C and stirred at room temperature for 2 h. The reaction mixture was quenched with saturated aqueous sodium thiosulfate solution (20 mL), extracted with DCM (2 x 50 mL) and combined organic layer was dried, filtered and evaporated affording a pale yellow solid (250 mg, crude). M/z = 319.1 $(M+H)^+$.

d. Tert-butyl N-[[4-[3-[(4-methylpiperazin-1-yl)methyl]phenyl]thiazol-2-yl]methyl]carbamate

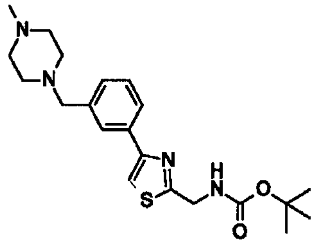

To a stirred solution of tert-butyl N-[[4-(3-formylphenyl)thiazol-2-yl]methyl]carbamate (250 mg, 0.78 mmol) in THF (5 mL) was added 1-methylpiperazine (118 mg, 1.17 mmol) and di n-butyltin dichloride (121 mg, 0.39 mmol) at room temperature and stirred for 12 h. Then phenylsilane (0.2 mL, 1.57 mmol) was added at room temperature and stirred for 4 h. The reaction mixture was diluted with water (20 mL), extracted with EtOAc (2 x 80 mL) and combined organic layer was dried, filtered and evaporated. The crude was chromatographed on silica eluting with 5-10% MeOH in DCM affording a pale yellow solid (190 mg, 48%). M/z 403.2 $(M+H)^+$.

e. 2-[2-[[4-[3-[(4-methylpiperazin-1-yl)methyl]phenyl]thiazol-2-yl]methylcarbamoyl]indan-2-yl]acetic acid

The title product was obtained following the procedure described in example 74 step-b to step-d. The crude compound was purified by preparative HPLC [YMC-TRIART-C18 (150*25), 10 u, Mobile phase: A: 0.1% Formic Acid in $H_2O$, B: MeCN] affording an off white solid (12 mg, 26%). M/z 505.3 $[(M+H]^+$. $^1H$ NMR (500 MHz, DMSO-$d_6$): δ 8.98 (1H, bs), 7.95 (1H, s), 7.85 (1H, s), 7.97 (1H, d, J = 7.5 Hz), 7.36 (1H, t, J = 7.5 Hz), 7.25 (1H, d, J = 7.5 Hz), 7.21-7.20 (2H, m), 7.14-7.12 (2H, m), 4.59 (2H, d, J = 5.5 Hz), 3.61 (2H, s), 3.45 (2H, d, J = 16 Hz), 3.00 (2H, d, J = 16 Hz), 2.72 (2H, s), 2.49-2.22 (8H, m), 2.15 (3H, s).

Other compounds have been prepared by Method B following the procedure described in example 160 from methyl 4-(2-bromoacetyl)benzoate using 1-methylpiperidine or dimethylamine in step-d, and purified in a similar manner by preparative HPLC are shown in the Table below, wherein R is the moiety:

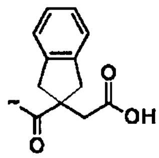

| Example | Structure | Name, MS and NMR data |
|---|---|---|
| **161** | 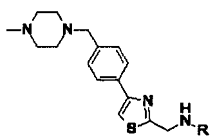 | 2-[2-[[4-[4-[(4-methylpiperazin-1-yl)methyl]phenyl]thiazol-2-yl] methylcarbamoyl]indan-2-yl] acetic acid<br>M/z 505.2 $(M+H)^+$<br>$^1H$ NMR (500 MHz, DMSO-$d_6$): δ 9.23 (1H, bs), 7.91 (1H, s), 7.86 (2H, d, J = 8.5 Hz), 7.34 (2H, d, J = 8.5 Hz), 7.21-7.19 (2H, m), 7.13-7.12 (2H, m), 4.59 (2H, d, J = 5.5 Hz), 3.50-3.31 (4H, m), 2.99 (2H, d, J = 16 Hz), 2.69 (2H, s), 2.36-2.22 (8H, m), 2.14 (3H, s). |
| **162** | 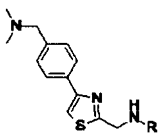 | 2-[2-[[4-[4-[(dimethylamino)methyl]phenyl]thiazol-2-yl]methylcarbamoyl]indan-2-yl]acetic acid<br>M/z 450.2 $(M+H)^+$<br>$^1H$ NMR (500 MHz, DMSO-$d_6$): δ 9.63 (1H, bs), 8.68 (1H, t, J = 6 Hz), 8.03 (1H, s), 7.98 (2H, d, J = 7.5 Hz), 7.49 (2H, d, J = 7.5 Hz), 7.22-7.20 (2H, m), 7.14-7.13 (2H, m), 4.59 (2H, d, J = 6 Hz), 4.05 (2H, bs), 3.50 (2H, d, J = 16 Hz), 3.01 (2H, d, J = 16 Hz), 2.76 (2H, s), 2.60 (6H, m). |

**Example 163**

**2-[2-[[4-[4-(2-aminoethoxy)phenyl]thiazol-2-yl]methylcarbamoyl]indan-2-yl]acetic acid**

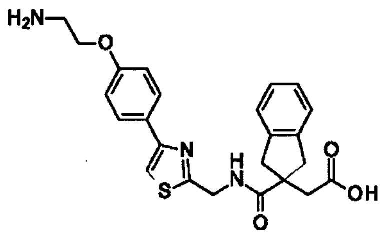

a. Allyl N-(2-bromoethyl)carbamate

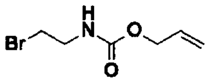

To a stirred solution of 2-bromoethanamine (5 g, 24.7 mmol) in DCM (50 mL) was added $Et_3N$ (12 mL, 86.6 mmol) at room temperature and stirred for 15 minutes. Then reaction mixture was cooled to 0 °C and allyl chloroformate solution (2.6 mL, 24.7 mmol) in DCM (3 mL) was added drop wise for 15 minutes. The reaction mixture was allowed to stir at room temperature for 16 h. The reaction mixture was diluted with cold water (150 mL), extracted with DCM (2 x 100 mL) and evaporated affording a colour less liquid (3 g, 58%).

b. Tert-butyl N-[[4-[4-[2-(allyloxycarbonylamino)ethoxy]phenyl]thiazol-2-yl]methyl]carbamate

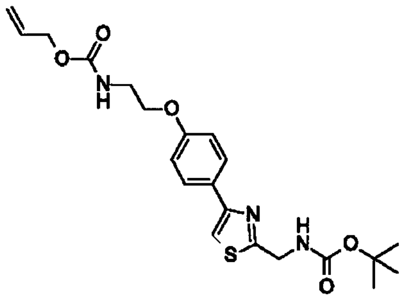

To a stirred solution of allyl 2-bromoethylcarbamate (3 g, 9.8 mmol) in acetone (30 mL) was added tert-butyl (4-(4-hydroxyphenyl) thiazol-2-yl) methylcarbamate (6 g, 29.4 mmol), $K_2CO_3$ (5.4 g, 39.2 mmol) at room temperature and heated at 70 °C for 16 h. The reaction mixture was evaporated and resulting residue was diluted with cold water (60 mL) and extracted with EtOAc (2 x 100 mL). The combined organic layer was dried, filtered and evaporated. The crude was chromatographed on silica eluting with 3% MeOH in DCM affording an off white solid (3.5 g, 83%). M/z 434.2 $(M+H)^+$.

c. Tert-butyl 2-[2-[[4-[4-[2-(allyloxycarbonylamino)ethoxy]phenyl]thiazol-2-yl]methylcarbamoyl]indan-2-yl]acetate

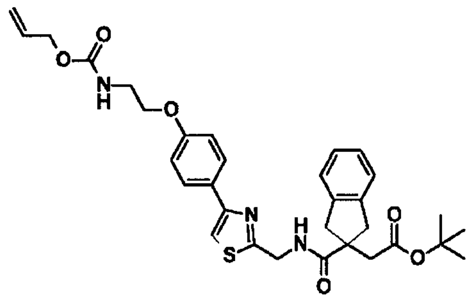

This was prepared following the procedure described in example 46 step-c and step-d. The crude was chromatographed on neutral alumina eluting with 3% MeOH in DCM affording an off white solid (1.8 g, 21%). M/z 592.2 $(M+H)^+$.

d. Tert-butyl 2-[2-[[4-[4-(2-aminoethoxy)phenyl]thiazol-2-yl]methylcarbamoyl]indan-2-yl]acetate

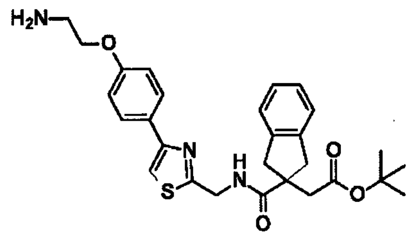

To a stirred solution of tert-butyl 2-[2-[[4-[4-[2-(allyloxycarbonylamino)ethoxy]phenyl]thiazol-2-yl]methylcarbamoyl]indan-2-yl]acetate (1.3 g, 2.19 mmol) in DCM (26 mL) was added phenylsilane (1.62 mL, 13.1 mmol), $Pd(PPh_3)_4$ (127 mg, 1.09 mmol) at room temperature and stirred for 2 h. The reaction mixture was diluted with cold water (30 mL), extracted with DCM (2 x 50 mL) and combined organic layer was dired, filtered and evaporated. The crude was chromatographed on neutral alumina eluting with 7% MeOH in DCM affording a brown solid (650 mg, 58%). M/z = 508.2 $(M+H)^+$.

e. 2-[2-[[4-[4-(2-aminoethoxy)phenyl]thiazol-2-yl]methylcarbamoyl]indan-2-yl]acetic acid

The title product was obtained following the procedure described in example 74 step-d. The crude compound was purified by preparative HPLC [SYMMETRY-C18 (300*19), 7 u, Mobile phase: 0.1% Formic Acid in $H_2O$:acetonitrile] affording an off white solid (5 mg, 12%). M/z 452.1 $(M+H)^+$. $^1H$ NMR (500 MHz, DMSO-$d_6$): δ 10.50 (2H, bs), 8.40 (1H, bs), 7.85 (2H, d, J = 8 Hz), 7.78 (1H, s), 7.19-7.17 (2H, m), 7.13-7.10 (2H, m), 7.00 (2H, d, J = 8 Hz), 4.58 (2H, d, J = 4.5 Hz), 4.02-4.00 (2H, m), 3.43 (2H, d, J =16.5 Hz), 3.32 (2H, bs), 2.94 (2H, d, J = 16.5 Hz), 2.58 (2H, s).

**Example 164**

**2-[2-[[4-[4-[2-(ethanimidoylamino)ethoxy]phenyl]thiazol-2-yl]methylcarbamoyl]indan-2-yl]acetic acid**

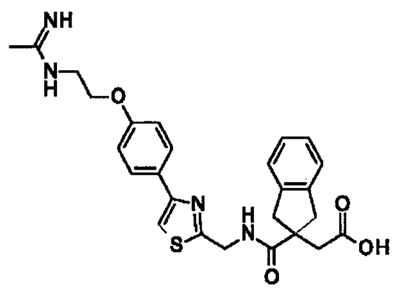

a. Tert-butyl2-[2-[[4-[4-[2-(ethanimidoylamino)ethoxy]phenyl]thiazol-2-yl]methylcarbamoyl]indan-2-yl] acetate

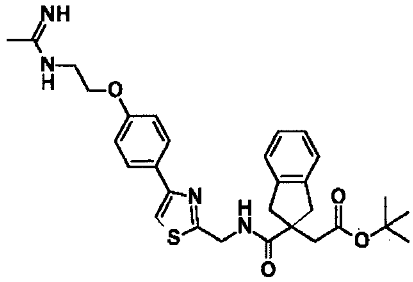

To a stirred solution of tert-butyl 2-[2-[[4-[4-(2-aminoethoxy)phenyl]thiazol-2-yl]methylcarbamoyl]indan-2-yl]acetate (500 mg, 0.98 mmol) in EtOH (10 mL) was added ethyl acetimidate hydrochloride (86 mg, 0.98 mmol), DIPEA (0.2 mL, 0.98 mmol) at room temperature and stirred for 1 h. The reaction mixture was evaporated and resulting residue was triturated with diethyl ether/n-pentane (30 mL, 1:1). The crude was purified by preparative HPLC [YMC TRIART-C18 (150x 25 mm), 10 u, Mobile phase: 0.1% Formic Acid in $H_2O$: acetonitrile] affording an off white solid (100 mg, 18%). M/z 549.2 $(M+H)^+$.

b. 2-[2-[[4-[4-[2-(ethanimidoylamino)ethoxy]phenyl]thiazol-2-yl]methylcarbamoyl]indan-2-yl]acetic acid

The title product was obtained following the procedure described in example 74 step-d. The crude compound was purified by preparative HPLC [KROMOSIL-C18 (150*25), 10 u, Mobile phase: 0.1% Formic Acid in $H_2O$: acetonitrile] affording an off white solid (40 mg, 66%). M/z 493.1 $(M+H)^+$. $^1H$ NMR (500 MHz, DMSO-$d_6$): δ 9.68 (1H, bs), 9.18 (1H, bs), 8.76 (1H, bs), 7.87 (2H, dd, J = 7 Hz, J = 2 Hz), 7.80 (1H, s), 7.20-7.18 (2H, m), 7.13-7.11 (2H, m), 7.00 (2H, dd, J = 7 Hz, J = 2 Hz), 4.57 (2H, d, J = 5.5 Hz), 4.17 (2H, t, J = 5 Hz), 3.62 (2H, t, J = 5 Hz), 3.43 (2H, d, J = 16.5 Hz), 2.96 (2H, d, J = 16.5 Hz), 2.65 (2H, s), 2.16 (3H, s).

**Example 165**

**2-[2-[[4-[4-(2-guanidinoethoxy)phenyl]thiazol-2-yl]methylcarbamoyl]indan-2-yl]acetic acid**

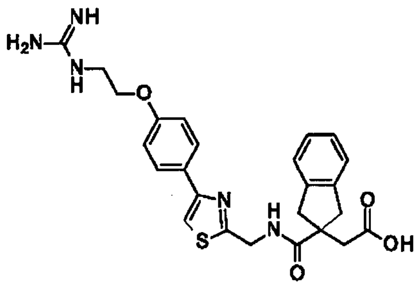

The title product was obtained following the procedure described in example 164 using pyrazole-1-carboxamidine hydrochloride in step-a. The crude compound was purified by MDAP affording an off white solid. M/z 494.1 $(M+H)^+$. $^1H$ NMR (500 MHz, DMSO-$d_6$): δ 11.09 (1H, bs), 9.08 (1H, bs), 7.77 (2H, d, J = 8.5 Hz), 7.70-7.50 (4H, m), 7.63 (1H, s), 7.17-7.16 (2H, m), 7.11-7.09 (2H, m), 6.90 (2H, d, J = 8.5 Hz), 4.56 (2H, d, J = 5.5 Hz), 4.04 (2H, t, J = 5.5 Hz), 3.54-4.51 (2H, m), 3.41 (2H, d, J = 16 Hz), 2.92 (2H, d, J = 16 Hz), 2.45 (2H, s).

**Example 166**

**2-[2-[(4-benzylthiazol-2-yl)methylcarbamoyl]indan-2-yl]acetic acid**

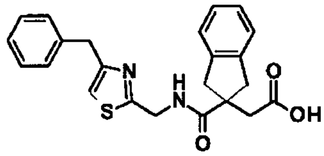

a. Tert-butyl N-[[4-(hydroxymethyl)thiazol-2-yl]methyl]carbamate

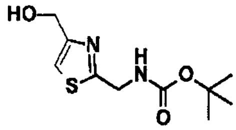

To a stirred solution of ethyl 2-(((tert-butoxycarbonyl)amino)methyl)thiazole-4-carboxylate (1 g, 3.49 mmol) in THF (20 mL) was added $LiAlH_4$ (7.0 mL, 6.98 mmol, 1M in THF) drop wise under nitrogen at 0 °C and the reaction mixture was allowed stir at room temperature for 4 h. The reaction mixture was cooled to 0 °C and quenched with EtOAc (30 mL) followed by saturated aqueous sodium sulphate solution. Then stirred at same temperature for 30 min., filtered and filtrate was evaporated. The crude was chromatographed on silica eluting with 40% EtOAc in petroleum ether affording a yellow solid (510 mg, 59%). M/z 245.1 $(M+H)^+$.

b. [2-[(tert-butoxycarbonylamino)methyl]thiazol-4-yl]methyl methanesulfonate

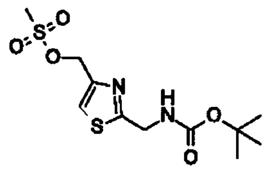

To a solution of tert-butyl N-[[4-(hydroxymethyl)thiazol-2-yl]methyl]carbamate (250 mg, 1.02 mmol) in DCM (10 mL) was added $Et_3N$ (0.28 mL, 2.04 mmol) and mesyl chloride (140 mg, 1.22 mmol) drop wise at 0 °C. The mixture was stirred at room temperature for 4 h. Then mixture was diluted with cold water (10 mL) and extracted with DCM (2 x 20 mL). The combined organic layer was dried, filtered and concentrated affording a yellow solid (320 mg, crude).

c. Tert-butyl N-[(4-benzylthiazol-2-yl)methyl]carbamate

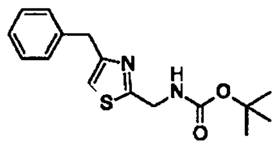

A solution of CuCN (250 mg, 2.78 mmol) in THF (15 mL) was added PhMgBr (1.8 mL, 5.59 mmol) drop wise at -25 °C and stirred for 15 minutes. Then allowed to stir at 0 °C for 15 minutes and further allowed to stir at room temperature for 30 minutes. The mixture was again cooled to -25 °C and added [2-[(tert-butoxycarbonylamino)methyl]thiazol-4-yl]methyl methanesulfonate solution (300 mg, 0.93 mmol) in THF (5 mL) drop wise for 15 minutes. The mixture was stirred at 0 °C for 16 h. The reaction mixture was quenched with ammonium hydroxide solution (10 mL) at 0 °C and extracted with EtOAc (2 x 20 mL). The combined organic layer was dried, filtered and evaporated. The crude was chromatographed on silica eluting with 15% EtOAc in petroleum ether affording a yellow solid (170 mg, 60%). M/z 305.1 $(M+H)^+$.

d. 2-[2-[(4-benzylthiazol-2-yl)methylcarbamoyl]indan-2-yl]acetic acid

The title product was obtained following the procedure described in example 74 step-c and step-d. The crude compound was purified by preparative HPLC [X-BRIDGE-C18 (150*30), 5 u, Mobile phase: A: 0.1% Formic Acid in $H_2O$, B: MeCN] affording an off white solid (32 mg, 30%). M/z 407.1 $(M+H)^+$. $^1H$ NMR (500 MHz, DMSO-$d_6$): δ 12.09 (1H, bs), 8.70 (1H, bs), 7.29-7.24 (4H, m), 7.20-7.17 (3H, m), 7.13-7.11 (3H, m), 4.46 (2H, d, J = 6 Hz), 3.98 (2H, s), 3.41 (2H, d, J = 16 Hz), 2.98 (2H, d, J = 16 Hz), 2.69 (2H, s).

**Example 167**

**2-[2-[(5-methyl-4-phenyl-thiazol-2-yl)methylcarbamoyl]indan-2-yl]acetic acid**

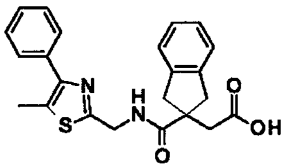

The title product was obtained following the procedure described in example 167 starting with ethyl 2-[(tert-butoxycarbonylamino)methyl]-5-methyl-thiazole-4-carboxylate and using phenylmagnesium bromide in step-c. The crude compound was purified by MDAP affording an off white solid. M/z 407.1 $(M+H)^+$. $^1H$ NMR (500 MHz, DMSO-$d_6$): δ 12.16 (1H, s), 8.58 (1H, t, J = 6 Hz), 7.64-7.62 (2H, m), 7.46-7.43 (2H, m), 7.36-7.33 (1H, m), 7.21-7.20 (2H, m), 7.14-7.12 (2H, m), 4.49 (2H, d, J = 6 Hz), 3.45 (2H, d, J = 16.5 Hz), 2.98 (2H, d, J = 16.5 Hz), 2.73 (2H, s), 2.48 (3H, s).

**Example 168**

**2-[2-[[4-(5-methyl-1H-1,2,4-triazol-3-yl)thiazol-2-yl]methylcarbamoyl]indan-2-yl]acetic acid**

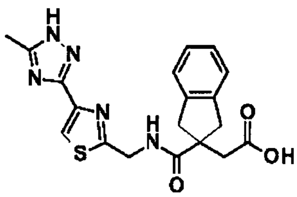

a. Tert-butyl N-[[4-(hydrazinecarbonyl)thiazol-2-yl]methyl]carbamate

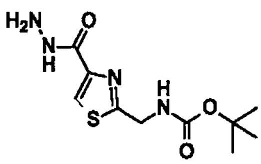

To a stirred solution of ethyl 2-(((tert-butoxycarbonyl) amino) methyl) thiazole-4-carboxylate (500 mg, 1.74 mmol) in dioxane (10 mL) was added hydrazine hydrate (3 mL) at room temperature and stirred for 3 h. The reaction mixture was diluted with water (30 mL), extracted with EtOAc (2 x 60 mL) and combined organic layer was dried, filtered and evaporated affording a pale yellow solid (520 mg, crude). M/z 273.1 $(M+H)^+$.

b. Tert-butyl N-[[4-(5-methyl-1H-1,2,4-triazol-3-yl)thiazol-2-yl]methyl]carbamate

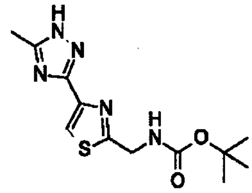

To a solution of tert-butyl N-[[4-(hydrazinecarbonyl)thiazol-2-yl]methyl]carbamate (500 mg, 1.83 mmol) in EtOH (10 mL) was added acetimidamide hydrochloride (260 mg, 2.75 mmol) at room temperature and heated at 100 °C for 24 h. The reaction mixture was evaporated and diluted with water (50 mL), extracted with DCM (2 x 80 mL) and evaporated. The crude was chromatographed on silica eluting with 2-3 % MeOH in DCM affording an off white solid (240 mg, 45%). M/z 296.1 $(M+H)^+$.

c. 2-[2-[[4-(5-methyl-1H-1,2,4-triazol-3-yl)thiazol-2-yl]methylcarbamoyl]indan-2-yl]acetic acid

The title product was obtained following the procedure described in example 74 step-c and step-d. The crude compound was purified by Preparative HPLC [YMC-TRIART-C18 (150*25), 10 u, Mobile phase: A: 0.1% Formic Acid in $H_2O$, B: MeCN] affording an off white solid (25 mg, 24%). M/z 398.1 $(M+H)^+$. $^1H$ NMR (500 MHz, DMSO-$d_6$): δ 13.56 (1H, bs), 9.52 (1H, bs), 7.94 (1H, s), 7.18-7.20 (2H, m), 7.11-7.13 (2H, m), 4.59 (2H, d, J = 5.5 Hz), 3.45 (2H, d, J = 16.5 Hz), 2.98 (2H, d, J = 16.5 Hz), 2.65 (2H, s), 2.33 (3H, s).

Other compounds according to the invention include the following. These compounds can be synthesized in accordance with the synthetic methodologies discussed above:

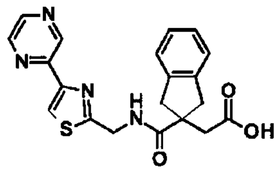

A. 2-[2-[(4-pyrazin-2-ylthiazol-2-yl)methylcarbamoyl]indan-2-yl]acetic acid

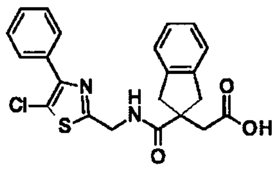

B. 2-[2-[(5-chloro-4-phenyl-thiazol-2-yl)methylcarbamoyl]indan-2-yl]acetic acid

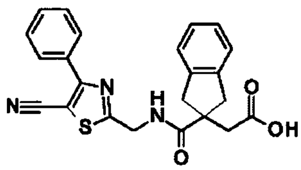

C. 2-[2-[(5-cyano-4-phenyl-thiazol-2-yl)methylcarbamoyl]indan-2-yl]acetic acid

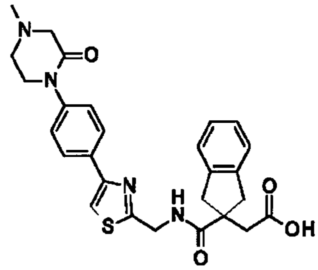

D. 2-[2-[[4-[4-(4-methyl-2-oxo-piperazin-1-yl)phenyl]thiazol-2-yl]methylcarbamoyl]indan-2-yl]acetic acid

**Example 169: LasB Inhibitory Activity Measurements**

The relevance of LasB to PA infection is illustrated in Figure 1, which shows lung burden in a rat model of chronic lung infection following infection with WT PA (which expresses LasB) and a mutant form of PA (*ΔlasB* PA) in which LasB is not expressed. It can be clearly seen in that following infection, whereas a wild type strain is able to persist at least for 14 days, a LasB deficient strain was not able to persist beyond day 5.

The relevance of LasB to PA biofilm development is shown in Figure 2. Biofilms formed after 3 days by both PA26 wt and PA26 *lasB* deletion strains were investigated by confocal imaging and subsequent analysis (with Comstat software). This study demonstrated that biofilms formed by the PA26 *lasB* deletion strain were highly reduced in thickness and biomass compared to the wt strain, demonstrating the essential role of LasB in PA biofilm development (Asterisks ** and *** indicates statistical difference for $P \leq 0.05$, $P \leq 0.01$; respectively).

Experiments were therefore conducted (1) to measure the potency of inhibition of compounds of the invention against purified *Pseudomonas aeruginosa* LasB enzyme and also experiments were conducted (2) to measure the ability of compounds of the invention to inhibit LasB-catalysed elastin degradation. The first assay uses a commercial fluorescent synthetic peptide and purified LasB enzyme. The LasB hydrolysis kinetics are measured allowing the determination of the IC50 and Ki of the inhibitors; the second is a more physiological assay using dialysed *Pseudomonas aeruginosa* supernatant as source of enzyme, plus its natural substrate Elastin. It is an "end point assay" that determines the percentage of LasB inhibition by each compound for one particular time point and inhibitor concentration. Technical details are described below:

*Fluorometric assay to determine Ki*

This assay uses commercially available substrate (Abz-Ala-Gly-Leu-Ala-p-Nitro-Benzyl-Amide (Ex: 340 nm, Em: 415 nm) from Peptide International) and purified LasB protein from P. *aeruginosa* (provided by Merck or Charles River Laboratories). It is performed to determine LasB elastase activity and assess compound inhibition in 96-well plate format. All compounds of Formula (I) were assessed using the method described below.

Method: 10 to 140 ng/ml purified LasB is incubated with 250 μM Abz-Ala-Gly-Leu-Ala-p-Nitro-Benzyl-Amide in 50 mM Tris-HCl pH 7.4, 2.5 mM CaCl2, 0.01% of Triton X100 at 37°C. LasB activity (corresponding to fluorescence emission induced by substrate hydrolysis) is measured over 30 min at 37°C with a fluorescence plate reader such as the Perkin Elmer Envision or similar. Different range of inhibitor concentrations are routinely assessed depending of inhibitor potency from 0.0016 to 200 μM (2-fold dilutions series) in order to determine IC50.

The equation used to calculate the Ki from IC50 is: $Ki = IC50 / (1+([S]/Km))$ where $[S] = 250$ μM and $Km = 214$ μM.

*Elastin assay to determine % inhibition*

The Elastin assay uses as source of enzyme dialysed supernatant from *P. aeruginosa* PAO1 and the Elastin Congo-Red as substrate. The natural LasB substrate, elastin, is complexed with the congo-red dye (Elastin Congo-Red, ECR). The elastolysis activity from the culture supernatant will degrade elastin and release the congo-red dye into the supernatant. This red dye release can be measured with a spectrophotometer.

All compounds of Formula (I) were assessed using the method described below.

Method: To determine LasB elastase activity and assess compound inhibition, an overnight culture of *P. aeruginosa* strain PAO1 is diluted in LB medium. After reaching an $OD_{600nm}$ of 0.6, this culture is diluted and incubated for additional 18-24 hours in a shaking incubator. Culture supernatants are recovered by centrifugation and filtrated through a 0.22 μM filter. These supernatants are dialysed (filtration molecules < 20kDa) into a 50 mM Tris-HCl pH 7.4, 2.5 mM $CaCl_2$ solution at 4°C under agitation for 24 hours. Supernatant dialysed is then mixed volume/volume with the ECR suspension (20 mg/mL of ECR in 100 mM Tris-HCl pH 7.4 buffer supplemented with 1 mM CaCl2) supplemented with

Triton X100 (final concentration of 0.01%) in presence of DMSO (positive control) and/or different concentrations of compound (routinely 50 to 1.56 μM). As a negative control, the dialysed supernatant is replaced by Tris-HCl solution (50 mM Tris-HCl pH 7.4, 2.5 mM $CaCl_2$). The mixed reaction is then incubated overnight in a 37°C shaking incubator. The reaction supernatant is recovered by centrifugation and the release of congo-red is measured by its absorbance at 495 nm ($OD_{495nm}$).

Percentage inhibition is determined using the following equation:

$$((OD_{495nm}\text{ value of positive control} - OD_{495nm}\text{ value of negative control}) - (OD_{495nm}\text{ value of treated supernatant} - OD_{495nm}\text{ value of negative control})) / (OD_{495nm}\text{ value of positive control} - OD_{495nm}\text{ value of negative control}) \times 100.$$

Results are shown in the Table below and categorised into A, B and C for both assays. The Ki values are grouped as A (Ki = 0.00 to 0.05 μM), B (Ki = 0.05 to 0.2 μM) and C (Ki = 0.2 to 10.00 μM). Similarly, for the elastase hydrolysis assay, values are grouped into A (>75% inhibition), B (60 to 75% inhibition) and C (10 to 60% inhibition) all at 25 μM inhibitor concentration. (n.d. not determined).

| Example | Ki (μM) | Elastin hydrolysis % inhibition @ 50 / 25 μM inhibitor concentration |
|---|---|---|
| **1** | B | A |
| **2** | C | C |
| **3** | A | A |
| **4** | C | ND |
| **5** | B | B |
| **6** | B | A |
| **7** | B | B |
| **8** | B | B |
| **9** | C | B |
| **10** | B | C |
| **11** | B | B |
| **12** | B | B |
| **13** | B | B |
| **14** | C | C |
| **15** | C | B |
| **16** | C | B |
| **17** | C | ND |
| **18** | C | ND |
| **19** | C | B |
| **20** | C | B |
| **21** | C | ND |
| **22** | C | ND |
| **23** | C | B |
| **24** | C | ND |
| **25** | C | ND |
| **26** | B | B |

(continued)

| Example | Ki (μM) | Elastin hydrolysis % inhibition @ 50 / 25 μM inhibitor concentration |
|---|---|---|
| **27** | B | B |
| **28** | C | B |
| **29** | C | ND |
| **30** | C | C |
| **31** | B | B |
| **32** | B | A |
| **33** | ND | ND |
| **34** | B | B |
| **35** | B | B |
| **36** | A | A |
| **37** | B | B |
| **38** | B | B |
| **39** | B | B |
| **40** | C | ND |
| **41** | C | ND |
| **42** | C | ND |
| **43** | C | B |
| **44** | B | B |
| **45** | B | B |
| **46** | B | A |
| **47** | C | B |
| **48** | C | ND |
| **49** | B | B |
| **50** | C | B |
| **51** | B | B |
| **52** | B | ND |
| **53** | C | ND |
| **54** | C | B |
| **55** | B | B |
| **56** | B | B |
| **57** | B | B |
| **58** | B | B |
| **59** | B | B |
| **60** | A | B |
| **61** | B | B |
| **62** | B | B |
| **63** | B | A |
| **64** | B | B |

(continued)

| Example | Ki (μM) | Elastin hydrolysis % inhibition @ 50 / 25 μM inhibitor concentration |
|---|---|---|
| **65** | C | ND |
| **66** | B | B |
| **67** | B | B |
| **68** | B | B |
| **69** | B | C |
| **70** | B | B |
| **71** | A | A |
| **72** | A | C |
| **73** | B | B |
| **74** | A | A |
| **75** | B | B |
| **76** | A | A |
| **77** | B | C |
| **78** | A | A |
| **79** | B | B |
| **80** | B | B |
| **81** | B | B |
| **82** | B | B |
| **83** | A | A |
| **84** | B | B |
| **85** | B | B |
| **86** | B | B |
| **87** | B | C |
| **88** | A | A |
| **89** | B | B |
| **90** | C | B |
| **91** | B | A |
| **92** | B | B |
| **93** | B | B |
| **94** | C | B |
| **95** | C | B |
| **96** | B | B |
| **97** | C | ND |
| **98** | C | B |
| **99** | B | B |
| **100** | c | ND |
| **101** | c | B |
| **102** | C | B |

(continued)

| Example | Ki (μM) | Elastin hydrolysis % inhibition @ 50 / 25 μM inhibitor concentration |
|---|---|---|
| **103** | C | B |
| **104** | B | B |
| **105** | B | B |
| **106** | B | B |
| **107** | C | ND |
| **108** | B | B |
| **109** | B | B |
| **110** | B | B |
| **111** | C | ND |
| **112** | B | B |
| **113** | C | ND |
| **114** | B | B |
| **115** | C | ND |
| **116** | B | B |
| **117** | C | ND |
| **118** | C | ND |
| **119** | B | B |
| **120** | B | B |
| **121** | B | B |
| **122** | B | A |
| **123** | A | B |
| **124** | B | B |
| **125** | B | C |
| **126** | B | B |
| **127** | B | A |
| **128** | C | C |
| **129** | C | B |
| **130** | B | B |
| **131** | C | ND |
| **132** | A | A |
| **133** | A | B |
| **134** | B | B |
| **135** | A | A |
| **136** | B | B |
| **137** | B | C |
| **138** | C | C |
| **139** | B | C |
| **140** | ND | ND |

(continued)

| Example | Ki (μM) | Elastin hydrolysis % inhibition @ 50 / 25 μM inhibitor concentration |
|---|---|---|
| **141** | B | B |
| **142** | A | A |
| **143** | B | C |
| **144** | C | ND |
| **145** | B | B |
| **146** | B | B |
| **147** | B | B |
| **148** | B | B |

*Cellular models to define inhibitory effect of the compounds on LasB deleterious effect on human lung epithelial cell models*

From Saint-Criq et al. (Saint Criq et al. Thorax, 2018), LasB has been shown to degrade/downregulate two factors involved in immune response against PA infection: the interleukin IL-6 (important anti-inflammation cytokine) and trappin-2, an important epithelial-derived antimicrobial molecule. These degradations are beneficial for P. aeruginosa pathogenesis by inhibiting antimicrobial peptide while impairing lung function of the host by promoting lung damage and inhibiting epithelial cell repair. In mice, intranasally instillated LasB induced significant inflammation, injury and death. St Criq et al. showed that overexpression of IL-6 and trappin-2 protect mice against PA-induced death demonstrating importance of both immune factors to control PA pathogenesis and the key role of LasB to circumvent immune answer to promote PA infection. Our compounds are therefore been tested in cellular models to define inhibitory effect of our series on LasB deleterious effect on human lung epithelial cell models (NCI-H292 and CFBE). Supernatants from PAO1 wild type and lasB mutant (PAO1ΔlasB) are pre-incubated with Example 1 at 10 times the final concentrations for 1h at 37°C. Then, supernatants are added to cell lines. After 6h incubation, cell culture supernatants are recovered, IL-6 and Trappin-2 contents are analysed by ELISA method. A Student t-Test is performed to assess the statistical significance between conditions ($p < 0.05$, *). NCI-H292 and CFBE cells express respectively Trappin-2 and IL-6. Whether treatment with culture supernatant from PAO1ΔlasB induces an overexpression of both immune factors, the treatment with PAO1 supernatant lead to strong reduction of IL-6 and Trappin-2 detected. LasB, hydrolyzing IL-6 and Trappin-2, hijacks the immune response against PA. Treatment with Example 1 restored in a dose response maneer level of both Trappin-2 and IL-6 similar to the control showing effective inhibition of LasB. By restoring immune response and secretion of IL-6 and Trappin-2, Example 1 and related LasB inhibitors may restore eradication of PA by the immune system.

## Claims

1. A compound which is an indane according to Formula (I), or a pharmaceutically acceptable salt thereof,

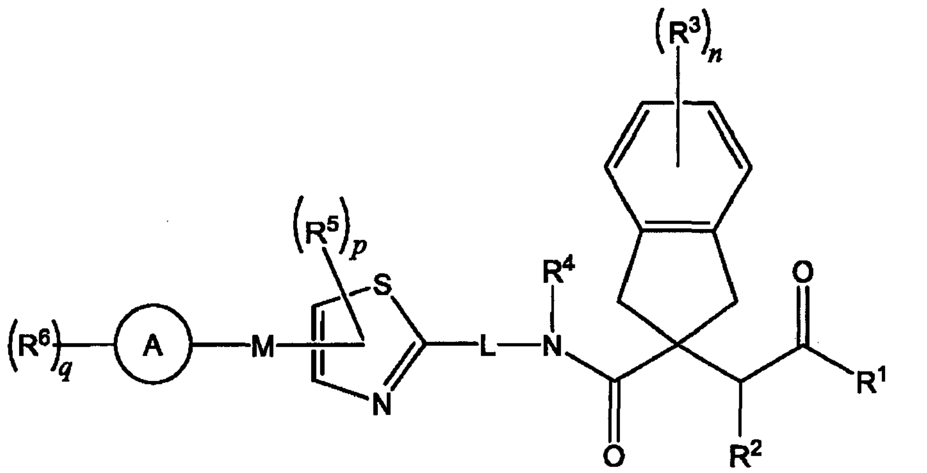

[FORMULA (I)]

wherein

- $R^1$ is selected from:

  - NHOH, OH, $OR^{1a}$ and $-OCH_2OC(O)R^{1a}$, wherein $R^{1a}$ is selected from an unsubstituted $C_1$ to $C_4$ alkyl group and phenyl; and
  - where the compound of Formula (I) contains a positively charged nitrogen atom, $R^1$ may be $O^-$, such that the compound forms a zwitterion;

- $R^2$ is selected from H and unsubstituted $C_1$ to $C_2$ alkyl;
- each $R^3$ group is independently selected from halogen, -OH, $-NH_2$, methyl and $-CF_3$;
- *n* is an integer from 0 to 4;
- $R^4$ is selected from H and unsubstituted $C_1$ to $C_2$ alkyl;
- L is selected from a bond and a $C_1$ to $C_3$ alkylene group which is unsubstituted or is substituted by one group selected from halogen, -OH, -OMe, $-NR^{20}R^{21}$; $-N^+R^{20}R^{21}R^{22}$, and $-CF_3$;
- M is selected from a bond and a $C_1$ to $C_3$ alkylene group which is unsubstituted or is substituted by one group selected from halogen, -OH, -OMe, $-NR^{20}R^{21}$; $-N^+R^{20}R^{21}W^2$, and $-CF_3$;
- Ⓐ is a cyclic group selected from phenyl, 5- to 10- membered heteroaryl, and 4- to 10-membered carbocyclic and heterocyclic groups; wherein when Ⓐ is a heterocyclic or heteroaryl group comprising at least one nitrogen atom, said nitrogen atom(s) are independently selected from secondary, tertiary and quaternary nitrogen atom(s);
- *p* is 0 or 1;
- $R^5$ is selected from halogen, -OH, -CN, $-NH_2$, methyl, $-CF_3$, and -OMe;
- *q* is 0, 1 or 2;
- each $R^6$ is independently selected from:

  - ◦ halogen, -CN, -OH;
  - ◦ oxo, providing that Ⓐ is a carbocyclic or heterocyclic group;
  - ◦ $-R^{6a}$, $-OR^{6a}$, $NR^{20}R^{6a}$, $-C(O)NR^{20}R^{6a}$, $-NR^{20}C(O)R^{6a}$, $-SO_2R^{6a}$, $-SO_2NR^{20}R^{6a}$, $-NR^{20}SO_2R^{6a}$, $C(O)R^{6a}$, $-OC(O)R^{6a}$, and $-C(O)OR^{6a}$; wherein each $R^{6a}$ is independently selected from $C_1$ to $C_4$ alkyl, $C_2$ to $C_4$ alkenyl and $C_2$ to $C_4$ alkynyl, and wherein each $R^{6a}$ is independently unsubstituted or is substituted with one, two or three groups selected from -OH, halogen; $-NR^{20}R^{21}$; $-N^+R^{20}R^{21}R^{22}$; $-NR^{20}C(NR^{21})NR^{22}R^{23}$; $-NR^{20}C(N^+R^{21}R^{22})NR^{23}R^{24}$; $-NR^{20}C(NR^{21})R^{22}$; $-NR^{20}C(N^+R^{21}R^{22})R^{23}$; $-C(NR^{20})NR^{21}R^{22}$; $-C(N^+R^{20}R^{21})NR^{22}R^{23}$; $-C(NR^{20})R^{21}$; $-C(N^+R^{20}R^{21})R^{22}$; $-C(O)NR^{20}R^{21}$; $-CON^+R^{20}R^{21}R^{22}$; $-C(O)-R^{20}$,and methoxy which is substituted by one, two or three halogen substituents;
  - ◦ $-R^7-(Het^1)_v-R^9-R^{R1}$ and $-R^7-R^{R1}-Het^2-R^{R2}$; wherein

    - Het[1] is selected from -O-, $-NR^{20}-$, -C(O)-, $-SO_2-$, $-C(O)NR^{20-}$, $-NR^{20}C(O)-$, $-O-R^8-C(O)-$, $-C(O)-R^8-O-$, $-SO_2NR^{20}-$, $-NR^{20}SO_2-$, -OC(O)-, and -C(O)O-;
    - Het[2] is selected from -C(O)-, $-R^8-C(O)-$ and $-C(O)-R^{8-}$;
    - *v* is 0 or 1;

- $R^{R1}$ and $R^{R2}$ are each independently a 4- to 10- membered heteroaryl or . heterocyclic group comprising at least one nitrogen atom, and said nitrogen atom(s) are independently selected from secondary, tertiary and quaternary nitrogen atom(s); and wherein each $R^{R1}$ and $R^{R2}$ is independently unsubstituted or is substituted with one, two or three groups independently selected from

◦ halogen, -CN;
◦ oxo, providing that said $R^{R1}$ or $R^{R2}$ group is a heterocyclic group;
◦ $-R^{20}$, $-R^7-OR^{20}$; $-R^7-NR^{20}R^{21}$; $-R^7-N^+R^{20}R^{21}R^{22}$; $-R^7-NR^{20}C(NR^{21})NR^{22}R^{23}$; $-R^7-NR^{20}C(N^+R^{21}R^{22})NR^{23}R^{24}$; $-R^7-NR^{20}C(NR^{21})R^{22}$; $-R^7-NR^{20}C(N^+R^{21}R^{22})R^{23}$; $-R^7-C(NR^{20})NR^{21}R^{22}$; $-R^7-C(N^+R^{21}R^{21})NR^{22}R^{23}$; $-R^7-C(NR^{20})R^{21}$; $-R^7-(N^+R^{20}R^{21})R^{22}$; $-R^7-C(O)-R^9-NR^{20}R^{21}$; $R^7-C(O)-R^9-N^+R^{20}R^{21}R^{22}$; $R^7-C(O)-R^{20}$, and methoxy which is substituted by one, two or three halogen substituents;

• $R^7$ is selected from a bond and unsubstituted $C_1$ to $C_3$ alkylene;
• $R^8$ is unsubstituted $C_1$ to $C_3$ alkylene;
• $R^9$ is selected from a bond and unsubstituted $C_1$ to $C_3$ alkylene;
• $R^{20}$, $R^{21}$, $R^{22}$, $R^{23}$ and $R^{24}$ are each independently selected from H and unsubstituted $C_1$ to $C_2$ alkyl.

**2.** A compound according to claim 1 wherein:

- $R^1$ is selected from OH and NHOH; or where the compound of Formula (I) contains a positively charged nitrogen atom, $R^1$ may be $O^-$, such that the compound forms a zwitterion;
- $R^2$ is selected from H and unsubstituted methyl; and
- $R^4$ is H.

**3.** A compound according to claim 1 or 2, wherein *n* is an integer from 0 to 2 and each $R^3$ group is independently selected from halogen and -OH.

**4.** A compound according to any one of claims 1 to 3 wherein

- L is an unsubstituted $C_1$ alkylene group;
and/or
- M is selected from a bond and an unsubstituted $C_1$ alkylene group.

**5.** A compound according to any one of claims 1 to 4 wherein

- $R^3$ is selected from halogen, -CN, and methyl;
or
- p is 0.

**6.** A compound according to any one of claims 1 to 5 wherein Ⓐ is selected from phenyl and 5- to 6-membered heteroaryl and heterocyclic groups, wherein when Ⓐ is a heterocyclic or heteroaryl group it comprises at least one nitrogen atom, said nitrogen atom(s) being independently selected from secondary, tertiary and quaternary nitrogen atom(s);

wherein preferably Ⓐ is selected from benzene, pyridine, pyrimidine, pyrazine, pyrazole, triazole, dihydropyridine, piperazine and piperidine.

**7.** A compound according to any one of claims 1 to 6 wherein Ⓐ is aromatic;

wherein preferably Ⓐ is phenyl.

**8.** A compound according to any one of claims 1 to 7 wherein each $R^6$ is independently selected from:

◦ halogen, -OH;
◦ oxo, providing that Ⓐ is a carbocyclic or heterocyclic group;
◦ $-R^{6a}$ , $-OR^{6a}$, $NR^{20R6a}$, $-C(O)NR^{20}R^{6a}$, $-NR^{20}C(O)R^{6a}$, $-SO_2R^{6a}$, $-SO_2NR^{20}R^{6A}$, and $-NR^{20}SO_2R^{6a}$; wherein

each $R^{6a}$ is independently selected from $C_1$ to $C_3$ alkyl and $C_2$ to $C_3$ alkynyl, and wherein each $R^{6a}$ is independently unsubstituted or is substituted with one or two groups selected from -OH; $-NR^{20}R^{21}$; $-N^+R^{20}R^{21}R^{22}$; $-NR^{20}C(NR^{21})NR^{22}R^{23}$; $-NR^{20}C(N^+R^{21}R^{22})NR^{23}R^{24}$; $-NR^{20}C(NR^{21})R^{22}$; $-NR^{20}C(N^+R^{21}R^{22})R^{23}$; $-C(NR^{20})NR^{21}R^{22}$; and $-C(N^+R^{20}R^{21})NR^{22}R^{23}$; or with one, two or three halogen groups

◦ $-R^7-(Het^1)_v-R^9-R^{R1}$ and $-R^7-R^{R1}-Het^2-R^{R2}$; wherein

$R^{R1}$ and $R^{R2}$ are each independently a 5- to 6- membered heteroaryl or 4- to 8-membered heterocyclic group comprising at least one nitrogen atom, and said nitrogen atom(s) are independently selected from secondary, tertiary and quaternary nitrogen atom(s); and wherein each $R^{R1}$ and $R^{R2}$ is independently unsubstituted or is substituted with one or two groups independently selected from

- oxo, providing that said $R^{R1}$ or $R^{R2}$ group is a heterocyclic group;
- $-R^{20}$, $-R^7-OR^{20}$; $-R^7-NR^{20}R^{21}$; $-R^7-N^+R^{20}R^{21}R^{22}$; $-R^7-NR^{20}C(NR^{21})NR^{22}R^{23}$; $-R^7-NR^{20}C(N^+R^{21}R^{22})NR^{23}R^{24}$; $-R^7-NR^{20}C(NR^{21})R^{22}$; $-R^7-NR^{20}C(N^+R^{21}R^{22})R^{23}$; $-R^7-C(NR^{20})NR^{21}R^{22}$; $-R^7-C(N^+R^{20}R^{21})NR^{22}R^{23}$; $-R^7-C(NR^{20})R^{21}$; $-R^7-(N^+R^{20}R^{21})R^{22}$; $-R^7-C(O)-R^9-NR^{20}R^{21}$; $R^7-C(O)-R^9-N^+R^{20}R^{21}R^{22}$; and $R^7-C(O)-R^{20}$;

or

- with one, two or three halogen groups;

**9.** A compound according to any one of claims 1 to 8 wherein each $R^6$ is independently selected from:

• halogen; -OH; $-OCF_3$; $-(CH_2)_z-H$; $-(CH_2)_z-OH$; $-(CH_2)_z-N(R^X)_2$; $-(CH_2)_z-N^+(R^X)_3$;$-O-(CH_2)_z-H$; $-O-(CH_2)_z-OH$; $-O-(CH_2)_z-N(R^X)_2$; $-O-(CH_2)_z-N^+(R^X)3$;$-O-(CH_2)_z-N(R^X)-C(NR^X)-CH_3$; $-O-(CH_2)_z-N(R^X)-C(NR^X)-N(R^X)_2$; $-C(O)NR^X-(CH_2)_z-N(R^X)_2$; $-C(O)NR^X-(CH_2)_z-N^+(R^X)_3$;$-NR^XC(O)-(CH_2)_z-N(R^X)_2$; $-NR^XC(O)-(CH_2)_z-N^+(R^x)_3$; $-SO_2-(CH_2)_z-H$; $-SO_2-NR^X-(CH_2)_z-N(R^X)_2$; $-SO_2-NR^X-(CH_2)_z-N^+(R^X)_3$; $-C{\equiv}C-CH_2-N(R^X)_2$ and $-C{\equiv}C-CH_2-N^+(R^X)_3$; wherein $R^X$ is H or Me and *z* is 1, 2 or 3;

or

• $-R^{R1}$; $-(CH_2)_z-R^{R1}$; $-O-(CH_2)_z--R^{R1}$; $-C(O)-R^{R1}$; $-(CH_2)_z-C(O)-R^{R1}$; $-N(R^X)-C(O)-R^{R1}$; $-N(R^X)-C(O)-(CH_2)_z-R^{R1}$; $-O-(CH2)_z-C(O)-R^{R1}$; $-SO_2-R^{R1}$; $R^{R1}-C(O)-R^{R2}$; $-(CH_2)_z-R^{R1}-C(O)-R^{R2}$; $-R^{R1}-(CH_2)_z-C(O)-R^{R2}$;

and

each $R^{R1}$ and $R^{R2}$ is independently unsubstituted or is substituted with one or two groups selected from -CH3; $-N(R^X)_2$; $-N^+(R^X)_3$; $-(CH_2)_z-N(R^X)_2$;$-(CH_2)_z-N^+(R^X)_3$; $-OCH_3$; oxo; $-C(O)-CH_3$; $-(CO)-N(R^X)_2$; $-(CO)-(CH_2)_z-N(R^X)_2$; $-C(NR^X)-N(R^X)_2$ and $-C(NR^X)-CH_3$;

wherein $R^X$ is H or Me and z is 1, 2 or 3.

**10.** A compound according to any one of claims 1 to 9 wherein each $R^{R1}$ and $R^{R2}$ if present are independently selected from azetidine, imidazole, morpholine, 1,4-oxazepane, octahydropyrrolo[3,4-c]pyrrole, piperazine, piperidine, pyridine, and pyrrolidine.

**11.** A compound according to claim 1, which compound is selected from

1. 2-[2-[(4-phenylthiazol-2-yl)methylcarbamoyl]indan-2-yl]acetic acid
2. 2-[2-[(5-phenylthiazol-2-yl)methylcarbamoyl]indan-2-yl]acetic acid
3. 2-[5,6-difluoro-2-[(4-phenylthiazol-2-yl)methylcarbamoyl]indan-2-yl]acetic acid
4. 2-(2-((benzo[d]thiazol-2-ylmethyl)carbamoyl)-5,6-dichloro-2,3-dihydro-1H-inden-2-yl)acetic acid
5. 2-[2-[[4-(3-methoxyphenyl)thiazol-2-yl]methylcarbamoyl]indan-2-yl]acetic acid
6. 2-[2-[[4-(4-methoxyphenyl)thiazol-2-yl]methylcarbamoyl]indan-2-yl]acetic acid
7. 2-[2-[[4-(4-chlorophenyl)thiazol-2-yl]methylcarbamoyl]indan-2-yl]acetic acid
8. 2-[2-[[4-(3-pyridyl)thiazol-2-yl]methylcarbamoyl]indan-2-yl]acetic acid
9. 2-[2-[[4-(2-pyridyl)thiazol-2-yl]methylcarbamoyl]indan-2-yl]acetic acid
10. 2-[2-[[4-(2-hydroxyphenyl)thiazol-2-yl]methylcarbamoyl]indan-2-yl]acetic acid
11. 2-[2-[[4-(4-hydroxyphenyl)thiazol-2-yl]methylcarbamoyl]indan-2-yl]acetic acid
12. 2-[2-[[4-(3-hydroxyphenyl)thiazol-2-yl]methylcarbamoyl]indan-2-yl]acetic acid
13. 2-[2-[[4-[2-(2-hydroxyethoxy)phenyl]thiazol-2-yl]methylcarbamoyl]indan-2-yl]acetic acid
14. 2-[2-[[4-[2-(2-aminoethoxy)phenyl]thiazol-2-yl]methylcarbamoyl]indan-2-yl]acetic acid
15. 2-[2-[[4-[4-[2-(dimethylamino)ethylcarbamoyl]phenyl]thiazol-2-yl]methylcarbamoyl]indan-2-yl]acetic acid

16. 2-[2-[[4-[4-(4-methylpiperazine-1-carbonyl)phenyl]thiazol-2-yl]methylcarbamoyl]indan-2-yl]acetic acid
17. 2-[2-[[4-[4-[4-(dimethylamino)piperidine-1-carbonyl]phenyl]thiazol-2-yl]methylcarbamoyl]indan-2-yl]acetic acid
18. 2-[2-[[4-[4-[(3S)-3-(dimethylamino)pyrrolidine-1-carbonyl]phenyl]thiazol-2-yl]methylcarbamoyl]indan-2-yl]acetic acid
19. 2-[2-[[4-[4-[(3R)-3-(dimethylamino)pyrrolidine-1-carbonyl]phenyl]thiazol-2-yl]methylcarbamoyl]indan-2-yl]acetic acid
20. 2-[2-[[4-[4-[3-(dimethylamino)azetidine-1-carbonyl]phenyl]thiazol-2-yl]methylcarbamoyl]indan-2-yl]acetic acid
21. 2-[2-[[4-[2-[3-(dimethylamino)propylcarbamoyl]phenyl]thiazol-2-yl]methylcarbamoyl]indan-2-yl]acetic acid
22. 2-[2-[[4-[4-(4,4-dimethylpiperazin-4-ium-1-carbonyl)phenyl]thiazol-2-yl]methylcarbamoyl]indan-2-yl]acetate
23. 2-[2-[[4-[3-(4-methylpiperazine-1-carbonyl)phenyl]thiazol-2-yl]methylcarbamoyl]indan-2-yl]acetic acid
24. 2-[2-[[4-[5-[2-(dimethylamino)ethylcarbamoy1]-2-methoxy-phenyl]thiazol-2-yl]methylcarbamoyl]indan-2-yl]acetic acid
25. 2-[2-[[4-[2-methoxy-5-(4-methylpiperazine-1-carbonyl)phenyl]thiazol-2-yl]methylcarbamoyl]indan-2-yl]acetic acid
26. 2-[2-[[4-[4-(4-methylpiperazin-1-yl)phenyl]thiazol-2-yl]methylcarbamoyl]indan-2-yl]acetic acid
27. 2-[2-[[4-[4-(4-methylpiperazine-1-carbonyl)phenyl]thiazol-2-yl]methylcarbamoyl]indan-2-yl]acetic acid
28. 2-[2-[[4-[3-(4-methylpiperazin-1-yl)phenyl]thiazol-2-yl]methylcarbamoyl]indan-2-yl]acetic acid
29. 2-[2-[[4-[3-[3-(dimethylamino)azetidin-1-yl]phenyl]thiazol-2-yl]methylcarbamoyl]indan-2-yl]acetic acid
30. 2-[2-[[4-[4-[(3aS,6aR)-2-methyl-1,3,3a,4,6,6a-hexahydropyrrolo[3,4-c]pyrrol-5-yl]phenyl]thiazol-2-yl]methylcarbamoyl]indan-2-yl]acetic acid
31. 2-[2-[[4-[2-methoxy-4-(4-methylpiperazin-1-yl)phenyl]thiazol-2-yl]methylcarbamoyl]indan-2-yl]acetic acid
32. 2-[2-[[4-[4-[3-(dimethylamino)azetidin-1-yl]-2-methoxy-phenyl]thiazol-2-yl]methylcarbamoyl]indan-2-yl]acetic acid
33. 2-[5,6-difluoro-2-[[4-[2-methoxy-4-[4-(trimethylammonio)-1-piperidyl]phenyl]thiazol-2-yl]methylcarbamoyl]indan-2-yl]acetate
34. 2-[2-[[4-[6-(4-methylpiperazin-1-yl)-3-pyridyl]thiazol-2-yl]methylcarbamoyl]indan-2-yl]acetic acid
35. 2-[2-[[4-[6-[3-(dimethylamino)azetidin-1-yl]-3-pyridyl]thiazol-2-yl]methylcarbamoyl]indan-2-yl]acetic acid
36. 2-[2-[[4-[4-[4-[2-(4-methylpiperazin-1-yl)-2-oxo-ethyl]piperazin-1-yl]phenyl]thiazol-2-yl]methylcarbamoyl]indan-2-yl]acetic acid
37. 2-[2-[[4-[4-[3-(dimethylamino)prop-1-ynyl]phenyl]thiazol-2-yl]methylcarbamoyl]indan-2-yl]acetic acid
38. 2-[2-[[4-[6-[2-(4-methylpiperazin-1-yl)-2-oxo-ethoxy]-3-pyridyl]thiazol-2-yl]methylcarbamoyl]indan-2-yl]acetic acid
39. 2-[2-[[4-[4-[[2-(dimethylamino)acetyl]amino]phenyl]thiazol-2-yl]methylcarbamoyl]indan-2-yl]acetic acid
40. 2-[2-[[4-[4-[[2-(trimethylammonio)acetyl]amino]phenyl]thiazol-2-yl]methylcarbamoyl]indan-2-yl]acetate
41. 2-[2-[[4-[3-[[2-(dimethylamino)acetyl]amino]phenyl]thiazol-2-yl]methylcarbamoyl]indan-2-yl]acetic acid
42. 2-[2-[[4-[3-[[2-(trimethylammonio)acetyl]amino]phenyl]thiazol-2-yl]methylcarbamoyl]indan-2-yl]acetate
43. 2-[2-[[4-[4-[(1-methylpiperidine-4-carbonyl)amino]phenyl]thiazol-2-yl]methylcarbamoyl]indan-2-yl]acetic acid
44. 2-[2-[[4-[4-[(4-methylpiperazine-1-carbonyl)amino]phenyl]thiazol-2-yl]methylcarbamoyl]indan-2-yl]acetic acid
45. 2-[2-[[4-[4-[[2-(4-methylpiperazin-1-yl)acetyl]amino]phenyl]thiazol-2-yl]methylcarbamoyl]indan-2-yl]acetic acid
46. 2-[2-[[4-[4-[2-(4-methylpiperazin-1-yl)-2-oxo-ethoxy]phenyl]thiazol-2-yl]methylcarbamoyl]indan-2-yl]acetic acid
47. 2-[2-[[4-[4-[2-(4,4-dimethylpiperazin-4-ium-1-yl)-2-oxo-ethoxy]phenyl]thiazol-2-yl]methylcarbamoyl]indan-2-yl]acetate
48. 2-[2-[[4-[4-[2-(dimethylamino)ethoxy]phenyl]thiazol-2-yl]methylcarbamoyl]indan-2-yl]acetic acid
49. 2-[2-[[4-[4-(2-morpholinoethoxy)phenyl]thiazol-2-yl]methylcarbamoyl]indan-2-yl]acetic acid
50. 2-[2-[[4-[4-[2-(4-methylmorpholin-4-ium-4-yl)ethoxy]phenyl]thiazol-2-yl]methylcarbamoyl]indan-2-yl]acetate
51. 2-[2-[[4-[4-[(1-methylpyridin-1-ium-4-yl)methoxy]phenyl]thiazol-2-yl]methylcarbamoyl]indan-2-yl]acetate
52. 2-[2-[[4-[4-[2-(4-amino-1-piperidyl)-2-oxo-ethoxy]phenyl]thiazol-2-yl]methylcarbamoyl]indan-2-yl]acetic acid
53. 2-[2-[[4-[3-[2-(dimethylamino)ethoxy]phenyl]thiazol-2-yl]methylcarbamoyl]indan-2-yl]acetic acid
54. 2-[2-[[4-[3-[2-(4-methylpiperazin-1-yl)-2-oxo-ethoxy]phenyl]thiazol-2-yl]methylcarbamoyl]indan-2-yl]acetic

acid

55. 2-[2-[[4-[4-(3-morpholinopropoxy)phenyl]thiazol-2-yl]methylcarbamoyl]indan-2-yl]acetic acid

56. 2-[2-[[4-[4-[3-(4-methylmorpholin-4-ium-4-yl)propoxy]phenyl]thiazol-2-yl]methylcarbamoyl]indan-2-yl]acetate

57. 2-[2-[[4-[4-[3-(4-methylpiperazin-1-yl)propoxy]phenyl]thiazol-2-yl]methylcarbamoyl]indan-2-yl]acetic acid

58. 2-[2-[[4-[4-(3-pyrrolidin-1-ylpropoxy)phenyl]thiazol-2-yl]methylcarbamoyl]indan-2-yl]acetic acid

59. 2-[2-[[4-[4-(3-pyridin-1-ium-1-ylpropoxy)phenyl]thiazol-2-yl]methylcarbamoyl]indan-2-yl]acetate

60. 2-[2-[[4-[3-methylsulfonyl-4-(3-morpholinopropoxy)phenyl]thiazol-2-yl]methylcarbamoyl]indan-2-yl]acetic acid

61. 2-[2-[[4-[4-[3-(4-methylmorpholin-4-ium-4-yl)propoxy]-3-methylsulfonyl-phenyl]thiazol-2-yl]methylcarbamoyl]indan-2-yl]acetate

62. 2-[5,6-difluoro-2-[[4-[4-[3-(4-methylmorpholin-4-ium-4-yl)propoxy]-3-methylsulfonyl-phenyl]thiazol-2-yl]methylcarbamoyl]indan-2-yl]acetate

63. 2-[2-[[4-[2-methoxy-4-[2-(4-methylpiperazin-1-yl)-2-oxo-ethoxy]phenyl]thiazol-2-yl]methylcarbamoyl]indan-2-yl]acetic acid

64. 2-[5,6-difluoro-2-[[4-[2-methoxy-4-[2-oxo-2-[3-(trimethylammonio)azetidin-1-yl]ethoxy]phenyl]thiazol-2-yl]methylcarbamoyl]indan-2-yl]acetate

65. 2-[2-[[4-[4-[2-[4-[3-(dimethylamino)propyl]piperazin-1-yl]-2-oxo-ethoxy]-2-methoxy-phenyl]thiazol-2-yl]methylcarbamoyl]indan-2-yl]acetic acid

66. 2-[2-[[4-[2-methoxy-4-[2-(2-methyl-1,3,3a,4,6,6a-hexahydropyrrolo[3,4-c]pyrrol-5-yl)-2-oxo-ethoxy]phenyl]thiazol-2-yl]methylcarbamoyl]indan-2-yl]acetic acid

67. 2-[2-[[4-[2-methoxy-4-[2-(4-methylmorpholin-4-ium-4-yl)ethoxy]phenyl]thiazol-2-yl]methylcarbamoyl]indan-2-yl]acetate

68. 2-[5,6-difluoro-2-[[4-[2-methoxy-4-[2-(4-methylmorpholin-4-ium-4-yl)ethoxy]phenyl]thiazol-2-yl]methylcarbamoyl]indan-2-yl]acetate

69. 2-[2-[[4-[2-methoxy-4-[3-(4-methylmorpholin-4-ium-4-yl)propoxy]phenyl]thiazol-2-yl]methylcarbamoyl]indan-2-yl]acetate

70. 2-[5,6-difluoro-2-[[4-[2-methoxy-4-[3-(4-methylmorpholin-4-ium-4-yl)propoxy]phenyl]thiazol-2-yl]methylcarbamoyl]indan-2-yl]acetate

71. 2-[2-[[4-[2-methoxy-4-(3-morpholinopropoxy)phenyl]thiazol-2-yl]methylcarbamoyl]indan-2-yl]acetic acid

72. 2-[5,6-difluoro-2-[[4-[2-methoxy-4-(3-pyrrolidin-1-ylpropoxy)phenyl]thiazol-2-yl]methylcarbamoyl]indan-2-yl]acetic acid

73. 2-[5,6-difluoro-2-[[4-[2-methoxy-4-[3-(1-methylpyrrolidin-1-ium-1-yl)propoxy]phenyl]thiazol-2-yl]methylcarbamoyl]indan-2-yl]acetate

74. 2-[2-[[4-[2-methoxy-4-(4-pyridylmethoxy)phenyl]thiazol-2-yl]methylcarbamoyl]indan-2-yl]acetic acid

75. 2-[2-[[4-[2-methoxy-4-[(1-methylpyridin-1-ium-4-yl)methoxy]phenyl]thiazol-2-yl]methylcarbamoyl]indan-2-yl]acetate

76. 2-[2-[[4-[2-methoxy-4-(2-morpholinoethoxy)phenyl]thiazol-2-yl]methylcarbamoyl]indan-2-yl]acetic acid

77. 2-[2-[[4-[2-methoxy-4-[(1-methylpyridin-1-ium-3-yl)methoxy]phenyl]thiazol-2-yl]methylcarbamoyl]indan-2-yl]acetate

78. 2-[2-[[4-[2-methoxy-4-(3-pyridylmethoxy)phenyl]thiazol-2-yl]methylcarbamoyl]indan-2-yl]acetic acid

79. 2-[2-[[4-[4-[2-(4,4-dimethylpiperazin-4-ium-1-yl)-2-oxo-ethoxy]-2-methoxyphenyl]thiazol-2-yl]methylcarbamoyl]indan-2-yl]acetate

80. 2-[2-[[4-[4-[3-(dimethylamino)propoxy]-2-methoxy-phenyl]thiazol-2-yl]methylcarbamoyl]-5,6-difluoro-indan-2-yl]acetic acid

81. 2-[5,6-difluoro-2-[[4-[2-methoxy-4-[3-(trimethylammonio)propoxy]phenyl]thiazol-2-yl]methylcarbamoyl]indan-2-yl]acetate

82. 2-[2-[[4-[4-[2-(4,4-dimethylpiperazin-4-ium-1-yl)-2-oxo-ethoxy]-2-methoxyphenyl]thiazol-2-yl]methylcarbamoyl]-5,6-difluoro-indan-2-yl]acetate

83. 2-[2-[[4-[2-methoxy-4-[2-(4-methyl-3-oxo-piperazin-1-yl)ethoxy]phenyl]thiazol-2-yl]methylcarbamoyl]indan-2-yl]acetic acid

84. 2-[2-[[4-[4-[2-(1,4-dimethyl-3-oxo-piperazin-1-ium-1-yl)ethoxy]-2-methoxyphenyl]thiazol-2-yl]methylcarbamoyl]indan-2-yl]acetate

85. 2-[2-[[4-[2-methoxy-4-[2-(1-methylimidazol-2-yl)ethoxy]phenyl]thiazol-2-yl]methylcarbamoyl]indan-2-yl]acetic acid

86. 2-[2-[[4-[4-[2-(1,3-dimethylimidazol-1-ium-2-yl)ethoxy]-2-methoxy-phenyl]thiazol-2-yl]methylcarbamoyl]-5,6-difluoro-indan-2-yl]acetate

87. 2-[2-[[4-[4-[2-(1,3-dimethylimidazol-1-ium-2-yl)ethoxy]phenyl]thiazol-2-yl]methylcarbamoyl]indan-2-yl]acetate

88. 2-[2-[[4-[4-[2-(4-acetylpiperazin-1-yl)ethoxy]-2-methoxy-phenyl]thiazol-2-yl]methylcarbamoyl]indan-2-yl]acetic acid

89. 2-[2-[[4-(1-methylpyrazol-4-yl)thiazol-2-yl]methylcarbamoyl]indan-2-yl]acetic acid

90. 2-[2-[[4-(1-methylpyrazol-3-yl)thiazol-2-yl]methylcarbamoyl]indan-2-yl]acetic acid

91. 2-[2-[[4-(1,5-dimethylpyrazol-4-yl)thiazol-2-yl]methylcarbamoyl]indan-2-yl]acetic acid

92. 2-[2-[[4-(2-methylpyrazol-3-yl)thiazol-2-yl]methylcarbamoyl]indan-2-yl]acetic acid

93. 2-[2-[[4-(1,3-dimethylpyrazol-4-yl)thiazol-2-yl]methylcarbamoyl]indan-2-yl]acetic acid

94. 2-[2-[(4-pyrimidin-5-ylthiazol-2-yl)methylcarbamoyl]indan-2-yl]acetic acid

95. 2-[2-[[4-(4-pyridyl)thiazol-2-yl]methylcarbamoyl]indan-2-yl]acetic acid

96. 2-[2-[[4-[1-(2-morpholinoethyl)pyrazol-4-yl]thiazol-2-yl]methylcarbamoyl]indan-2-yl]acetic acid

97. 2-[2-[[4-[1-[2-(dimethylamino)ethyl]pyrazol-4-yl]thiazol-2-yl]methylcarbamoyl]indan-2-yl]acetic acid

98. 2-[2-[[4-(5-methyl-1H-pyrazol-3-yl)thiazol-2-yl]methylcarbamoyl]indan-2-yl]acetic acid

99. 2-[2-[[4-[1-(3-morpholinopropyl)pyrazol-4-yl]thiazol-2-yl]methylcarbamoyl]indan-2-yl]acetic acid

100. 2-[2-[[4-[1-[3-(4-methylmorpholin-4-ium-4-yl)propyl]pyrazol-4-yl]thiazol-2-yl]methylcarbamoyl]indan-2-yl]acetate

101. 2-[2-[[4-[1-(3-morpholinopropyl)pyrazol-4-yl]thiazol-2-yl]methylcarbamoyl]indan-2-yl]acetic acid

102. 2-[2-[[4-[1-(1-methyl-4-piperidyl)pyrazol-4-yl]thiazol-2-yl]methylcarbamoyl]indan-2-yl]acetic acid

103. 2-[2-[[4-[1-[1-[2-(dimethylamino)acetyl]-4-piperidyl]pyrazol-4-yl]thiazol-2-yl]methylcarbamoyl]indan-2-yl]acetic acid

104. 2-[2-[[4-[1-[1-(4-methylpiperazine-1-carbonyl)-4-piperidyl]pyrazol-4-yl]thiazol-2-yl]methylcarbamoyl]indan-2-yl]acetic acid

105. 2-[2-[[4-[1-[1-[2-(4-methylpiperazin-1-yl)-2-oxo-ethyl]-4-piperidyl]pyrazol-4-yl]thiazol-2-yl]methylcarbamoyl]indan-2-yl]acetic acid

106. 2-[2-[[4-[1-[3-(4-acetylpiperazin-1-yl)propyl]pyrazol-4-yl]thiazol-2-yl]methylcarbamoyl]indan-2-yl]acetic acid

107. 2-[2-[[4-[1-[3-(4-acetyl-1-methyl-piperazin-1-ium-1-yl)propyl]pyrazol-4-yl]thiazol-2-yl]methylcarbamoyl]indan-2-yl]acetate

108. 2-[2-[[4-[1-[3-(4-methylpiperazin-1-yl)propyl]pyrazol-4-yl]thiazol-2-yl]methylcarbamoyl]indan-2-yl]acetic acid

109. 2-[2-[[4-[1-[3-(1,4-oxazepan-4-yl)propyl]pyrazol-4-yl]thiazol-2-yl]methylcarbamoyl]indan-2-yl]acetic acid

110. 2-[2-[[4-[1-[3-(4-methoxy-1-piperidyl)propyl]pyrazol-4-yl]thiazol-2-yl]methylcarbamoyl]indan-2-yl]acetic acid

111. 2-[2-[[4-[1-[3-(1-piperidyl)propyl]pyrazol-4-yl]thiazol-2-yl]methylcarbamoyl]indan-2-yl]acetic acid

112. 2-[2-[[4-[1-[3-[4-(4-methylpiperazine-1-carbonyl)piperazin-1-yl]propyl]pyrazol-4-yl]thiazol-2-yl]methylcarbamoyl]indan-2-yl]acetic acid

113. 2-[2-[[4-[1-[3-[4-(dimethylcarbamoyl)-1-piperidyl]propyl]pyrazol-4-yl]thiazol-2-yl]methylcarbamoyl]indan-2-yl]acetic acid

114. 2-[2-[[4-[1-[3-[4-(1-methylpiperidine-4-carbonyl)piperazin-1-yl]propyl]pyrazol-4-yl]thiazol-2-yl]methylcarbamoyl]indan-2-yl]acetic acid

115. 2-[2-[[4-[1-[3-[4-(1,1-dimethylpiperidin-1-ium-4-carbonyl)-1-methyl-piperazin-1-ium-1-yl]propyl]pyrazol-4-yl]thiazol-2-yl]methylcarbamoyl]indan-2-yl]acetate

116. 2-[2-[[4-[1-[3-(4-methyl-3-oxo-piperazin-1-yl)propyl]pyrazol-4-yl]thiazol-2-yl]methylcarbamoyl]indan-2-yl]acetic acid

117. 2-[2-[[4-[1-[2-(1-methylpyridin-1-ium-4-yl)ethyl]pyrazol-4-yl]thiazol-2-yl]methylcarbamoyl]indan-2-yl]acetate

118. 2-[2-[[4-[1-[3-(1-methylpyridin-1-ium-4-yl)propyl]pyrazol-4-yl]thiazol-2-yl]methylcarbamoyl]indan-2-yl]acetate

119. 2-[2-[[4-[1-[2-(4-acetylpiperazin-1-yl)ethyl]pyrazol-4-yl]thiazol-2-yl]methylcarbamoyl]indan-2-yl]acetic acid

120. 2-[2-[[4-[1-[(1-methylpyridin-1-ium-4-yl)methyl]pyrazol-4-yl]thiazol-2-yl]methylcarbamoyl]indan-2-yl]acetate

121. 2-[2-[[4-[3-[[4-(dimethylamino)-1-piperidyl]sulfonyl]-4-methoxy-phenyl]thiazo1-2-yl]methylcarbamoyl]indan-2-yl]acetic acid

122. 2-[2-[[4-[3-[[4-(dimethylamino)-1-piperidyl]sulfonyl]-4-methoxy-phenyl]thiazol-2-yl]methylcarbamoyl]indan-2-yl]acetic acid

123. 2-[2-[[4-[3-[[4-(dimethylamino)-1-piperidyl]sulfonyl]-4-methoxy-phenyl]thiazol-2-yl]methylcarbamoyl]-5,6-difluoro-indan-2-yl]acetic acid

124. 2-[5,6-difluoro-2-[[4-[4-methoxy-3-[[4-(trimethylammonio)-1-piperidyl]sulfonyl]phenyl]thiazol-2-yl]methylcarbamoyl]indan-2-yl]acetate
125. 2-[2-[[4-[5-[[4-(dimethylamino)-1-piperidyl]sulfonyl]-2-methoxy-phenyl]thiazol-2-yl]methylcarbamoyl]-5,6-difluoro-indan-2-yl]acetic acid
126. 2-[5,6-difluoro-2-[[4-[2-methoxy-5-[[4-(trimethylammonio)-1-piperidyl]sulfonyl]phenyl]thiazol-2-yl]methylcarbamoyl]indan-2-yl]acetate
127. 2-[2-[[4-[3-(4-methylpiperazin-1-yl)sulfonylphenyl]thiazol-2-yl]methylcarbamoyl]indan-2-yl]acetic acid
128. 2-[2-[[4-[3-(4,4-dimethylpiperazin-4-ium-1-yl)sulfonylphenyl]thiazol-2-yl]methylcarbamoyl]indan-2-yl]acetate
129. 2-[2-[[4-[3-[2-(dimethylamino)ethylsulfamoyl]phenyl]thiazol-2-yl]methylcarbamoyl]indan-2-yl]acetic acid
130. 2-[2-[[4-[4-(4-methylpiperazin-1-yl)sulfonylphenyl]thiazol-2-yl]methylcarbamoyl]indan-2-yl]acetic acid
131. 2-[2-[[4-[4-[2-(dimethylamino)ethylsulfamoyl]phenyl]thiazol-2-yl]methylcarbamoyl]indan-2-yl]acetic acid
132. 2-[2-[[4-[3-(4-methylpiperazin-1-yl)sulfonyl-4-(2-morpholinoethoxy)phenyl]thiazol-2-yl]methylcarbamoyl]indan-2-yl]acetic acid
133. 2-[2-[[4-[3-(4-methylpiperazin-1-yl)sulfonyl-4-(3-morpholinopropoxy)phenyl]thiazol-2-yl]methylcarbamoyl]indan-2-yl]acetic acid
134. 2-[2-[[4-[3-[[4-(dimethylamino)-1-piperidyl]sulfonyl]-4-(2-morpholinoethoxy)phenyl]thiazol-2-yl]methylcarbamoyl]-5,6-difluoro-indan-2-yl]acetic acid
135. 2-[2-[[4-[3-methylsulfonyl-4-(2-morpholinoethoxy)phenyl]thiazol-2-yl]methylcarbamoyl]indan-2-yl]acetic acid
136. 2-[2-[[4-[4-[2-(4-methylmorpholin-4-ium-4-yl)ethoxy]-3-methylsulfonyl-phenyl]thiazol-2-yl]methylcarbamoyl]indan-2-yl]acetate
137. 2-[2-[[4-[3-[4-(dimethylamino)piperidine-1-carbonyl]-4-methoxy-phenyl]thiazol-2-yl]methylcarbamoyl]indan-2-yl]acetic acid
138. 2-[2-[[4-[4-methoxy-3-[4-(trimethylammonio)piperidine-1-carbonyl]phenyl]thiazol-2-yl]methylcarbamoyl]indan-2-yl]acetate
139. 2-[2-[[4-[3-[[4-(dimethylamino)-1-piperidyl]sulfonyl]-4-(3-pyrrolidin-1-ylpropoxy)phenyl]thiazol-2-yl]methylcarbamoyl]-5,6-difluoro-indan-2-yl]acetic acid
140. 2-[2-[[4-[6-(2-morpholinoethyl)-3-pyridyl]thiazol-2-yl]methylcarbamoyl]indan-2-yl]acetic acid
141. 2-[2-[[4-[6-[2-(4-methylmorpholin-4-ium-4-yl)ethyl]-3-pyridyl]thiazol-2-yl]methylcarbamoyl]indan-2-yl]acetate
142. 2-[2-[[4-[6-(4-pyridylmethoxy)-3-pyridyl]thiazol-2-yl]methylcarbamoyl]indan-2-yl]acetic acid
143. 2-[2-[[4-[6-[(l-methylpyridm-1-ium-4-yl)methoxy]-3-pyridyl]thiazol-2-yl]methylcarbamoyl]indan-2-yl]acetate
144. 2-[2-[[4-[5-(4-methylpiperazine-1-carbonyl)-3-pyridyl]thiazol-2-yl]methylcarbamoyl]indan-2-yl]acetic acid
145. 2-[2-[[4-[2-(trifluoromethoxy)phenyl]thiazol-2-yl]methylcarbamoyl]indan-2-yl]acetic acid
146. 2-[2-[[4-(2-methoxyphenyl)thiazol-2-yl]methylcarbamoyl]indan-2-yl]acetic acid
147. 2-[2-[[4-(2-ethylphenyl)thiazol-2-yl]methylcarbamoyl]indan-2-yl]acetic acid
148. 2-[2-[[4-[2-(hydroxymethyl)phenyl]thiazol-2-yl]methylcarbamoyl]indan-2-yl]acetic acid
149. 2-[2-[[4-[3-(hydroxymethyl)phenyl]thiazol-2-yl]methylcarbamoyl]indan-2-yl]acetic acid
150. 2-[2-[[4-(2-methoxy-3-pyridyl)thiazol-2-yl]methylcarbamoyl]indan-2-yl]acetic acid
151. 2-[2-[[4-(4-methoxy-3-pyridyl)thiazol-2-yl]methylcarbamoyl]indan-2-yl]acetic acid
152. 2-[2-[[4-(2-oxo-1H-pyridin-3-yl)thiazol-2-yl]methylcarbamoyl]indan-2-yl]acetic acid
153. 2-[2-[[4-[1-(4-methylpiperazine-1-carbonyl)-4-piperidyl]thiazol-2-yl]methylcarbamoyl]indan-2-yl]acetic acid
154. 2-[2-[[4-[1-(4-methylpiperazin-1-yl)sulfonyl-3-piperidyl]thiazol-2-yl]methylcarbamoyl]indan-2-yl]acetic acid
155. 2-[2-[[4-[4-(4-carbamimidoylpiperazin-1-yl)phenyl]thiazol-2-yl]methylcarbamoyl]indan-2-yl]acetic acid
156. 2-[2-[[4-[4-(4-ethanimidoylpiperazin-1-yl)phenyl]thiazol-2-yl]methylcarbamoyl]indan-2-yl]acetic acid
157. 2-[2-(hydroxyamino)-2-oxo-ethyl]-N-[(4-phenylthiazol-2-yl)methyl]indane-2-carboxamide
158. 2-[2-(hydroxyamino)-2-oxo-ethyl]-N-[[4-[1-(2-morpholinoethyl)pyrazol-4-yl]thiazol-2-yl]methyl]indane-2-carboxamide
159. 2-[2-(hydroxyamino)-2-oxo-ethyl]-N-[[4-[1-[2-(4-methylpiperazin-1-yl)-2-oxoethyl]pyrazol-4-yl]thiazol-2-yl]methyl]indane-2-carboxamide
160. 2-[2-[[4-[3-[(4-methylpiperazin-1-yl)methyl]phenyl]thiazol-2-yl]methylcarbamoyl]indan-2-yl]acetic acid
161. 2-[2-[[4-[4-[(4-methylpiperazin-1-yl)methyl]phenyl]thiazol-2-yl]methylcarbamoyl]indan-2-yl]acetic acid
162. 2-[2-[[4-[4-[(dimethylamino)methyl]phenyl]thiazol-2-yl]methylcarbamoyl]indan-2-yl]acetic acid
163. 2-[2-[[4-[4-(2-aminoethoxy)phenyl]thiazol-2-yl]methylcarbamoyl]indan-2-yl]acetic acid
164. 2-[2-[[4-[4-[2-(ethanimidoylamino)ethoxy]phenyl]thiazol-2-yl]methylcarbamoyl]indan-2-yl]acetic acid
165. 2-[2-[[4-[4-(2-guanidinoethoxy)phenyl]thiazol-2-yl]methylcarbamoyl]indan-2-yl]acetic acid

166. 2-[2-[(4-benzylthiazol-2-yl)methylcarbamoyl]indan-2-yl]acetic acid
167. 2-[2-[(5-methyl-4-phenyl-thiazol-2-yl)methylcarbamoyl]indan-2-yl]acetic acid
168. 2-[2-[[4-(5-methyl-1H-1,2,4-triazol-3-yl)thiazol-2-yl]methylcarbamoyl]indan-2-yl]acetic acid

A. 2-[2-[(4-pyrazin-2-ylthiazol-2-yl)methylcarbamoyl]indan-2-yl]acetic acid
B. 2-[2-[(5-chloro-4-phenyl-thiazol-2-yl)methylcarbamoyl]indan-2-yl]acetic acid
C. 2-[2-[(5-cyano-4-phenyl-thiazol-2-yl)methylcarbamoyl]indan-2-yl]acetic acid
D. 2-[2-[[4-[4-(4-methyl-2-oxo-piperazin-1-yl)phenyl]thiazol-2-yl]methylcarbamoyl]indan-2-yl]acetic acid

or a pharmaceutically acceptable salt thereof.

**12.** A pharmaceutical composition comprising (i) a compound according to any one of the preceding claims and (ii) at least one pharmaceutically acceptable carrier or diluent; and optionally further comprising (iii) an antibiotic agent; wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

**13.** A combination of (i) a compound according to any one of claims 1 to 11 and (ii) an antibiotic agent;

wherein preferably the antibiotic agent is selected from tobramycin, neomycin, streptomycin, gentamycin, ceftazidime, ticarcillin, piperacillin, tazobactam, imipenem, meropenem, rifampicin, ciprofloxacin, amikacin, colistin, aztreonam and levofloxacin.

**14.** A compound according to any one of claims 1 to 11; a composition according to claim 12 or a combination according to claim 13 for use in medicine.

**15.** A compound according to any one of claims 1 to 11; a composition according to claim 12 or a combination according to claim 13 for use in treating or preventing bacterial infection in a subject;

wherein preferably

- the bacterial infection is caused by *Bacillus*, *Pseudomonas*, *Staphylococcus*, *Streptococcus*, *Listeria*, *Burkholderia or Escherichia*; wherein more preferably the bacterial infection is caused by *Pseudomonas aeruginosa*;
- the compound for use, composition for use or combination for use is for use in the treatment or prevention of pneumonia; and/or
- the subject suffers from cystic fibrosis.

Figure 1

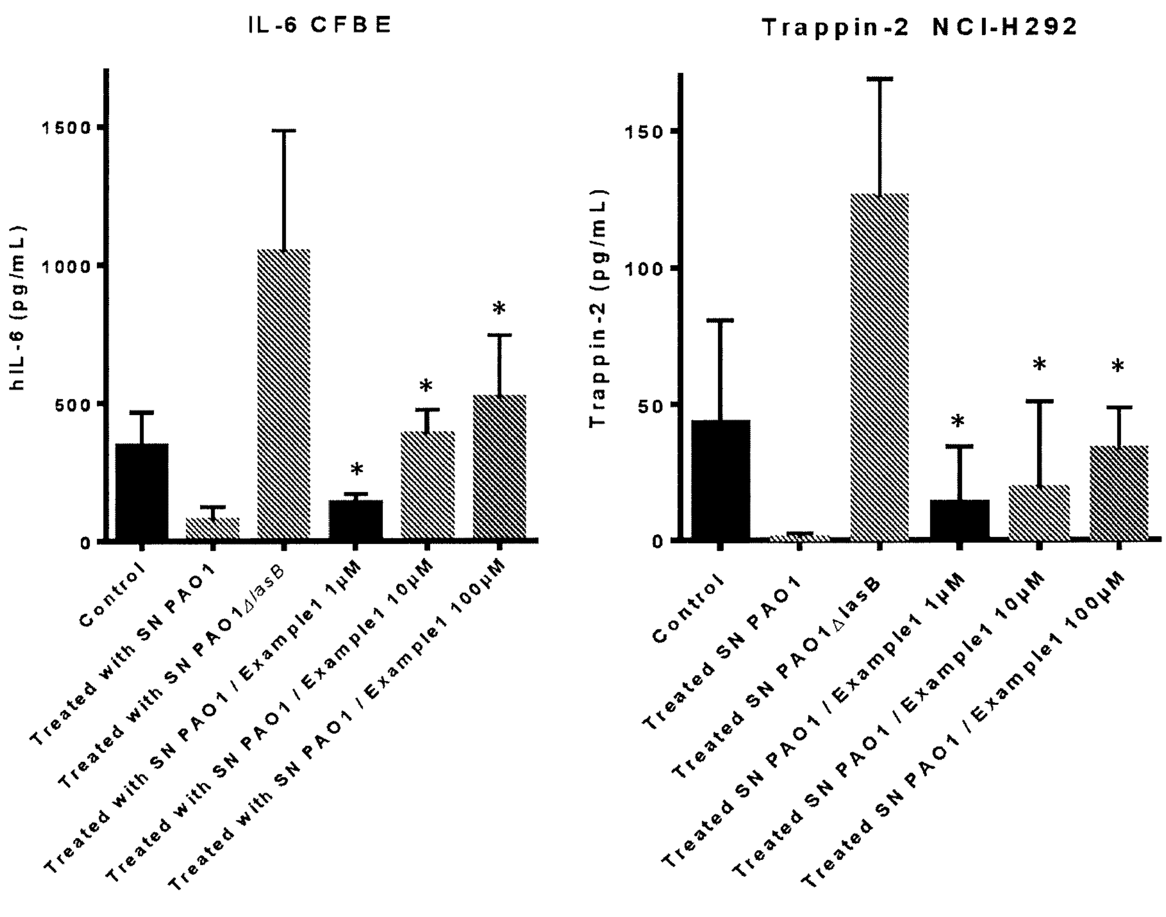
IL-6 CFBE
hIL-6 (pg/mL)
1500
1000
500
0
Control
Treated with SN PAO1
Treated with SN PAO1Δ/asB
Treated with SN PAO1 / Example1 1µM
Treated with SN PAO1 / Example1 10µM
Treated with SN PAO1 / Example1 100µM
Trappin-2 NCI-H292
Trappin-2 (pg/mL)
150
100
50
0
Control
Treated SN PAO1
Treated SN PAO1ΔlasB
Treated SN PAO1 / Example1 1µM
Treated SN PAO1 / Example1 10µM
Treated SN PAO1 / Example1 100µM

Europäisches Patentamt
European Patent Office
Office européen des brevets

# EUROPEAN SEARCH REPORT

Application Number
EP 18 29 0105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | G. R. A. CATHCART ET AL: "Novel Inhibitors of the Pseudomonas aeruginosa Virulence Factor LasB: a Potential Therapeutic Approach for the Attenuation of Virulence Mechanisms in Pseudomonal Infection", ANTIMICROBIAL AGENTS AND CHEMOTHERAPY, vol. 55, no. 6, 1 June 2011 (2011-06-01), pages 2670-2678, XP055156158, ISSN: 0066-4804, DOI: 10.1128/AAC.00776-10 | 1-10, 12-15 | INV. C07D417/12 C07D417/14 C07D277/28 A61P25/00 A61P31/04 A61K31/427 A61K31/4523 A61K31/505 |
| A | * figure 1; table 1 * | 11 | |
| A | WO 2014/083033 A1 (BAYER CROPSIENCE AG [DE]) 5 June 2014 (2014-06-05) * page 30, line 33; claims 1-11 * | 1-15 | |
| A | US 2012/122764 A1 (KARKI RAJESHRI GANESH [US] ET AL) 17 May 2012 (2012-05-17) * precursor of example 11-1; claims 1-18 * | 1-15 | |

TECHNICAL FIELDS SEARCHED (IPC)

C07D
A61P
A61K

The present search report has been drawn up for all claims

2

| Place of search | Date of completion of the search | Examiner |
|---|---|---|
| Munich | 5 November 2018 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 29 0105

This annex lists the patent family members relating to the patent documents cited in the above-mentioned European search report. The members are as contained in the European Patent Office EDP file on
The European Patent Office is in no way liable for these particulars which are merely given for the purpose of information.

05-11-2018

| Patent document cited in search report | Publication date | Patent family member(s) | Publication date |
|---|---|---|---|
| WO 2014083033 A1 | 05-06-2014 | AR 093674 A1 | 17-06-2015 |
| | | BR 112015012054 A2 | 11-07-2017 |
| | | CA 2892702 A1 | 05-06-2014 |
| | | CN 104837351 A | 12-08-2015 |
| | | EA 201500584 A1 | 30-11-2015 |
| | | EP 2925137 A1 | 07-10-2015 |
| | | JP 6367214 B2 | 01-08-2018 |
| | | JP 2016503431 A | 04-02-2016 |
| | | UA 117820 C2 | 10-10-2018 |
| | | US 2015282483 A1 | 08-10-2015 |
| | | WO 2014083033 A1 | 05-06-2014 |
| US 2012122764 A1 | 17-05-2012 | AR 083872 A1 | 27-03-2013 |
| | | CN 103249715 A | 14-08-2013 |
| | | EP 2640689 A1 | 25-09-2013 |
| | | JP 2014507372 A | 27-03-2014 |
| | | TW 201300353 A | 01-01-2013 |
| | | US 2012122764 A1 | 17-05-2012 |
| | | WO 2012065953 A1 | 24-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200629153 A
- WO 2008151211 A
- US 2006223830 A
- WO 2006125511 A


### Non-patent literature cited in the description


- **ROBINSON, R.P. et al.** *Bioorganic and Medicinal Chemistry Letters,* 1996, 1719
- *Synthesis,* 2017, vol. 22, 3535-3541
- **GANESHPURKAR, A. et al.** *Current Organic Syntheses,* 2018, vol. 15, 154-165
- **DING, C. et al.** *Bioorg. Med. Chem. Lett,* 2017, vol. 25, 27-37
- **MERRITT, E. et al.** *Synthesis,* 2017, vol. 22, 3535-3541
- **BUCHWALD, S.L.** *Chem. Rev.,* 2016, vol. 116, 12564-12649
- **ORLANDI, M et al.** *Organic Process Research and Development,* 2018, vol. 22, 430-445
- **MOLANDER, G.A.** *JACS,* 2012, vol. 134, 11667-11673
- **CAO, S. et al.** *Chemistry - A European Journal,* 2013, vol. 19, 9050-9058
- **SUZUKI, A.** *Chem. Rev.,* 1995, vol. 95, 2457-2483
- **CARPINO, L.A. et al.** JCS Chemical Commun. Teoc group, 1978, 358